# EUROPEAN PATENT SPECIFICATION

(11) **EP 3 224 362 B1**
(45) Date of publication and mention of the grant of the patent: **25.09.2024**
(21) Application number: 15863015.2
(22) Date of filing: 27.11.2015
(51) Int. Cl.: C12N 15/79, C12N 15/86, C12N 15/864, C07K 14/47, C12N 5/00

(54) **THERAPEUTIC COMPOSITIONS COMPRISING TRANSCRIPTION FACTORS AND METHODS OF MAKING AND USING THE SAME**
THERAPEUTISCHE ZUSAMMENSETZUNGEN MIT TRANSKRIPTIONSFAKTOREN SOWIE VERFAHREN ZUR HERSTELLUNG UND VERWENDUNG DAVON
COMPOSITIONS THÉRAPEUTIQUES COMPRENANT DES FACTEURS DE TRANSCRIPTION ET LEURS PROCÉDÉS DE PRÉPARATION ET D'UTILISATION

(30) Priority: 26.11.2014 US 201462085177 P
(43) Date of publication of application: 04.10.2017
(73) Proprietor: The Regents of the University of California, Oakland, CA 94607 (US)
(72) Inventor: WILLENBRING, Holger, San Francisco, California 94117 (US); DUMONT, Laure, San Francisco, California 94122 (US); MALATO, Yann, San Francisco, California 94115 (US)
(74) Representative: Leonard, Thomas Charles
(86) International application number: PCT/US2015/062841
(87) International publication number: WO 2016/086227

(56) References cited:
- WO-A1-2011/141405
- WO-A1-2015/120776
- US-A1- 2012 093 775
- US-A1- 2012 093 775
- US-A1- 2013 244 331
- US-A1- 2013 244 331
- US-A1- 2013 251 694
- US-A1- 2013 251 694
- US-A1- 2014 249 209
- REZVANI MILAD ET AL: "In Vivo Hepatic Reprogramming of Myofibroblasts with AAV Vectors as a Therapeutic Strategy for Liver Fibrosis", CELL STEM CELL, ELSEVIER, CELL PRESS, AMSTERDAM, NL, vol. 18, no. 6, 2 June 2016 (2016-06-02), pages 809 - 816, XP029567588, ISSN: 1934-5909, DOI: 10.1016/J.STEM.2016.05.005

## Description

### RELATED APPLICATIONS

This application claims priority to U.S. Provisional Serial No. 62/085,177, filed November 26, 2014.

### STATEMENT REGARDING FEDERALLY SPONSORED RESEARCH

This invention was made with government support under grant number NIAAA R21 AA022158 awarded by the National Institutes of Health. The United States government has certain rights in this invention.

### FIELD OF THE INVENTION

The invention relates generally to compositions comprising viral vectors comprising one or a plurality of mammalian transcription factors. The invention also relates to method of making and using the same for treating or preventing fibrosis of the liver in a subject.

### BACKGROUND OF THE INVENTION

Hepatocyte proliferation is effective in sustaining liver function in normal and acutely injured liver. However, repeated hepatocyte death, as in chronic liver diseases, can exceed the regenerative capabilities of hepatocytes. This deficiency leads to liver fibrosis, a form of scarring characterized by replacement of hepatocytes by collagen produced by myofibroblasts (MFs) [1-4]. The structural and molecular changes associated with liver fibrosis further impair liver function, eventually leading to liver failure. The only cure for liver fibrosis is liver transplantation, but donor organs are scarce [5]. Liver cell therapy is ineffective because cell engraftment is impaired in the fibrotic liver [6].

### SUMMARY OF ILLUSTRATIVE EMBODIMENTS

The invention is defined in the appended set of claims. The description may disclose additional technical information, which although not part of the claimed invention, is provided to place the invention in a broader technical context and to illustrate possible related technical developments.

The present invention provides a viral vector comprising:
a viral capsid comprising a nucleic acid molecule encapsulated within the viral capsid, wherein the nucleic acid molecule comprises:
a first nucleic acid sequence that encodes HNF4α; and
a second nucleic acid sequence that encodes one or more transcription factors selected from the group consisting of: FOXA1, FOXA2, FOXA3, HNF1α, HNF6, GATA4, HLF, CEBPA, PROX1, and ATF5A;
wherein the viral capsid comprises the viral capsid polypeptides VP1 of AAV6, VP2 of AAV6, and VP3 of AAV6.

The present invention further provides a composition comprising
a) one or a plurality of viral vectors of the invention; and/or
b) a plurality of viral vectors comprising
   i) a first viral vector comprising a viral capsid comprising a nucleic acid molecule encapsulated within the viral capsid, wherein the nucleic acid molecule comprises a nucleic acid sequence that encodes mammalian HNF4α and wherein the viral capsid comprises the viral capsid polypeptides VP1 of AAV6, VP2 of AAV6, and VP3 of AAV6; and ii) a second viral vector comprising a viral capsid comprising a nucleic acid molecule encapsulated within the viral capsid, wherein the nucleic acid molecule comprises a nucleic acid sequence that encodes one or more mammalian transcription factors selected from the group consisting of: FOXA1, FOXA2, FOXA3, HNF1α, HNF6, GATA4, HLF, CEBPA, PROX1, and ATF5A and wherein the viral capsid comprises the viral capsid polypeptides VP1 of AAV6, VP2 of AAV6, and VP3 of AAV6.

The present invention further provides a pharmaceutical composition comprising: a therapeutically effective amount of the viral vector or the composition of the invention; and a pharmaceutically acceptable carrier.

The present invention further provides the pharmaceutical composition of the invention for use in a method of treating and/or preventing liver fibrosis in a subject in need thereof.

The present invention further provides the pharmaceutical composition of the invention for use in a method of restoring tissue-specific function to fibrotic tissue in an organ in a subject suspected of having, diagnosed as having, or genetically predisposed to acquiring fibrotic tissue in an organ.

The present invention also provides the pharmaceutical composition of the invention for use in a method of inducing differentiation of a myofibroblast into a hepatocyte *in vivo* or a method of inducing proliferation of hepatocytes in a liver of a subject.

The present disclosure relates to a viral vector comprising: a viral capsid comprising a plurality of one or more viral capsid polypeptides and one or more nucleic acid molecules encapsulated within the viral capsid, wherein the one or more nucleic acid molecules comprise: a first nucleic acid sequence that encodes HNF4α or a functional fragment thereof; and a second nucleic acid sequence that encodes one or more transcription factors selected from the group consisting of thereof chosen from: FOXA1, FOXA2, FOXA3, HNF1α, HNF6, GATA4, HLF, CEBPA, PROX1, ATF5A and functional fragments thereof. The disclosure also relates to a composition or pharmaceutical composition comprising at least one viral vector comprising any of the disclosed nucleic acid sequence encoding one or more expressible genes, wherein the nucleic acid sequence comprises at last one or a plurality of regulatory sequences in operable communication with the one or more expressible genes. In some embodiments, the pharmaceutical compositions or compositions disclosed herein comprise a heterogeneous group of viral vectors, the contents of which include any one or combination of nucleic acid sequence disclosed herein.

In some embodiments, the viral capsid is derived from *Parvovirus.* In some embodiments, the viral capsid is derived from an adeno-associated virus (AAV). In embodiments of the invention, the viral capsid comprises the viral capsid polypeptides VP1 of AAV6, VP2 of AAV6, and VP3 of AAV6. In some embodiments of the disclosure, the present disclosure relates to any composition or pharmaceutical composition comprising one or a plurality of viral vectors wherein at least one viral vector comprises a viral capsid comprising at least one VP polypeptide comprising at least about 70% sequence identity to any of VP1, VP2 and/or VP3 of AAV6. In some embodiments, the viral capsid comprises at least one VP polypeptide comprising at least about 70% sequence identity to VP1 of any one or combination of AAV6, AAV7 and AAV8. In some embodiments, the viral capsid comprises at least one VP polypeptide comprising at least about 70% sequence identity to VP2 of any one or combination of AAV6, AAV7 and AAV8. In some embodiments, the viral capsid comprises at least one VP polypeptide comprising at least about 70% sequence identity to VP3 of any one or combination of AAV6, AAV7 and AAV8.

In some embodiments, the viral vector is free of an expressible gene from a lentivirus. In some embodiments, the one or more nucleic acid molecules are free of regulatory sequences from a lentivirus. In some embodiments, the viral vector is free of structural proteins or peptides from a lentivirus. In some embodiments, the one or more viral capsid polypeptides are derived from *Parvovirus.*

In some embodiments, the disclosure relates to a composition or pharmaceutical composition comprising one or a plurality of viral vectors comprising at least one or a plurality of nucleic acid sequences encoding one or a combination of any of the transcription factors disclosed herein. In some embodiments, the viral vector comprises a nucleic acid sequence encoding an amino acid sequence that is at least about 70% homologous to FOXA2 or a functional fragment thereof. In some embodiments, the one or more viral capsid polypeptides are selected from one or a combination of VP1, VP2, or VP3 polypeptides derived from any of AAV6, AAV7, and AAV8. In some embodiments, the viral capsid comprises VP1, VP2, and VP3 capsid proteins derived from AAV6.

The present disclosure also relates to a composition comprising a) a plurality of viral particles of claim 1; and/or b) a plurality of viral particles comprising: i) a first viral particle comprising a viral capsid comprising plurality of one or more viral capsid polypeptides and one or more nucleic acid molecules encapsulated within the viral capsid, wherein the one or more nucleic acid molecules comprises a nucleic acid sequence that encodes HNF4α or a functional fragment thereof; and ii) a second viral particle comprising a viral capsid comprising plurality of one or more viral capsid polypeptides and one or more nucleic acid molecules encapsulated within the viral capsid, wherein the one or more nucleic acid molecules comprises a nucleic acid sequence that encodes one or more transcription factors selected from the group consisting of thereof chosen from: FOXA1, FOXA2, FOXA3, HNF1α, HNF6, GATA4, HLF, CEBPA, PROX1, ATF5A and functional fragments thereof.

In some embodiments, the viral capsid is derived from *Parvovirus.* In some embodiments, the viral capsid is derived from an adeno-associated virus (AAV). In some embodiments, the viral capsid comprises at least one VP polypeptide comprising about 70%, 75%, 80%, 85%, 90%, 95%, 96%, 97%, 98%, or 99% sequence identity to any of VP1, VP2 or VP3 of AAV6. In some embodiments, the viral particle is also free of an expressible gene from a lentivirus. In some embodiments, the one or more nucleic acid molecules are free of regulatory sequences from a lentivirus. In some embodiments, the viral particle is free of an expressible gene from a retrovirus. In some embodiments, the one or more nucleic acid molecules are free of regulatory sequences from a retrovirus.

In some embodiments, the second nucleic acid sequences encodes an amino acid sequence that is at least about 70% homologous to a mammalian FOXA2 polypeptide or a functional fragment thereof. In some embodiments, the one or more viral capsid polypeptides are selected from a combination of VP1, VP2, or VP3 polypeptides derived from any of AAV6, AAV7, and AAV8. In some embodiments, the viral capsid comprises a combination of VP1, VP2, and VP3 capsid proteins derived from an AAV6 serotype.

The present disclosure also relates to a pharmaceutical composition comprising: any one or plurality of viral particles disclosed herein or any composition disclosed herein; and a pharmaceutically acceptable carrier.

In some embodiments, the viral capsid comprises at least one viral capsid polypeptide derived from *Parvovirus.* In some embodiments, the viral capsid comprises at least one viral capsid polypeptide derived from AAV. In some embodiments, the viral capsid comprises at least one viral capsid polypeptide derived from AAV6. In some embodiments, the viral capsid comprises at least one viral capsid polypeptide that has at least 70% sequence identity to VP1, VP2, or VP3 of any of AAV6, AAV7 or AAV8. In some embodiments, the viral capsid comprises at least one viral capsid polypeptide that has at least 70% sequence identity to VP1 of AAV6, at least one viral capsid polypeptide that has at least 70% sequence identity to VP2 of AAV6, and at least one viral capsid polypeptide that has at least 70% sequence identity to VP3 of AAV6. In some embodiments, the pharmaceutical composition is free of a short-hairpin RNA (shRNA), a nucleic acid sequence encoding a shRNA, a short inhibitory RNA (siRNA), and a nucleic acid sequence encoding a shRNA. In some embodiments, the pharmaceutical composition is sterile and pyrogen free.

The present disclosure also relates to a method of inducing differentiation of a fibroblast, such as a myofibroblast or a portal fibroblast, *in vivo* comprising contacting a fibroblast *in vivo* with the pharmaceutical composition disclosed herein with an amount of pharmaceutical composition sufficient to differentiate the fibroblast. The present disclosure also relates to a method of inducing differentiation of a fibroblast *in vivo* comprising contacting a fibroblast *in vivo* with the pharmaceutical composition in an amount sufficient to differentiate the fibroblast into a hepatocyte. In some embodiments, the pharmaceutical composition is administered to a subject via intravenous injection. In some embodiments, the fibroblast is a fibroblast of the subject's liver.

The present disclosure also relates to a method of inhibiting the deposition of collagen in a subject in need thereof comprising: contacting a fibroblast *in vivo* with the pharmaceutical composition in an amount sufficient to inhibit deposition of collagen. In some embodiments, the pharmaceutical composition is administered to a subject via intravenous injection. In some embodiments, the fibroblast is a fibroblast of the subject's liver.

The present disclosure also relates to a method of altering the phenotype of a fibroblast in a subject comprising: contacting a fibroblast of the subject liver in vivo with the pharmaceutical composition in an amount sufficient to alter the phenotype of the fibroblast in the subject.

The present disclosure also relates to a method of treating and/or preventing liver fibrosis in a subject in need thereof comprising: administering a therapeutic or prophylactically effective amount of the pharmaceutical composition. In some embodiments, the step of administering is performed via intravenous injection. Any reference in this specification to a method of treatment is to be interpreted as referring to the composition for use in the recited treatment.

The present disclosure also relates to a method of inducing proliferation of hepatocytes in a subject comprising: contacting a fibroblast of the subject liver in vivo with the pharmaceutical composition in an amount sufficient to induce proliferation of hepatocytes in a liver of the subject. In some embodiments, the pharmaceutical composition is administered to a subject via intravenous injection.

The present disclosure also relates to a method of targeting a fibroblast in the liver of a subject comprising contacting a fibroblast of the subject liver in vivo with the pharmaceutical composition in an amount sufficient to transduce the fibroblast in the liver.

The present disclosure also relates to a method of restoring tissue-specific function to fibrotic tissue in an organ comprising administering into a subject: a therapeutically effective amount of a first nucleic acid sequence encoding HNF4α or a functional fragment thereof; and a therapeutically effective amount of a second nucleic acid sequence that encodes one or a plurality of transcription factors or functional fragments thereof chosen from: FOXA1, FOXA2, FOXA3, HNF1α, HNF6, GATA4, HLF, CEBPA, PROX1, and ATF5A.

In some embodiments, the methods performed herein are performed by simultaneous or sequential administration of any one or more viral particles disclosed herein comprising any one or a plurality of any of the nucleic acid sequences disclosed herein.

The present disclosure also relates to a composition comprising a) a plurality of viral particles of claim 1; and/or b) a plurality of viral particles comprising: (i) a first viral particle comprising a viral capsid comprising a plurality of one or more viral capsid polypeptides and one or more nucleic acid molecules encapsulated within the viral capsid, wherein the one or more nucleic acid molecules comprises a nucleic acid sequence that encodes HNF4α or a functional fragment thereof; and ii) a second viral particle comprising a viral capsid comprising plurality of one or more viral capsid polypeptides and one or more nucleic acid molecules encapsulated within the viral capsid, wherein the one or more nucleic acid molecules comprises a nucleic acid sequence that encodes one or more transcription factors selected from the group consisting of: FOXA1, FOXA2, FOXA3, HNF1α, HNF6, GATA4, HLF, CEBPA, PROX1, ATF5A and functional fragments thereof. In some embodiments, the nucleic acid sequence encodes two or more transcription factors selected from the group consisting of: FOXA1, FOXA2, FOXA3, HNF1α, HNF6, GATA4, HLF, CEBPA, PROX1, ATF5A and functional fragments thereof. In some embodiments, the nucleic acid sequence encodes three or more transcription factors selected from the group consisting of: FOXA1, FOXA2, FOXA3, HNF1α, HNF6, GATA4, HLF, CEBPA, PROX1, ATF5A and functional fragments thereof. In some embodiments, the nucleic acid sequence encodes four or more transcription factors selected from the group consisting of: FOXA1, FOXA2, FOXA3, HNF1α, HNF6, GATA4, HLF, CEBPA, PROX1, ATF5A and functional fragments thereof. In some embodiments, the nucleic acid sequence encodes five or more transcription factors selected from the group consisting of: FOXA1, FOXA2, FOXA3, HNF1α, HNF6, GATA4, HLF, CEBPA, PROX1, ATF5A and functional fragments thereof. In some embodiments, the nucleic acid sequence encodes six or more transcription factors selected from the group consisting of: FOXA1, FOXA2, FOXA3, HNF1α, HNF6, GATA4, HLF, CEBPA, PROX1, ATF5A and functional fragments thereof. In some embodiments, the nucleic acid sequence encodes seven or more transcription factors selected from the group consisting of: FOXA1, FOXA2, FOXA3, HNF1α, HNF6, GATA4, HLF, CEBPA, PROX1, ATF5A and functional fragments thereof. In some embodiments, the nucleic acid sequence encodes eight or more transcription factors selected from the group consisting of: FOXA1, FOXA2, FOXA3, HNF1α, HNF6, GATA4, HLF, CEBPA, PROX1, ATF5A and functional fragments thereof. In some embodiments, the nucleic acid sequence encodes nine or more transcription factors selected from the group consisting of: FOXA1, FOXA2, FOXA3, HNF1α, HNF6, GATA4, HLF, CEBPA, PROX1, ATF5A and functional fragments thereof. In some embodiments, the nucleic acid sequence encodes ten or more transcription factors selected from the group consisting of: FOXA1, FOXA2, FOXA3, HNF1α, HNF6, GATA4, HLF, CEBPA, PROX1, ATF5A and functional fragments thereof. In some embodiments, the nucleic acid sequence encodes the transcription factor FOXA1 and one or more transcription factors selected from the group consisting of: FOXA2, FOXA3, HNF1α, HNF6, GATA4, HLF, CEBPA, PROX1, ATF5A and functional fragments thereof. In some embodiments, the nucleic acid sequence encodes the transcription factor FOXA2 and one or more transcription factors selected from the group consisting of: FOXA1, FOXA3, HNF1α, HNF6, GATA4, HLF, CEBPA, PROX1, ATF5A and functional fragments thereof. In some embodiments, the nucleic acid sequence encodes the transcription factor FOXA3 and one or more transcription factors selected from the group consisting of: FOXA1, FOXA2, HNF1α, HNF6, GATA4, HLF, CEBPA, PROX1, ATF5A and functional fragments thereof. In some embodiments, the nucleic acid sequence encodes the transcription factor HNF1α and one or more transcription factors selected from the group consisting of: FOXA1, FOXA2, FOXA3, HNF6, GATA4, HLF, CEBPA, PROX1, ATF5A and functional fragments thereof. In some embodiments, the nucleic acid sequence encodes the transcription factor HNF6 and one or more transcription factors selected from the group consisting of: FOXA1, FOXA2, FOXA3, HNF1α, GATA4, HLF, CEBPA, PROX1, ATF5A and functional fragments thereof. In some embodiments, the nucleic acid sequence encodes the transcription factor GATA4 and one or more transcription factors selected from the group consisting of: FOXA1, FOXA2, FOXA3, HNF1α, HNF6, HLF, CEBPA, PROX1, ATF5A and functional fragments thereof. In some embodiments, the nucleic acid sequence encodes the transcription factor HLF and one or more transcription factors selected from the group consisting of: FOXA1, FOXA2, FOXA3, HNF1α, HNF6, GATA4, CEBPA, PROX1, ATF5A and functional fragments thereof. In some embodiments, the nucleic acid sequence encodes the transcription factor CEBPA and one or more transcription factors selected from the group consisting of: FOXA1, FOXA2, FOXA3, HNF1α, HNF6, GATA4, HLF, PROX1, ATF5A and functional fragments thereof. In some embodiments, the nucleic acid sequence encodes the transcription factor PROX1 and one or more transcription factors selected from the group consisting of: FOXA1, FOXA2, FOXA3, HNF1α, HNF6, GATA4, HLF, CEBPA, ATF5A and functional fragments thereof. In some embodiments, the nucleic acid sequence encodes the transcription factor ATF5A and one or more transcription factors selected from the group consisting of: FOXA1, FOXA2, FOXA3, HNF1α, HNF6, GATA4, HLF, CEBPA, PROX1, and functional fragments thereof.

The present disclosure also relates to a method of restoring tissue-specific function to fibrotic tissue in an organ comprising administering into a subject suspected of having, diagnosed as having, or genetically predisposed to acquiring fibrotic tissue in an organ: (a) the pharmaceutical composition; and/ or (b) a first nucleic acid sequence encoding HNF4α or a functional fragment thereof; and a second nucleic acid sequence that encodes one or a plurality of transcription factors or functional fragments thereof chosen from: FOXA1, FOXA2, FOXA3, HNF1α, HNF6, GATA4, HLF, CEBPA, PROX1, and ATF5A.

In some embodiments, the pharmaceutical composition is free of a lentiviral vector or a lentiviral regulatory sequence but comprises one or more viral regulatory sequences in operable communication with any one or combination of expressible genes, either in trans or in cis. In some embodiments, the pharmaceutical composition is free of a retroviral vector or a retroviral regulatory sequence.

### BRIEF DESCRIPTION OF DRAWINGS

FIG. 1: Adult wildtype mice received 12 doses of CCl4 (over the course of six weeks) to induce liver fibrosis. Afterwards the mice were intravenously injected with 4 × 10¹¹ viral genomes of an AAV-EYFP vector pseudotyped with one of the eight candidate capsids. Livers were analyzed four weeks later.
FIG. 2: Co-immunostainings for EYFP and α-SMA of fibrotic livers of mice intravenously injected with AAV-EYFP vectors pseudotyped with AAV 6, 7 or 8 capsids show transduced MFs. Size bars, 62.5 µm.
FIG. 3: Co-immunostainings for α-SMA and EYFP show that the AAV1P4, AAV2, AAV9, AAV2(Y444,500,730F) and AAV-DJ capsids transduce hepatocytes but not MFs in the liver. Size bars, 75 µm.
FIG. 4: Quantification of transduced MFs *in vivo.* Results are means ± s.e.m. for biological replicates (n = 3).
FIG. 5: Immunostainings for EYFP show that AAV6-EYFP efficiently transduces liver and skeletal muscle (M. extensor iliotibialis anticus). Size bars, 75 µm.
FIG. 6: Direct fluorescence shows EYFP expression in MFs-generated by culturing wildtype HSCs on plastic for 10 days-transduced *in vitro* with AAV6-EYFP but not in vehicle-treated control cells. Size bars, 200 µm.
FIG. 7: Quantification of transduced MFs *in vitro.* Results are means ± s.e.m. for biological replicates (n = 3) and technical replicates (n = 3). All results were replicated in two independent experiments that each used three biological replicates.
FIG. 8: qRT-PCR shows overexpression of *Foxa1, Foxa2, Foxa3, Gata4, Hnf1a* and *Hnf4a* in MFs transduced with AAV6-6TFs *in vitro* relative to nontransduced MFs (Vehicle). Results are means ± s.e.m. for biological replicates (n = 3) and technical replicates (n = 3). Student's *t*-test, one asterisk, *P* < 0.05; two asterisks, *P* < 0.01.
FIG. 9: qRT-PCR shows reduced expression of the MF markers *Acta2, Des, Col1a1* and *Col1a2* in MFs transduced with AAV6-6TFs *in vitro* relative to nontransduced MFs (Vehicle). Results are means ± s.e.m. for biological replicates (n = 3) and technical replicates (n = 3). Student's *t*-test, two asterisks, *P* < 0.01; three asterisks, *P* < 0.001. All results were replicated in two independent experiments that each used three biological replicates.
FIG. 10: Adult Pdgfrb-Cre, R26R-EYFP mice received 12 doses of CCl4 (over the course of six weeks) to generate a mouse model of MF lineage tracing in liver fibrosis. Livers were analyzed three days after the last CCl4 dose.
FIG. 11: Coimmunostaining for α-SMA/DES and EYFP shows efficient lineage tracing of MFs. Size bars, 75 µm.
FIG. 12: Co-immunostaining for MUP and EYFP shows absence of unspecific lineage tracing of hepatocytes. Size bars, 75 µm.
FIG. 13: After receiving 12 doses of CCl4 (over the course of six weeks) adult Pdgfrb-Cre, R26R-EYFP mice were intravenously injected with 4 × 10¹¹ viral genomes of AAV6-6TFs to reprogram MFs into MF-iHeps. Livers were analyzed four weeks after AAV6-6TFs injection.
FIG. 14: Co-immunostainings for FAR and EYFP show clusters of three MF-iHeps in different liver sections. Size bars, 75 µm. All results were replicated in four independent experiments that each used three biological replicates.
FIG. 15: Co-immunostainings for MUP and EYFP show clusters of three MF-iHeps in different liver sections. Size bars, 75 µm. All results were replicated in four independent experiments that each used three biological replicates.
FIG. 16: P2 FRG pups were intrahepatically injected with 250,000 Pdgfrb-Cre, R26R-EYFP HSCs to generate a mouse model in which MFi-Heps have a selective growth advantage.
FIG. 17: Immunostaining for EYFP shows high engraftment efficiency of Pdgfrb-Cre, R26R-EYFP HSCs eight weeks after intrahepatic injection into FRG mice. Size bars, 75 µm.
FIG. 18: After receiving five doses of CCl4 (over the course of two weeks) six weeks later to prompt HSCs to become MFs, mice were intravenously injected with 4 × 10¹¹ viral genomes of AAV6-6TFs to reprogram MFs into MF-iHeps. Four weeks later the mice were cycled off/on NTBC twice to induce MF-iHep proliferation. Livers were analyzed at the end of the last cycle off NTBC. Coimmunostaining for FAH and EYFP shows a cluster of four MF-iHeps. Size bars, 40 µm.
FIG. 19: After receiving five doses of CCl4 (over the course of two weeks) six weeks later to prompt HSCs to become MFs, mice were intravenously injected with 4 × 10¹¹ viral genomes of AAV6-6TFs to reprogram MFs into MF-iHeps. Four weeks later the mice were cycled off/on NTBC twice to induce MF-iHep proliferation. Livers were analyzed at the end of the last cycle off NTBC. Coimmunostaining for MUP and EYFP shows a different cluster of four MF-iHeps. Size bars, 40 µm.
FIG. 20: Co-immunostaining shows EYFPpositive MF-iHeps with residual DES expression. Size bars, 40 µm.
FIG. 21: Co-immunostaining shows EYFP-positive MF-iHeps lacking VIM expression. Size bars, 75 µm.
FIG. 22: Coimmunostaining shows EYFP-positive MF-iHeps lacking COL1A1 expression. Size bars, 75 µm.
FIG. 23: After receiving five doses of CCl4 (over the course of two weeks) six weeks later to prompt HSCs to become MFs, mice were intravenously injected with 4 × 10¹¹ viral genomes of AAV6-6TFs to reprogram MFs into MF-iHeps. Four weeks later the mice were cycled off/on NTBC five times to induce MF-iHep proliferation. Livers were analyzed at the end of the last cycle off NTBC. Co-immunostaining for FAR and EYFP shows a nodule of 64 MF-iHeps. Size bars, 40 µm.
FIG. 24: After receiving five doses of CCl4 (over the course of two weeks) six weeks later to prompt HSCs to become MFs, mice were intravenously injected with 4 × 10¹¹ viral genomes of AAV6-6TFs to reprogram MFs into MF-iHeps. Four weeks later the mice were cycled off/on NTBC five times to induce MF-iHep proliferation. Livers were analyzed at the end of the last cycle off NTBC. Co-immunostaining for FAH and Ki67 shows proliferating MF-iHeps in a nodule of 32 MF-iHeps. Size bars, 40 µm.
FIG. 25: Adult Pdgfrb-Cre, R26R-RFP mice received six doses of CCl4 (over the course of three weeks) to generate a mouse model of MF lineage-tracing in early liver fibrosis. Afterwards the mice were intravenously injected with 4 × 10¹¹ viral genomes of AAV6-6TFs to reprogram MFs into MFiHeps. Livers were analyzed five weeks after AAV6-6TFs injection.
FIG. 26: Adult Pdgfrb-Cre, R26-RFP mice received 20 doses of CCl4 (over the course of 10 weeks) to generate a mouse model of MF lineage-tracing in advanced liver fibrosis. Afterwards the mice were intravenously injected with 4 × 10¹¹ viral genomes of AAV6-6TFs to reprogram MFs into MF-iHeps. Livers were analyzed five weeks after AAV6-6TFs injection.
FIG. 27: Immunostaining for MUP and direct RFP fluorescence shows a cluster of two MF-iHeps. Size bars, 75 µm.
FIG. 28: Immunostaining for MUP and direct RFP fluorescence shows a cluster of 16 MF-iHeps. Size bars, 75 µm.
FIG. 29: Modeling of early fibrosis. Immunostainings for COL1A1 show early liver fibrosis in Pdgfrb-Cre, R26R-EYFP mice treated with six doses of CCl4. Size bars, 150 µm. All results were replicated in two independent experiments that each used three biological replicates.
FIG. 30: Modeling of advanced fibrosis. Immunostainings for COL1A1 show advanced liver fibrosis in Pdgfrb-Cre, R26R-EYFP mice treated with 20 doses of CCl4. Size bars, 150 µm. All results were replicated in two independent experiments that each used three biological replicates.
FIG. 31: Advanced liver fibrosis and ongoing liver injury increase MF-iHep proliferation and formation efficiency. Graph shows a positive correlation between MF-iHep clone size and number of CCl4 doses in Pdgfrb-Cre, R26R-RFP/EYFP mice after intravenous injection of AAV6-6TFs. The number of reprogramming events is not correlated with the number of CCl4 doses but increases in mice that received CCl4 not only before but also after intravenous injection of AAV6-6TFs.
FIG. 32: Adult Pdgfrb-Cre, R26R-EYFP mice received 16 doses of CCl4 (over the course of eight weeks) to generate a mouse model of MF lineage-tracing in advanced liver fibrosis. Afterwards the mice were intravenously injected with 4 × 10¹¹ viral genomes of AAV6-6TFs to reprogram MFs into MF-iHeps. Two weeks after AAV6-6TFs injection the mice received additional 12 doses of CCl4 (over the course of six weeks) to model persistent liver injury. Livers were analyzed two days after the last CCl4 dose.
FIG. 33: Co-immunostaining for MUP and EYFP shows a nodule of 82 MF-iHeps. Size bars, 75 µm.
FIG. 34: FIG. 34: Modeling of bridging fibrosis. Immunostainings for COL1A1 show bridging liver fibrosis in Pdgfrb-Cre, R26R-EYFP mice treated with 28 (16 followed by an additional 12) doses of CCl4. Size bars, 150 µm. All results were replicated in two independent experiments that each used three biological replicates.
FIG. 35: Immunostaining for EYFP shows lineage-traced MFs in a section of a liver lobe of a Pdgfrb-Cre, R26R-EYFP mouse that received 28 doses of CCl4 in two phases, 16 doses before and 12 doses after a 2-week-long interval at the beginning of which AAV6-6TFs were intravenously injected. White arrowheads indicate 16 MF-iHep clones originating from separate reprogramming events. Size bar, 500 µm. All results were replicated in two independent experiments that each used three biological replicates.
FIG. 36: Quantification of immunostainings for COL1A1 of livers of Pdgfrb-Cre, R26R-EYFP mice that received 28 doses of CCl4 shows less collagen deposition in areas where MF-iHeps are located as compared to areas where only injured primary hepatocytes are located. Results are means ± s.e.m. for technical replicates (n = 15). Student's t-test, two asterisks, *P* < 0.01.
FIG. 37: Lasercapture microdissection followed by qRT-PCR shows both MF-iHeps and primary hepatocytes express *Des* in livers of Pdgfrb-Cre, R26RRFP mice that received 20 doses of CCl4. HSCs and hepatocytes from noninjured Pdgfrb-Cre, R26R-RFP mice were used as controls. ND, not detected. Results are means ± s.e.m. for biological replicates (n = 3) and technical replicates (n = 3). Student's *t*-test, one asterisk, *P* < 0.05; three asterisks, *P* < 0.001.
FIG. 38: Lasercapture microdissection followed by qRT-PCR shows only MF-iHeps express traces of *Acta2* in livers of Pdgfrb-Cre, R26RRFP mice that received 20 doses of CCl4. HSCs and hepatocytes from noninjured Pdgfrb-Cre, R26R-RFP mice were used as controls. ND, not detected. Results are means ± s.e.m. for biological replicates (n = 3) and technical replicates (n = 3). Student's t-test, one asterisk, *P* < 0.05; three asterisks, *P* < 0.001.
FIG. 39: qRT-PCR shows expression levels of the hepatic TF genes *Hnf4a* and *Foxa3* and *Serpina1a* and *Alb,* genes encoding secreted proteins, in MF-iHeps and primary hepatocytes in livers of Pdgfrb-Cre, R26R-RFP mice that received 20 doses of CCl4. Hepatocytes from noninjured Pdgfrb-Cre, R26R-RFP mice were used as control. Results are means ± s.e.m. for biological replicates (n = 3) and technical replicates (n = 3). Student's t-test, three asterisks, *P* < 0.001. All results were replicated in two independent experiments that each used three biological replicates.

### DETAILED DESCRIPTION OF ILLUSTRATIVE EMBODIMENTS

The terminology used herein is for the purpose of describing particular embodiments only and is not intended to be limiting. Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The following terms or definitions are provided solely to aid in the understanding of the invention. Unless specifically defined herein, all terms used herein have the same meaning as they would to one skilled in the art of the present invention. Practitioners are particularly directed to Sambrook et al., Molecular Cloning: A Laboratory Manual, 2nd ed., Cold Spring Harbor Press, Plainsview, N.Y. (1989); and Ausubel et al., Current Protocols in Molecular Biology (Supplement 47), John Wiley & Sons, New York (1999), for definitions and terms of the art. The definitions provided herein should not be construed to have a scope less than understood by a person of ordinary skill in the art.

As used in the specification and the appended claims, the singular forms "a," "an" and "the" include plural referents unless the context clearly dictates otherwise.

"AAV virion" refers to a complete virus particle, such as for example a wild type AAV virion particle, which comprises single stranded genome DNA packaged into AAV capsid proteins. In embodiments of the invention, the viral capsid comprises the viral capsid polypeptides VP1 of AAV6, VP2 of AAV6, and VP3 of AAV6. The single stranded nucleic acid molecule is either sense strand or antisense strand, as both strands are equally infectious. A "rAAV virion" refers to a recombinant AAV virus particle, i.e. a particle which is infectious but replication defective. It is composed of an AAV protein shell and comprises a rAAV vector. In the context of the present invention the protein shell may be of a different serotype than the rAAV vector. An AAV virion of the invention may thus be composed a protein shell, i.e. the icosahedral capsid, which comprises capsid proteins (VPl, VP2, and/or VP3) of one AAV serotype, e.g. AAV serotype 6, whereas the rAAV vector contained in that AAV6 virion may be any of the rAAVX vectors described above, including a rAAV6 vector. An "rAAV6 virion" comprises capsid proteins of AAV serotype 6, while e.g. a rAAV2 virion comprises capsid proteins of AAV serotype 2, whereby either may comprise any of rAAVX vectors of the invention. "AAV helper functions" generally refers to the corresponding AAV functions required for rAAV replication and packaging supplied to the rAAV virion or rAAV vector in trans. AAV helper functions complement the AAV functions which are missing in the rAAV vector, but they lack AAV ITRs (which are provided by the rAAV vector). AAV helper functions include the two major ORFs of AAV, namely the rep coding region and the cap coding region or functional substantially identical sequences thereof. Rep and Cap regions are well known in the art, see e.g. Chiorini et al. (1999, J. of Virology, Vol 73(2): 1309-1319) or US 5,139,941. The AAV helper functions can be supplied on a AAV helper construct. Introduction of the helper construct by into the host cell can occur e.g. by transformation or transduction prior to or concurrently with the introduction of the rAAV vector. The AAV helper constructs of the invention may thus be chosen such that they produce the desired combination of serotypes for the rAAV virion' s capsid proteins on the one hand and for the rAAV vector replication and packaging on the other hand.

"AAV helper virus" provides additional functions required for AAV replication and packaging. Suitable AAV helper viruses include adenoviruses, herpes simplex viruses (such as HSV types 1 and 2) and vaccinia viruses. The additional functions provided by the helper virus can also be introduced into the host cell via vectors, as described in US 6,531,456.

The term "about" as used herein when referring to a measurable value such as an amount, a temporal duration, and the like, is meant to encompass variations of ±20%, ±10%, ±5%,, ±1%, or ±0.1% from the specified value, as such variations are appropriate to perform the disclosed methods. For recitation of numeric ranges herein, each intervening number therebetween with the same degree of precision is explicitly contemplated. For example, for the range of 6-9, the numbers 7 and 8 are contemplated in addition to 6 and 9, and for the range 6.0-7.0, the numbers 6.0, 6.1, 6.2, 6.3, 6.4, 6.5, 6.6, 6.7, 6.8, 6,9, and 7.0 are explicitly contemplated.

"Cell type" means the organism, organ, and/or tissue type from which the cell is derived or sourced, state of development, phenotype or any other categorization of a particular cell that appropriately forms the basis for defining it as "similar to" or "different from" another cell or cells.

"Coding sequence" or "encoding nucleic acid" as used herein may mean refers to the nucleic acid (RNA, DNA, or RNA/DNA hybrid molecule) that comprises a nucleotide sequence which encodes a protein. The coding sequence may further include initiation and termination signals operably linked to regulatory elements including a promoter and polyadenylation signal capable of directing expression in the cells of an individual or mammal to whom the nucleic acid is administered.

"Complement" or "complementary" as used herein may mean a nucleic acid may mean Watson-Crick (e.g., A-T/U and C-G) or Hoogsteen base pairing between nucleotides or nucleotide analogs of nucleic acid molecules.

As used herein, the term "functional fragment" means any portion of a polypeptide that is of a sufficient length to retain at least partial biological function that is similar to or substantially similar to the wild-type polypeptide upon which the fragment is based. In some embodiments, a functional fragment of a polypeptide is a polypeptide that comprises or possesses 80, 85, 90, 95, 96, 97, 98, or 99% sequence identity to any polypeptide disclosed in Table 3 and has sufficient length to retain at least partial binding affinity to one or a plurality of ligands that bind to the polypeptides in Table 3. In some embodiments, a functional fragment of a nucleic acid is a nucleic acid that comprises or possesses 80, 85, 90, 95, 96, 97, 98, or 99% sequence identity to any nucleic acid to which it is being compared and has sufficient length to retain at least partial function related to the nucleic acid to which it is being compared. In some embodiments, the fragment is a fragment of any polypeptide disclosed in Table 3 and has a length of at least about 10, about 20, about 30, about 40, about 50 , about 60, about 70, about 80, about 90, or about 100 contiguous amino acids. In some embodiments, the fragment is a fragment of any polypeptide disclosed in Table 3 and has a length of at least about 50 amino acids. In some embodiments, the fragment is a fragment of any polypeptide disclosed in Table 3 and has a length of at least about 100 amino acids. In some embodiments, the fragment is a fragment of any polypeptide disclosed in Table 3 and has a length of at least about 150 amino acids. In some embodiments, the fragment is a fragment of any polypeptide disclosed in Table 3 and has a length of at least about 200 amino acids. In some embodiments, the fragment is a fragment of any polypeptide disclosed in Table 3 and has a length of at least about 250 amino acids. In some embodiments, the fragment is a fragment of any polypeptide disclosed in Table 3 and has a length of at least about 300 amino acids. In some embodiments, the fragment is a fragment of any polypeptide disclosed in Table 3 and has a length of at least about 350 amino acids. In some embodiments, the fragment is a fragment of any polypeptide disclosed in Table 3 and has a length of at least about 400 amino acids. In some embodiments, the fragment is a fragment of any polypeptide disclosed in Table 3 and has a length of at least about 450 amino acids. In some embodiments, the fragment is a fragment of any polypeptide disclosed in Table 3 and has a length of at least about 500 amino acids. In some embodiments, the fragment is a fragment of any polypeptide disclosed in Table 3 and has a length of at least about 550 amino acids. In some embodiments, the fragment is a fragment of any polypeptide disclosed in Table 3 and has a length of at least about 600 amino acids. In some embodiments, the fragment is a fragment of any polypeptide disclosed in Table 3 and has a length of at least about 650 amino acids. In some embodiments, the fragment is a fragment of any polypeptide disclosed in Table 3 and has a length of at least about 700 amino acids. In some embodiments, the fragment is a fragment of any polypeptide disclosed in Table 3 and has a length of at least about 750 amino acids. In some embodiments, the fragment is a fragment of any polypeptide disclosed in Table 3 and has a length of at least about 800 amino acids. In some embodiments, the fragment is a fragment of any polypeptide disclosed in Table 3 and has a length of at least about 850 amino acids. In some embodiments, the fragment is a fragment of any polypeptide disclosed in Table 3 and has a length of at least about 900 amino acids. In some embodiments, the fragment is a fragment of any polypeptide disclosed in Table 3 and has a length of at least about 950 amino acids. In some embodiments, the fragment is a fragment of any polypeptide disclosed in Table 3 and has a length of at least about 1000 amino acids. In some embodiments, the fragment is a fragment of any polypeptide disclosed in Table 3 and has a length of at least about 1050 amino acids. In some embodiments, the fragment is a fragment of any polypeptide disclosed in Table 3 and has a length of at least about 1250 amino acids. In some embodiments, the fragment is a fragment of any polypeptide disclosed in Table 3 and has a length of at least about 1500 amino acids. In some embodiments, the fragment is a fragment of any polypeptide disclosed in Table 3 and has a length of at least about 1750 amino acids. In some embodiments, the fragment is a fragment of any polypeptide disclosed in Table 3 and has a length of at least about 2000 amino acids. In some embodiments, the fragment is a fragment of any polypeptide disclosed in Table 3 and has a length of at least about 2250 amino acids. In some embodiments, the fragment is a fragment of any polypeptide disclosed in Table 3 and has a length of at least about 2500 amino acids. In some embodiments, the fragment is a fragment of any polypeptide disclosed in Table 3 and has a length of at least about 2750 amino acids. In some embodiments, the fragment is a fragment of any polypeptide disclosed in Table 3 and has a length of at least about 3000 amino acids.

The term "polypeptide" encompasses two or more naturally or non-naturally-occurring amino acids joined by a covalent bond (e.g., an amide bond). Polypeptides as described herein include full-length proteins (e.g., fully processed pro-proteins or full-length synthetic polypeptides) as well as shorter amino acid sequences (e.g., fragments of naturally-occurring proteins or synthetic polypeptide fragments).

As used herein, the terms "polypeptide sequence associated with a hepatocyte" means any polypeptide or fragment thereof, modified or unmodified by any macromolecule (such as a sugar molecule or macromolecule) that is produced naturally by hepatocytes in any multicellular organism or whose structure is based upon an polypeptide expressed by a cell of a hepatocyte lineage. In some embodiments, a polypeptide sequence associated with the hepatocyte is any polypeptide or fragment thereof, modified or unmodified by any macromolecule (such as a sugar molecule or macromolecule) that is produced naturally by epithelial hepatocytes in any multicellular organism or whose structure is based upon an polypeptide expressed by an epithelial cell of with a hepatocyte lineage. In some embodiments, a polypeptide sequence associated with the hepatocyte does not comprise a polypeptide or fragment thereof, modified or unmodified by any macromolecule (such as a sugar molecule or macromolecule) that is produced naturally by fibroblasts within the liver of any multicellular organism or whose structure is based upon an polypeptide expressed by a fibroblast, even within the liver. In some embodiments, a polypeptide sequence associated with the hepatocyte is any polypeptide sequence comprising any one or plurality of the polypeptides disclosed in Table 3. In some embodiments, a polypeptide sequence associated with the heptaocyte is any polypeptide sequence comprising any of the polypeptides disclosed in Table 3 or a sequence that shares 85,90,95, 96, 97, 98, or 99% sequence identity with the polypeptides disclosed in Table 3 or a functional fragment thereof. In some embodiments, a polypeptide sequence associated with the extracellular matrix consists of any of the polypeptides disclosed in Table 3 or a sequence that shares 85,90,95, 96, 97, 98, or 99% sequence identity with the polypeptides disclosed in Table 3.

As used herein, "sequence identity" is determined by using the stand-alone executable BLAST engine program for blasting two sequences (bl2seq), which can be retrieved from the National Center for Biotechnology Information (NCBI) ftp site, using the default parameters (Tatusova and Madden, FEMS Microbiol Lett., 1999, 174, 247-250).

The term "subject" is used throughout the specification to describe an animal from which a cell sample is taken or an animal to which a disclosed virus or viral vector has been administered. In some embodiment, the animal is a human. For diagnosis of those conditions which are specific for a specific subject, such as a human being, the term "patient" may be interchangeably used. In some instances in the description of the present invention, the term "patient" will refer to human patients suffering from a particular disease or disorder. In some embodiments, the subject may be a human suspected of having or being identified as at risk to develop fibrosis or cirrhosis of the liver. In some embodiments, the subject may be diagnosed as having fibrosis of the liver or being identified as at risk to develop fibrosis of the liver. In some embodiments, the subject is suspected of having or has been diagnosed with fibrosis of the liver. In some embodiments, the subject may be a human suspected of having or being identified as at risk to develop fibrosis of the liver. In some embodiments, the subject may be a mammal which functions as a source of the isolated cell sample. In some embodiments, the subject may be a non-human animal from which a cell sample is isolated or provided, such as a mammal. The term "mammal" encompasses both humans and non-humans and includes but is not limited to humans, non-human primates, canines, felines, murines, bovines, equines, and porcines.

"Nucleic acid" or "oligonucleotide" or "polynucleotide" as used herein may mean at least two nucleotides covalently linked together. The depiction of a single strand also defines the sequence of the complementary strand. Thus, a nucleic acid also encompasses the complementary strand of a depicted single strand. Many variants of a nucleic acid may be used for the same purpose as a given nucleic acid. Thus, a nucleic acid also encompasses substantially identical nucleic acids and complements thereof. A single strand provides a probe that may hybridize to a target sequence under stringent hybridization conditions. Thus, a nucleic acid also encompasses a probe that hybridizes under stringent hybridization conditions.

Nucleic acids may be single stranded or double stranded, or may contain portions of both double stranded and single stranded sequence. The nucleic acid may be DNA, both genomic and cDNA, RNA, or a hybrid, where the nucleic acid may contain combinations of deoxyribo- and ribonucleotides, and combinations of bases including uracil, adenine, thymine, cytosine, guanine, inosine, xanthine hypoxanthine, isocytosine and isoguanine. Nucleic acids may be obtained by chemical synthesis methods or by recombinant methods. In some embodiments, the nucleic acid is isolated from an organism.

"Operably linked" as used herein may mean that expression of a gene is under the control of a promoter with which it is spatially connected. A promoter may be positioned 5' (upstream) or 3' (downstream) of a gene under its control. The distance between the promoter and a gene may be approximately the same as the distance between that promoter and the gene it controls in the gene from which the promoter is derived. As is known in the art, variation in this distance may be accommodated without loss of promoter function.

"Promoter" as used herein may mean a synthetic or naturally-derived molecule which is capable of conferring, activating or enhancing expression of a nucleic acid in a cell. A promoter may comprise one or more specific transcriptional regulatory sequences to further enhance expression and/or to alter the spatial expression and/or temporal expression of same. A promoter may also comprise distal enhancer or repressor elements, which can be located as much as several thousand base pairs from the start site of transcription. A promoter may be derived from sources including viral, bacterial, fungal, plants, insects, and animals. A promoter may regulate the expression of a gene component constitutively, or differentially with respect to cell, the tissue or organ in which expression occurs or, with respect to the developmental stage at which expression occurs, or in response to external stimuli such as physiological stresses, pathogens, metal ions, or inducing agents. Representative examples of promoters include the bacteriophage T7 promoter, bacteriophage T3 promoter, SP6 promoter, lac operator-promoter, tac promoter, SV40 late promoter, SV40 early promoter, RSV-LTR promoter, CMV IE promoter, SV40 early promoter or SV40 late promoter and the CMV IE promoter.

"rAAV vector" as used herein refers to a recombinant vector derived from an adeno-associated virus serotype, such as AAV1, AAV2, AAV3, AAV4, AAV5, AAV6, AAV7, AAV8 and others. rAAV vectors have one or preferably all wild type AAV genes deleted, but still comprise functional ITR nucleic acid sequences. Functional ITR sequences are necessary for the replication, rescue and packaging of AAV virions. The ITR sequences may be wild type sequences or substantially identical sequences (as defined below) or may be altered by for example in insertion, mutation, deletion or substitution of nucleotides, as long as they remain functional. "rAAV vector" as used herein also refers to a recombinant AAV vector comprising the ITR nucleic acid sequences of any of the AAV serotypes, or nucleic acid sequences being substantially identical to the particular AAV serotype wild type ITR sequences, as long as they remain functional. Nucleotide sequences of choice are inserted between the AAV ITR sequences, for example expression constructs comprising an expression regulatory element operably linked to a coding sequence and a 3' termination sequence. The term "rAAV vector" as used herein also refers to a recombinant AAV vector comprising the ITR nucleic acid sequences of the AAV serotype, or nucleic acid sequences being substantially identical to the AAV serotype wild type ITR sequences, as long as they remain functional. The term "rAAV5 vector" or "rAAV2 vector" is thus used to indicate a rAAV5 or rAAV2 vector comprising respectively the ITR nucleic acid sequences of AAV serotype 5 or serotype 2, or nucleic acid sequences substantially identical thereto.

"Signal peptide" and "leader sequence" are used interchangeably herein and refer to an amino acid sequence that can be linked at the amino terminus of a protein set forth herein. Signal peptides/leader sequences typically direct localization of a protein. Signal peptides/leader sequences used herein preferably facilitate secretion of the protein from the cell in which it is produced. Signal peptides/leader sequences are often cleaved from the remainder of the protein, often referred to as the mature protein, upon secretion from the cell. Signal peptides/leader sequences are linked at the N terminus of the protein.

"Stringent hybridization conditions" as used herein may mean conditions under which a first nucleic acid sequence (e.g., probe) will hybridize to a second nucleic acid sequence (e.g., target), such as in a complex mixture of nucleic acids. Stringent conditions are sequence-dependent and will be different in different circumstances. Stringent conditions may be selected to be about 5-10°C lower than the thermal melting point (Tₘ) for the specific sequence at a defined ionic strength pH. The Tₘ may be the temperature (under defined ionic strength, pH, and nucleic concentration) at which 50% of the probes complementary to the target hybridize to the target sequence at equilibrium (as the target sequences are present in excess, at Tₘ, 50%> of the probes are occupied at equilibrium). Stringent conditions may be those in which the salt concentration is less than about 1.0 M sodium ion, such as about 0.01-1.0 M sodium ion concentration (or other salts) at pH 7.0 to 8.3 and the temperature is at least about 30°C for short probes (e.g., about 10-50 nucleotides) and at least about 60°C for long probes (e.g., greater than about 50 nucleotides). Stringent conditions may also be achieved with the addition of destabilizing agents such as formamide. For selective or specific hybridization, a positive signal may be at least 2 to 10 times background hybridization. Exemplary stringent hybridization conditions include the following: 50%> formamide, 5x SSC, and 1% SDS, incubating at 42°C, or, 5x SSC, 1% SDS, incubating at 65°C, with wash in 0.2x SSC, and 0.1% SDS at 65°C.

"Substantially complementary" as used herein may mean that a first sequence is at least 60%, 65%, 70%, 75%, 80%, 85%, 90%, 95%, 96%, 97%, 98% or 99% identical to the complement of a second sequence over a region of 8, 9, 10, 11, 12, 13, 14, 15, 16, 17, 18, 19, 20, 21, 22, 23, 24, 25, 30, 35, 40, 45, 50, 55, 60, 65, 70, 75, 80, 85, 90, 95, 100 or more nucleotides or amino acids, or that the two sequences hybridize under stringent hybridization conditions.

"Substantially identical" as used herein may mean that, in respect to a first and a second sequence, a first and second sequence are at least 60%, 65%, 70%, 75%, 80%, 85%, 90%, 95%, 96%, 97%, 98% or 99% identical over a region of 8, 9, 10, 11, 12, 13, 14, 15, 16, 17, 18, 19, 20, 21, 22, 23, 24, 25, 30, 35, 40, 45, 50, 55, 60, 65, 70, 75, 80, 85, 90, 95, 100 or more nucleotides or amino acids, or with respect to nucleic acids, if the first sequence is substantially complementary to the complement of the second sequence.

"Subtype" or "serotype": as used herein, interchangeably, and in reference to AAV, means genetic variants of an AAV such that one subtype is less recognized by an immune system of a subject apart from a different subtype. In some embodiments, the viral vector comprises at least one *cap* polypeptide from an AAV serotype chosen from: AAV1, AAV2, AAV3, AAV4, AAV5, AAV6, AAV7, AAV8, AAV9, AAV10, AAV11, and AAV12. In some embodiments the viral vector comprises a polypeptide comprising VP1 from an AAV serotype chosen from: AAV1, AAV2, AAV3, AAV4, AAV5, AAV6, AAV7, AAV8, AAV9, AAV10, AAV11, and AAV12. In some embodiments the viral vector comprises a polypeptide comprising VP2 from an AAV serotype chosen from: AAV1, AAV2, AAV3, AAV4, AAV5, AAV6, AAV7, AAV8, AAV9, AAV10, AAV11, and AAV12. In some embodiments the viral vector comprises a polypeptide comprising VP3 from an AAV serotype chosen from: AAV1, AAV2, AAV3, AAV4, AAV5, AAV6, AAV7, AAV8, AAV9, AAV10, AAV11, and AAV12. In some embodiments, the viral vector comprises VP1, VP2 and VP3 polypeptides that is at least 60%, 65%, 70%, 75%, 80%, 85%, 90%, 95%, 96%, 97%, 98% or 99% identical over the VP1, VP2, and/or VP3 polypeptides from AAV6. In some embodiments, the viral vector comprises VP1, VP2 and VP3 polypeptides that is at least 60%, 65%, 70%, 75%, 80%, 85%, 90%, 95%, 96%, 97%, 98% or 99% identical over the VP1, VP2, and/or VP3 polypeptides from AAV7. In some embodiments, the viral vector comprises VP1, VP2 and VP3 polypeptides that is at least 60%, 65%, 70%, 75%, 80%, 85%, 90%, 95%, 96%, 97%, 98% or 99% identical over the VP1, VP2, and/or VP3 polypeptides from AAV8.

The term "effective amount" or "therapeutically effective amount" means that amount of compound, composition or agent that will elicit the biological or medical response of a subject that is being sought. In some embodiments, the therapeutically effective amount is administered by a medical doctor or other clinician. In particular, with regard to treating a liver-related disorder, the term "effective amount" is intended to mean that amount of a compound, composition or agent that will elicit the biological or medical response of a subject that is being sought with regard to alleviating, suspending, curing or partially curing a cause of the disease, symptom or set of symptoms, due to a dysfunction or scarring of liver tissue in a subject. In some embodiments, the term "effective amount" is intended to mean that effective amount of an compound or agent that will bring about a biologically meaningful increase in the hepatocyte mass in the liver of a subject.

"Variant" used herein with respect to a nucleic acid means (i) a portion or fragment of a referenced nucleotide sequence; (ii) the complement of a referenced nucleotide sequence or portion thereof; (iii) a nucleic acid that is substantially identical to a referenced nucleic acid or the complement thereof; or (iv) a nucleic acid that hybridizes under stringent conditions to the referenced nucleic acid, complement thereof, or a sequences substantially identical thereto. "Variant" with respect to a peptide or polypeptide that differs in amino acid sequence by the insertion, deletion, or conservative substitution of amino acids, but retain at least one biological activity. Variant may also mean a protein with an amino acid sequence that is substantially identical to a referenced protein with an amino acid sequence that retains at least one biological activity. A conservative substitution of an amino acid, i.e., replacing an amino acid with a different amino acid of similar properties (e.g., hydrophilicity, degree and distribution of charged regions) is recognized in the art as typically involving a minor change. These minor changes can be identified, in part, by considering the hydropathic index of amino acids, as understood in the art. Kyte et al., J. Mol. Biol. 157: 105-132 (1982). The hydropathic index of an amino acid is based on a consideration of its hydrophobicity and charge. It is known in the art that amino acids of similar hydropathic indexes can be substituted and still retain protein function. In one aspect, amino acids having hydropathic indexes of ±2 are substituted. The hydrophilicity of amino acids can also be used to reveal substitutions that would result in proteins retaining biological function. A consideration of the hydrophilicity of amino acids in the context of a peptide permits calculation of the greatest local average hydrophilicity of that peptide, a useful measure that has been reported to correlate well with antigenicity and immunogenicity. U.S. Patent No. 4,554,101. Substitution of amino acids having similar hydrophilicity values can result in peptides retaining biological activity, for example immunogenicity, as is understood in the art. Substitutions may be performed with amino acids having hydrophilicity values within ±2 of each other. Both the hyrophobicity index and the hydrophilicity value of amino acids are influenced by the particular side chain of that amino acid. Consistent with that observation, amino acid substitutions that are compatible with biological function are understood to depend on the relative similarity of the amino acids, and particularly the side chains of those amino acids, as revealed by the hydrophobicity, hydrophilicity, charge, size, and other properties.

Nucleic acid molecules or nucleic acid sequences of the disclosure include those coding sequences comprising one or more of: FOXA1, FOXA2, FOXA3, HNF1α, HNF6, GATA4, HLF, CEBPA, PROX1, ATF5A and functional fragments thereof that possess no less than 65, 70, 75, 80, 85, 90, 95, 96, 97, 98, or 99% sequence identity with the coding sequences of the transcription factors disclosed herein.

"Vector" used herein means, in respect to a nucleic acid sequence, a nucleic acid sequence comprising a regulatory nucleic acid sequence that controls the replication of an expressible gene. A vector may be either a self-replicating, extrachromosomal vector or a vector which integrates into a host genome. Alternatively, a vector may also be a vehicle comprising the aforementioned nucleic acid sequence. A vector may be a plasmid, bacteriophage, viral particle (isolated, attenuated, recombinant, etc.). A vector may comprise a double-stranded or single-stranded DNA, RNA, or hybrid DNA/RNA sequence comprising double-stranded and/or single-stranded nucleotides. In some embodiments, the vector is a viral vector that comprises a nucleic acid sequence that is a viral packaging sequence responsible for packaging one or plurality of nucleic acid sequence that encode one or a plurality of polypeptides. In some embodiments, the vector comprises a viral particle comprising a nucleic acid sequence operably linked to a regulatory sequence, wherein the nucleic acid sequence encodes a fusion protein comprising one or a plurality of AAV VP polypeptides or fragments thereof. In embodiments of the invention, the viral capsid comprises the viral capsid polypeptides VP1 of AAV6, VP2 of AAV6, and VP3 of AAV6.

"Viral vector" as disclosed herein means, in respect to a vehicle, any virus, virus-like particle, virion, viral particle, or pseudotyped virus that comprises a nucleic acid sequence that directs packaging of a nucleic acid sequence in the virus, virus-like particle, virion, viral particle, or pseudotyped virus. In some embodiments, the virus, virus-like particle, virion, viral particle, or pseudotyped virus is capable of transferring a vector (such as a nucleic acid vector) into and/or between host cells. In some embodiments, the virus, virus-like particle, virion, viral particle, or pseudotyped virus is capable of transferring a vector (such as a nucleic acid vector) into and/or between target cells, such as a myofibroblast in the liver of a subject.

The chimeric vectors of the present invention do not necessarily increase the risks presently associated with either retroviral or adenoviral vectors. However, it allows the exploitation of the in vivo infectivity of adenoviruses and the long-term expression from retroviruses. It also provides unique advantages. For example, as with other adenoviral vectors, the chimeric vector preferentially targets hepatocytes. Expression of the retroviral components in the transduced hepatocytes leads to their elimination by the immune system. This would result in a cellular void that would stimulate de novo liver regeneration. The regeneration may provide the required dividing cell targets for the locally produced retroviral vectors. Furthermore, a chimeric vector construct that encodes all the functional components of a vector may obviate the need for repeat vector administrations.

The description of Retroviridae, Adenoviridae, and Parvoviridae (which include adeno-associated viruses) including genome organization and replication, is detailed in references known in the art, such as Fields Virology (Fields et al., eds.).

A "viral particle" as that term is used herein, means a small particle of about ten nanometers to about one micrometer, comprising a structural viral protein (such as a viral core protein), around which one or a plurality of nucleic acid molecules are contained. Viral particles comprise a group of particles called lipoparticles which include enveloped virus-like particles. In some preferred embodiments, the lipoparticles are enveloped virus-like particles which comprise an enveloped viral core protein, a lipid bilayer, and an additional polypeptide on its surface. The viral particle may be about ten nm to about 500 nm, about 100 to about 500 nm, about 200 to about 400 nm, about 300 to about 399 nm, about 500 nm to about 1000 nm, about 600 to about 900 nm, or about 700 to about 800 nm. In some embodiments, the viral particle does not encompass or comprise (free of) cell membrane vesicles, which are typically produced using empirical methods and which are usually heterogeneous in size. In some embodiments, the lipoparticle also does not encompass liposomes, which typically lack core proteins that induce their formation. In some embodiments, the lipoparticle is dense, spherical, and/or homogeneous in size.

The lipoparticle is based on retrovirus structures and enables structurally intact cellular proteins to be purified away from the cell. Briefly, when a retrovirus is produced from a cell, the protein core of the virus buds through the membrane of the cell. As a consequence, the virus becomes enwrapped by the cellular membrane. Once the membrane 'pinches' off, the virus particle is free to diffuse. Normally, the virus also produces its own membrane protein (Envelope) that is expressed on the cell surface and that becomes incorporated into the virus. However, if the gene for the viral membrane protein is deleted, virus assembly and budding can still occur. Under these conditions, the membrane enwrapping the virus contains a number of cellular proteins.

The term "retrovirus" as used herein is defined as an RNA virus of the Retroviridae family, which includes the subfamilies Oncovirinae, Lentivirinae and Spumavirinae. A skilled artisan is aware that the Oncovirinae subfamily further includes the groups Avian leukosis-sarcoma, which further includes such examples as Rous ssarcoma virus (RSV), Avian myeloblastosis virus (AMV) and, Rous-associated virus (RAV)-1 to 50. A skilled artisan is also aware that the Oncovirinae subfamily also includes the Mammalian C-type viruses, such as Moloney murine leukemia virus (Mo-MLV), Harvey murine sarcoma virus (Ha-MSV), Abelson murine leukemia virus (A-MuLV), AKR-MuLV, Feline leukemia virus (FeLV), Simian sarcoma virus, Reticuloendotheliosis virus (REV), and spleen necrosis virus (SNV). A skilled artisan is also aware of the Oncovirinae subfamily includes the B-type viruses, such as Mouse mammary tumor virus (MMTV), D-type viruses, such as Mason-Pfizer monkey virus (MPMV) or "SAIDS" virus, and the HTLV-BLV group, such as Human T-cell leukemia (or lymphotropic) virus (HTLV). A skilled artisan is also aware the Lentivirinae subfamily includes Lentiviruses such as Human immunodeficiency virus (HIV-1 and -2), Simian immunodeficiency virus (SIV), Feline immunodeficiency virus (FIV), Visna/maedi virus, Equine infectious anemia virus (EIAV) and Caprine arthritis-encephalitis virus (CAEV). A skilled artisan is also aware of the Spumavirinae subfamily includes "Foamy" viruses such as simian foamy virus (SFV).

A skilled artisan is aware that adeno-associated viruses (AAV) utilized in the present invention are included in the Dependovirus genus of the Parvoviridae family. The AAV genome has an inverted terminal repeat of 145 nucleotides, the first 125 or which form a palindromic sequence which may be further identified as containing two internal palindromes flanked by a more extensive palindrome. The AAV virions contain three coat proteins, including VP-1 (87,000 daltons), VP-2 (73,000 daltons) and VP-3 (62,000 daltons). It is known that VP-1 and VP-3 contain several sub-species. Furthermore, the three coat proteins are relatively acidic and are likely encoded by a common DNA sequence, or nucleic acid region. In some embodiments, the compositions or pharmaceutical compositions disclosed herein are viral particles derived from the Dependovirus genus.

In an embodiment, the cell to be transfected by an AAV, for replication, viral vector or manufacturing requirements, must also be infected by a helper adeno- or herpesvirus. Alternatively, a cell line, which has been subjected to various chemical or physical treatments known in the art, is utilized which permits AAV infection in the absence of helper virus coinfection. In some embodiments, the compositions or pharmaceutical compositions or methods disclosed herein are free of helper virus or helper phage or any step that requires helper virus. In some embodiments, the vectors described herein lack DNA encoding adenoviral proteins and/or preferably lack DNA encoding a selectable marker. Also generated from a cell, present in a cell or transfected into a cell is a helper virus. In such a process, a helper virus remains at a level which is sufficient to support vector replication, yet at a low enough level whereby the vector is not diluted out of virus preparations produced during a scale-up process. The vectors of the invention may be separated or purified from the helper virus by conventional means such as equilibrium density centrifugation, which may be conducted, for example, on a CsCl gradient. In order to enable such separation, it is preferred that the adenoviral vector has a number of base pairs which is different from that of the helper virus. For example, the adenoviral vector has a number of base pairs which is less than that of the helper virus.

In one embodiment, the helper virus includes a mutated packaging signal. The term "mutated" as used herein means that one or more base pairs of the packaging signal have been deleted or changed, whereby the helper virus is packaged less efficiently than wild-type adenovirus. The helper virus, which has a mutated packaging signal, is packaged less efficiently than the adenoviral vector (e.g., from about 10 to about 100 times less efficiently than the adenoviral vector).

In one embodiment, the nucleic acid of interest encodes a therapeutic agent. The term "therapeutic" is used in a generic sense and includes treating agents, prophylactic agents, and replacement agents. A therapeutic agent may be considered therapeutic if it improves or prevents at least one symptom of a disease or medical condition. Genetic diseases which may be treated with vectors and/or methods of the present invention include those in which long-term expression of the therapeutic nucleic acid is desired. This includes chronic liver disease, cirrhosis, liver cancer, and liver fibrosis.

In a specific embodiment, a therapeutic nucleic acid is utilized whose product (a polypeptide or RNA) would be circulating in the body of an organism. That is, the therapeutic product is provided not to replace or repair a defective copy present endogenously within a cell but instead enhances or augments an organism at the cellular level. This includes EPO, an antibody, GNCF, growth hormones, etc.

A skilled artisan is aware of repositories for cells and plasmids. The American Type Culture Collection (http://phage.atcc.org/searchengine/all.html) contains the cells and other biological entities utilized herein and would be aware of means to identify other cell lines which would work equally well in the methods of the present invention. The HEK 293 cells may be obtained therein with the identifier ATCC 45504, and the C3 cells may be obtained with the ATCC CRL-10741 identifier. The HepG2 cells mentioned herein are obtained with ATCC HB-8065. Many adenovirus genomes, which may be utilized in vectors of the invention, include those available from the American Type Culture Collection: adenovirus type 1 (ATCC VR-1), adenovirus type 2 (ATCC CR-846), adenovirus type 3 (ATCC VR-3 or ATCC VR-847), adenovirus type 5 (ATCC VR-5), etc.

In a specific embodiment, the vectors of the present invention are utilized for gene therapy for the treatment of liver disease. In one aspect of this embodiment the gene therapy is directed to a nucleic acid sequence selected from the group consisting of ras, myc, raf erb, src, fms, jun, trk, ret, gsp, hst, bcl abl, Rb, CFTR, p16, p21, p27, p53, p57, p73, C-CAM, APC, CTS-1, zac1, scFV ras, DCC, NF-1, NF-2, WT-1, MEN-I, MEN-II, BRCA1, VHL, MMAC1, FCC, MCC, BRCA2, IL-1, IL-2, IL-3, IL4, IL-5, IL-6, IL7, IL-8, IL-9, IL-10, IL-11 IL-12, GM-CSF G-CSF and thymidine kinase. A skilled artisan is aware these sequences and any others which may be used in the invention are readily obtainable by searching a nucleic acid sequence repository such as GenBank which is available online at http://www.ncbi.nlm.nih.gov/Genbank/GenbankSearch.html.

### Nucleic Acid-Based Expression Systems

The disclosure relates to nucleic acid-based expression systems and kits comprising the same. The expression systems include one or a plurality of vectors that encode individually or collectively one or a plurality of transcription factors or fragments thereof. The kits and expressions systems may include one or a plurality of plasmids, such as helper plasmids, empty shuttle vectors (e.g. nucleic acid based vectors), and, optionally one or more containers or vials of cells suitable for recombinant production of the viral particles disclosed herein. The nucleic acid molecules that may be contained in the one or more kits, compositions, or expression systems are described below and may comprise one or more of the elements described below. In some embodiments, the expression systems, compositions, and/or kits of the disclosure comprise one or a plurality of viral particles described herein and one or more nucleic acid molecules described herein. In some embodiments, the expression systems, compositions, and/or kits of the disclosure comprise one or a plurality of viral particles described herein and one or more nucleic acid molecules described herein. The nucleic acid molecules of the oscosure include vectors and viral vectors that encode one or more therapeutic agent in addition to the viral particle comprising the one or more transcription factors disclosed herein.

### Vectors

The present invention provides a viral vector comprising:
a viral capsid comprising a nucleic acid molecule encapsulated within the viral capsid, wherein the nucleic acid molecule comprises:
a first nucleic acid sequence that encodes HNF4α; and
a second nucleic acid sequence that encodes one or more transcription factors selected from the group consisting of: FOXA1, FOXA2, FOXA3, HNF1α, HNF6, GATA4, HLF, CEBPA, PROX1, and ATF5A;
wherein the viral capsid comprises the viral capsid polypeptides VP1 of AAV6, VP2 of AAV6, and VP3 of AAV6.

The term "vector" is used to refer to a carrier molecule into which a nucleic acid sequence can be inserted for introduction into a cell. in some embodiments, such as those methods related to manufacturing the viral particles of the disclosure, the vectors can be inserted for introduction into a cell where it can be replicated. In some embodiments the carrier molecule is a nucleic acid. A nucleic acid sequence can be "exogenous," which means that it is foreign to the cell into which the vector is being introduced or that the sequence is homologous to a sequence in the cell but in a position within the host cell nucleic acid in which the sequence is ordinarily not found. One of skill in the art would be well equipped to construct a vector through standard recombinant techniques, which are described in Maniatis et al, 1988 and Ausubel et al., 1994.

The term "expression vector" refers to a vector containing a nucleic acid sequence coding for at least part of a gene product capable of being transcribed. In some cases, RNA molecules are then translated into a protein, polypeptide, or peptide. In other cases, these sequences are not translated, for example, in the production of antisense molecules or ribozymes. Expression vectors can contain a variety of "control sequences," which refer to nucleic acid sequences necessary for the transcription and possibly translation of an operably linked coding sequence in a particular host organism. In addition to control sequences that govern transcription and translation, vectors and expression vectors may contain nucleic acid sequences that serve other functions as well and are described herein.

In some embodiments, the nucleic acid molecules packaged in the viral particles disclosed herein comprise 1, 2, 3, 4 or more regulatory sequences (such as promoter sequences) that are operably linked with one or more expressible genes disclosed herein. A "promoter" is a control sequence that is a region of a nucleic acid sequence at which initiation and rate of transcription are controlled. It may contain genetic elements at which regulatory proteins and molecules may bind such as RNA polymerase and other transcription factors. The phrases "operatively positioned," "operatively linked," "under control," and "under transcriptional control" mean that a promoter is in a correct functional location and/or orientation in relation to a nucleic acid sequence to control transcriptional initiation and/or expression of that sequence. A promoter may or may not be used in conjunction with an "enhancer," which refers to a cis-acting regulatory sequence involved in the transcriptional activation of a nucleic acid sequence.

A promoter may be one naturally associated with a gene or sequence, as may be obtained by isolating the 5' non-coding sequences located upstream of the coding segment and/or exon. Such a promoter can be referred to as "endogenous." Similarly, an enhancer may be one naturally associated with a nucleic acid sequence, located either downstream or upstream of that sequence. Alternatively, certain advantages will be gained by positioning the coding nucleic acid segment under the control of a recombinant or heterologous promoter, which refers to a promoter that is not normally associated with a nucleic acid sequence in its natural environment. A recombinant or heterologous enhancer refers also to an enhancer not normally associated with a nucleic acid sequence in its natural environment. Such promoters or enhancers may include promoters or enhancers of other genes, and promoters or enhancers isolated from any other prokaryotic, viral, or eukaryotic cell, and promoters or enhancers not "naturally occurring," i.e., containing different elements of different transcriptional regulatory regions, and/or mutations that alter expression. In addition to producing nucleic acid sequences of promoters and enhancers synthetically, sequences are produced using recombinant cloning and/or nucleic acid amplification technology, including PCR, in connection with the compositions disclosed herein (see U.S. Pat. No. 4,683,202, U.S. Pat. No. 5,928,906). Furthermore, it is contemplated the control sequences that direct transcription and/or expression of sequences within non-nuclear organelles such as mitochondria, chloroplasts, and the like, can be employed as well.

In an embodiment of the present invention there is a vector comprising a bidirectional promoter such as the aldehyde reductase promoter described by Barski et al. (1999), in which two gene products (RNA or polypeptide) or lastly are transcribed from the same regulatory sequence. This permits production of two gene products in relatively equivalent stoichiometric amounts.

Naturally, it is important to employ a promoter and/or enhancer that effectively directs the expression of the DNA segment in the cell type, organelle, and organism chosen for expression. Those of skill in the art of molecular biology generally know the use of promoters, enhancers, and cell type combinations for protein expression, for example, see Sambrook et al. (1989). The promoters employed may be constitutive, tissue-specific, inducible, and/or useful under the appropriate conditions to direct high level expression of the introduced DNA segment, such as is advantageous in the large-scale production of recombinant proteins and/or peptides. The promoter may be heterologous or endogenous.

Table 1 lists several elements/promoters that may be employed, in the context of the present invention, to regulate the expression of a gene. This list is not intended to be exhaustive of all the possible elements involved in the promotion of expression but, merely, to be exemplary thereof. Table 2 provides examples of inducible elements, which are regions of a nucleic acid sequence that can be activated in response to a specific stimulus.

**TABLE 1: Promoter and/or Enhancer Elements**

| Promoter/Enhancer | References |
|---|---|
| A-Smooth muscle actin (aSMA) | |
| Lecithin retinol acyltransferase (LRAT) | |
| Human elongation factor-1 alpha (EF1a) | |
| Glioma-associated oncogene 1 (GLI1) | |
| Immunoglobulin Heavy Chain | Banerji et al., 1983; Gilles et al., 1983; Grosschedl et al., 1985; Atchinson et al., 1986, 1987; Imler et al., 1987; Weinberger |
| | et al., 1984; Kiledjian et al., 1988; Porton et al.; 1990 |
| Immunoglobulin Light Chain | Queen et al., 1983; Picard et al., 1984 |
| T-Cell Receptor | Luria et al., 1987; Winoto et al., 1989; Redondo et al.; 1990 |
| HLA DQ a and/or DQ beta | Sullivan et al., 1987 |
| beta-Interferon | Goodbourn et al., 1986; Fujita et al., 1987; Goodbourn et al., 1988 |
| Interleukin-2 | Greene et al. , 1989 |
| Interleukin-2 | Receptor Greene et al., 1989; Lin et al., 1990 MHC Class II 5 Koch et al., 1989 |
| MHC Class II HLA-DRa | Sherman et al., 1989 |
| β-Actin | Kawamoto et al., 1988; Ng et al.; 1989 |
| Muscle Creatine Kinase (MCK) | Jaynes et al., 1988; Horlick et al., 1989; Johnson et al., 1989 |
| Prealbumin (Transthyretin) | Costa et al., 1988 |
| Elastase I | Omitz et al., 1987 |
| Metallothionein (MTII) | Karin et al., 1987; Culotta et al., 1989 |
| Collagenase | Pinkert et al., 1987; Angel et al., 1987 |
| Albumin | Pinkert et al., 1987; Tronche et al., 1989, 1990 |
| α-Fetoprotein | Godbout et al., 1988; Campere et al., 1989 |
| t-Globin | Bodine et al., 1987; Perez-Stable et al., 1990 |
| β-Globin | Trudel et al., 1987 |
| c-fos | Cohen et al., 1987 |
| c-HA-ras | Triesman, 1986; Deschamps et al., 1985 |
| Insulin | Edlund et al., 1985 |
| Neural Cell Adhesion Molecule | Hirsh et al., 1990 |
| α₁-Antitrypsin | Latimer et al., 1990 |
| H2B (TH2B) Histone | Hwang et al., 1990 |
| Mouse and/or Human Collagen I | Ripe et al., 1989 |
| Mouse and/or Human Collagen II | |
| Mouse and/or Human Collagen III | |
| Mouse and/or Human Collagen IV | |
| Mouse and/or Human Collagen V | |
| Regulated Proteins | Chang et al., 1989 |
| Rat Growth Hormone | Larsen et al., 1986 |
| Human Serum Amyloid A (SAA) | Edbrooke et al., 1989 |
| Troponin I (TN I) | Yutzey et al. , 1989 |
| Platelet-Derived Growth Factor Receptor β | Pech et al., 1989 |
| Duchenne Muscular Dystrophy | Klamut et al., 1990 |
| SV40 | Banerji et al., 1981; Moreau et al., 1981; Sleigh et al., 1985; Firak et al., 1986; Herr et al., 1986; Imbra et al., 1986; Kadesch et al., 1986; Wang et al., 1986; Ondek et al., 1987; Kuhl et al., 1987; Schaffner et al., 1988 |
| Polyoma | Swartzendruber et al., 1975; Vasseur et al., 1980; Katinka et al., 1980, 1981; Tyndell et al., 1981; Dandolo et al., 1983; de Villiers et al., 1984; Hen et al., 1986; Satake et al., 1988; Campbell and/or Villarreal, 1988 |
| Retroviruses | Kriegler et al., 1982, 1983; Levinson et al., 1982; Kriegler et al., 1983, 1984a, b, 1988; Bosze et al., 1986; Miksicek et al., 1986; Celander et al., 1987; Thiesen et al., 1988; Celander et al., 1988; Chol et al., 1988; Reisman et al., 1989 |
| Papilloma Virus | Campo et al., 1983; Lusky et al., 1983; Spandidos and/or Wilkie, 1983; Spalholz et al., 1985; Lusky et al., 1986; Cripe et al., 1987; Gloss et al., 1987; Hirochika et al., 1987; Stephens et al., 1987; Glue et al., 1988 |
| Hepatitis B Virus | Bulla et al., 1986; Jameel et al., 1986; Shaul et al., 1987; Spandau et al., 1988; Vannice et al., 1988 |
| Human Immunodeficiency Virus | Muesing et al., 1987; Hauber et al., 1988; Jakobovits et al., 1988; Feng et al., 1988; Takebe et al., 1988; Rosen et al., 1988; Berkhout et al., 1989; Laspia et al., 1989; Sharp et al., 1989; Braddock et al., 1989 |
| Cytomegalovirus (CMV) | Weber et al., 1984; Boshart et al., 1985; Foecking et al., 1986 |
| Gibbon Ape Leukemia Virus | Holbrook et al., 1987; Quinn et al., 1989 |

**TABLE 2: Inducible Elements**

| Element | Inducer | References |
|---|---|---|
| MT II | Phorbol Ester (TFA) Heavy metals | Palmiter et al., 1982; Haslinger et al., 1985; Searle et al., 1985; Stuart et al., 1985; Imagawa et al., 1987, Karin et al., 1987; Angel et al., 1987b; McNeall et al., 1989 |
| MMTV | Glucocorticoids | Huang et al., 1981; Lee et al., 1981; Majors et al., 1983; Chandler et al., 1983; Lee et al., 1984; Ponta et al., 1985; Sakai et al., 1988 |
| β-Interferon | poly(rI)x | Tavernier et al., 1983 |
| | poly(rc) | |
| Adenovirus 5 | E2 E1A | Imperiale et al., 1984 |
| Collagenase | Phorbol Ester (TPA) | Angel et al., 1987a |
| Stromelysin | Phorbol Ester (TPA) | Angel et al., 1987b |
| SV40 | Phorbol Ester (TPA) | Angel et al., 1987b |
| Murine MX Gene | Interferon, Newcastle | Hug et al., 1988 |
| | Disease Virus | GRP78 Gene A23187 Resendez et al., 1988 |
| a-2-Macroglobulin | IL-6 | Kunz et al., 1989 |
| Vimentin MHC Class I | Serum | Rittling et al., 1989 |
| Gene H-2.kappa.b | Interferon | Blanar et al., 1989 |
| HSP70 | E1A, SV40 Large T | Taylor et al., 1989, 1990a., 1990b |
| | Antigen | |
| Proliferin Tumor Necrosis | Phorbol Ester-TPA | Mordacq et al., 1989 |
| Factor | PMA | Hensel et al., 1989 |
| Thyroid Stimulating Hormone-a. Gene | Thyroid Hormone | Chatterjee et al., 1989 |

The identity of tissue-specific promoters or elements, as well as assays to characterize their activity, is well known to those of skill in the art. Examples of such regions include the human LINM2 gene (Nomoto et al. 1999), the somatostatin receptor 2 gene (Kraus et al., 1998), murine epididymal retinoic acid-binding gene (Lareyre et al., 1999), human CD4 (Zhao-Emonet et al., 1998), mouse alpha2 (XI) collagen (Tsumaki, et al., 1998), D1A dopamine receptor gene (Lee, et al., 1997), insulin-like growth factor II (Wu et al., 1997), human platelet endothelial cell adhesion molecule-1 (Almendro et al., 1996).

### Initiation Signals and Internal Ribosome Binding Sites

A specific initiation signal also may be required for efficient translation of coding sequences. These signals include the ATG initiation codon or adjacent sequences. Exogenous translational control signals, including the ATG initiation codon, may need to be provided. One of ordinary skill in the art would readily be capable of determining this and providing the necessary signals. It is well known that the initiation codon must be "in-frame" with the reading frame of the desired coding sequence to ensure translation of the entire insert. The exogenous translational control signals and initiation codons can be either natural or synthetic. The efficiency of expression may be enhanced by the inclusion of appropriate transcription enhancer elements.

In certain embodiments of the invention, the use of internal ribosome entry sites (IRES) elements are used to create multigene, or polycistronic, messages. IRES elements are able to bypass the ribosome scanning model of 5' methylated Cap dependent translation and begin translation at internal sites (Pelletier and Sonenberg, 1988). IRES elements from two members of the picornavirus family polio and encephalomyocarditis) have been described (Pelletier and Sonenberg, 1988), as well an IRES from a mammalian message (Macejak and Sarnow, 1991). IRES elements can be linked to heterologous open reading frames. Multiple open reading frames can be transcribed together, each separated by an IRES, creating polycistronic messages. By virtue of the IRES element, each open reading frame is accessible to ribosomes for efficient translation. Multiple genes can be efficiently expressed using a single promoter/enhancer to transcribe a single message (see U.S. Pat. Nos. 5,925,565 and 5,935,819).

### Multiple Cloning Sites

Vectors can include a multiple cloning site (MCS), which is a nucleic acid region that contains multiple restriction enzyme sites, any of which can be used in conjunction with standard recombinant technology to digest the vector. (See Carbonelli et al., 1999, Levenson et al., 1998, and Cocea, 1997.) "Restriction enzyme digestion" refers to catalytic cleavage of a nucleic acid molecule with an enzyme that functions only at specific locations in a nucleic acid molecule. Many of these restriction enzymes are commercially available. Use of such enzymes is widely understood by those of skill in the art. Frequently, a vector is linearized or fragmented using a restriction enzyme that cuts within the MCS to enable exogenous sequences to be ligated to the vector. "Ligation" refers to the process of forming phosphodiester bonds between two nucleic acid fragments, which may or may not be contiguous with each other. Techniques involving restriction enzymes and ligation reactions are well known to those of skill in the art of recombinant technology.

### Splicing Sites

Most transcribed eukaryotic RNA molecules will undergo RNA splicing to remove introns from the primary transcripts. Vectors containing genomic eukaryotic sequences may require donor and/or acceptor splicing sites to ensure proper processing of the transcript for protein expression. (See Chandler et al., 1997.)

### Polyadenylation Signals

In expression, one will typically include a polyadenylation signal to effect proper polyadenylation of the transcript. The nature of the polyadenylation signal is not believed to be crucial to the successful practice of the invention, and/or any such sequence may be employed. Preferred embodiments include the SV40 polyadenylation signal and/or the bovine growth hormone polyadenylation signal, convenient and/or known to function well in various target cells. Also contemplated as an element of the expression cassette is a transcriptional termination site. These elements can serve to enhance message levels and/or to minimize read through from the cassette into other sequences.

### Origins of Replication

In order to propagate a vector in a host cell, it may contain one or more origins of replication sites (often termed "on"), which is a specific nucleic acid sequence at which replication is initiated.

### Selectable and Screenable Markers

In certain embodiments of the invention, wherein cells contain a nucleic acid construct of the present invention, a cell may be identified in vitro or in vivo by including a marker in the expression vector. Such markers would confer an identifiable change to the cell permitting easy identification of cells containing the expression vector. Generally, a selectable marker is one that confers a property that allows for selection. A positive selectable marker is one in which the presence of the marker allows for its selection, while a negative selectable marker is one in which its presence prevents its selection. An example of a positive selectable marker is a drug resistance marker.

Usually the inclusion of a drug selection marker aids in the cloning and identification of transformants, for example, genes that confer resistance to neomycin, puromycin, hygromycin, DHFR, GPT, zeocin and histidinol are useful selectable markers. In addition to markers conferring a phenotype that allows for the discrimination of transformants based on the implementation of conditions, other types of markers including screenable markers such as GFP, whose basis is colorimetric analysis, are also contemplated. Alternatively, screenable enzymes such as herpes simplex virus thymidine kinase (tk) or chloramphenicol acetyltransferase (CAT) may be utilized. One of skill in the art would also know how to employ inmmunologic markers, possibly in conjunction with FACS analysis. The marker used is not believed to be important, so long as it is capable of being expressed simultaneously with the nucleic acid encoding a gene product. Further examples of selectable and screenable markers are well known to one of skill in the art.

### Host Cells

The method of making a viral vector comprising the nucleic acid disclosed herein involves using a cell. Hence in some embodiments the method of making the viral vector involves expression of at least a competent portion of the genome of an virus disclosed herein in a cell. As used herein, the terms "cell," "cell line," and "cell culture" may be used interchangeably. All of these terms also include their progeny, which is any and all subsequent generations. It is understood that all progeny may not be identical due to deliberate or inadvertent mutations. In the context of expressing a heterologous nucleic acid sequence, "host cell" refers to a prokaryotic or eukaryotic cell, and it includes any transformable organisms that is capable of replicating a vector and/or expressing a heterologous gene encoded by a vector. A host cell can, and has been, used as a recipient for vectors. A host cell may be "transfected" or "transformed," which refers to a process by which exogenous nucleic acid is transferred or introduced into the host cell. A transformed cell includes the primary subject cell and its progeny.

In some embodiments, the host cell is the target of the viral particle, virion, or pseudovirus disclosed herein. In some embodiments, the target of the viral particle, virion, or pseudovirus disclosed herein is any type of fibroblast. In some embodiments, the target of the viral particle, virion, or pseudovirus disclosed herein is any type of myofibroblast, hepatic stellate cell, portal fibroblast, or a cell derived therefrom. In some embodiments, the cell is a myofibroblast.

Host cells may be derived from prokaryotes or eukaryotes, depending upon whether the desired result is replication of the vector or expression of part or all of the vector-encoded nucleic acid sequences. Numerous cell lines and cultures are available for use as a host cell, and they can be obtained through the American Type Culture Collection (ATCC), which is an organization that serves as an archive for living cultures and genetic materials (www.atcc.org). An appropriate host can be determined-by one of skill in the art based on the vector backbone and the desired result. A plasmid or cosmid, for example, can be introduced into a prokaryote host cell for replication of many vectors. Bacterial cells used as host cells for vector replication and/or expression include DH5.alpha., JM109, and KC8, as well as a number of commercially available bacterial hosts such as SURE^{®}. Competent Cells and SOLOPACK^{™} Gold Cells (STRATAGENE. ^{®}, La Jolla). Alternatively, bacterial cells such as E. coli LE392 could be used as host cells for phage viruses.

Examples of eukaryotic host cells for replication and/or expression of a vector include HeLa, NIH3T3, Jurkat, 293, Cos, CHO, Saos, and PC12. Many host cells from various cell types and organisms are available and would be known to one of skill in the art. Similarly, a viral vector may be used in conjunction with either a eukaryotic or prokaryotic host cell, particularly one that is permissive for replication or expression of the vector.

Some vectors may employ control sequences that allow it to be replicated and/or expressed in both prokaryotic and eukaryotic cells. One of skill in the art would further understand the conditions under which to incubate all of the above described host cells to maintain them and to permit replication of a vector. Also understood and known are techniques and conditions that would allow large-scale production of vectors, as well as production of the nucleic acids encoded by vectors and their cognate polypeptides, proteins, or peptides.

### Expression Systems

Numerous expression systems exist that comprise at least a part or all of the compositions discussed above. Prokaryote- and/or eukaryote-based systems can be employed for use with the present invention to produce nucleic acid sequences, or their cognate polypeptides, proteins and peptides. Many such systems are commercially and widely available.

The insect cell/baculovirus system can produce a high level of protein expression of a heterologous nucleic acid segment, such as described in U.S. Pat. Nos. 5,871,986, 4,879,236, and which can be bought, for example, under the name MAXBAC^{®} 2.0 from INVITROGEN^{®} and

### BACPACK^{™} BACULOVIRUS EXPRESSION SYSTEM FROM CLONTECH^{®}.

Other examples of expression systems include STRATAGENE^{®} 's COMPLETE CONTROL^{™}. Inducible Mammalian Expression System, which involves a synthetic ecdysone-inducible receptor, or its pET Expression System, an E. coli expression system. Another example of an inducible expression system is available from INVITROGEN^{®}, which carries the T-REX^{™} (tetracycline-regulated expression) System, an inducible mammalian expression system that uses the full-length CMV promoter. INVITROGEN^{®} also provides a yeast expression system called the *Pichia methanolica* Expression System, which is designed for high-level production of recombinant proteins in the methylotrophic yeast *Pichia methanolica.* One of skill in the art would know how to express a vector, such as an expression construct, to produce a nucleic acid sequence or its cognate polypeptide, protein, or peptide.

### Nucleic Acid Detection

In addition to their use in directing the expression a polypeptide from a nucleic acid of interest including proteins, polypeptides and/or peptides, the nucleic acid sequences disclosed herein have a variety of other uses. For example, they have utility as probes or primers for embodiments involving nucleic acid hybridization. In one embodiments, the disclosure relates to a method of detecting the presence, absence, or quantity of expression of an exogenous nucleic acid in a subject.

### Hybridization

The use of a probe or primer of between 13 and 100 nucleotides, between 17 and 100 nucleotides in length, or in some aspects of the invention up to 1-2 kilobases or more in length, allows the formation of a duplex molecule that is both stable and selective. Molecules having complementary sequences over contiguous stretches greater than 20 bases in length are generally preferred, to increase stability and/or selectivity of the hybrid molecules obtained. One will generally prefer to design nucleic acid molecules for hybridization having one or more complementary sequences of 20 to 30 nucleotides, or even longer where desired. Such fragments may be readily prepared, for example, by directly synthesizing the fragment by chemical means or by introducing selected sequences into recombinant vectors for recombinant production.

Accordingly, the nucleotide sequences of the invention, or fragments or derivatives thereof, may be used for their ability to selectively form duplex molecules with complementary stretches of DNAs and/or RNAs or to provide primers for amplification of DNA or RNA from samples. Depending on the application envisioned, one would desire to employ varying conditions of hybridization to achieve varying degrees of selectivity of the probe or primers for the target sequence.

For applications requiring high selectivity, one will typically desire to employ relatively high stringency conditions to form the hybrids. For example, relatively low salt and/or high temperature conditions, such as provided by about 0.02 M to about 0.10 M NaCl at temperatures of about 50°C. to about 70°C. Such high stringency conditions tolerate little, if any, mismatch between the probe or primers and the template or target strand and would be particularly suitable for isolating specific genes or for detecting specific mRNA transcripts. It is generally appreciated that conditions can be rendered more stringent by the addition of increasing amounts of formamide.

For certain applications, for example, site-directed mutagenesis, it is appreciated that lower stringency conditions are preferred. Under these conditions, hybridization may occur even though the sequences of the hybridizing strands are not perfectly complementary, but are mismatched at one or more positions. Conditions may be rendered less stringent by increasing salt concentration and/or decreasing temperature. For example, a medium stringency condition could be provided by about 0.1 to 0.25 M NaCl at temperatures of about 37. degree. C. to about 55. degree. C., while a low stringency condition could be provided by about 0.15 M to about 0.9 M salt, at temperatures ranging from about 20. degree. C. to about 55. degree. C. Hybridization conditions can be readily manipulated depending on the desired results.

In other embodiments, hybridization may be achieved under conditions of, for example, 50 mM Tris-HCl (pH 8.3), 75 mM KCl, 3 mM MgCl.sub.2, 1.0 mM dithiothreitol, at temperatures between approximately 20°C. to about 37°C. Other hybridization conditions utilized could include approximately 10 mM Tris-HCl (pH 8.3), 50 mMKCl, 1.5 mM MgCl.sub.2, at temperatures ranging from approximately 40°C. to about 72°C.

In certain embodiments, it will be advantageous to employ nucleic acids of defined sequences of the present invention in combination with an appropriate means, such as a label, for determining hybridization. A wide variety of appropriate indicator means are known in the art, including fluorescent, radioactive, enzymatic or other ligands, such as avidin/biotin, which are capable of being detected. In preferred embodiments, one may desire to employ a fluorescent label or an enzyme tag such as urease, alkaline phosphatase or peroxidase, instead of radioactive or other environmentally undesirable reagents. In the case of enzyme tags, colorimetric indicator substrates are known that can be employed to provide a detection means that is visibly or spectrophotometricaily detectable, to identify specific hybridization with complementary nucleic acid containing samples.

In general, it is envisioned that the probes or primers described herein will be useful as reagents in solution hybridization, as in PCR for detection of expression of corresponding genes, as well as in embodiments employing a solid phase. In embodiments involving a solid phase, the test DNA (or RNA) is adsorbed or otherwise affixed to a selected matrix or surface. This fixed, single-stranded nucleic acid is then subjected to hybridization with selected probes under desired conditions. The conditions selected will depend on the particular circumstances (depending, for example, on the G+C content, type of target nucleic acid, source of nucleic acid, size of hybridization probe, etc.). Optimization of hybridization conditions for the particular application of interest is well known to those of skill in the art. After washing of the hybridized molecules to remove non-specifically bound probe molecules, hybridization is detected, and/or quantified, by determining the amount of bound label. Representative solid phase hybridization methods are disclosed in U.S. Pat. Nos. 5,843,663, 5,900,481 and 5,919,626. Other methods of hybridization that may be used in the practice of the present invention are disclosed in U.S. Pat. Nos. 5,849,481, 5,849,486 and 5,851,772.

### Amplification of Nucleic Acids

Nucleic acids used as a template for amplification may be isolated from cells, tissues or other samples according to standard methodologies (Sambrook et al., 1989). In certain embodiments, analysis is performed on whole cell or tissue homogenates or biological fluid samples without substantial purification of the template nucleic acid. The nucleic acid may be genomic DNA or fractionated or whole cell RNA. Where RNA is used, it may be desired to first convert the RNA to a complementary DNA.

The term "primer," as used herein, is meant to encompass any nucleic acid that is capable of priming the synthesis of a nascent nucleic acid in a template-dependent process. Typically, primers are oligonucleotides from ten to twenty and/or thirty base pairs in length, but longer sequences can be employed. Primers may be provided in double- stranded and/or single-stranded form, although the single-stranded form is preferred.

Pairs of primers designed to selectively hybridize to nucleic acids corresponding to a vector or nucleic acid sequence of interest are contacted with the template nucleic acid under conditions that permit selective hybridization. Depending upon the desired application, high stringency hybridization conditions may be selected that will only allow hybridization to sequences that are completely complementary to the primers. In other embodiments, hybridization may occur under reduced stringency to allow for amplification of nucleic acids contain one or more mismatches with the primer sequences. Once hybridized, the template-primer complex is contacted with one or more enzymes that facilitate template-dependent nucleic acid synthesis. Multiple rounds of amplification, also referred to as "cycles," are conducted until a sufficient amount of amplification product is produced.

The amplification product may be detected or quantified. In certain applications, the detection may be performed by visual means. Alternatively, the detection may involve indirect identification of the product via chemiluminescence, radioactive scintigraphy of incorporated radiolabel or fluorescent label or even via a system using electrical and/or thermal impulse signals.

A number of template dependent processes are available to amplify the oligonucleotide sequences present in a given template sample. One of the best known amplification methods is the polymerase chain reaction (referred to as PCR) which is described in detail in U.S. Pat. Nos. 4,683,195, 4,683,202 and 4,800,159. A reverse transcriptase PCR amplification procedure may be performed to quantify the amount of mRNA amplified. Methods of reverse transcribing RNA into cDNA are well known and described in Sambrook et al., 1989. Alternative methods for reverse transcription utilize thermostable DNA polymerases. These methods are described in WO 90/07641. Polymerase chain reaction methodologies are well known in the art. Representative methods of RT-PCR are described in U.S. Pat. No. 5,882,864.

Alternative methods for amplification of target nucleic acid sequences that may be used in the practice of the present invention are disclosed in U.S. Pat. Nos. 5,843,650, 5,846,709, 5,846,783, 5,849,546, 5,849,497, 5,849,547, 5,858,652, 5,866,366, 5,916,776, 5,922,574, 5,928,905, 5,928,906, 5,932,451, 5,935,825, 5,939,291 and 5,942,391, GB Application No. 2 202 328, and in PCT Application No. PCT/US89/01025.

Qbeta Replicase, described in PCT Application No. PCT/US87/00880, may also be used as an amplification method in the present invention. In this method, a replicative sequence of RNA that has a region complementary to that of a target is added to a sample in the presence of an RNA polymerase. The polymerase will copy the replicative sequence which may then be detected.

An isothermal amplification method, in which restriction endonucleases and ligases are used to achieve the amplification of target molecules that contain nucleotide 5'-[alpha-thio]-triphosphates in one strand of a restriction site may also be useful in the amplification of nucleic acids in the present invention (Walker et al., 1992). Strand Displacement Amplification (SDA), disclosed in U.S. Pat. No. 5,916,779, is another method of carrying out isothermal amplification of nucleic acids, which involves multiple rounds of strand displacement and synthesis, i.e., nick translation.

Other nucleic acid amplification procedures include transcriptien-based amplification systems (TAS), including nucleic acid sequence based amplification (NASBA) and 3SR (Kwoh et al., 1989; Gingeras et al., PCT Application WO 88/10315). Davey et al., European Application No. 329 822 disclose a nucleic acid amplification process involving cyclically synthesizing single-stranded RNA ("ssRNA"), ssDNA, and double-stranded DNA (dsDNA), which may be used in accordance with the present invention.

Miller et al., PCT Application WO 89/06700 disclose a nucleic acid sequence amplification scheme based on the hybridization of a promoter region/primer sequence to a target single-stranded DNA ("ssDNA") followed by transcription of many RNA copies of the sequence. This scheme is not cyclic, i.e., new templates are not produced from the resultant RNA transcripts. Other amplification methods include "race" and "one-sided PCR" (Frobman, 1990; Ohara et al., 1989).

### Detection of Nucleic Acids

Following any amplification, it may be desirable to separate the amplification product from the template and/or the excess primer. In one embodiment, amplification products are separated by agarose, agarose-acrylamide or polyacrylamide gel electrophoresis using standard methods (Sambrook et al., 1989). Separated amplification products may be cut out and eluted from the gel for further manipulation. Using low melting point agarose gels, the separated band may be removed by heating the gel, followed by extraction of the nucleic acid.

Separation of nucleic acids may also be effected by chromatographic techniques known in art. There are many kinds of chromatography which may be used in the practice of the present invention, including adsorption, partition, ion-exchange, hydroxylapatite, molecular sieve, reverse-phase, column, paper, thin-layer, and gas chromatography as well as HPLC.

In certain embodiments, the amplification products are visualized. A typical visualization method involves staining of a gel with ethidium bromide and visualization of bands under UV light. Alternatively, if the amplification products are integrally labeled with radio- or fluorometrically-labeled nucleotides, the separated amplification products can be exposed to x-ray film or visualized under the appropriate excitatory spectra.

In one embodiment, following separation of amplification products, a labeled nucleic acid probe is brought into contact with the amplified marker sequence. The probe preferably is conjugated to a chromophore but may be radiolabeled. In another embodiment, the probe is conjugated to a binding partner, such as an antibody or biotin, or another binding partner carrying a detectable moiety.

In particular embodiments, detection is by Southern blotting and hybridization with a labeled probe. The techniques involved in Southern blotting are well known to those of skill in the art. See Sambrook et al., 1989. One example of the foregoing is described in U.S. Pat. No. 5,279,721, which discloses an apparatus and method for the automated electrophoresis and transfer of nucleic acids. The apparatus permits electrophoresis and blotting without external manipulation of the gel and is ideally suited to carrying out methods according to the present invention.

Other methods of nucleic acid detection that may be used in the practice of the instant invention are disclosed in U.S. Pat. Nos. 5,840,873, 5,843,640, 5,843,651, 5,846,708, 5,846,717, 5,846,726, 5,846,729, 5,849,487, 5,853,990, 5,853,992, 5,853,993, 5,856,092, 5,861,244, 5,863,732, 5,863,753, 5,866,331, 5,905,024, 5,910,407, 5,912,124, 5,912,145, 5,919,630, 5,925,517, 5,928,862, 5,928,869, 5,929,227, 5,932,413 and 5,935,791.

Alternative methods for detection of deletion, insertion or substititution mutations that may be used in the practice of the present invention are disclosed in U.S. Pat. Nos. 5,849,483, 5,851,770, 5,866,337, 5,925,525 and 5,928,870.

The present disclosure relates to one or a plurality of nucleic acid molecules encoding one or a plurality of transcription factors disclosed herein. The compositions or pharmaceutical compositions disclosed herein may contain one, two, three, four, five, six, seven or more separate nucleic acid molecules each one of the nucleic acid molecules encoding one or more of the transcription factors disclosed herein. In some embodiments, the viral particles or virons disclosed herein may comprise the one, two, three, four, five, six, seven or more separate nucleic acid molecules each one of the nucleic acid molecules encoding one or more of the transcription factors disclosed herein. In some embodiments, the compositions or pharmaceutical compositions disclosed herein may comprise a single population of virions or viral particles in which the same nucleic acid molecules are contained. In some embodiments, the compositions or pharmaceutical compositions disclosed herein may comprise a mixed or hetergenous population of viral particle or virons disclosed herein such that there are a variable number or type of expressible genes contained within one or a plurality of viral particles or virions but, collectively the one or plurality of virons can express any one r plurality of transcription factors disclosed herein upon transfection into one or more cells, such as a myofibroblast.

### Kits

All the essential materials and/or reagents required for detecting or administering a vector sequence of the present invention in a sample may be assembled together in a kit to facilitate detection or administration. Such kits are disclosed, but not claimed. This generally will comprise a probe or primers designed to hybridize specifically to individual nucleic acids of interest in the practice of the present invention, including a nucleic acid sequence of interest. Also included may be enzymes suitable for amplifying nucleic acids, including various polymerases (reverse transcriptase, Taq, etc.), deoxynucleotides and buffers to provide the necessary reaction mixture for amplification. Such kits may also include enzymes and other reagents suitable for detection of specific nucleic acids or amplification products. Such kits generally will comprise, in suitable means, distinct containers for each individual reagent or enzyme as well as for each probe or primer pair.

### Administration

For gene therapy, a skilled artisan would be cognizant that the vector to be utilized must contain the gene of interest operably linked or operatively limited to a promoter. For antisense gene therapy, the antisense sequence of the gene of interest would be operatively linked to a promoter. One skilled in the art recognizes-that in certain instances other sequences such as a regulatory sequences are useful in expressing the gene of interest. Where appropriate, the gene therapy vectors can be formulated into preparations in solid, semisolid, liquid or gaseous forms in the ways known in the art for their respective route of administration. Means known in the art can be utilized to prevent release and absorption of the composition until it reaches the target organ or to ensure timed-release of the composition. A pharmaceutically acceptable form should be employed which does not ineffectuate the compositions of the present invention. In pharmaceutical dosage forms, the compositions can be used alone or in appropriate association, as well as in combination, with other pharmaceutically active compounds. A therapeutically effective amount of vector containing the therapeutic nucleic acid sequence must be administered to provide a pharmacologically effective dose of the gene product.

One skilled in the art recognizes that different methods of delivery may be utilized to administer a vector into a cell. Examples include: (1) methods utilizing physical means, such as electroporation (electricity), a gene gun (physical force) or applying large volumes of a liquid (pressure); and (2) methods wherein said vector is complexed to another entity, such as a liposome or transporter molecule; and (3) intravenously or intrahepatically administering any pharmaceutically effective amount of.

Accordingly, the present invention provides a method of transferring a therapeutic gene to a subject, which comprises administering the vector of the present invention, preferably as part of a composition, using any of the aforementioned routes of administration or alternative routes known to those skilled in the art and appropriate for a particular application. Effective gene transfer of a vector to a host cell in accordance with the present invention to a host cell can be monitored in terms of a therapeutic effect (e.g. alleviation of some symptom associated with the particular disease being treated) or, further, by evidence of the transferred gene or expression of the gene within the host (e.g., using the polymerase chain reaction in conjunction with sequencing, Northern or Southern hybridizations, or transcription assays to detect the nucleic acid in host cells, or using immunoblot analysis, antibody-mediated detection, mRNA or protein half-life studies, or particularized assays to detect protein or polypeptide encoded by the transferred nucleic acid, or impacted in level or function due to such transfer).

These methods described herein are by no means all-inclusive, and farther methods to suit the specific application will be apparent to the ordinary skilled artisan. Moreover, the effective amount of the compositions can be further approximated through analogy to compounds known to exert the desired effect.

Furthermore, the actual dose and schedule can vary depending on whether the compositions are administered in combination with other pharmaceutical compositions, or depending on individual differences in pharmacokinetics, drug disposition, and metabolism. Similarly, amounts can vary in in vitro applications depending on the particular cell line utilized (e.g., based on the number of vector receptors present on the cell surface, or the ability of the particular vector employed for gene transfer to replicate in that cell line). Furthermore, the amount of vector to be added per cell will likely vary with the length and stability of the therapeutic gene inserted in the vector, as well as also the nature of the sequence, and is particularly a parameter which needs to be determined empirically, and can be altered due to factors not inherent to the methods of the present invention (for instance, the cost associated with synthesis). One skilled in the art can easily make any necessary adjustments in accordance with the exigencies of the particular situation.

The dosage of the vectors of the present invention can be appropriately determined by those skilled in the art, although it varies depending on the disease, patient's weight, age, sex, symptom, objective of administration, form of composition administered, administration method, type of gene to be introduced, and such. The route of administration can be appropriately selected, and includes, for example, percutaneous, intranasal, intrahepatically, transbronchial, intramuscular, intraperitoneal, intravenous, intraarticular, intraspinal, and/or subcutaneous administrations, but is not limited thereto. In some embodiments, the mode of administration is intranasal administration. The administration may be local and/or systemic. In some embodiments, the step of administering the vectors comprises administering a therapeutically effective dosage from about 10⁵ CIU/ml to about 10¹¹ ClU/ml, from about 10⁷ CIU/ml to about 10⁹ CIU/ml, or from about 1 ×10⁸ CIU/ml to about 5 ×10⁸ CIU/ml, together with a pharmaceutically acceptable carrier. In some embodiments, a single dose for human is preferably 2 ×10⁵ CIU to 2 ×10¹⁰ CIU. The frequency of administration can be once or more, and within the range of clinically acceptable side effects. The same applies to the daily administration frequency. For protein preparations produced using vectors of the present invention, the protein dosage may be, for example, within the range of 10 ng/kg to 100 µg/kg, preferably 100 ng/kg to 50 µg/kg, and more preferably 1 µg/kg to 5 µg/kg. For non-human animals, for example, the dosage to be administered can be converted from the above-described dosage based on the body weight ratio or volume ratio (e.g., average value) of the target site for administration between the animal of interest and human. In some embodiments, the administration is daily. In some embodiments, the administration is once a week. In some embodiments, the administration is twice a week. In some embodiments, the administration is three times a week. In some embodiments, the administration is four times a week. In some embodiments, the administration is five times a week. In some embodiments, the administration is six times a week. In some embodiments, the administration is once a month. In some embodiments, the administration is twice a month. In some embodiments, the administration is three times a month. In some embodiments, the administration is four times a month. In some embodiments, the administration is once a year. In some embodiments, the administration is twice a year. In some embodiments, the administration is three times a year. In some embodiments, the administration is four times a year.

### Combination Treatments

In yet another embodiment, the pharmaceutical composition, composition or kit disclosed herein comprises a secondary treatment such as a second gene therapy vector in which a second therapeutic agent is administered before, after, or at the same time a first viral particle is administered comprising all of part of an exogenous nucleic acid sequence of interest. Delivery of a vector encoding either a full-length or truncated amino acid sequence of interest in conduction with a second vector encoding one of the following gene products will have a combined anti-hyperproliferative effect on target tissues. In some embodiments, CAR- Tcells may be administered to any of the disclosed subjects in any of the disclosed methods. Alternatively, a single vector encoding two or more genes may be used. A variety of proteins are encompassed within the invention, some of which are described below.

### Inducers of Cellular Proliferation

The proteins that induce cellular proliferation further fall into various categories dependent on function. The commonality of all of these proteins is their ability to regulate cellular proliferation. For example, a form of PDGF, the sis oncogene, is a secreted growth factor. Oncogenes rarely arise from genes encoding growth factors, and at the present, sis is the only known naturally-occurring oncogenic growth factor. In one embodiment of the present invention, it is contemplated that anti-sense MRNA directed to a particular inducer of cellular proliferation is used to prevent expression of the inducer of cellular proliferation.

The proteins FMS, ErbA, ErbB and neu are growth factor receptors. Mutations to these receptors result in loss of regulatable function. For example, a point mutation affecting the transmembrane domain of the Neu receptor protein results in the neu oncogene. The erbA oncogene is derived from the intracellular receptor for thyroid hormone. The modified oncogenic ErbA receptor is believed to compete with the endogenous thyroid hormone receptor, causing uncontrolled growth.

The largest class of oncogenes includes the signal transducing proteins (e.g., Src, Ab1 and Ras). The protein Src is a cytoplasmic protein-tyrosine linase, and its transformation from proto-oncogene to oncogene in some cases, results via mutations at tyrosine residue 527. In contrast, transformation of GTPase protein ras from proto-oncogene to oncogene, in one example, results from a valine to glycine mutation at amino acid 12 in the sequence, reducing ras GTPase activity.

The proteins Jun, Fos and Myc are proteins that directly exert their effects on nuclear functions as transcription factors.

### b. Inhibitors of Cellular Proliferation

The tumor suppressor oncogenes function to inhibit excessive cellular proliferation. The inactivation of these genes destroys their inhibitory activity, resulting in unregulated proliferation. The tumor suppressors p53, p16 and C-CAM are described below.

High levels of mutant p53 have been found in many cells transformed by chemical carcinogenesis, ultraviolet radiation, and several viruses. The p53 gene is a frequent target of mutational inactivation in a wide variety of human tumors and is already documented to be the most frequently mutated gene in common human cancers. It is mutated in over 50% of human NSCLC (Hollstein et al., 1991) and in a wide spectrum of other tumors.

The p53 gene encodes a 393-amino acid phosphoprotein that can form complexes with host proteins such as large-T antigen and E1B. The protein is found in normal tissues and cells, but at concentrations which are minute by comparison with transformed cells or tumor tissue

Wild-type p53 is recognized as an important growth regulator in many cell types. Missense mutations are common for the p53 gene and are essential for the transforming ability of the oncogene. A single genetic change prompted by point mutations can create carcinogenic p53. Unlike other oncogenes, however, p53 point mutations are known to occur in at least 30 distinct codons, often creating dominant alleles that produce shifts in cell phenotype without a reduction to homozygosity. Additionally, many of these dominant negative alleles appear to be tolerated in the organism and passed on in the germ line. Various mutant alleles appear to range from minimally dysfunctional to strongly penetrant, dominant negative alleles (Weinberg, 1991).

Another inhibitor of cellular proliferation is p16. The major transitions of the eukaryotic cell cycle are triggered by cyclin-dependent kinases, or CDK's. One CDK, cyclin-dependent kinase 4 (CDK4), regulates progression through the G. sub. 1. The activity of this enzyme may be to phosphorylate Rb at late G.sub. 1. The activity of CDK4 is controlled by an activating subunit, D-type cyclin, and by an inhibitory subunit, the p16.sup.INK4 has been biochemically characterized as a protein that specifically binds to and inhibits CDK4, and thus may regulate Rb phosphorylation (Serrano et al., 1993; Serrano et al., 1995). Since the p16.sup.INK4 protein is a CDK4 inhibitor (Serrano, 1993), deletion of this gene may increase the activity of CDK4, resulting in hyperphosphorylation of the Rb protein. p16 also is known to regulate the function of CDK6.

p16 ^{INK4}belongs to a described class of CDK-inhibitory proteins, p16 ^{INK4}gene maps to 9p21, a chromosome region frequently deleted in many tumor types. Homozygous deletions and mutations of the p16 ^{INK4} gene are frequent in human tumor cell lines. This evidence suggests that the p16^{INK4} gene is a tumor suppressor gene. This interpretation has been challenged, however, by the observation that the frequency of the p16.sup.INK4 gene alterations is much lower in primary uncultured tumors than in cultured cell lines (Caldas et al., 1994; Cheng et al., 1994; Hussussian et al., 1994; Kamb et al., 1994; Kamb et al., 1994; Mori et al., 1994; Okamoto et al., 1994; Nobori et al., 1995; Orlow et al., 1994; Arap et al., 1995). Restoration of wild-type p16.sup.INK4 function by transfection with a plasmid expression vector reduced colony formation by some human cancer cell lines (Okamoto, 1994; Arap, 1995).

Other genes that may be employed according to the present invention include Rb, APC, DCC, NF-1, NF-2, WT-1, MEN-I, MEN-II, zac1, p73, VHL, MMAC1/PTEN, DBCCR-1, FCC, rsk-3, p.sup.27, p.sup.27/p16 fusions, p21/p27 fusions, anti-thrombotic genes (e.g., COX-1, TFPI), PGS, Dp, E2F, ras, myc, neu, raf, erb, fms, trk, ret, gsp, hst, abl, E1A, p300, genes involved in angiogenesis (e.g., VEGF, FGF, thrombospondin, BAI-1, GDAIF, or their receptors) and MCC.

### c. Other Agents

It is contemplated that other agents may be used in combination with the present invention to improve the therapeutic efficacy of treatment. These additional agents include immunomodulatory agents, agents that affect the upregulation of cell surface receptors and GAP junctions, cytostatic and differentiation agents, inhibitors of cell adehesion, or agents that increase the sensitivity of the hyperproliferative cells to apoptotic inducers. Immunomodulatory agents include tumor necrosis factor; interferon alpha, beta, and gamma; IL-2 and other cytokines; F42K and other cytokine analogs; or MIP-1, MIP-1beta, MCP-1, RANTES, and other chemokines. It is further contemplated that the upregulation of cell surface receptors or their ligands such as Fas/Fas ligand, DR4 or DR5/TRAIL would potentiate the apoptotic inducing abililties of the present invention by establishment of an autocrine or paracrine effect on hyperproliferative cells. Increases intercellular signaling by elevating the number of GAP junctions would increase the anti-hyperproliferative effects on the neighboring hyperproliferative cell population. In other embodiments, cytostatic or differentiation agents can be used in combination with the present invention to improve the anti-hyerproliferative efficacy of the treatments. Inhibitors of cell adehesion are contemplated to improve the efficacy of the present invention. Examples of cell adhesion inhibitors are focal adhesion kinase (FAKs) inhibitors and Lovastatin. It is further contemplated that other agents that increase the sensitivity of a hyperproliferative cell to apoptosis, such as the antibody c225, could be used in combination with the present invention to improve the treatment efficacy.

Hormonal therapy may also be used in conjunction with the present invention or in combination with any other therapy previously described. The use of hormones may be employed in the treatment of certain cancers such as breast, prostate, ovarian, or cervical cancer to lower the level or block the effects of certain hormones such as testosterone or estrogen. This treatment is often used in combination with at least one other cancer therapy as a treatment option or to reduce the risk of metastases.

In some embodiments, any one or plurality of CRISPR complex components may be administered with or within the viral particles, virions, or viral vectors disclosed herein. In some embodiments, an sgRNA or tracr/mate RNAs may be packaged with one or more reprogramming factors. In some embodiments, sgRNA molecules encapsulated by the viral particles, virions, or viral vectors may be packaged with one or more reprogramming factors.

With respect to general information on CRISPR-Cas Systems, components thereof and delivery of such components, including methods, materials, delivery vehicles, vectors, particles, AAV, and making and using thereof, including as to amounts and formulations, all useful in the practice of the instant invention, reference is made to: US Patents Nos. 8,697,359, 8,771,945, 8,795,965, 8,865,406 and 8,871,445; US Patent Publications US 2014-0287938 A1 (U.S. App. Ser. No. 14/213,991 ), US 2014-0273234 A1 (U.S. App. Ser. No. 14/293,674); US2014-0273232 A1 (U.S. App. Ser. No. 14/290,575), US 2014-0273231 (U.S. App. Ser. No. 14/259,420), US 2014-0256046 A1 (U.S. App. Ser. No. 14/226,274), US 2014-0248702 A1 (U.S. App. Ser. No. 14/258,458), US 2014-0242700 A1 (U.S. App. Ser. No. 14/222,930), US 2014-0242699 A1 (U.S. App. Ser. No. 14/183,512), US 2014-0242664 A1 (U.S. App. Ser. No. 14/104,990), US 2014-0234972 A1 (U.S. App. Ser. No. 14/183,471), US 2014-0227787 A1 (U.S. App. Ser. No. 14/256,912), US 2014-0189896 A1 (U.S. App. Ser. No. 14/105,035), US 2014-0186958 (U.S. App. Ser. No. 14/105,01 7), US 2014-0186919 A1 (U.S. App. Ser. No. 14/104,977), US 2014-0186843 A1 (U.S. App. Ser. No. 14/104,900), US 2014-0179770 A1 (U.S. App. Ser. No. 14/104,837) and US 2014-0179006 A1 (U.S. App. Ser. No. 14/183,486); PCT Patent Publications WO 2014/093661 (PCT/US2013/074743), WO 2014/093694 (PCT/US2013/074790), WO 2014/093595 (PCT/US2013/074611), WO 2014/09371 8 (PCT/US2013/074825), WO 2014/093709 (PCT/US2013/074812), WO 2014/093622 (PCT/US2013/074667), WO 2014/093635 (PCT/US2013/074691), WO 2014/093655 (PCT/US2013/074736), WO 2014/093712 (PCT/US2013/074819), WO2014/093701 (PCT/US2013/074800), and WO2014/018423 (PCT/US2013/051418); US provisional patent applications 61/961,980 and 61/963,643 each entitled FUNCTIONAL GENOMICS USING CRISPR-CAS SYSTEMS, COMPOSITIONS, METHODS, SCREENS AND APPLICATIONS THEREOF, filed October 28 and December 9, 2013 respectively; PCT/US2014/041806, filed June 10, 2014, US provisional patent applications 61/836, 123, 61/960,777 and 61/995,636, filed on June 17, 2013, September 25, 2013 and April 15, 2014, and PCT/US 13/74800, filed December 12, 2013. : Reference is also made to US provisional patent applications 61 /736,527, 61/748,427, 61/791,409 and 61/835,931, filed on December 12, 2012, January 2, 2013, March 15, 2013 and June 17, 2013, respectively. Reference is also made to US provisional applications 61/757,972 and 61/768,959, filed on January 29, 2013 and February 25, 2013, respectively. Reference is also made to US provisional patent applications 61/835,931, 61/835,936, 61/836,127, 61/836,101, 61/836,080 and 61/835,973, each filed June 17, 2013. Each of these applications, and all documents cited therein or during their prosecution ("appln cited documents") and ail documents cited or referenced in the appln cited documents, together with any instructions, descriptions, product specifications, and product sheets for any products mentioned therein or in any document therein, may be employed in the practice of the invention. Citations for documents cited herein may also be found in the foregoing herein-cited documents, as well as those hereinbelow cited.

### Methods

Some embodiments of the disclosure relate to methods for locally delivering nucleic acid molecules to fibroblasts within or associated with fibrotic tissues in the liver of a subject, in particular to one or a plurality of myofibroblasts, which can be derived from hepatic stellate cells or portal fibroblasts. Some embodiments of the disclosure provide methods for locally delivering nucleic acid molecules to fibroblasts within or associated with fibrotic tissues in the liver of a subject in need thereof, in particular to one or a plurality of myofibroblasts. The embodiments are based upon the discovery, among other things, that AAV viral vectors comprising a set of transcription factors disclosed herein may reprogram a fibroblast upon transduction of the fibroblast in vivo such that expression of exogenous genes or functional gene fragments thereof cause direct differentiation of fibroblasts into hepatocytes. Altering state of the cell from a myofibroblast to a hepatocyte results in differentiated hepatocytes that lose the myofibroblast's function to produce and deposit extracellular matrix such as collagen and acquire the function of a primary hepatocyte in the liver of a subject. Not only does the transduction of myofibroblasts within the liver of the subject cause a reduction of fibrotic tissue but it also creates a subpopulation of newly differentiated hepatocytes that have a growth advantage over damaged primary hepatocytes in the liver of a subject. Furthermore, the disclosure relates to the demonstration that the transduction of myofibroblasts reduces the deposition of extracellular matrix (ECM) materials, such as collagen. In some embodiments, the disclosure relates to a method of progressive repopulation of cells in the liver of a subject. In some embodiments, the subject has been diagnosed with, is susceptible to, or is has a likelihood of developing liver cirrhosis, liver fibrosis, liver cancer, and/or portal hypertension. More generally, any gene may be delivered to the myofibroblast of a subject in vivo through administration of any of the viral particles disclosed herein by directional contact of the viral particle to the myofibroblast. Such viral particles may comprise nucleic acid sequences comprising regulatory sequences operably lined to a coding sequence, wherein the regulatory sequence allows for directional expression of the coding sequence in a fibroblast, hepatic stellate cell, or portal fibroblast in the subject.

In some embodiments, the methods provided enable the efficient transduction of nucleic acid molecules encoding therapeutic proteins into myofibroblasts cells and tissues in a therapeutically effective amount and for a therapeutically effective time period. In some embodiments, the methods provided enable the efficient transduction of nucleic acid molecules encoding therapeutic proteins into portal fibroblasts cells and tissues in a therapeutically effective amount and for a therapeutically effective time period. The methods of the invention provide improved, sustained (long term) high level expression of therapeutic proteins in target cells. Without limiting the scope of the invention, it is especially the transduction efficiency of the AAV6, AAV7 and AAV8 virions, (optionally mixed populations of viral vectors or viral vectors with mixed populations of AAV VP proteins) in combination with the rAAV vectors of the invention, which enables efficient in vivo gene delivery. Although rAAV virions comprising capsid proteins of AAV serotype 6 may advantageously be used in the present invention, rAAV virions comprising at least one capsid protein of AAV serotype 6 (rAAV6 virions) are contemplated for use in the methods and compositions of the disclosure. The methods of the invention comprise the steps of (a) providing a recombinant AAV virion (rAAV) comprising capsid proteins of AAV serotypes disclosed herein, wherein the rAAV virion comprises a rAAV vector of a fragment of an AAV vector, the rAAV vector comprising an expression element operably linked to a nucleic acid sequence; and (b) bringing the rAAV virion into contact with one or more myofibroblasts, whereby transduction of the rAAV vector results in expression of the nucleic acid sequence in the transduced fibroblasts or tissue comprising fibroblasts. Preferably in the method, the nucleic acid sequence is delivered to the fibroblast or tissue comprising fibroblasts in vivo, by administration of the rAAV virion to a patient. In some embodiments, the method comprises administering a viral vector to a subject in need thereof by injecting the viral vector into a vein, such as a hepatic portal vein of the subject. In some embodiments, the method comprises administering a viral vector to a subject in need thereof. In some embodiments, methods comprise administering the composition, pharmaceutical composition, or viral vector disclosed herein intranasally, sublingually, intraperitoneally, intramuscularlly, or intravenously. Alternatively, in the method, the rAAV virion is brought into contact with cells or cell cultures or cell lines comprising fiibroblasts cells ex vivo, and whereby optionally the transduced cells are selected. In some embodiments, after ex vivo contact, the contacted fibroblasts are transplanted into a subject with liver disease. An alternative embodiment further comprises the step of administering the transduced cells to the bloodstream of a subject. In these methods the expression of the nucleic acid sequence in the in vivo or ex vivo transduced fibroblasts cell results in differentiation of the cell into a hepatocyte and reduction of symptoms of fibrosis.

In some embodiments, the method comprises contacting a composition comprising myofibroblasts ex vivo, selecting for the transduced myofibroblasts, and injecting the transduced cells into a subject. In some embodiments, the method comprises contacting a composition comprising myofibroblasts ex vivo, selecting for the transduced myofibroblasts, and injecting the transduced cells into a subject in need thereof.

Some embodiments of the disclosure relate to administering an amount of viral particle, compositions, pharmaceutical compositions, viral vector or transduced cells to a subject in an amount sufficient to cause the biological result that is desired. For instance, in some embodiments, if the desired biological result is to induce expression of a gene within the liver or muscle of subject in vivo, the amount of viral particle, compositions, pharmaceutical compositions, viral vector administered is in a sufficient amount to transduce a cell within the liver and induce expression of the gene. All methods disclosed herein contemplate the administration of a therapeutically effective amount or amount sufficient to result in the desired biological effect. In some embodiments, the methods relate to cause a recited biological effect in vivo.

The present disclosure also relates to a method of inducing differentiation of a fibroblast *in vivo* comprising contacting a fibroblast *in vivo* with the pharmaceutical composition in an amount sufficient to differentiate the fibroblast. The present disclosure also relates to a method of inducing differentiation of a fibroblast *in vivo* comprising contacting a fibroblast *in vivo* with the pharmaceutical composition in an amount sufficient to differentiate the fibroblast into a hepatocyte. In some embodiments, the pharmaceutical composition is administered to a subject via intravenous injection. In some embodiments, the fibroblast is a myofibroblast of the subject's liver. In some embodiments, the fibroblast is a portal fibroblast of the subject's liver.

The present disclosure also relates to a method of inhibiting the deposition of collagen in a subject comprising: contacting a fibroblast *in vivo* with the pharmaceutical composition in an amount sufficient to inhibit deposition of collagen. In some embodiments, the pharmaceutical composition is administered to a subject via intravenous injection. In some embodiments, the fibroblast is a myofibroblast of the subject's liver.

The present disclosure also relates to a method of altering the phenotype of a fibroblast in a subject comprising: contacting a fibroblast of the subject liver in vivo with the pharmaceutical composition in an amount sufficient to alter the phenotype of the fibroblast.

The present invention provides a pharmaceutical composition of the invention for use in a method of treating and/or preventing liver fibrosis in a subject in need thereof. The present disclosure also relates to a method of treating and/or preventing liver fibrosis in a subject in need thereof comprising: administering a therapeutic or prophylactically effective amount of the pharmaceutical composition. The present disclosure also relates to a method of treating and/or preventing liver cirrhosis in a subject in need thereof comprising: administering a therapeutic or prophylactically effective amount of the pharmaceutical composition. In some embodiments, the step of administering is performed via intravenous injection.

The present disclosure relates to a method of inducing proliferation of hepatocytes in a subject comprising: contacting a fibroblast of the subject liver in vivo with the pharmaceutical composition in an amount sufficient to induce differentiation and proliferation of hepatocytes in a liver of the subject. In some embodiments, the pharmaceutical composition is administered to a subject via intravenous injection.

The present disclosure relates to a method of targeting a myofibroblast in the liver of a subject comprising contacting a myofibroblast of the subject liver in vivo with the pharmaceutical composition in an amount sufficient to transduce the myofibroblast in the liver.

The present disclosure also relates to a method of restoring tissue-specific function to fibrotic tissue in an organ comprising administering into a subject: a first nucleic acid sequence encoding HNF4α or a functional fragment thereof; and a second nucleic acid sequence that encodes one or a plurality of transcription factors or functional fragments thereof chosen from: FOXA1, FOXA2, FOXA3, HNF1α, HNF6, GATA4, HLF, CEBPA, PROX1, and ATF5A. The present disclosure also relates to a method of restoring tissue-specific function to fibrotic tissue in an organ comprising administering into a subject: a first nucleic acid sequence encoding HNF4α or a functional fragment thereof; and a second nucleic acid sequence that encodes one or a plurality of transcription factors or functional fragments thereof chosen from: FOXA1, FOXA2, FOXA3, HNF1α, HNF6, GATA4, and HLF. The present disclosure also relates to a method of restoring tissue-specific function to fibrotic tissue in an organ comprising administering into a subject: a first nucleic acid sequence encoding HNF4α or a functional fragment thereof; and a second nucleic acid sequence that encodes one or a plurality of transcription factors or functional fragments thereof chosen from: FOXA1, FOXA2, FOXA3, HNF1α, HNF6, and GATA4. The present disclosure also relates to a method of restoring tissue-specific function to fibrotic tissue in an organ comprising administering into a subject: a first nucleic acid sequence encoding HNF4α or a functional fragment thereof; and a second nucleic acid sequence that encodes one or a plurality of transcription factors or functional fragments thereof chosen from: FOXA1, FOXA2, FOXA3, and HNF1α. The present disclosure also relates to a method of restoring tissue-specific function to fibrotic tissue in an organ comprising administering into a subject: a first nucleic acid sequence encoding HNF4α or a functional fragment thereof; and a second nucleic acid sequence that encodes one or a plurality of transcription factors or functional fragments thereof chosen from: FOXA1, FOXA2, and FOXA3.

One of ordinary skill in art would readily understand that combinations of pharmaceutical compositions are acceptable. The present disclosure also relates to a method of restoring tissue-specific function to fibrotic tissue in an organ of a subject comprising administering into the subject suspected of having, diagnosed as having, or genetically predisposed to acquiring fibrotic tissue in an organ: (a) a pharmaceutical composition comprising any of the disclosed viral particles disclosed herein; and/ or (b) a first nucleic acid sequence encoding HNF4α or a functional fragment thereof; and a second nucleic acid sequence that encodes one or a plurality of transcription factors or functional fragments thereof chosen from: FOXA1, FOXA2, FOXA3, HNF1α, HNF6, GATA4, HLF, CEBPA, PROX1, and ATF5A. Any of the above-mentioned sequences may be on one, two, three, four, five or more separate nucleic acid molecules each of which capable of expressing the one or plurality of expressible genes under conditions sufficient to express the gene upon introduction of the one or plurality of nucleic acid molecules in a cell. Another aspect of the invention relates to a method of restoring tissue-specific function to fibrotic tissue in an organ of a subject comprising administering into the subject suspected of having, diagnosed as having, or genetically predisposed to acquiring fibrotic tissue in an organ: a pharmaceutical composition comprising: a first nucleic acid sequence encoding HNF4α or a functional fragment thereof; and a second nucleic acid sequence that encodes one or a plurality of transcription factors or functional fragments thereof chosen from: FOXA1, FOXA2, FOXA3, HNF1α, HNF6, GATA4, HLF, CEBPA, PROX1, and ATF5A. Another aspect of the invention relates to a method of restoring tissue-specific function to fibrotic tissue in an organ of a subject comprising administering into the subject suspected of having, diagnosed as having, or genetically predisposed to acquiring fibrotic tissue in an organ: a pharmaceutical composition comprising: a first nucleic acid sequence encoding HNF4α or a functional fragment thereof; and a second nucleic acid sequence that encodes one or a plurality of transcription factors or functional fragments thereof chosen from: FOXA1, FOXA2, FOXA3, HNF1α, HNF6, GATA4 or functional fragment thereof.

In some embodiments, the methods relates to administration of any pharmaceutical composition, cell, vector, virion, viral particle disclosed herein in a therapeutically effective amount or amount sufficient to cause the recited desired effect, wherein the pharmaceutical composition, cell, vector, virion, viral particle is free of a coding sequence for HLF, CEBPA, PROX1, and/or ATF5A or a functional fragment thereof.

Another aspect of the disclosure relates to repeating a dose of an amount of the pharmaceutical composition, cell, vector, virion, viral particle disclosed herein sufficient to cause the desired biological effect. Repetition of the dose can occur daily, weekly, monthly, or annually. In some embodiments, the methods comprise administering a second dosage of the the pharmaceutical composition, cell, vector, virion, viral particle disclosed herein sufficient to cause the desired biological effect. Another aspect of the disclosure relates to repeating a dose of an amount of the pharmaceutical composition, cell, vector, virion, viral particle disclosed herein sufficient to cause the desired biological effect no more once, twice, three times, four times, five times, six times, seven times, eight times, nine times, or ten times or more.

Another aspect of the disclosure relates to repeating a dose of an amount of the pharmaceutical composition, cell, vector, virion, viral particle disclosed herein sufficient to cause the desired biological effect and administering a second pharmaceutical composition, cell, vector, virion, viral particle comprising a second agent before, contemporaneous with, or after administration of the pharmaceutical composition, cell, vector, virion, viral particle disclosed herein sufficient to cause the desired biological effect no. In some embodiments, the second agent may be a radionucleotide, a small molecular compound (such as a steroid), a polypeptide, or another gene therapy agent (such as a second viral particle comprising one or a plurality of genes).

In still another aspect of the disclosure, the disclosure relates to a method in vivo reprogramming of any nonhepatocyte in a liver of a subject into a hepatocytes.

The disclosure relates to a method of inducing expression of a gene in a myofibroblast in a subject comprising administering an amount of the pharmaceutical composition, cell, vector, virion, viral particle disclosed herein to the subject sufficient to transduce the myofibroblast. In some embodiments, the pharmaceutical composition or cell comprises a nucleic acid based vector comprising a regulatory sequence that is myofibroblast specific, such that the presence of the regulatory sequences become active operably through trans-acting regulatory proteins in the myofibroblast.

In some embodiments, the methods disclosed herein are free of a step in which the viral vector become stably integrated in the genomic DNA of the subject.

Some embodiments of the disclosure provide methods for treating and/or preventing fibrosis cirrhosis in a subject in need thereof by administering any viral particles disclosed herein (AAV6, AAV7, AAV8, or hybrid synthetic viruses derived therefrom) for elimination or directed killing of one or more myofibroblasts in the liver of the subject. In some embodiments, such viral particles comprise any nucleic acid sequence that encodes a cellular toxin such that transduction of the myofibroblast results in directed killing of the cell.

### Compositions

The invention relates to a composition comprising
a) one or a plurality of viral vectors of claim 1; and/or
b) a plurality of viral vectors comprising
   i) a first viral vector comprising a viral capsid comprising a nucleic acid molecule encapsulated within the viral capsid, wherein the nucleic acid molecule comprises a nucleic acid sequence that encodes mammalian HNF4α and wherein the viral capsid comprises the viral capsid polypeptides VP1 of AAV6, VP2 of AAV6, and VP3 of AAV6; and
   ii) a second viral vector comprising a viral capsid comprising a nucleic acid molecule encapsulated within the viral capsid, wherein the nucleic acid molecule comprises a nucleic acid sequence that encodes one or more mammalian transcription factors selected from the group consisting of: FOXA1, FOXA2, FOXA3, HNF1α, HNF6, GATA4, HLF, CEBPA, PROX1, and ATF5A and wherein the viral capsid comprises the viral capsid polypeptides VP1 of AAV6, VP2 of AAV6, and VP3 of AAV6.

The disclosure relates to viral vectors comprising one or more nucleic acid sequences encoding one or a plurality of transcription factors disclosed herein, and compositions and pharmaceutical compositions comprising the viral vectors disclosed herein. In some embodiments, the compositions or pharmaceutical compositions disclosed herein comprise one or a nucleic acid sequences that encode no more than 2, 3, 4, 5, 6, 7, 8, 9, or 10 of transcription factors disclosed herein, or functional fragments thereof. The disclosure also relates to compositions and pharmaceutical compositions comprising the nucleic acid molecules disclosed herein. Such nucleic acid molecules may have one or a plurality of coding sequences that encode for one or a plurality of transcription factors disclosed herein. The nucleic acid sequences encoding the one or plurality of transcription factors may be aligned in any order sequentially on a single nucleic acid molecule or may be one, two, three or more distinct nucleic acid molecules packaged within a viral particle or virion disclosed herein and/or including another vehicle to deliver nucleic acid sequences. The disclosure contemplates, for instance, pharmaceutical compositions, and compositions comprising 1, 2, 3, 4, 5, or 6 nucleic acids, each nucleic acid sequence encoding a single transcription factor disclosed herein and, optionally each nucleic acid sequence comprising one or a plurality to regulatory sequence operably linked to the encdable nucleic acid sequence. In alternative embodiments, the pharmaceutical compositions and/or compositions comprise one or a plurality of nucleic acid sequences encoding more than one coding sequence of the transcription factors disclosed herein. In some embodiments the pharmaceutical compositions disclosed herein comprise several different subpopulations of viral particles, each viral particle containing a single nucleic acid molecule that expresses one or more of the transcription factors disclosed herein. For instance, it is contemplated that, in some embodiments, a pharmaceutical composition comprises one viral particle comprising a nucleic acid that encodes a transcription factor and another viral particle comprising a second nucleic acid encoding another transcription factor. All permutations or combinations of viral particles 9with one, two, three or more VP amino acid sequences) comprising 1, 2, 3, 4, 5, 6, 7, 8, 9, or 10 or more coding sequences (transcription factors) disclosed herein are contemplated by the disclosure. For example, the compositions or pharmaceutical compositions disclosed herein may consist of one or more nucleic acid sequences disclosed herein and/or be free of any one or more of the nucleic acid sequences encoding other transcription factors. The disclosure relates to the compositions or the pharmaceutical compositions disclosed herein may consist of one or more nucleic acid sequences that are at least 70, 75, 80, 85, 90, 91, 92, 93, 94, 95, 96, 97, 98, 99, or 100% homologous to the nucleic acid sequences of Table 3 or functional fragments thereof. In some embodiments, a nucleic acid molecule comprises one or more nucleic acid sequences that are at least 70, 75, 80, 85, 90, 91, 92, 93, 94, 95, 96, 97, 98, 99, or 100% homologous to the nucleic acid sequences disclosed in Table 3 or functional frgaments thereof, further comprising one or a plurality of regulatory elements operably linked to the one or more nucleic acid sequences, such that under sufficient conditions, the nucleic acid sequences encode one or more transcription factors. In some embodiments, compositions or pharmaceutical compositions comprise one or a plurality of viral particles, virions, or vectors that comprise the aforementioned one or more nucleic acid sequences within their capsids on one or more nucleic acid molecules. In some embodiments, a nucleic acid molecule comprises one or more nucleic acid sequences that are at least 70, 75, 80, 85, 90, 91, 92, 93, 94, 95, 96, 97, 98, 99, or 100% homologous to the nucleic acid sequences disclosed in the Examples or functional frgaments thereof. In some embodiments, a nucleic acid molecule comprises one or more nucleic acid sequences that are at least 70, 75, 80, 85, 90, 91, 92, 93, 94, 95, 96, 97, 98, 99, or 100% homologous to the nucleic acid sequences amplified by the primers or functional frgaments thereof disclosed in the examples section. In some embodiments, the one or plurality of viral particles, virions, or viral vectors comprising one or a plurality of nucleic acid molecules that are at least 70, 75, 80, 85, 90, 91, 92, 93, 94, 95, 96, 97, 98, 99, or 100% homologous to SEQ ID Nos:67 - 72 the nucleic acid sequences or functional fragments thereof. Any pharmaceutical composition disclosed herein may comprise one or a plurality of viral particles, virions, or viral vectors comprising one or a plurality of nucleic acid molecules that are at least 70, 75, 80, 85, 90, 91, 92, 93, 94, 95, 96, 97, 98, 99, or 100% homologous to SEQ ID Nos:67 - 72 the nucleic acid sequences or functional fragments thereof, such that compositions may comprise viral vectors, virons, or viral particles with different nucleic acid molecules but, collectively, the composition comprise a pahramceutically effective amount of any combination of the nucleic acid molecules disclosed herein. Any pharmaceutical composition disclosed herein may comprise one or a plurality of viral particles, virions, or viral vectors comprising one or a plurality of nucleic acid molecules that are at least 70, 75, 80, 85, 90, 91, 92, 93, 94, 95, 96, 97, 98, 99, or 100% homologous to SEQ ID Nos:67 - 72 the nucleic acid sequences or functional fragments thereof, such that compositions may comprise viral vectors, virons, or viral particles with different nucleic acid molecules but, collectively, the composition comprise a pharmaceutically effective amount of any combination of the nucleic acid sequences disclosed herein. The viral particle, virions, or viral vectors of the disclosure may comprise any permutations of VP polypeptides from AAV serotypes. For instance any combination of VP1, 2, and/or 3 may be contemplated such that the AAV particle may comprise AAV2, 6, 7, and/or 8 VP polypeptides and may comprise any nucleica cid seqeunce encoding the seqeunces of Table 3, either as separate or sinlge nucleic acid molecules.

All permutations of the presence of a transcription factors disclosed in this disclosure are contemplated and any seqeunces substantially complementary to those seqeunces or functional fragments thereof. The disclosure also relates to compositions and pharmaceutical compositions comprising isolated nucleic acid molecules disclosed herein, wherein the nucleic acid sequence encoding one or a plurality of transcription factors are operably linked to one or more regulatory sequences. In some embodiments, the regulatory sequences drive expression of the one or plurality of transcription factors in a host cell.

In some embodiments, the compositions and pharmaceutical compositions comprise a viral vector comprising one or a plurality of nucleic acid sequences encoding one or a plurality of transcription factors disclosed herein. In some embodiments, the viral vector is a recombinant AAV pseduo-virus , virion, or viral particle. The recombinant AAV virion, including one of the rAAV vectors, is produced using methods known in the art, as described in Pan et al. (J. of Virology 1999, Vol 73(4):3410-3417) and Clark et al. (Human Gene Therapy, 1999, 10:1031-1039). In short, the methods generally involve (a) the introduction of the rAAV vector into a host cell, (b) the introduction of an AAV helper construct into the host cell, wherein the helper construct comprises the viral functions missing from the rAAV vector and (c) introducing a helper virus into the host cell. Functions for rAAV virion replication and packaging need to be present, to achieve replication and packaging of the rAAV vector into rAAV virions. The introduction into the host cell can be carried out using standard virological techniques and can be simultaneously or sequentially. Finally, the host cells are cultured to produce rAAV virions and are purified using standard techniques such as CsCl gradients (Xiao et al. 1996, J. Virol. 70: 8098-8108). Residual helper virus activity can be inactivated using known methods, such as for example heat inactivation. The purified rAAV virion is then ready for use in the methods. High titres of more than 10¹² particles per ml and high purity (free of detectable helper and wild type viruses) can be achieved (Clark et al. supra and Flotte et al. 1995, Gene Ther. 2: 29-37).

The rAAV vector comprises at least the nucleotide sequences of the inverted terminal repeat regions (ITR) of one of the AAV serotypes, or nucleotide sequences substantially identical thereto, and at least one nucleotide sequence encoding one or a plurality of therapeutic proteins (under control of a suitable regulatory element) inserted between the two ITRs.

The complete genome of AAV5 and other AAV serotypes has been sequenced (Chiorini et al. 1999, J. of Virology Vol. 73, No.2, pl309-1319) and the nucleotide sequence is available in GenBank (Accession No. AF085716). The ITR nucleotide sequences of AAV serotypes are thus readily available to a skilled person. They can be either cloned or made by chemical synthesis as known in the art, using for example an oligonucleotide synthesizer as supplied e.g. by Applied Biosystems Inc. (Fosters, CA, USA) or by standard molecular biology techniques. The ITRs can be cloned from the AAV viral genome or excised from a vector comprising the AAV ITRs. The ITR nucleotide sequences can be either ligated at either end to the nucleotide sequence encoding one or more therapeutic proteins using standard molecular biology techniques, or the wild type AAV sequence between the ITRs can be replaced with the desired nucleotide sequence. In some embodiments, the desired nucleotide sequence comprises a coding sequence for 1, 2, 3, 4, 5, 6, 7, 8, 9, or 10 of the transcription factors disclosed in Table 3 or functional fragments thereof.

Preferably, the rAAV nucleic acid vector is free of any nucleotide sequences encoding viral proteins, such as the rep (replication) or cap (capsid) genes of AAV. The rAAV nucleic acid vector may further comprise a marker or reporter gene, such as a gene for example encoding an antibiotic resistance gene, a fluorescent protein (e.g. gfp) or a gene encoding a chemically, enzymatically or otherwise detectable and/or selectable product (e.g. lacZ, aph, etc.) known in the art.

The rAAV nucleic acid vector further comprises a promoter sequence operably linked to the nucleotide sequence encoding a therapeutic protein and/or a transcription factor. Suitable promoter sequences are promoters which confer expression in cells of the liver, such as fibroblasts and/or other cells that responsible for scarring in the liver. Suitable promoters are for example the promoters of genes known to be expressed in liver cells, such as the CMV promoter (cytomegalovirus), the promoter of the IL-6 gene or the SV40 promoter, and others, as readily determined by a skilled person.

A suitable 3' non-translated sequence may also be operably linked to the nucleotide sequence encoding the therapeutic protein. Suitable 3' non-translated regions may be those naturally associated with the nucleotide sequence or may be derived from different genes, such as for example the bovine growth hormone 3' non-translated region (BGH poly A) sequence.

The total size of the DNA molecule inserted into the rAAV vector between the ITR regions is generally smaller than 5 kilobases (kb) in size. It is also envisaged that the rAAV vector comprises nucleotide sequences encoding two therapeutic proteins (e.g. therapeutic proteins having a synergistic effect). These may either comprise a suitable promoter and suitable 3'nontranslated region each, or they may be linked by an IRES (internal ribosome entry sites) element, providing a bicistronic transcript under control of a single promoter. Suitable IRES elements are described in e.g. Hsieh et al. (1995, Biochemical Biophys. Res. Commun. 214:910-917).

Some embodiments of the disclosure relate to compositions or pharmaceutical compositions comprising one or a plurality of vrial particles, virion, or pseudoviruses disclosed herein and one or a plurality of additional gene therapy vectors or vaccines.

Gene therapy vectors include, for example, viral vectors, lipoparticles, liposomes and other lipid-containing complexes, catanionic vesicles and other macromolecular complexes capable of mediating delivery of a gene to a host cell. Open reading frames useful in gene therapy vectors include but are not limited to those described in U.S. patent application Ser. No. 10/788,906, entitled "METHOD AND APPARATUS FOR DEVICE CONTROLLED GENE EXPRESSION". Vectors can also comprise other components or functionalities that further modulate gene delivery and/or gene expression, or that otherwise provide beneficial properties to the targeted cells. Such other components include, for example, components that influence binding or targeting to cells (including components that mediate cell-type or tissue-specific binding); components that influence uptake of the vector by the cell; components that influence localization of the transferred gene within the cell after uptake (such as agents mediating nuclear localization); and components that influence expression of the gene. Such components also might include markers, such as detectable and/or selectable markers that can be used to detect or select for cells that have taken up and are expressing the nucleic acid delivered by the vector. Such components can be provided as a natural feature of the vector (such as the use of certain viral vectors which have components or functionalities mediating binding and uptake), or vectors can be modified to provide such functionalities. Selectable markers can be positive, negative or bifunctional. Positive selectable markers allow selection for cells carrying the marker, whereas negative selectable markers allow cells carrying the marker to be selectively eliminated. A variety of such marker genes have been described, including bifunctional (i.e., positive/negative) markers (see, e.g., WO 92/08796; and WO 94/28143). Such marker genes can provide an added measure of control that can be advantageous in gene therapy contexts. A large variety of such vectors are known in the art and are generally available.

Gene therapy vectors within the scope of the invention include, but are not limited to, isolated nucleic acid, e.g., plasmid-based vectors which may be extrachromosomally maintained, and viral vectors, e.g., recombinant adenovirus, retrovirus, lentivirus, herpesvirus, poxvirus, papilloma virus, or adeno-associated virus, including viral and non-viral vectors which are present in liposomes, e.g., neutral or cationic liposomes, such as DOSPA/DOPE, DOGS/DOPE or DMRIE/DOPE liposomes, and/or associated with other molecules such as DNA-anti-DNA antibody-cationic lipid (DOTMA/DOPE) complexes. Gene therapy vectors of the disclosure may also include surfactant vesicles that envelope a nucleic acid sequence. Exemplary gene therapy vectors are described below. Gene therapy vectors may be administered via any route including, but not limited to, intramuscular, buccal, rectal, intravenous administration or administration through the hepatic portal vein, and transfer to cells may be enhanced using electroporation and/or iontophoresis. In some embodiments, the gene therapy vector may be free of retroviral or lentiviral protein or retroviral or lentiviral nucleic acid sequences.

In some embodiments, the viral particles, compositions, pharmaceutical compositions disclosed herein are free of a nucleic acid that encodes HNF4α or functional fragments thereof.

### Adeno-Associated Virus Vectors

Recombinant adeno-associated viruses (rAAV) are derived from nonpathogenic parvoviruses, evoke essentially no cellular immune response, and produce transgene expression lasting months in most systems. Moreover, like adenovirus, adeno-associated virus vectors also have the capability to infect replicating and nonreplicating cells and are believed to be nonpathogenic to humans. In some embodiments, the viral vector comprises plasmid DNA.

In some embodiments, the viral vector comprises antisense oligonucleotides, which are short (approximately 10 to 30 nucleotides in length), chemically synthesized DNA molecules that are designed to be complementary to the coding sequence of an RNA of interest. These agents may enter cells by diffusion or liposome-mediated transfer and possess relatively high transduction efficiency. These agents are useful to reduce or ablate the expression of a targeted gene while unmodified oligonucleotides have a short half-life in vivo, modified bases, sugars or phosphate groups can increase the half-life of oligonucleotide. For unmodified nucleotides, the efficacy of using such sequences is increased by linking the antisense segment with a specific promoter of interest, e.g., in an adenoviral construct. In one embodiment, electroporation and/or liposomes are employed to deliver plasmid vectors. Synthetic oligonucleotides may be delivered to cells as part of a macromolecular complex, e.g., a liposome, and delivery may be enhanced using techniques such as electroporation.

### Targeted Vectors

The present disclosure contemplates the use of cell targeting not only by local delivery of the transgene or recombinant cell, but also by use of targeted vector constructs having features that tend to target gene delivery and/or gene expression to particular host cells or host cell types. Such targeted vector constructs would thus include targeted delivery vectors and/or targeted vectors, as described herein. Restricting delivery and/or expression can be beneficial as a means of further focusing the potential effects of gene therapy. The potential usefulness of further restricting delivery/expression depends in large part on the type of vector being used and the method and place of introduction of such vector. In addition, using vectors that do not result in transgene integration into a replicon of the host cell (such as adeno-associated virus and numerous other vectors).

Targeted delivery vectors include, for example, vectors (such as viruses, non-viral protein-based vectors and lipid-based vectors) having surface components (such as a member of a ligand-receptor pair, the other half of which is found on a host cell to be targeted) or other features that mediate preferential binding and/or gene delivery to particular host cells or host cell types. As is known in the art, a number of vectors of both viral and non-viral origin have inherent properties facilitating such preferential binding and/or have been modified to effect preferential targeting (see, e.g., Miller, et al., FASEB Journal, 9:190 (1995); Chonn et al., Curr. Opin. Biotech., 6:698 (1995); Schofield et al., British Med. Bull., 51:56 (1995); Schreier, Pharmaceutica Acta Helvetiae, 68:145 (1994); Ledley, Human Gene Therapy, 6:1129 (1995); WO 95/34647; WO 95/28494; and WO 96/00295).

Targeted vectors include vectors (such as viruses, non-viral protein-based vectors and lipid-based vectors) in which delivery results in transgene expression that is relatively limited to particular host cells or host cell types. For example, transgenes can be operably linked to heterologous tissue-specific enhancers or promoters thereby restricting expression to cells in that particular tissue.

The disclosure further provides a pharmaceutical composition that increases hepatocyte mass in the liver of a subject in need thereof comprising a therapeutically effective amount of a vector as described herein, in admixture with a pharmaceutically acceptable carrier. Another embodiment is a pharmaceutical composition for the treatment or prevention of a condition involving an increase of fibrotic tissue in the liver of a subject in need thereof, the decrease of hepatocyte mass in the liver of a subject, or a susceptibility to the condition, comprising an amount of viral vector sufficient to differentiate myofibroblasts in the liver of the subject and/or enhance the growth of hepatocytes in the liver of the subject. In some embodiments, the pharmaceutical composition comprises a therapeutic agent, such as a polypeptide, nucleic acid sequence, small chemical compound, prodrug, or pharmaceutically acceptable salts thereof. In some embodiments, the pharmaceutical compositions of the disclosure comprise pharmaceutically acceptable salts, hydrates, solvates, or prodrugs thereof in admixture with a pharmaceutically acceptable carrier.

The pharmaceutical composition may be solid, liquid, gel, or other form, in which the compound, polynucleotide, vector, and antibody of the disclosure is maintained in an active form, e.g., in a form sufficient to effect a biological activity. For example, in some embodiments, the pharmaceutical composition comprises a therapeutically effective amount of a viral vector comprising the nucleic acid sequences disclosed herein to differentiate a fibroblast in the liver of a subject, induce proliferation of hepatocytes in the liver of a subject, and/or reduce collagen deposition in the liver of a subject.

Such compositions can be formulated for administration by topical, oral, parenteral, intranasal, subcutaneous, and intraocular, routes. Parenteral administration is meant to include intravenous injection, intramuscular injection, intraarterial injection or infusion techniques. The composition may be administered parenterally in dosage unit formulations containing standard, well-known non-toxic physiologically acceptable carriers, adjuvants and vehicles as desired.

Pharmaceutical compositions for oral administration can be formulated using pharmaceutically acceptable carriers well known in the art in dosages suitable for oral administration. Such carriers enable the pharmaceutical compositions to be formulated as tablets, pills, dragees, capsules, liquids, gels, syrups, slurries, suspensions and the like, for ingestion by the patient. Pharmaceutical compositions for oral use can be prepared by combining active compounds with solid excipient, optionally grinding a resulting mixture, and processing the mixture of granules, after adding suitable auxiliaries, if desired, to obtain tablets or dragee cores. Suitable excipients are carbohydrate or protein fillers, such as sugars, including lactose, sucrose, mannitol, or sorbitol; starch from corn, wheat, rice, potato, or other plants; cellulsoe, such as methyl cellulose, hydorxypropylmethyl-cellulose, or sodium carboxymethyl-cellulose; gums including arabic and tragacanth; and proteins such as gelatin and collagen. If desired, disintegrating or solubilizing agents may be added, such as the cross-linked polyvinyl pyrrolidone, agar, alginic acid, or a salt thereof, such as sodium alginate. Dragee cores may be used in conjunction with suitable coatings, such as concentrated sugar solutions, which may also contain gum arabic, talc, polyvinyl-pyrrolidone, carbopol gel, polyethylene glycol, and/or titanium dioxide, lacquer solutions, and suitable organic solvents or solvent mixtures. Dyestuffs or pigments may be added to the tablets or dragee coatings for product identification or to characterize the quantity of active compound, i.e., dosage.

Pharmaceutical preparations that can be used orally include push-fit capsules made of gelatin, as well as soft, sealed capsules made of gelatin and a coating, such as glycerol or sorbitol. Push-fit capsules can contain active ingredients mixed with filler or binders, such as lactose or starches, lubricants, such as talc or magnesium stearate, and, optionally, stabilizers. In soft capsules, the active compounds may be dissolved or suspended in suitable liquids, such as fatty oils, liquid, or liquid polyethylene glycol with or without stabilizers.

Preferred sterile injectable preparations can be a solution or suspension in a non-toxic parenterally acceptable solvent or diluent. Examples of pharmaceutically acceptable carriers are saline, buffered, saline, isotonic saline (e.g., monosodium or disodium phosphate, sodium, potassium; calcium or magnesium chloride, or mixtures of such salts). Ringer's solution, dextrose, water, sterile water, glycerol, ethanol, and combinations thereof 1,3-butanediol and sterile fixed oils are conveniently employed as solvents or suspending media, Any bland fixed oil can be employed, including synthetic mono- or di-glycerides. Fatty acids such as oleic acid also find use in the preparation of injectables. In some embodiments, the dosages are sterile and pyrogen-free.

The composition medium can also be a hydrogel, which is prepared from any biocompatible or non-cytotoxic homo- or hetero-polymer, such as a hychrophilic polyaorylic acid polymer that can act as a drug absorbing sponge. Certain of them, such as, in particular, shore obtained from ethylene and/or propylene oxide are commercially available, A hydrogel can be deposited directly onto the surface of the tissue to be treated, for example, during surgical intervention or transplant procedure.

Embodiments of pharmaceutical compositions of the present disclosure comprise a replication defective recombinant viral vector encoding the transcription factors of the present disclosure and a transfection enhancer, such as poloxamer. An example of a poloxamer is Poloxamer 407, which is commercially available (BASF, Parsippany, N.J.) and is a non-toxic, biocompatible polyol. A polyoxamer impregnated with recombinant viruses may be deposited directly on the surface of the tissue to be treated, for example, during a surgical intervention. Poloxamer possesses essentially the same advantages as hydrogel while having a lower viscosity.

The nucleic acid or vector comprising the nucleic acid agent may also be entrapped in microcapsules prepared, for example, by interfacial polymerization, for example, hydroxymethylcellulose or gelatin-microcapsules and poly-(methlymethacylate) mocrocapsules, respectively, in colloidal drug delivery systems (for example, liposomes, albumin microspheres, microemulsions, nano-articles and nanocapsules) or in macroemulsion. Such techniques are disclosed in Remington's Pharmaceutical Sciences (1980) 16th edition, Osol, A. Ed.

Sustained-release preparations may be prepared. Suitable examples of sustained-release preparations include semi-permeable matrices of solid hydrophobic polymers containing the antibody, which matrices are in the form of shaped articles, e.g. films, or microcapsules. Examples oil sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and gamma-ethyl-L-glutamate, non-degradable ethylene-vinyl acetate, degradable lactic acid-glycolic acid copolymers such as the LUPRON DEPOT^{™}. (injectable microspheres composed of lactic acid-glycolic acid copolymer and leuprolide acetate), and poly-D-(-)-3-Hydroxybutyric acid. While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37.degree. C., resulting in a loss of biological activity and possible changes in immunogenicity. Rational strategies can be devised for stabilization depending on the mechanism involved. For example, if the aggregation mechanism is discovered to be intermolecular S--S bond formation through thio-disulfide interchange, stabilization may be achieved by modifying sulfhydryl residues, lyophilizing from acidic solutions, controlling moisture content, using appropriate additives, and developing specific polymer matrix compositions.

As defined above, therapeutically effective dose means that amount of protein, polynucleotide, peptide or antibodies against such peptides, virus or nucleic acid which ameliorate the symptoms or condition. Therapeutic efficacy and toxicity of such compounds can be determined by standard pharmaceutical procedures in cell cultures or experimental animals, e.g., ED50 (the dose therapeutically effective in 50% of the population) and LD50 (the dose lethal to 50% of the population). The dose ration of toxic to therapeutic effects is the therapeutic index, and it can be expressed as the ratio, LD50/ED50. Pharmaceutical compositions that exhibit large therapeutic indices are preferred. The data obtained from cell culture assays and animal studies is used in formulating a range of dosage for human use. The dosage of such compounds lies preferably within a range of circulating concentrations that include the ED50 with little or no toxicity. The dosage varies within this range depending upon the dosage form employed, sensitivity of the patient, and the route of administration.

For any compound, the therapeutically effective dose can be estimated initially either in cell culture assays or in animal models, such as mice, rabbits, dogs, or pigs. The animal model is also used to achieve a desirable concentration range and route of administration. Such information can then be used to determine useful doses and routes for administration in humans. The exact dosage is chosen by the individual physician in view of the patient to be treated. Dosage and administration are adjusted to provide sufficient levels of the active moiety or to maintain the desired effect. Additional factors which may be taken into account include the severity of the disease state, age, weight and gender of the patient; diet, desired duration of treatment, method of administration, time and frequency of administration, drag combination(s), reaction sensitivities, and tolerance/response to therapy. Long acting pharmaceutical compositions might be administered every 3 to 4 days, every week, or once every two weeks depending on half-life and clearance rate of the particular formulation.

The pharmaceutical compositions according to this disclosure may be administered to a subject by a variety of methods. They may be added directly to target tissues, complexed with cationic lipids, packaged within liposomes, or delivered to target cells by other methods known in the art. Localized administration to the desired tissues may be done by catheter, infusion pump or stent. The DNA, DNA/vehicle complexes, or the recombinant virus particles are locally administered to the site of treatment. Alternative routes of delivery include, but are not limited to, intravenous injection, intramuscular injection, subcutaneous injection, aerosol inhalation, oral (tablet or pill form), topical, systemic, ocular, intraperitoneal and/or intrathecal delivery. Examples of ribozyme delivery and administration are provided in Sullivan et al. WO 94/02595. As discussed hereinabove, recombinant viruses may be used to introduce DNA encoding polynucleotide agents useful in the present disclosure. Recombinant viruses according to the disclosure are generally formulated and administered in the form of doses of from about 10⁴ to about 10¹⁴ pfu. In some embodiments, doses are from about 10⁶ to about 10¹¹ pfu. In some embodiments, doses are from about 10⁵ to about 10¹¹ pfu. In some embodiments, doses are from about 10⁷ to about 10¹¹ pfu. In some embodiments, doses are from about 10⁸ to about 10¹¹ pfu. In some embodiments, doses are from about 10⁹ to about 10¹¹ pfu. In some embodiments, doses are from about 10⁹ to about 10¹¹ pfu. The term pfu ("plaque-forming unit") corresponds to the infective power of a suspension of virions and is determined by infecting an appropriate cell culture and measuring the number of plaques formed. The techniques for determining the pfu titre of a viral solution are well documented in the prior art. In some embodiments, the therapeutically effective dosage ranges from about 2 x 10¹¹ viral genomes (vg)/kg body weight (BW) (low dose) to about 6 x 10¹¹ vg/kg BW (intermediate dose) and/or to about 2 x 10¹² vg/kg BW (high dose). All doses were tolerated without complications. In some embodiments, the therapeutically effective dosage ranges from about 2 x 10¹¹ viral genomes (vg)/kg body weight (BW) (low dose) to about 2 x 10¹² vg/kg BW (high dose). In some embodiments, the therapeutically effective dosage ranges from about 1 x 10¹¹ viral genomes (vg)/kg body weight (BW) (low dose) to about 3 x 10¹² vg/kg BW (high dose). In some embodiments, the therapeutically effective dosage ranges from about 0.1 x 10¹¹ viral genomes (vg)/kg body weight (BW) (low dose) to about 1 x 10¹³ vg/kg BW (high dose).

A further aspect of the disclosure relates to a method of treating or preventing liver disease involving fibrotic tissue, comprising administering to said subject a pharmaceutical composition as described herein.

A further aspect of the disclosure relates to a method of treating or preventing liver disease comprising administering to said subject a pharmaceutical composition as described herein.

A further aspect of the disclosure relates to a method of treating or preventing Nonalcoholic Steatohepatitis (NASH) involving fibrotic tissue, comprising administering to said subject a pharmaceutical composition as described herein.

A further aspect of the disclosure relates to a method of treating or preventing alcoholic hepatitis in a subject in need thereof, comprising administering to said subject a pharmaceutical composition as described herein.

A further aspect of the disclosure relates to a method of treating or preventing cirrhosis involving fibrotic tissue in a subject in need thereof, comprising administering to said subject a pharmaceutical composition as described herein.

A further aspect of the disclosure relates to a method of treating or preventing liver fibrosis in a subject in need thereof, comprising administering to said subject a pharmaceutical composition as described herein.

The disclosure also relates to a method of *in vivo* reprogramming of myofibroblasts into hepatocytes comprising contacting one or more myofibroblasts with a pharmaceutical composition as disclosed herein. The disclosure also relates to a method of simultenously replenishing the number of hepatocytes in the liver of a subject in need thereof and suppressing collagen production by myofibroblasts in the subject in need thereof comprising administering to said subject a pharmaceutical composition as described herein. In some embodiments of any of the disclosed methods, the method is free of converting any myofibroblast into a pluripotent state - in other words, the reprogramming step alters the phenotype of the cells contacted by the viral vectors or pharmaceutical compositions disclosed herein directly from a myofibroblast into a hepatocyte. The disclosure relates to a method of inducing the AAV vector-mediated expression of exogenous or recombinantly engineered amino acids in myofibroblsts in a subject (in vivo) comprising comprising administering to said subject a pharmaceutical composition as described herein. The disclosure relates to a method of inducing the AAV vector-mediated expression of exogenous or recombinantly engineered amino acids in myofibroblsts in a subject (in vivo) comprising comprising administering to said subject one or a plurality of nucleic acid seqeunces encoding transcription factors disclosed herein. Any of the methods disclosed herein may comprise the step of: (i) administering a pharmaceutically effective amount of a virion, viral particle, or virus-like particle comprising one or a plurality of nucleic acid seqeunces encoding transcription factors disclosed herein to a subject; or (ii) contacting a pharmaceutically effective amount of a virion, viral particle, or virus-like particle comprising one or a plurality of nucleic acid seqeunces encoding transcription factors disclosed herein to a myofibroblast in a subject. The subject may be a human, or non-human mammal.

The disclosure relates to a method of preventing liver failure, portal hypertension and/or liver cancer by administering to said subject a pharmaceutical composition as described herein. increased myofibroblast production in the liver of patients

The disclosure also relates to a method of stably reprogamming myofibroblasts by transient expression of one or more nucleic acid sequences disclosed herein or functional fragments thereof or variants thereof comprising administering to a subject a pharmaceutically effective amount of a virion, viral particle, or virus-like particle comprising one or a plurality of nucleic acid seqeunces encoding transcription factors disclosed herein (or functional fragments or variants thereof). Any of the methods herein may comprise the step of administering any dosage amount of the viral particles or virions disclosed herein. Any of the methods disclosed herein may comprise a step of isolating one or a plurality of virions, viral particles or viral vectors comprising any one or plurality nucleic acids disclosed herein by removing the virions, viral particles, or viral vectors from a cell culture. In some embodiments, any of the methods disclosed herein comprise a step of in vitro selection of commercially available pooled human antisera to eliminate viral particles, virions or viral vectors comprising prevalent epitopes recognized by human antibodies. This step should increase the potency of the pharmaceutical compositions when administered to a subject, such as a human or other mammal.

**TABLE 3, AAV sequences and Transcription Factor**

| **AAV capsid sequences (nucleotide + protein)** |
|---|
| **AAV6** |
| **AAV6 - VP1 nucleotide sequence (SEQ ID NO:1):** |
| ATGGCTGCCGATGGTTATCTTCCAGATTGGCTCGAGGACAACCTCTCTGAGGGCATT CGCGAGTGGTGGGACTTGAAACCTGGAGCCCCGAAACCCAAAGCCAACCAGCAAAA |
| GCAGGACGACGGCCGGGTCTGGTGCTTCCTGGCTACAAGTACCTCGGACCCTTCAA CGGACTCGACAAGGGGGAGCCGTCAACGCGGCGGATGCAGCGGCCCTCGAGCACG ACAAGGCCTACGACCAGCAGCTCAAAGCGGGTGACAATCCGTACCTGCGGTATAAC CACGCCGACGCCGAGTTTCAGGAGCGTCTGCAAGAAGATACGTCTTTGGGGGCAA CCTCGGGCGAGCAGTCTTCCAGGCCAAGAAGAGGGTTCTCGAACCTTTTGGTCTGGT TGAGGAAGGTGCTAAGACGGCTCCTGGAAAGAAACGTCCGGTAGAGCAGTCGCCAC AAGAGCCAGACTCCTCCTCGGGCATTGGCAAGACAGGCCAGCAGCCCGCTAAAAAG AGACTCAATTTTGGTCAGACTGGCGACTCAGAGTCAGTCCCCGACCCACAACCTCTC GGAGAACCTCCAGCAACCCCCGCTGCTGTGGGACCTACTACAATGGCTTCAGGCGG TGGCGCACCAATGGCAGACAATAACGAAGGCGCCGACGGAGTGGGTAATGCCTCAG GAAATTGGCATTGCGATTCCACATGGCTGGGCGACAGAGTCATCACCACCAGCACC CGAACATGGGCCTTGCCCACCTATAACAACCACCTCTACAAGCAAATCTCCAGTGCT TCAACGGGGGCCAGCAACGACAACCACTACTTCGGCTACAGCACCCCCTGGGGGTA TTTTGATTTCAACAGATTCCACTGCCATTTCTCACCACGTGACTGGCAGCGACTCATC AACAACAATTGGGGATTCCGGCCCAAGAGACTCAACTTCAAGCTCTTCAACATCCA AGTCAAGGAGGTCACGACGAATGATGGCGTCACGACCATCGCTAATAACCTTACCA GCACGGTTCAAGTCTTCTCGGACTCGGAGTACCAGTTGCCGTACGTCCTCGGCTCTG CGCACCAGGGCTGCCTCCCTCCGTTCCCGGCGGACGTGTTCATGATTCCGCAGTACG GCTACCTAACGCTCAACAATGGCAGCCAGGCAGTGGGACGGTCATCCTTTTACTGCC TGGAATATTTCCCATCGCAGATGCTGAGAACGGGCAATAACTTTACCTTCAGCTACA CCTTCGAGGACGTGCCTTTCCACAGCAGCTACGCGCACAGCCAGAGCCTGGACCGG CTGATGAATCCTCTCATCGACCAGTACCTGTATTACCTGAACAGAACTCAGAATCAG TCCGGAAGTGCCCAAAACAAGGACTTGCTGTTTAGCCGGGGGTCTCCAGCTGGCATG TCTGTTCAGCCCAAAAACTGGCTACCTGGACCCTGTTACCGGCAGCAGCGTTTCT AAAACAAAAACAGACAACAACAACAGCAACTTTACCTGGACTGGTGCTTCAAAATA TAACCTTAATGGGCGTGAATCTATAATCAACCCTGGCACTGCTATGGCCTCACACAA AGACGACAAAGACAAGTTCTTTCCCATGAGCGGTGTCATGATTTTGGAAAGGAGA GCGCCGGAGCTTCAAACACTGCATTGGACAATGTCATGATCACAGACGAAGAGGAA ATCAAAGCCACTAACCCCGTGGCCACCGAAAGATTTGGGACTGTGGCAGTCAATCT CCAGAGCAGCAGCACAGACCCTGCGACCGGAGATGTGCATGTTATGGGAGCCTTAC CTGGAATGGTGTGGCAAGACAGAGACGTATACCTGCAGGGTCCTATTTGGGCCAAA ATTCCTCACACGGATGGACACTTTCACCCGTCTCCTCTCATGGGCGGCTTTGGACTTA AGCACCCGCCTCCTCAGATCCTCATCAAAAACACGCCTGTTCCTGCGAATCCTCCGG CAGAGTTTTCGGCTACAAAGTTTGCTTCATTCATCACCCAGTATTCCACAGGACAAG TGAGCGTGGAGATTGAATGGGAGCTGCAGAAAGAAAACAGCAAACGCTGGAATCCC GAAGTGCAGTATACATCTAACTATGCAAAATCTGCCAACGTTGATTTCACTGTGGAC AACAATGGACTTTATACTGAGCCTCGCCCCATTGGCACCCGTTACCTCACCCGTCCC CTGTAA |
| **AAV6 - VP1 protein sequence (SEQ ID NO:2):** |
| MAADGYLPDWLEDNLSEGIREWWDLKPGAPKPKANQQKQDDGRGLVLPGYKYLGPF NGLDKGEPVNAADAAALEHDKAYDQQLKAGDNPYLRYNHADAEFQERLQEDTSFGGN LGRAVFQAKKRVLEPFGLVEEGAKTAPGKKRPVEQSPQEPDSSSGIGKTGQQPAKKRLN FGQTGDSESVPDPQPLGEPPATPAAVGPTTMASGGGAPMADNNEGADGVGNASGNWH CDSTWLGDRVITTSTRTWALPTYNNHLYKQISSASTGASNDNHYFGYSTPWGYFDFNRF HCHFSPRDWQRLINNNWGFRPKRLNFKLFNIQVKEVTTNDGVTTIANNLTSTVQVFSDS EYQLPYVLGSAHQGCLPPFPADVFMIPQYGYLTLNNGSQAVGRSSFYCLEYFPSQMLRT GNNFTFSYTFEDVPFHSSYAHSQSLDRLMNPLIDQYLYYLNRTQNQSGSAQNKDLLFSR GSPAGMSVQPKNWLPGPCYRQQRVSKTKTDNNNSNFTWTGASKYNLNGRESIINPGTA MASHKDDKDKFFPMSGVMIFGKESAGASNTALDNVMITDEEEIKATNPVATERFGTVA VNLQSSSTDPATGDVHVMGALPGMVWQDRDVYLQGPIWAKIPHTDGHFHPSPLMGGF GLKHPPPQILIKNTPVPANPPAEFSATKFASFITQYSTGQVSVEIEWELQKENSKRWNPEV QYTSNYAKSANVDFTVDNNGLYTEPRPIGTRYLTRPL* |
| **AAV6 - VP2 nucleotide sequence (SEQ ID NO:3):** |
| ACGGCTCCTGGAAAGAAACGTCCGGTAGAGCAGTCGCCACAAGAGCCAGACTCCTC CTCGGGCATTGGCAAGACAGGCCAGCAGCCCGCTAAAAAGAGACTCAATTTTGGTC AGACTGGCGACTCAGAGTCAGTCCCCGACCCACAACCTCTCGGAGAACCTCCAGCA ACCCCCGCTGCTGTGGGACCTACTACAATGGCTTCAGGCGGTGGCGCACCAATGGC AGACAATAACGAAGGCGCCGACGGAGTGGGTAATGCCTCAGGAAATTGGCATTGCG ATTCCACATGGCTGGGCGACAGAGTCATCACCACCAGCACCCGAACATGGGCCTTG CCCACCTATAACAACCACCTCTACAAGCAAATCTCCAGTGCTTCAACGGGGGCCAGC AACGACAACCACTACTTCGGCTACAGCACCCCCTGGGGGTATTTTGATTTCAACAGA TTCCACTGCCATTTCTCACCACGTGACTGGCAGCGACTCATCAACAACAATTGGGGA TTCCGGCCCAAGAGACTCAACTTCAAGCTCTTCAACATCCAAGTCAAGGAGGTCACG ACGAATGATGGCGTCACGACCATCGCTAATAACCTTACCAGCACGGTTCAAGTCTTC TCGGACTCGGAGTACCAGTTGCCGTACGTCCTCGGCTCTGCGCACCAGGGCTGCCTC CCTCCGTTCCCGGCGGACGTGTTCATGATTCCGCAGTACGGCTACCTAACGCTCAAC AATGGCAGCCAGGCAGTGGGACGGTCATCCTTTTACTGCCTGGAATATTTCCCATCG CAGATGCTGAGAACGGGCAATAACTTTACCTTCAGCTACACCTTCGAGGACGTGCCT TTCCACAGCAGCTACGCGCACAGCCAGAGCCTGGACCGGCTGATGAATCCTCTCATC GACCAGTACCTGTATTACCTGAACAGAACTCAGAATCAGTCCGGAAGTGCCCAAAA CAAGGACTTGCTGTTTAGCCGGGGGTCTCCAGCTGGCATGTCTGTTCAGCCCAAAAA CTGGCTACCTGGACCCTGTTACCGGCAGCAGCGCGTTTCTAAAACAAAAACAGACA ACAACAACAGCAACTTTACCTGGACTGGTGCTTCAAAATATAACCTTAATGGGCGTG AATCTATAATCAACCCTGGCACTGCTATGGCCTCACACAAAGACGACAAAGACAAG TTCTTTCCCATGAGCGGTGTCATGATTTTTGGAAAGGAGAGCGCCGGAGCTTCAAAC |
| ACTGCATTGGACAATGTCATGATCACAGACGAAGAGGAAATCAAAGCCACTAACCC CGTGGCCACCGAAAGATTTGGGACTGTGGCAGTCAATCTCCAGAGCAGCACAG ACCCTGCGACCGGAGATGTGCATGTTATGGGAGCCTTACCTGGAATGGTGTGGCAA GACAGAGACGTATACCTGCAGGGTCCTATTTGGGCCAAAATTCCTCACACGGATGG ACACTTTCACCCGTCTCCTCTCATGGGCGGCTTTGGACTTAAGCACCCGCCTCCTCAG ATCCTCATCAAAAACACGCCTGTTCCTGCGAATCCTCCGGCAGAGTTTTCGGCTACA AAGTTTGCTTCATTCATCACCCAGTATTCCACAGGACAAGTGAGCGTGGAGATTGAA TGGGAGCTGCAGAAAGAAAACAGCAAACGCTGGAATCCCGAAGTGCAGTATACATC TAACTATGCAAAATCTGCCAACGTTGATTTCACTGTGGACAACAATGGACTTTATAC TGAGCCTCGCCCCATTGGCACCCGTTACCTCACCCGTCCCCTGTAA |
| **AAV6 - VP2 protein sequence (SEQ ID NO:4):** |
| TAPGKKRPVEQSPQEPDSSSGIGKTGQQPAKKRLNFGQTGDSESVPDPQPLGEPPATPAA VGPTTMASGGGAPMADNNEGADGVGNASGNWHCDSTWLGDRVITTSTRTWALPTYN NHLYKQISSASTGASNDNHYFGYSTPWGYFDFNRFHCHFSPRDWQRLINNNWGFRPKR LNFKLFNIQVKEVTTNDGVTTIANNLTSTVQVFSDSEYQLPYVLGSAHQGCLPPFPADVF MIPQYGYLTLNNGSQAVGRSSFYCLEYFPSQMLRTGNNFTFSYTFEDVPFHSSYAHSQSL DRLMNPLIDQYLYYLNRTQNQSGSAQNKDLLFSRGSPAGMSVQPKNWLPGPCYRQQR VSKTKTDNNNSNFTWTGASKYNLNGRESIINPGTAMASHKDDKDKFFPMSGVMIFGKE SAGASNTALDNVMITDEEEIKATNPVATERFGTVAVNLQSSSTDPATGDVHVMGALPG MVWQDRDVYLQGPIWAKIPHTDGHFHPSPLMGGFGLKHPPPQILIKNTPVPANPPAEFS ATKFASFITQYSTGQVSVEIEWELQKENSKRWNPEVQYTSNYAKSANVDFTVDNNGLY TEPRPIGTRYLTRPL* |
| **AAV6 - VP3 nucleotide sequence (SEQ ID NO:5):** |
| ATGGCTTCAGGCGGTGGCGCACCAATGGCAGACAATAACGAAGGCGCCGACGGAGT GGGTAATGCCTCAGGAAATTGGCATTGCGATTCCACATGGCTGGGCGACAGAGTCA TCACCACCAGCACCCGAACATGGGCCTTGCCCACCTATAACAACCACCTCTACAAGC AAATCTCCAGTGCTTCAACGGGGGCCAGCAACGACAACCACTACTTCGGCTACAGC ACCCCCTGGGGGTATTTTGATTTCAACAGATTCCACTGCCATTTCTCACCACGTGACT GGCAGCGACTCATCAACAACAATTGGGGATTCCGGCCCAAGAGACTCAACTTCAAG CTCTTCAACATCCAAGTCAAGGAGGTCACGACGAATGATGGCGTCACGACCATCGC TAATAACCTTACCAGCACGGTTCAAGTCTTCTCGGACTCGGAGTACCAGTTGCCGTA CGTCCTCGGCTCTGCGCACCAGGGCTGCCTCCCTCCGTTCCCGGCGGACGTGTTCAT GATTCCGCAGTACGGCTACCTAACGCTCAACAATGGCAGCCAGGCAGTGGGACGGT CATCCTTTTACTGCCTGGAATATTTCCCATCGCAGATGCTGAGAACGGGCAATAACT TTACCTTCAGCTACACCTTCGAGGACGTGCCTTTCCACAGCAGCTACGCGCACAGCC |
| AGAGCCTGGACCGGCTGATGAATCCTCTCATCGACCAGTACCTGTATTACCTGAACA GAACTCAGAATCAGTCCGGAAGTGCCCAAAACAAGGACTTGCTGTTTAGCCGGGGG TCTCCAGCTGGCATGTCTGTTCAGCCCAAAAACTGGCTACCTGGACCCTGTTACCGG CAGCAGCGCGTTTCTAAAACAAAAACAGACAACAACAACAGCAACTTTACCTGGAC TGGTGCTTCAAAATATAACCTTAATGGGCGTGAATCTATAATCAACCCTGGCACTGC TATGGCCTCACACAAAGACGACAAAGACAAGTTCTTTCCCATGAGCGGTGTCATGAT TTTTGGAAAGGAGAGCGCCGGAGCTTCAAACACTGCATTGGACAATGTCATGATCA CAGACGAAGAGGAAATCAAAGCCACTAACCCCGTGGCCACCGAAAGATTTGGGACT GTGGCAGTCAATCTCCAGAGCAGCAGCACAGACCCTGCGACCGGAGATGTGCATGT TATGGGAGCCTTACCTGGAATGGTGTGGCAAGACAGAGACGTATACCTGCAGGGTC CTATTTGGGCCAAAATTCCTCACACGGATGGACACTTTCACCCGTCTCCTCTCATGG GCGGCTTTGGACTTAAGCACCCGCCTCCTCAGATCCTCATCAAAAACACGCCTGTTC CTGCGAATCCTCCGGCAGAGTTTTCGGCTACAAAGTTTGCTTCATTCATCACCCAGT ATTCCACAGGACAAGTGAGCGTGGAGATTGAATGGGAGCTGCAGAAAGAAAACAG CAAACGCTGGAATCCCGAAGTGCAGTATACATCTAACTATGCAAAATCTGCCAACGT TGATTTCACTGTGGACAACAATGGACTTTATACTGAGCCTCGCCCCATTGGCACCCG TTACCTCACCCGTCCCCTGTAA |
| **AAV6 - VP3 protein sequence (SEQ ID NO:6):** |
| MASGGGAPMADNNEGADGVGNASGNWHCDSTWLGDRVITTSTRTWALPTYNNHLYK QISSASTGASNDNHYFGYSTPWGYFDFNRFHCHFSPRDWQRLINNNWGFRPKRLNFKLF NIQVKEVTTNDGVTTIANNLTSTVQVFSDSEYQLPYVLGSAHQGCLPPFPADVFMIPQYG YLTLNNGSQAVGRSSFYCLEYFPSQMLRTGNNFTFSYTFEDVPFHSSYAHSQSLDRLMN PLIDQYLYYLNRTQNQSGSAQNKDLLFSRGSPAGMSVQPKNWLPGPCYRQQRVSKTKT DNNNSNFTWTGASKYNLNGRESIINPGTAMASHKDDKDKFFPMSGVMIFGKESAGASN TALDNVMITDEEEIKATNPVATERFGTVAVNLQSSSTDPATGDVHVMGALPGMVWQDR DVYLQGPIWAKIPHTDGHFHPSPLMGGFGLKHPPPQILIKNTPVPANPPAEFSATKFASFI TQYSTGQVSVEIEWELQKENSKRWNPEVQYTSNYAKSANVDFTVDNNGLYTEPRPIGT RYLTRPL* |

| **AAV7** |
|---|
| **AAV7 - VP1 nucleotide sequence (SEQ ID NO:7):** |
| ATGGCTGCCGATGGTTATCTTCCAGATTGGCTCGAGGACAACCTCTCTGAGGGCATT CGCGAGTGGTGGGACCTGAAACCTGGAGCCCCGAAACCCAAAGCCAACCAGCAAA AGCAGGACAACGGCCGGGGTCTGGTGCTTCCTGGCTACAAGTACCTCGGACCCTTCA ACGGACTCGACAAGGGGGAGCCCGTCAACGCGGCGGACGCAGCGGCCCTCGAGCA CGACAAGGCCTACGACCAGCAGCTCAAAGCGGGTGACAATCCGTACCTGCGGTATA ACCACGCCGACGCCGAGTTTCAGGAGCGTCTGCAAGAAGATACGTCATTTGGGGGC AACCTCGGGCGAGCAGTCTTCCAGGCCAAGAAGCGGGTTCTCGAACCTCTCGGTCTG GTTGAGGAAGGCGCTAAGACGGCTCCTGCAAAGAAGAGACCGGTAGAGCCGTCACC TCAGCGTTCCCCCGACTCCTCCACGGGCATCGGCAAGAAAGGCCAGCAGCCCGCCA GAAAGAGACTCAATTTCGGTCAGACTGGCGACTCAGAGTCAGTCCCCGACCCTCAA CCTCTCGGAGAACCTCCAGCAGCGCCCTCTAGTGTGGGATCTGGTACAGTGGCTGCA GGCGGTGGCGCACCAATGGCAGACAATAACGAAGGTGCCGACGGAGTGGGTAATGC CTCAGGAAATTGGCATTGCGATTCCACATGGCTGGGCGACAGAGTCATTACCACCAG CACCCGAACCTGGGCCCTGCCCACCTACAACAACCACCTCTACAAGCAAATCTCCAG TGAAACTGCAGGTAGTACCAACGACAACACCTACTTCGGCTACAGCACCCCCTGGG GGTATTTTGACTTTAACAGATTCCACTGCCACTTCTCACCACGTGACTGGCAGCGAC TCATCAACAACAACTGGGGATTCCGGCCCAAGAAGCTGCGGTTCAAGCTCTTCAAC ATCCAGGTCAAGGAGGTCACGACGAATGACGGCGTTACGACCATCGCTAATAACCT TACCAGCACGATTCAGGTATTCTCGGACTCGGAATACCAGCTGCCGTACGTCCTCGG CTCTGCGCACCAGGGCTGCCTGCCTCCGTTCCCGGCGGACGTCTTCATGATTCCTCA GTACGGCTACCTGACTCTCAACAATGGCAGTCAGTCTGTGGGACGTTCCTCCTTCTA CTGCCTGGAGTACTTCCCCTCTCAGATGCTGAGAACGGGCAACAACTTTGAGTTCAG CTACAGCTTCGAGGACGTGCCTTTCCACAGCAGCTACGCACACAGCCAGAGCCTGG ACCGGCTGATGAATCCCCTCATCGACCAGTACTTGTACTACCTGGCCAGAACACAGA GTAACCCAGGAGGCACAGCTGGCAATCGGGAACTGCAGTTTTACCAGGGCGGGCCT TCAACTATGGCCGAACAAGCCAAGAATTGGTTACCTGGACCTTGCTTCCGGCAACAA AGAGTCTCCAAAACGCTGGATCAAAACAACAACAGCAACTTTGCTTGGACTGGTGC CACCAAATATCACCTGAACGGCAGAAACTCGTTGGTTAATCCCGGCGTCGCCATGGC AACTCACAAGGACGACGAGGACCGCTTTTTCCCATCCAGCGGAGTCCTGATTTTTGG AAAAACTGGAGCAACTAACAAAACTACATTGGAAAATGTGTTAATGACAAATGAAG AAGAAATTCGTCCTACTAATCCTGTAGCCACGGAAGAATACGGGATAGTCAGCAGC AACTTACAAGCGGCTAATACTGCAGCCCAGACACAAGTTGTCAACAACCAGGGAGC CTTACCTGGCATGGTCTGGCAGAACCGGGACGTGTACCTGCAGGGTCCCATCTGGGC CAAGATTCCTCACACGGATGGCAACTTTCACCCGTCTCCTTTGATGGGCGGCTTTGG ACTTAAACATCCGCCTCCTCAGATCCTGATCAAGAACACTCCCGTTCCCGCTAATCC TCCGGAGGTGTTTACTCCTGCCAAGTTTGCTTCGTTCATCACACAGTACAGCACCGG |
| ACAAGTCAGCGTGGAAATCGAGTGGGAGCTGCAGAAGGAAAACAGCAAGCGCTGG AACCCGGAGATTCAGTACACCTCCAACTTTGAAAAGCAGACTGGTGTGGACTTTGCC GTTGACAGCCAGGGTGTTTACTCTGAGCCTCGCCCTATTGGCACTCGTTACCTCACC CGTAATCTGTAA |
| **AAV7 - VP1 protein sequence (SEQ ID NO:8):** |
| MAADGYLPDWLEDNLSEGIREWWDLKPGAPKPKANQQKQDNGRGLVLPGYKYLGPF NGLDKGEPVNAADAAALEHDKAYDQQLKAGDNPYLRYNHADAEFQERLQEDTSFGGN LGRAVFQAKKRVLEPLGLVEEGAKTAPAKKRPVEPSPQRSPDSSTGIGKKGQQPARKRL NFGQTGDSESVPDPQPLGEPPAAPSSVGSGTVAAGGGAPMADNNEGADGVGNASGNW HCDSTWLGDRVITTSTRTWALPTYNNHLYKQISSETAGSTNDNTYFGYSTPWGYFDFNR FHCHFSPRDWQRLINNNWGFRPKKLRFKLFNIQVKEVTTNDGVTTIANNLTSTIQVFSDS EYQLPYVLGSAHQGCLPPFPADVFMIPQYGYLTLNNGSQSVGRSSFYCLEYFPSQMLRT GNNFEFSYSFEDVPFHSSYAHSQSLDRLMNPLIDQYLYYLARTQSNPGGTAGNRELQFY QGGPSTMAEQAKNWLPGPCFRQQRVSKTLDQNNNSNFAWTGATKYHLNGRNSLVNPG VAMATHKDDEDRFFPSSGVLIFGKTGATNKTTLENVLMTNEEEIRPTNPVATEEYGIVSS NLQAANTAAQTQVVNNQGALPGMVWQNRDVYLQGPIWAKIPHTDGNFHPSPLMGGFG LKHPPPQILIKNTPVPANPPEVFTPAKFASFITQYSTGQVSVEIEWELQKENSKRWNPEIQ YTSNFEKQTGVDFAVDSQGVYSEPRPIGTRYLTRNL* |
| **AAV7 - VP2 nucleotide sequence (SEQ ID NO:9):** |
| ACGGCTCCTGCAAAGAAGAGACCGGTAGAGCCGTCACCTCAGCGTTCCCCCGACTC CTCCACGGGCATCGGCAAGAAAGGCCAGCAGCCCGCCAGAAAGAGACTCAATTTCG GTCAGACTGGCGACTCAGAGTCAGTCCCCGACCCTCAACCTCTCGGAGAACCTCCAG CAGCGCCCTCTAGTGTGGGATCTGGTACAGTGGCTGCAGGCGGTGGCGCACCAATG GCAGACAATAACGAAGGTGCCGACGGAGTGGGTAATGCCTCAGGAAATTGGCATTG CGATTCCACATGGCTGGGCGACAGAGTCATTACCACCAGCACCCGAACCTGGGCCC TGCCCACCTACAACAACCACCTCTACAAGCAAATCTCCAGTGAAACTGCAGGTAGT ACCAACGACAACACCTACTTCGGCTACAGCACCCCCTGGGGGTATTTTGACTTTAAC AGATTCCACTGCCACTTCTCACCACGTGACTGGCAGCGACTCATCAACAACAACTGG GGATTCCGGCCCAAGAAGCTGCGGTTCAAGCTCTTCAACATCCAGGTCAAGGAGGT CACGACGAATGACGGCGTTACGACCATCGCTAATAACCTTACCAGCACGATTCAGG TATTCTCGGACTCGGAATACCAGCTGCCGTACGTCCTCGGCTCTGCGCACCAGGGCT GCCTGCCTCCGTTCCCGGCGGACGTCTTCATGATTCCTCAGTACGGCTACCTGACTCT CAACAATGGCAGTCAGTCTGTGGGACGTTCCTCCTTCTACTGCCTGGAGTACTTCCC CTCTCAGATGCTGAGAACGGGCAACAACTTTGAGTTCAGCTACAGCTTCGAGGACGT GCCTTTCCACAGCAGCTACGCACACAGCCAGAGCCTGGACCGGCTGATGAATCCCCT |
| CATCGACCAGTACTTGTACTACCTGGCCAGAACACAGAGTAACCCAGGAGGCACAG CTGGCAATCGGGAACTGCAGTTTACCAGGGCGGGCCTTCAACTATGGCCGAACAA GCCAAGAATTGGTTACCTGGACCTTGCTTCCGGCAACAAAGAGTCTCCAAAACGCTG GATCAAAACAACAACAGCAACTTTGCTTGGACTGGTGCCACCAAATATCACCTGAA CGGCAGAAACTCGTTGGTTAATCCCGGCGTCGCCATGGCAACTCACAAGGACGACG AGGACCGCTTTTTCCCATCCAGCGGAGTCCTGATTTTTGGAAAAACTGGAGCAACTA ACAAAACTACATTGGAAAATGTGTTAATGACAAATGAAGAAGAAATTCGTCCTACT AATCCTGTAGCCACGGAAGAATACGGGATAGTCAGCAGCAACTTACAAGCGGCTAA TACTGCAGCCCAGACACAAGTTGTCAACAACCAGGGAGCCTTACCTGGCATGGTCT GGCAGAACCGGGACGTGTACCTGCAGGGTCCCATCTGGGCCAAGATTCCTCACACG GATGGCAACTTTCACCCGTCTCCTTTGATGGGCGGCTTTGGACTTAAACATCCGCCTC CTCAGATCCTGATCAAGAACACTCCCGTTCCCGCTAATCCTCCGGAGGTGTTTACTC CTGCCAAGTTTGCTTCGTTCATCACACAGTACAGCACCGGACAAGTCAGCGTGGAAA TCGAGTGGGAGCTGCAGAAGGAAAACAGCAAGCGCTGGAACCCGGAGATTCAGTAC ACCTCCAACTTTGAAAAGCAGACTGGTGTGGACTTTGCCGTTGACAGCCAGGGTGTT TACTCTGAGCCTCGCCCTATTGGCACTCGTTACCTCACCCGTAATCTGTAA |
| **AAV7 - VP2 protein sequence (SEQ ID NO:10):** |
| TAPAKKRPVEPSPQRSPDSSTGIGKKGQQPARKRLNFGQTGDSESVPDPQPLGEPPAAPS SVGSGTVAAGGGAPMADNNEGADGVGNASGNWHCDSTWLGDRVITTSTRTWALPTY NNHLYKQISSETAGSTNDNTYFGYSTPWGYFDFNRFHCHFSPRDWQRLINNNWGFRPK KLRFKLFNIQVKEVTTNDGVTTIANNLTSTIQVFSDSEYQLPYVLGSAHQGCLPPFPADV FMIPQYGYLTLNNGSQSVGRSSFYCLEYFPSQMLRTGNNFEFSYSFEDVPFHSSYAHSQS LDRLMNPLIDQYLYYLARTQSNPGGTAGNRELQFYQGGPSTMAEQAKNWLPGPCFRQQ RVSKTLDQNNNSNFAWTGATKYHLNGRNSLVNPGVAMATHKDDEDRFFPSSGVLIFGK TGATNKTTLENVLMTNEEEIRPTNPVATEEYGIVSSNLQAANTAAQTQVVNNQGALPG MVWQNRDVYLQGPIWAKIPHTDGNFHPSPLMGGFGLKHPPPQILIKNTPVPANPPEVFTP AKFASFITQYSTGQVSVEIEWELQKENSKRWNPEIQYTSNFEKQTGVDFAVDSQGVYSE PRPIGTRYLTRNL* |
| **AAV7 - VP3 nucleotide sequence (SEQ ID NO:11):** |
| GTGGCTGCAGGCGGTGGCGCACCAATGGCAGACAATAACGAAGGTGCCGACGGAGT GGGTAATGCCTCAGGAAATTGGCATTGCGATTCCACATGGCTGGGCGACAGAGTCA TTACCACCAGCACCCGAACCTGGGCCCTGCCCACCTACAACAACCACCTCTACAAGC AAATCTCCAGTGAAACTGCAGGTAGTACCAACGACAACACCTACTTCGGCTACAGC ACCCCCTGGGGGTATTTTGACTTTAACAGATTCCACTGCCACTTCTCACCACGTGACT GGCAGCGACTCATCAACAACAACTGGGGATTCCGGCCCAAGAAGCTGCGGTTCAAG |
| CTCTTCAACATCCAGGTCAAGGAGGTCACGACGAATGACGGCGTTACGACCATCGC TAATAACCTTACCAGCACGATTCAGGTATTCTCGGACTCGGAATACCAGCTGCCGTA CGTCCTCGGCTCTGCGCACCAGGGCTGCCTGCCTCCGTTCCCGGCGGACGTCTTCAT GATTCCTCAGTACGGCTACCTGACTCTCAACAATGGCAGTCAGTCTGTGGGACGTTC CTCCTTCTACTGCCTGGAGTACTTCCCCTCTCAGATGCTGAGAACGGGCAACAACTT TGAGTTCAGCTACAGCTTCGAGGACGTGCCTTTCCACAGCAGCTACGCACACAGCCA GAGCCTGGACCGGCTGATGAATCCCCTCATCGACCAGTACTTGTACTACCTGGCCAG AACACAGAGTAACCCAGGAGGCACAGCTGGCAATCGGGAACTGCAGTTTTACCAGG GCGGGCCTTCAACTATGGCCGAACAAGCCAAGAATTGGTTACCTGGACCTTGCTTCC GGCAACAAAGAGTCTCCAAAACGCTGGATCAAAACAACAACAGCAACTTTGCTTGG ACTGGTGCCACCAAATATCACCTGAACGGCAGAAACTCGTTGGTTAATCCCGGCGTC GCCATGGCAACTCACAAGGACGACGAGGACCGCTTTTTCCCATCCAGCGGAGTCCT GATTTTTGGAAAAACTGGAGCAACTAACAAAACTACATTGGAAAATGTGTTAATGA CAAATGAAGAAGAAATTCGTCCTACTAATCCTGTAGCCACGGAAGAATACGGGATA GTCAGCAGCAACTTACAAGCGGCTAATACTGCAGCCCAGACACAAGTTGTCAACAA CCAGGGAGCCTTACCTGGCATGGTCTGGCAGAACCGGGACGTGTACCTGCAGGGTC CCATCTGGGCCAAGATTCCTCACACGGATGGCAACTTTCACCCGTCTCCTTTGATGG GCGGCTTTGGACTTAAACATCCGCCTCCTCAGATCCTGATCAAGAACACTCCCGTTC CCGCTAATCCTCCGGAGGTGTTTACTCCTGCCAAGTTTGCTTCGTTCATCACACAGTA CAGCACCGGACAAGTCAGCGTGGAAATCGAGTGGGAGCTGCAGAAGGAAAACAGC AAGCGCTGGAACCCGGAGATTCAGTACACCTCCAACTTTGAAAAGCAGACTGGTGT GGACTTTGCCGTTGACAGCCAGGGTGTTTACTCTGAGCCTCGCCCTATTGGCACTCG TTACCTCACCCGTAATCTGTAA |
| **AAV7 - VP3 protein sequence (SEQ ID NO:12):** |
| VAAGGGAPMADNNEGADGVGNASGNWHCDSTWLGDRVITTSTRTWALPTYNNHLYK QISSETAGSTNDNTYFGYSTPWGYFDFNRFHCHFSPRDWQRLINNNWGFRPKKLRFKLF NIQVKEVTTNDGVTTIANNLTSTIQVFSDSEYQLPYVLGSAHQGCLPPFPADVFMIPQYG YLTLNNGSQSVGRSSFYCLEYFPSQMLRTGNNFEFSYSFEDVPFHSSYAHSQSLDRLMNP LIDQYLYYLARTQSNPGGTAGNRELQFYQGGPSTMAEQAKNWLPGPCFRQQRVSKTLD QNNNSNFAWTGATKYHLNGRNSLVNPGVAMATHKDDEDRFFPSSGVLIFGKTGATNK TTLENVLMTNEEEIRPTNPVATEEYGIVSSNLQAANTAAQTQVVNNQGALPGMVWQNR DVYLQGPIWAKIPHTDGNFHPSPLMGGFGLKHPPPQILIKNTPVPANPPEVFTPAKFASFI TQYSTGQVSVEIEWELQKENSKRWNPEIQYTSNFEKQTGVDFAVDSQGVYSEPRPIGTR YLTRNL* |

| **AAV8** |
|---|
| **AAV8 - VP1 nucleotide sequence (SEQ ID NO:13):** |
| ATGGCTGCCGATGGTTATCTTCCAGATTGGCTCGAGGACAACCTCTCTGAGGGCATT CGCGAGTGGTGGGCGCTGAAACCTGGAGCCCCGAAGCCCAAAGCCAACCAGCAAA AGCAGGACGACGGCCGGGGTCTGGTGCTTCCTGGCTACAAGTACCTCGGACCCTTCA ACGGACTCGACAAGGGGGAGCCCGTCAACGCGGCGGACGCAGCGGCCCTCGAGCA CGACAAGGCCTACGACCAGCAGCTGCAGGCGGGTGACAATCCGTACCTGCGGTATA ACCACGCCGACGCCGAGTTTCAGGAGCGTCTGCAAGAAGATACGTCTTTTGGGGGC AACCTCGGGCGAGCAGTCTTCCAGGCCAAGAAGCGGGTTCTCGAACCTCTCGGTCTG GTTGAGGAAGGCGCTAAGACGGCTCCTGGAAAGAAGAGACCGGTAGAGCCATCACC CCAGCGTTCTCCAGACTCCTCTACGGGCATCGGCAAGAAAGGCCAACAGCCCGCCA GAAAAAGACTCAATTTTGGTCAGACTGGCGACTCAGAGTCAGTTCCAGACCCTCAAC CTCTCGGAGAACCTCCAGCAGCGCCCTCTGGTGTGGGACCTAATACAATGGCTGCAG GCGGTGGCGCACCAATGGCAGACAATAACGAAGGCGCCGACGGAGTGGGTAGTTCC TCGGGAAATTGGCATTGCGATTCCACATGGCTGGGCGACAGAGTCATCACCACCAG CACCCGAACCTGGGCCCTGCCCACCTACAACAACCACCTCTACAAGCAAATCTCCAA CGGGACATCGGGAGGAGCCACCAACGACAACACCTACTTCGGCTACAGCACCCCCT GGGGGTATTTTGACTTTAACAGATTCCACTGCCACTTTTCACCACGTGACTGGCAGC GACTCATCAACAACAACTGGGGATTCCGGCCCAAGAGACTCAGCTTCAAGCTCTTCA ACATCCAGGTCAAGGAGGTCACGCAGAATGAAGGCACCAAGACCATCGCCAATAAC CTCACCAGCACCATCCAGGTGTTTACGGACTCGGAGTACCAGCTGCCGTACGTTCTC GGCTCTGCCCACCAGGGCTGCCTGCCTCCGTTCCCGGCGGACGTGTTCATGATTCCC CAGTACGGCTACCTAACACTCAACAACGGTAGTCAGGCCGTGGGACGCTCCTCCTTC TACTGCCTGGAATACTTTCCTTCGCAGATGCTGAGAACCGGCAACAACTTCCAGTTT ACTTACACCTTCGAGGACGTGCCTTTCCACAGCAGCTACGCCCACAGCCAGAGCTTG GACCGGCTGATGAATCCTCTGATTGACCAGTACCTGTACTACTTGTCTCGGACTCAA ACAACAGGAGGCACGGCAAATACGCAGACTCTGGGCTTCAGCCAAGGTGGGCCTAA TACAATGGCCAATCAGGCAAAGAACTGGCTGCCAGGACCCTGTTACCGCCAACAAC GCGTCTCAACGACAACCGGGCAAAACAACAATAGCAACTTTGCCTGGACTGCTGGG ACCAAATACCATCTGAATGGAAGAAATTCATTGGCTAATCCTGGCATCGCTATGGCA ACACACAAAGACGACGAGGAGCGTTTTTTCCCAGTAACGGGATCCTGATTTTTGGC AAACAAAATGCTGCCAGAGACAATGCGGATTACAGCGATGTCATGCTCACCAGCGA GGAAGAAATCAAAACCACTAACCCTGTGGCTACAGAGGAATACGGTATCGTGGCAG ATAACTTGCAGCAGCAAAACACGGCTCCTCAAATTGGAACTGTCAACAGCCAGGGG GCCTTACCCGGTATGGTCTGGCAGAACCGGGACGTGTACCTGCAGGGTCCCATCTGG GCCAAGATTCCTCACACGGACGGCAACTTCCACCCGTCTCCGCTGATGGGCGGCTTT GGCCTGAAACATCCTCCGCCTCAGATCCTGATCAAGAACACGCCTGTACCTGCGGAT CCTCCGACCACCTTCAACCAGTCAAAGCTGAACTCTTTCATCACGCAATACAGCACC |
| GGACAGGTCAGCGTGGAAATTGAATGGGAGCTGCAGAAGGAAAACAGCAAGCGCT GGAACCCCGAGATCCAGTACACCTCCAACTACTACAAATCTACAAGTGTGGACTTTG CTGTTAATACAGAAGGCGTGTACTCTGAACCCCGCCCCATTGGCACCCGTTACCTCA CCCGTAATCTGTAA |
| **AAV8 - VP1 protein sequence (SEQ ID NO:14):** |
| MAADGYLPDWLEDNLSEGIREWWALKPGAPKPKANQQKQDDGRGLVLPGYKYLGPF NGLDKGEPVNAADAAALEHDKAYDQQLQAGDNPYLRYNHADAEFQERLQEDTSFGGN LGRAVFQAKKRVLEPLGLVEEGAKTAPGKKRPVEPSPQRSPDSSTGIGKKGQQPARKRL NFGQTGDSESVPDPQPLGEPPAAPSGVGPNTMAAGGGAPMADNNEGADGVGSSSGNW HCDSTWLGDRVITTSTRTWALPTYNNHLYKQISNGTSGGATNDNTYFGYSTPWGYFDF NRFHCHFSPRDWQRLINNNWGFRPKRLSFKLFNIQVKEVTQNEGTKTIANNLTSTIQVFT DSEYQLPYVLGSAHQGCLPPFPADVFMIPQYGYLTLNNGSQAVGRSSFYCLEYFPSQML RTGNNFQFTYTFEDVPFHSSYAHSQSLDRLMNPLIDQYLYYLSRTQTTGGTANTQTLGF SQGGPNTMANQAKNWLPGPCYRQQRVSTTTGQNNNSNFAWTAGTKYHLNGRNSLAN PGIAMATHKDDEERFFPSNGILIFGKQNAARDNADYSDVMLTSEEEIKTTNPVATEEYGI VADNLQQQNTAPQIGTVNSQGALPGMVWQNRDVYLQGPIWAKIPHTDGNFHPSPLMG GFGLKHPPPQILIKNTPVPADPPTTFNQSKLNSFITQYSTGQVSVEIEWELQKENSKRWNP EIQYTSNYYKSTSVDFAVNTEGVYSEPRPIGTRYLTRNL* |
| **AAV8 - VP2 nucleotide sequence (SEQ ID NO:15):** |
| ACGGCTCCTGGAAAGAAGAGACCGGTAGAGCCATCACCCCAGCGTTCTCCAGACTC CTCTACGGGCATCGGCAAGAAAGGCCAACAGCCCGCCAGAAAAAGACTCAATTTG GTCAGACTGGCGACTCAGAGTCAGTTCCAGACCCTCAACCTCTCGGAGAACCTCCAG CAGCGCCCTCTGGTGTGGGACCTAATACAATGGCTGCAGGCGGTGGCGCACCAATG GCAGACAATAACGAAGGCGCCGACGGAGTGGGTAGTTCCTCGGGAAATTGGCATTG CGATTCCACATGGCTGGGCGACAGAGTCATCACCACCAGCACCCGAACCTGGGCCC TGCCCACCTACAACAACCACCTCTACAAGCAAATCTCCAACGGACATCGGGAGGA GCCACCAACGACAACACCTACTTCGGCTACAGCACCCCCTGGGGGTATTTTGACTTT AACAGATTCCACTGCCACTTTTCACCACGTGACTGGCAGCGACTCATCAACAACAAC TGGGGATTCCGGCCCAAGAGACTCAGCTTCAAGCTCTTCAACATCCAGGTCAAGGA GGTCACGCAGAATGAAGGCACCAAGACCATCGCCAATAACCTCACCAGCACCATCC AGGTGTTTACGGACTCGGAGTACCAGCTGCCGTACGTTCTCGGCTCTGCCCACCAGG GCTGCCTGCCTCCGTTCCCGGCGGACGTGTTCATGATTCCCCAGTACGGCTACCTAA CACTCAACAACGGTAGTCAGGCCGTGGGACGCTCCTCCTTCTACTGCCTGGAATACT TTCCTTCGCAGATGCTGAGAACCGGCAACAACTTCCAGTTTACTTACACCTTCGAGG ACGTGCCTTTCCACAGCAGCTACGCCCACAGCCAGAGCTTGGACCGGCTGATGAATC |
| CTCTGATTGACCAGTACCTGTACTACTTGTCTCGGACTCAAACAACAGGAGGCACGG CAAATACGCAGACTCTGGGCTTCAGCCAAGGTGGGCCTAATACAATGGCCAATCAG GCAAAGAACTGGCTGCCAGGACCCTGTTACCGCCAACAACGCGTCTCAACGACAAC CGGGCAAAACAACAATAGCAACTTTGCCTGGACTGCTGGGACCAAATACCATCTGA ATGGAAGAAATTCATTGGCTAATCCTGGCATCGCTATGGCAACACACAAAGACGAC GAGGAGCGTTTTTTCCCAGTAACGGGATCCTGATTTTGGCAAACAAAATGCTGCC AGAGACAATGCGGATTACAGCGATGTCATGCTCACCAGCGAGGAAGAAATCAAAAC CACTAACCCTGTGGCTACAGAGGAATACGGTATCGTGGCAGATAACTTGCAGCAGC AAAACACGGCTCCTCAAATTGGAACTGTCAACAGCCAGGGGGCCTTACCCGGTATG GTCTGGCAGAACCGGGACGTGTACCTGCAGGGTCCCATCTGGGCCAAGATTCCTCAC ACGGACGGCAACTTCCACCCGTCTCCGCTGATGGGCGGCTTTGGCCTGAAACATCCT CCGCCTCAGATCCTGATCAAGAACACGCCTGTACCTGCGGATCCTCCGACCACCTTC AACCAGTCAAAGCTGAACTCTTTCATCACGCAATACAGCACCGGACAGGTCAGCGT GGAAATTGAATGGGAGCTGCAGAAGGAAAACAGCAAGCGCTGGAACCCCGAGATC CAGTACACCTCCAACTACTACAAATCTACAAGTGTGGACTTTGCTGTTAATACAGAA GGCGTGTACTCTGAACCCCGCCCCATTGGCACCCGTTACCTCACCCGTAATCTGTAA |
| **AAV8 - VP2 protein sequence (SEQ ID NO:16):** |
| TAPGKKRPVEPSPQRSPDSSTGIGKKGQQPARKRLNFGQTGDSESVPDPQPLGEPPAAPS GVGPNTMAAGGGAPMADNNEGADGVGSSSGNWHCDSTWLGDRVITTSTRTWALPTY NNHLYKQISNGTSGGATNDNTYFGYSTPWGYFDFNRFHCHFSPRDWQRLINNNWGFRP KRLSFKLFNIQVKEVTQNEGTKTIANNLTSTIQVFTDSEYQLPYVLGSAHQGCLPPFPAD VFMIPQYGYLTLNNGSQAVGRSSFYCLEYFPSQMLRTGNNFQFTYTFEDVPFHSSYAHS QSLDRLMNPLIDQYLYYLSRTQTTGGTANTQTLGFSQGGPNTMANQAKNWLPGPCYRQ QRVSTTTGQNNNSNFAWTAGTKYHLNGRNSLANPGIAMATHKDDEERFFPSNGILIFGK QNAARDNADYSDVMLTSEEEIKTTNPVATEEYGIVADNLQQQNTAPQIGTVNSQGALPG MVWQNRDVYLQGPIWAKIPHTDGNFHPSPLMGGFGLKHPPPQILIKNTPVPADPPTTFN QSKLNSFITQYSTGQVSVEIEWELQKENSKRWNPEIQYTSNYYKSTSVDFAVNTEGVYSE PRPIGTRYLTRNL* |
| **AAV8 - VP3 nucleotide sequence (SEQ ID NO:17):** |
| ATGGCTGCAGGCGGTGGCGCACCAATGGCAGACAATAACGAAGGCGCCGACGGAG TGGGTAGTTCCTCGGGAAATTGGCATTGCGATTCCACATGGCTGGGCGACAGAGTCA TCACCACCAGCACCCGAACCTGGGCCCTGCCCACCTACAACAACCACCTCTACAAGC AAATCTCCAACGGGACATCGGGAGGAGCCACCAACGACAACACCTACTTCGGCTAC AGCACCCCCTGGGGGTATTTTGACTTTAACAGATTCCACTGCCACTTTTCACCACGTG ACTGGCAGCGACTCATCAACAACAACTGGGGATTCCGGCCCAAGAGACTCAGCTTC |
| AAGCTCTTCAACATCCAGGTCAAGGAGGTCACGCAGAATGAAGGCACCAAGACCAT CGCCAATAACCTCACCAGCACCATCCAGGTGTTTACGGACTCGGAGTACCAGCTGCC GTACGTTCTCGGCTCTGCCCACCAGGGCTGCCTGCCTCCGTTCCCGGCGGACGTGTT CATGATTCCCCAGTACGGCTACCTAACACTCAACAACGGTAGTCAGGCCGTGGGAC GCTCCTCCTTCTACTGCCTGGAATACTTTCCTTCGCAGATGCTGAGAACCGGCAACA ACTTCCAGTTTACTTACACCTTCGAGGACGTGCCTTTCCACAGCAGCTACGCCCACA GCCAGAGCTTGGACCGGCTGATGAATCCTCTGATTGACCAGTACCTGTACTACTTGT CTCGGACTCAAACAACAGGAGGCACGGCAAATACGCAGACTCTGGGCTTCAGCCAA GGTGGGCCTAATACAATGGCCAATCAGGCAAAGAACTGGCTGCCAGGACCCTGTTA CCGCCAACAACGCGTCTCAACGACAACCGGGCAAAACAACAATAGCAACTTTGCCT GGACTGCTGGGACCAAATACCATCTGAATGGAAGAAATTCATTGGCTAATCCTGGC ATCGCTATGGCAACACACAAAGACGACGAGGAGCGTTTTTTCCCAGTAACGGGAT CCTGATTTTTGGCAAACAAAATGCTGCCAGAGACAATGCGGATTACAGCGATGTCAT GCTCACCAGCGAGGAAGAAATCAAAACCACTAACCCTGTGGCTACAGAGGAATACG GTATCGTGGCAGATAACTTGCAGCAGCAAAACACGGCTCCTCAAATTGGAACTGTC AACAGCCAGGGGGCCTTACCCGGTATGGTCTGGCAGAACCGGGACGTGTACCTGCA GGGTCCCATCTGGGCCAAGATTCCTCACACGGACGGCAACTTCCACCCGTCTCCGCT GATGGGCGGCTTTGGCCTGAAACATCCTCCGCCTCAGATCCTGATCAAGAACACGCC TGTACCTGCGGATCCTCCGACCACCTTCAACCAGTCAAAGCTGAACTCTTTCATCAC GCAATACAGCACCGGACAGGTCAGCGTGGAAATTGAATGGGAGCTGCAGAAGGAA AACAGCAAGCGCTGGAACCCCGAGATCCAGTACACCTCCAACTACTACAAATCTAC AAGTGTGGACTTTGCTGTTAATACAGAAGGCGTGTACTCTGAACCCCGCCCCATTGG CACCCGTTACCTCACCCGTAATCTGTAA |
| **AAV8 - VP3 protein sequence (SEQ ID NO:18):** |
| MAAGGGAPMADNNEGADGVGSSSGNWHCDSTWLGDRVITTSTRTWALPTYNNHLYK QISNGTSGGATNDNTYFGYSTPWGYFDFNRFHCHFSPRDWQRLINNNWGFRPKRLSFKL FNIQVKEVTQNEGTKTIANNLTSTIQVFTDSEYQLPYVLGSAHQGCLPPFPADVFMIPQY GYLTLNNGSQAVGRSSFYCLEYFPSQMLRTGNNFQFTYTFEDVPFHSSYAHSQSLDRLM NPLIDQYLYYLSRTQTTGGTANTQTLGFSQGGPNTMANQAKNWLPGPCYRQQRVSTTT GQNNNSNFAWTAGTKYHLNGRNSLANPGIAMATHKDDEERFFPSNGILIFGKQNAARD NADYSDVMLTSEEEIKTTNPVATEEYGIVADNLQQQNTAPQIGTVNSQGALPGMVWQN RDVYLQGPIWAKIPHTDGNFHPSPLMGGFGLKHPPPQILIKNTPVPADPPTTFNQSKLNSF ITQYSTGQVSVEIEWELQKENSKRWNPEIQYTSNYYKSTSVDFAVNTEGVYSEPRPIGTR YLTRNL* |

| **AAV1** |
|---|
| **AAV1 - VP1 nucleotide sequence (SEQ ID NO:19):** |
| ATGGCTGCCGATGGTTATCTTCCAGATTGGCTCGAGGACAACCTCTCTGAGGGCATT CGCGAGTGGTGGGACTTGAAACCTGGAGCCCCGAAGCCCAAAGCCAACCAGCAAAA GCAGGACGACGGCCGGGGTCTGGTGCTTCCTGGCTACAAGTACCTCGGACCCTTCAA CGGACTCGACAAGGGGGAGCCCGTCAACGCGGCGGACGCAGCGGCCCTCGAGCAC GACAAGGCCTACGACCAGCAGCTCAAAGCGGGTGACAATCCGTACCTGCGGTATAA CCACGCCGACGCCGAGTTTCAGGAGCGTCTGCAAGAAGATACGTCTTTTGGGGGCA ACCTCGGGCGAGCAGTCTTCCAGGCCAAGAAGCGGGTTCTCGAACCTCTCGGTCTGG TTGAGGAAGGCGCTAAGACGGCTCCTGGAAAGAAACGTCCGGTAGAGCAGTCGCCA CAAGAGCCAGACTCCTCCTCGGGCATCGGCAAGACAGGCCAGCAGCCCGCTAAAAA GAGACTCAATTTTGGTCAGACTGGCGACTCAGAGTCAGTCCCCGATCCACAACCTCT CGGAGAACCTCCAGCAACCCCCGCTGCTGTGGGACCTACTACAATGGCTTCAGGCG GTGGCGCACCAATGGCAGACAATAACGAAGGCGCCGACGGAGTGGGTAATGCCTCA GGAAATTGGCATTGCGATTCCACATGGCTGGGCGACAGAGTCATCACCACCAGCAC CCGCACCTGGGCCTTGCCCACCTACAATAACCACCTCTACAAGCAAATCTCCAGTGC TTCAACGGGGGCCAGCAACGACAACCACTACTTCGGCTACAGCACCCCCTGGGGGT ATTTTGATTTCAACAGATTCCACTGCCACTTTTCACCACGTGACTGGCAGCGACTCAT CAACAACAATTGGGGATTCCGGCCCAAGAGACTCAACTTCAAACTCTTCAACATCCA AGTCAAGGAGGTCACGACGAATGATGGCGTCACAACCATCGCTAATAACCTTACCA GCACGGTTCAAGTCTTCTCGGACTCGGAGTACCAGCTTCCGTACGTCCTCGGCTCTG CGCACCAGGGCTGCCTCCCTCCGTTCCCGGCGGACGTGTTCATGATTCCGCAATACG GCTACCTGACGCTCAACAATGGCAGCCAAGCCGTGGGACGTTCATCCTTTTACTGCC TGGAATATTTCCCTTCTCAGATGCTGAGAACGGGCAACAACTTTACCTTCAGCTACA CCTTTGAGGAAGTGCCTTTCCACAGCAGCTACGCGCACAGCCAGAGCCTGGACCGG CTGATGAATCCTCTCATCGACCAATACCTGTATTACCTGAACAGAACTCAAAATCAG TCCGGAAGTGCCCAAAACAAGGACTTGCTGTTTAGCCGTGGGTCTCCAGCTGGCATG TCTGTTCAGCCCAAAAACTGGCTACCTGGACCCTGTTATCGGCAGCAGCGTTTCT AAAACAAAAACAGACAACAACAACAGCAATTTTACCTGGACTGGTGCTTCAAAATA TAACCTCAATGGGCGTGAATCCATCATCAACCCTGGCACTGCTATGGCCTCACACAA AGACGACGAAGACAAGTTCTTTCCCATGAGCGGTGTCATGATTTTTGGAAAAGAGA GCGCCGGAGCTTCAAACACTGCATTGGACAATGTCATGATTACAGACGAAGAGGAA ATTAAAGCCACTAACCCTGTGGCCACCGAAAGATTTGGGACCGTGGCAGTCAATTTC CAGAGCAGCAGCACAGACCCTGCGACCGGAGATGTGCATGCTATGGGAGCATTACC TGGCATGGTGTGGCAAGATAGAGACGTGTACCTGCAGGGTCCCATTTGGGCCAAAA TTCCTCACACAGATGGACACTTTCACCCGTCTCCTCTTATGGGCGGCTTTGGACTCAA GAACCCGCCTCCTCAGATCCTCATCAAAAACACGCCTGTTCCTGCGAATCCTCCGGC GGAGTTTTCAGCTACAAAGTTTGCTTCATTCATCACCCAATACTCCACAGGACAAGT |
| GAGTGTGGAAATTGAATGGGAGCTGCAGAAAGAAAACAGCAAGCGCTGGAATCCC GAAGTGCAGTACACATCCAATTATGCAAAATCTGCCAACGTTGATTTTACTGTGGAC AACAATGGACTTTATACTGAGCCTCGCCCCATTGGCACCCGTTACCTTACCCGTCCC CTGTAA |
| **AAV1 - VP1 protein sequence (SEQ ID NO:20):** |
| MAADGYLPDWLEDNLSEGIREWWDLKPGAPKPKANQQKQDDGRGLVLPGYKYLGPF NGLDKGEPVNAADAAALEHDKAYDQQLKAGDNPYLRYNHADAEFQERLQEDTSFGGN LGRAVFQAKKRVLEPLGLVEEGAKTAPGKKRPVEQSPQEPDSSSGIGKTGQQPAKKRLN FGQTGDSESVPDPQPLGEPPATPAAVGPTTMASGGGAPMADNNEGADGVGNASGNWH CDSTWLGDRVITTSTRTWALPTYNNHLYKQISSASTGASNDNHYFGYSTPWGYFDFNRF HCHFSPRDWQRLINNNWGFRPKRLNFKLFNIQVKEVTTNDGVTTIANNLTSTVQVFSDS EYQLPYVLGSAHQGCLPPFPADVFMIPQYGYLTLNNGSQAVGRSSFYCLEYFPSQMLRT GNNFTFSYTFEEVPFHSSYAHSQSLDRLMNPLIDQYLYYLNRTQNQSGSAQNKDLLFSR GSPAGMSVQPKNWLPGPCYRQQRVSKTKTDNNNSNFTWTGASKYNLNGRESIINPGTA MASHKDDEDKFFPMSGVMIFGKESAGASNTALDNVMITDEEEIKATNPVATERFGTVA VNFQSSSTDPATGDVHAMGALPGMVWQDRDVYLQGPIWAKIPHTDGHFHPSPLMGGF GLKNPPPQILIKNTPVPANPPAEFSATKFASFITQYSTGQVSVEIEWELQKENSKRWNPEV QYTSNYAKSANVDFTVDNNGLYTEPRPIGTRYLTRPL* |
| **AAV1 - VP2 nucleotide sequence (SEQ ID NO:21):** |
| ACGGCTCCTGGAAAGAAACGTCCGGTAGAGCAGTCGCCACAAGAGCCAGACTCCTC CTCGGGCATCGGCAAGACAGGCCAGCAGCCCGCTAAAAGAGACTCAATTTGGTC AGACTGGCGACTCAGAGTCAGTCCCCGATCCACAACCTCTCGGAGAACCTCCAGCA ACCCCCGCTGCTGTGGGACCTACTACAATGGCTTCAGGCGGTGGCGCACCAATGGC AGACAATAACGAAGGCGCCGACGGAGTGGGTAATGCCTCAGGAAATTGGCATTGCG ATTCCACATGGCTGGGCGACAGAGTCATCACCACCAGCACCCGCACCTGGGCCTTGC CCACCTACAATAACCACCTCTACAAGCAAATCTCCAGTGCTTCAACGGGGGCCAGC AACGACAACCACTACTTCGGCTACAGCACCCCCTGGGGGTATTTTGATTTCAACAGA TTCCACTGCCACTTTTCACCACGTGACTGGCAGCGACTCATCAACAACAATTGGGGA TTCCGGCCCAAGAGACTCAACTTCAAACTCTTCAACATCCAAGTCAAGGAGGTCACG ACGAATGATGGCGTCACAACCATCGCTAATAACCTTACCAGCACGGTTCAAGTCTTC TCGGACTCGGAGTACCAGCTTCCGTACGTCCTCGGCTCTGCGCACCAGGGCTGCCTC CCTCCGTTCCCGGCGGACGTGTTCATGATTCCGCAATACGGCTACCTGACGCTCAAC AATGGCAGCCAAGCCGTGGGACGTTCATCCTTTTACTGCCTGGAATATTTCCCTTCTC AGATGCTGAGAACGGGCAACAACTTTACCTTCAGCTACACCTTTGAGGAAGTGCCTT TCCACAGCAGCTACGCGCACAGCCAGAGCCTGGACCGGCTGATGAATCCTCTCATC |
| GACCAATACCTGTATTACCTGAACAGAACTCAAAATCAGTCCGGAAGTGCCCAAAA CAAGGACTTGCTGTTTAGCCGTGGGTCTCCAGCTGGCATGTCTGTTCAGCCCAAAAA CTGGCTACCTGGACCCTGTTATCGGCAGCAGCGTTTCTAAAACAAAAACAGACA ACAACAACAGCAATTTTACCTGGACTGGTGCTTCAAAATATAACCTCAATGGGCGTG AATCCATCATCAACCCTGGCACTGCTATGGCCTCACACAAAGACGACGAAGACAAG TTCTTTCCCATGAGCGGTGTCATGATTTTTGGAAAAGAGAGCGCCGGAGCTTCAAAC ACTGCATTGGACAATGTCATGATTACAGACGAAGAGGAAATTAAAGCCACTAACCC TGTGGCCACCGAAAGATTTGGGACCGTGGCAGTCAATTTCCAGAGCAGCAGCACAG ACCCTGCGACCGGAGATGTGCATGCTATGGGAGCATTACCTGGCATGGTGTGGCAA GATAGAGACGTGTACCTGCAGGGTCCCATTTGGGCCAAAATTCCTCACACAGATGG ACACTTTCACCCGTCTCCTCTTATGGGCGGCTTTGGACTCAAGAACCCGCCTCCTCA GATCCTCATCAAAAACACGCCTGTTCCTGCGAATCCTCCGGCGGAGTTTTCAGCTAC AAAGTTTGCTTCATTCATCACCCAATACTCCACAGGACAAGTGAGTGTGGAAATTGA ATGGGAGCTGCAGAAAGAAAACAGCAAGCGCTGGAATCCCGAAGTGCAGTACACAT CCAATTATGCAAAATCTGCCAACGTTGATTTTACTGTGGACAACAATGGACTTTATA CTGAGCCTCGCCCCATTGGCACCCGTTACCTTACCCGTCCCCTGTAA |
| **AAV1 - VP2 protein sequence (SEQ ID NO:22):** |
| TAPGKKRPVEQSPQEPDSSSGIGKTGQQPAKKRLNFGQTGDSESVPDPQPLGEPPATPAA VGPTTMASGGGAPMADNNEGADGVGNASGNWHCDSTWLGDRVITTSTRTWALPTYN NHLYKQISSASTGASNDNHYFGYSTPWGYFDFNRFHCHFSPRDWQRLINNNWGFRPKR LNFKLFNIQVKEVTTNDGVTTIANNLTSTVQVFSDSEYQLPYVLGSAHQGCLPPFPADVF MIPQYGYLTLNNGSQAVGRSSFYCLEYFPSQMLRTGNNFTFSYTFEEVPFHSSYAHSQSL DRLMNPLIDQYLYYLNRTQNQSGSAQNKDLLFSRGSPAGMSVQPKNWLPGPCYRQQR VSKTKTDNNNSNFTWTGASKYNLNGRESIINPGTAMASHKDDEDKFFPMSGVMIFGKES AGASNTALDNVMITDEEEIKATNPVATERFGTVAVNFQSSSTDPATGDVHAMGALPGM VWQDRDVYLQGPIWAKIPHTDGHFHPSPLMGGFGLKNPPPQILIKNTPVPANPPAEFSAT KFASFITQYSTGQVSVEIEWELQKENSKRWNPEVQYTSNYAKSANVDFTVDNNGLYTEP RPIGTRYLTRPL* |
| **AAV1 - VP3 nucleotide sequence (SEQ ID NO:23):** |
| ATGGCTTCAGGCGGTGGCGCACCAATGGCAGACAATAACGAAGGCGCCGACGGAGT GGGTAATGCCTCAGGAAATTGGCATTGCGATTCCACATGGCTGGGCGACAGAGTCA TCACCACCAGCACCCGCACCTGGGCCTTGCCCACCTACAATAACCACCTCTACAAGC AAATCTCCAGTGCTTCAACGGGGGCCAGCAACGACAACCACTACTTCGGCTACAGC ACCCCCTGGGGGTATTTTGATTTCAACAGATTCCACTGCCACTTTTCACCACGTGACT GGCAGCGACTCATCAACAACAATTGGGGATTCCGGCCCAAGAGACTCAACTTCAAA |
| CTCTTCAACATCCAAGTCAAGGAGGTCACGACGAATGATGGCGTCACAACCATCGC TAATAACCTTACCAGCACGGTTCAAGTCTTCTCGGACTCGGAGTACCAGCTTCCGTA CGTCCTCGGCTCTGCGCACCAGGGCTGCCTCCCTCCGTTCCCGGCGGACGTGTTCAT GATTCCGCAATACGGCTACCTGACGCTCAACAATGGCAGCCAAGCCGTGGGACGTT CATCCTTTTACTGCCTGGAATATTTCCCTTCTCAGATGCTGAGAACGGGCAACAACTT TACCTTCAGCTACACCTTTGAGGAAGTGCCTTTCCACAGCAGCTACGCGCACAGCCA GAGCCTGGACCGGCTGATGAATCCTCTCATCGACCAATACCTGTATTACCTGAACAG AACTCAAAATCAGTCCGGAAGTGCCCAAAACAAGGACTTGCTGTTTAGCCGTGGGT CTCCAGCTGGCATGTCTGTTCAGCCCAAAAACTGGCTACCTGGACCCTGTTATCGGC AGCAGCGCGTTTCTAAAACAAAAACAGACAACAACAACAGCAATTTTACCTGGACT GGTGCTTCAAAATATAACCTCAATGGGCGTGAATCCATCATCAACCCTGGCACTGCT ATGGCCTCACACAAAGACGACGAAGACAAGTTCTTTCCCATGAGCGGTGTCATGATT TTTGGAAAAGAGAGCGCCGGAGCTTCAAACACTGCATTGGACAATGTCATGATTAC AGACGAAGAGGAAATTAAAGCCACTAACCCTGTGGCCACCGAAAGATTTGGGACCG TGGCAGTCAATTTCCAGAGCAGCAGCACAGACCCTGCGACCGGAGATGTGCATGCT ATGGGAGCATTACCTGGCATGGTGTGGCAAGATAGAGACGTGTACCTGCAGGGTCC CATTTGGGCCAAAATTCCTCACACAGATGGACACTTTCACCCGTCTCCTCTTATGGG CGGCTTTGGACTCAAGAACCCGCCTCCTCAGATCCTCATCAAAAACACGCCTGTTCC TGCGAATCCTCCGGCGGAGTTTTCAGCTACAAAGTTTGCTTCATTCATCACCCAATA CTCCACAGGACAAGTGAGTGTGGAAATTGAATGGGAGCTGCAGAAAGAAAACAGC AAGCGCTGGAATCCCGAAGTGCAGTACACATCCAATTATGCAAAATCTGCCAACGTT GATTTTACTGTGGACAACAATGGACTTTATACTGAGCCTCGCCCCATTGGCACCCGT TACCTTACCCGTCCCCTGTAA |
| **AAV1 - VP3 protein sequence (SEQ ID NO:24):** |
| MASGGGAPMADNNEGADGVGNASGNWHCDSTWLGDRVITTSTRTWALPTYNNHLYK QISSASTGASNDNHYFGYSTPWGYFDFNRFHCHFSPRDWQRLINNNWGFRPKRLNFKLF NIQVKEVTTNDGVTTIANNLTSTVQVFSDSEYQLPYVLGSAHQGCLPPFPADVFMIPQYG YLTLNNGSQAVGRSSFYCLEYFPSQMLRTGNNFTFSYTFEEVPFHSSYAHSQSLDRLMN PLIDQYLYYLNRTQNQSGSAQNKDLLFSRGSPAGMSVQPKNWLPGPCYRQQRVSKTKT DNNNSNFTWTGASKYNLNGRESIINPGTAMASHKDDEDKFFPMSGVMIFGKESAGASN TALDNVMITDEEEIKATNPVATERFGTVAVNFQSSSTDPATGDVHAMGALPGMVWQDR DVYLQGPIWAKIPHTDGHFHPSPLMGGFGLKNPPPQILIKNTPVPANPPAEFSATKFASFI TQYSTGQVSVEIEWELQKENSKRWNPEVQYTSNYAKSANVDFTVDNNGLYTEPRPIGT RYLTRPL* |

| **AAV2** |
|---|
| **AAV2 - VP1 nucleotide sequence (SEQ ID NO:25):** |
| ATGGCTGCCGATGGTTATCTTCCAGATTGGCTCGAGGACACTCTCTCTGAAGGAATA AGACAGTGGTGGAAGCTCAAACCTGGCCCACCACCACCAAAGCCCGCAGAGCGGCA TAAGGACGACAGCAGGGGTCTTGTGCTTCCTGGGTACAAGTACCTCGGACCCTTCAA CGGACTCGACAAGGGAGAGCCGGTCAACGAGGCAGACGCCGCGGCCCTCGAGCAC GACAAAGCCTACGACCGGCAGCTCGACAGCGGAGACAACCCGTACCTCAAGTACAA CCACGCCGACGCGGAGTTTCAGGAGCGCCTTAAAGAAGATACGTCTTTTGGGGGCA ACCTCGGACGAGCAGTCTTCCAGGCGAAAAAGAGGGTTCTTGAACCTCTGGGCCTG GTTGAGGAACCTGTTAAGACGGCTCCGGGAAAAAAGAGGCCGGTAGAGCACTCTCC TGTGGAGCCAGACTCCTCCTCGGGAACCGGAAAGGCGGGCCAGCAGCCTGCAAGAA AAAGATTGAATTTTGGTCAGACTGGAGACGCAGACTCAGTACCTGACCCCCAGCCTC TCGGACAGCCACCAGCAGCCCCCTCTGGTCTGGGAACTAATACGATGGCTACAGGC AGTGGCGCACCAATGGCAGACAATAACGAGGGCGCCGACGGAGTGGGTAATTCCTC GGGAAATTGGCATTGCGATTCCACATGGATGGGCGACAGAGTCATCACCACCAGCA CCCGAACCTGGGCCCTGCCCACCTACAACAACCACCTCTACAAACAAATTTCCAGCC AATCAGGAGCCTCGAACGACAATCACTACTTTGGCTACAGCACCCCTTGGGGGTATT TTGACTTCAACAGATTCCACTGCCACTTTTCACCACGTGACTGGCAAAGACTCATCA ACAACAACTGGGGATTCCGACCCAAGAGACTCAACTTCAAGCTCTTTAACATTCAAG TCAAAGAGGTCACGCAGAATGACGGTACGACGACGATTGCCAATAACCTTACCAGC ACGGTTCAGGTGTTTACTGACTCGGAGTACCAGCTCCCGTACGTCCTCGGCTCGGCG CATCAAGGATGCCTCCCGCCGTTCCCAGCAGACGTCTTCATGGTGCCACAGTATGGA TACCTCACCCTGAACAACGGGAGTCAGGCAGTAGGACGCTCTTCATTTTACTGCCTG GAGTACTTTCCTTCTCAGATGCTGCGTACCGGAAACAACTTTACCTTCAGCTACACTT TTGAGGACGTTCCTTTCCACAGCAGCTACGCTCACAGCCAGAGTCTGGACCGTCTCA TGAATCCTCTCATCGACCAGTACCTGTATTACTTGAGCAGAACAAACACTCCAAGTG GAACCACCACGCAGTCAAGGCTTCAGTTTTCTCAGGCCGGAGCGAGTGACATTCGG GACCAGTCTAGGAACTGGCTTCCTGGACCCTGTTACCGCCAGCAGCGAGTATCAAA GACATCTGCGGATAACAACAACAGTGAATACTCGTGGACTGGAGCTACCAAGTACC ACCTCAATGGCAGAGACTCTCTGGTGAATCCGGGCCCGGCCATGGCAAGCCACAAG GACGATGAAGAAAAGTTTTTTCCTCAGAGCGGGGTTCTCATCTTTGGGAAGCAAGGC TCAGAGAAAACAAATGTGGACATTGAAAAGGTCATGATTACAGACGAAGAGGAAAT CAGGACAACCAATCCCGTGGCTACGGAGCAGTATGGTTCTGTATCTACCAACCTCCA GAGAGGCAACAGACAAGCAGCTACCGCAGATGTCAACACACAAGGCGTTCTTCCAG GCATGGTCTGGCAGGACAGAGATGTGTACCTTCAGGGGCCCATCTGGGCAAAGATT CCACACACGGACGGACATTTTCACCCCTCTCCCCTCATGGGTGGATTCGGACTTAAA CACCCTCCTCCACAGATTCTCATCAAGAACACCCCGGTACCTGCGAATCCTTCGACC ACCTTCAGTGCGGCAAAGTTTGCTTCCTTCATCACACAGTACTCCACGGGACAGGTC AGCGTGGAGATCGAGTGGGAGCTGCAGAAGGAAAACAGCAAACGCTGGAATCCCG |
| AAATTCAGTACACTTCCAACTACAACAAGTCTGTTAATGTGGACTTTACTGTGGACA CTAATGGCGTGTATTCAGAGCCTCGCCCATTGGCACCAGATACCTGACTCGTAATC TGTAA |
| **AAV2 - VP1 protein sequence (SEQ ID NO:26):** Note: 3x Y mutated to F in AAV2(Y444,500,730F) |
| * |
| **AAV2 - VP2 nucleotide sequence (SEQ ID NO:27):** |
| ACGGCTCCGGGAAAAAGAGGCCGGTAGAGCACTCTCCTGTGGAGCCAGACTCCTC CTCGGGAACCGGAAAGGCGGCCAGCAGCCTGCAAGAAAAGATTGAATTTTGGTC AGACTGGAGACGCAGACTCAGTACCTGACCCCCAGCCTCTCGGACAGCCACCAGCA GCCCCCTCTGGTCTGGGAACTAATACGATGGCTACAGGCAGTGGCGCACCAATGGC AGACAATAACGAGGGCGCCGACGGAGTGGGTAATTCCTCGGGAAATTGGCATTGCG ATTCCACATGGATGGGCGACAGAGTCATCACCACCAGCACCCGAACCTGGGCCCTG CCCACCTACAACAACCACCTCTACAAACAAATTTCCAGCCAATCAGGAGCCTCGAA CGACAATCACTACTTTGGCTACAGCACCCCTTGGGGGTATTTTGACTTCAACAGATT CCACTGCCACTTTTCACCACGTGACTGGCAAAGACTCATCAACAACAACTGGGGATT CCGACCCAAGAGACTCAACTTCAAGCTCTTTAACATTCAAGTCAAAGAGGTCACGC AGAATGACGGTACGACGACGATTGCCAATAACCTTACCAGCACGGTTCAGGTGTTTA CTGACTCGGAGTACCAGCTCCCGTACGTCCTCGGCTCGGCGCATCAAGGATGCCTCC CGCCGTTCCCAGCAGACGTCTTCATGGTGCCACAGTATGGATACCTCACCCTGAACA ACGGGAGTCAGGCAGTAGGACGCTCTTCATTTTACTGCCTGGAGTACTTTCCTTCTC AGATGCTGCGTACCGGAAACAACTTTACCTTCAGCTACACTTTTGAGGACGTTCCTT TCCACAGCAGCTACGCTCACAGCCAGAGTCTGGACCGTCTCATGAATCCTCTCATCG |
| ACCAGTACCTGTATTACTTGAGCAGAACAAACACTCCAAGTGGAACCACCACGCAG TCAAGGCTTCAGTTTCTCAGGCCGGAGCGAGTGACATTCGGGACCAGTCTAGGAAC TGGCTTCCTGGACCCTGTTACCGCCAGCAGCGAGTATCAAAGACATCTGCGGATAAC AACAACAGTGAATACTCGTGGACTGGAGCTACCAAGTACCACCTCAATGGCAGAGA CTCTCTGGTGAATCCGGGCCCGGCCATGGCAAGCCACAAGGACGATGAAGAAAAGT TTTTTCCTCAGAGCGGGGTTCTCATCTTTGGGAAGCAAGGCTCAGAGAAAACAAATG TGGACATTGAAAAGGTCATGATTACAGACGAAGAGGAAATCAGGACAACCAATCCC GTGGCTACGGAGCAGTATGGTTCTGTATCTACCAACCTCCAGAGAGGCAACAGACA AGCAGCTACCGCAGATGTCAACACACAAGGCGTTCTTCCAGGCATGGTCTGGCAGG ACAGAGATGTGTACCTTCAGGGGCCCATCTGGGCAAAGATTCCACACACGGACGGA CATTTTCACCCCTCTCCCCTCATGGGTGGATTCGGACTTAAACACCCTCCTCCACAGA TTCTCATCAAGAACACCCCGGTACCTGCGAATCCTTCGACCACCTTCAGTGCGGCAA AGTTTGCTTCCTTCATCACACAGTACTCCACGGGACAGGTCAGCGTGGAGATCGAGT GGGAGCTGCAGAAGGAAAACAGCAAACGCTGGAATCCCGAAATTCAGTACACTTCC AACTACAACAAGTCTGTTAATGTGGACTTTACTGTGGACACTAATGGCGTGTATTCA GAGCCTCGCCCCATTGGCACCAGATACCTGACTCGTAATCTGTAA |
| **AAV2 - VP2 protein sequence (SEQ ID NO:30):** |
| TAPGKKRPVEHSPVEPDSSSGTGKAGQQPARKRLNFGQTGDADSVPDPQPLGQPPAAPS GLGTNTMATGSGAPMADNNEGADGVGNSSGNWHCDSTWMGDRVITTSTRTWALPTY NNHLYKQISSQSGASNDNHYFGYSTPWGYFDFNRFHCHFSPRDWQRLINNNWGFRPKR LNFKLFNIQVKEVTQNDGTTTIANNLTSTVQVFTDSEYQLPYVLGSAHQGCLPPFPADVF MVPQYGYLTLNNGSQAVGRSSFYCLEYFPSQMLRTGNNFTFSYTFEDVPFHSSYAHSQS LDRLMNPLIDQYLYYLSRTNTPSGTTTQSRLQFSQAGASDIRDQSRNWLPGPCYRQQRV SKTSADNNNSEYSWTGATKYHLNGRDSLVNPGPAMASHKDDEEKFFPQSGVLIFGKQG SEKTNVDIEKVMITDEEEIRTTNPVATEQYGSVSTNLQRGNRQAATADVNTQGVLPGMV WQDRDVYLQGPIWAKIPHTDGHFHPSPLMGGFGLKHPPPQILIKNTPVPANPSTTFSAAK FASFITQYSTGQVSVEIEWELQKENSKRWNPEIQYTSNYNKSVNVDFTVDTNGVYSEPR PIGTRYLTRNL* |
| **AAV2 - VP3 nucleotide sequence (SEQ ID NO:31):** |
| ATGGCTACAGGCAGTGGCGCACCAATGGCAGACAATAACGAGGGCGCCGACGGAG TGGGTAATTCCTCGGGAAATTGGCATTGCGATTCCACATGGATGGGCGACAGAGTCA TCACCACCAGCACCCGAACCTGGGCCCTGCCCACCTACAACAACCACCTCTACAAAC AAATTTCCAGCCAATCAGGAGCCTCGAACGACAATCACTACTTTGGCTACAGCACCC CTTGGGGGTATTTTGACTTCAACAGATTCCACTGCCACTTTTCACCACGTGACTGGCA AAGACTCATCAACAACAACTGGGGATTCCGACCCAAGAGACTCAACTTCAAGCTCT |
| TTAACATTCAAGTCAAAGAGGTCACGCAGAATGACGGTACGACGACGATTGCCAAT AACCTTACCAGCACGGTTCAGGTGTTACTGACTCGGAGTACCAGCTCCCGTACGTC CTCGGCTCGGCGCATCAAGGATGCCTCCCGCCGTTCCCAGCAGACGTCTTCATGGTG CCACAGTATGGATACCTCACCCTGAACAACGGGAGTCAGGCAGTAGGACGCTCTTC ATTTTACTGCCTGGAGTACTTTCCTTCTCAGATGCTGCGTACCGGAAACAACTTTACC TTCAGCTACACTTTTGAGGACGTTCCTTTCCACAGCAGCTACGCTCACAGCCAGAGT CTGGACCGTCTCATGAATCCTCTCATCGACCAGTACCTGTATTACTTGAGCAGAACA AACACTCCAAGTGGAACCACCACGCAGTCAAGGCTTCAGTTTTCTCAGGCCGGAGC GAGTGACATTCGGGACCAGTCTAGGAACTGGCTTCCTGGACCCTGTTACCGCCAGCA GCGAGTATCAAAGACATCTGCGGATAACAACAACAGTGAATACTCGTGGACTGGAG CTACCAAGTACCACCTCAATGGCAGAGACTCTCTGGTGAATCCGGGCCCGGCCATG GCAAGCCACAAGGACGATGAAGAAAAGTTTTTCCTCAGAGCGGGGTTCTCATCTTT GGGAAGCAAGGCTCAGAGAAAACAAATGTGGACATTGAAAAGGTCATGATTACAG ACGAAGAGGAAATCAGGACAACCAATCCCGTGGCTACGGAGCAGTATGGTTCTGTA TCTACCAACCTCCAGAGAGGCAACAGACAAGCAGCTACCGCAGATGTCAACACACA AGGCGTTCTTCCAGGCATGGTCTGGCAGGACAGAGATGTGTACCTTCAGGGGCCCAT CTGGGCAAAGATTCCACACACGGACGGACATTTTCACCCCTCTCCCCTCATGGGTGG ATTCGGACTTAAACACCCTCCTCCACAGATTCTCATCAAGAACACCCCGGTACCTGC GAATCCTTCGACCACCTTCAGTGCGGCAAAGTTTGCTTCCTTCATCACACAGTACTC CACGGGACAGGTCAGCGTGGAGATCGAGTGGGAGCTGCAGAAGGAAAACAGCAAA CGCTGGAATCCCGAAATTCAGTACACTTCCAACTACAACAAGTCTGTTAATGTGGAC TTTACTGTGGACACTAATGGCGTGTATTCAGAGCCTCGCCCCATTGGCACCAGATAC CTGACTCGTAATCTGTAA |
| **AAV2 - VP3 protein sequence (SEQ ID NO:32):** |
| MATGSGAPMADNNEGADGVGNSSGNWHCDSTWMGDRVITTSTRTWALPTYNNHLYK QISSQSGASNDNHYFGYSTPWGYFDFNRFHCHFSPRDWQRLINNNWGFRPKRLNFKLFN IQVKEVTQNDGTTTIANNLTSTVQVFTDSEYQLPYVLGSAHQGCLPPFPADVFMVPQYG YLTLNNGSQAVGRSSFYCLEYFPSQMLRTGNNFTFSYTFEDVPFHSSYAHSQSLDRLMN PLIDQYLYYLSRTNTPSGTTTQSRLQFSQAGASDIRDQSRNWLPGPCYRQQRVSKTSAD NNNSEYSWTGATKYHLNGRDSLVNPGPAMASHKDDEEKFFPQSGVLIFGKQGSEKTNV DIEKVMITDEEEIRTTNPVATEQYGSVSTNLQRGNRQAATADVNTQGVLPGMVWQDRD VYLQGPIWAKIPHTDGHFHPSPLMGGFGLKHPPPQILIKNTPVPANPSTTFSAAKFASFIT QYSTGQVSVEIEWELQKENSKRWNPEIQYTSNYNKSVNVDFTVDTNGVYSEPRPIGTRY LTRNL* |

| **AAV9** |
|---|
| **AAV9 - VP1 nucleotide sequence (SEQ ID NO:33):** |
| ATGGCTGCCGATGGTTATCTTCCAGATTGGCTCGAGGACAACCTTAGTGAAGGAATT CGCGAGTGGTGGGCTTTGAAACCTGGAGCCCCTCAACCCAAGGCAAATCAACAACA TCAAGACAACGCTCGAGGTCTTGTGCTTCCGGGTTACAAATACCTTGGACCCGGCAA CGGACTCGACAAGGGGGAGCCGGTCAACGCAGCAGACGCGGCGGCCCTCGAGCAC GACAAGGCCTACGACCAGCAGCTCAAGGCCGGAGACAACCCGTACCTCAAGTACAA CCACGCCGACGCCGAGTTCCAGGAGCGGCTCAAAGAAGATACGTCTTTTGGGGGCA ACCTCGGGCGAGCAGTCTTCCAGGCCAAAAAGAGGCTTCTTGAACCTCTTGGTCTGG TTGAGGAAGCGGCTAAGACGGCTCCTGGAAAGAAGAGGCCTGTAGAGCAGTCTCCT CAGGAACCGGACTCCTCCGCGGGTATTGGCAAATCGGGTGCACAGCCCGCTAAAAA GAGACTCAATTTCGGTCAGACTGGCGACACAGAGTCAGTCCCAGACCCTCAACCAA TCGGAGAACCTCCCGCAGCCCCCTCAGGTGTGGGATCTCTTACAATGGCTTCAGGTG GTGGCGCACCAGTGGCAGACAATAACGAAGGTGCCGATGGAGTGGGTAGTTCCTCG GGAAATTGGCATTGCGATTCCCAATGGCTGGGGGACAGAGTCATCACCACCAGCAC CCGAACCTGGGCCCTGCCCACCTACAACAATCACCTCTACAAGCAAATCTCCAACAG CACATCTGGAGGATCTTCAAATGACAACGCCTACTTCGGCTACAGCACCCCCTGGGG GTATTTTGACTTCAACAGATTCCACTGCCACTTCTCACCACGTGACTGGCAGCGACT CATCAACAACAACTGGGGATTCCGGCCTAAGCGACTCAACTTCAAGCTCTTCAACAT TCAGGTCAAAGAGGTTACGGACAACAATGGAGTCAAGACCATCGCCAATAACCTTA CCAGCACGGTCCAGGTCTTCACGGACTCAGACTATCAGCTCCCGTACGTGCTCGGGT CGGCTCACGAGGGCTGCCTCCCGCCGTTCCCAGCGGACGTTTTCATGATTCCTCAGT ACGGGTATCTGACGCTTAATGATGGAAGCCAGGCCGTGGGTCGTTCGTCCTTTTACT GCCTGGAATATTTCCCGTCGCAAATGCTAAGAACGGGTAACAACTTCCAGTTCAGCT ACGAGTTTGAGAACGTACCTTTCCATAGCAGCTACGCTCACAGCCAAAGCCTGGACC GACTAATGAATCCACTCATCGACCAATACTTGTACTATCTCTCAAAGACTATTAACG GTTCTGGACAGAATCAACAAACGCTAAAATTCAGTGTGGCCGGACCCAGCAACATG GCTGTCCAGGGAAGAAACTACATACCTGGACCCAGCTACCGACAACAACGTGTCTC AACCACTGTGACTCAAAACAACAACAGCGAATTTGCTTGGCCTGGAGCTTCTTCTTG GGCTCTCAATGGACGTAATAGCTTGATGAATCCTGGACCTGCTATGGCCAGCCACAA AGAAGGAGAGGACCGTTTCTTTCCTTTGTCTGGATCTTTAATTTTTGGCAAACAAGG AACTGGAAGAGACAACGTGGATGCGGACAAAGTCATGATAACCAACGAAGAAGAA ATTAAAACTACTAACCCGGTAGCAACGGAGTCCTATGGACAAGTGGCCACAAACCA CCAGAGTGCCCAAGCACAGGCGCAGACCGGCTGGGTTCAAAACCAAGGAATACTTC CGGGTATGGTTTGGCAGGACAGAGATGTGTACCTGCAAGGACCCATTTGGGCCAAA ATTCCTCACACGGACGGCAACTTTCACCCTTCTCCGCTGATGGGAGGGTTTGGAATG AAGCACCCGCCTCCTCAGATCCTCATCAAAAACACACCTGTACCTGCGGATCCTCCA ACGGCCTTCAACAAGGACAAGCTGAACTCTTTCATCACCCAGTATTCTACTGGCCAA |
| GTCAGCGTGGAGATCGAGTGGGAGCTGCAGAAGGAAAACAGCAAGCGCTGGAACC CGGAGATCCAGTACACTTCCAACTATTACAAGTCTAATAATGTTGAATTTGCTGTTA ATACTGAAGGTGTATATAGTGAACCCCGCCCCATTGGCACCAGATACCTGACTCGTA ATCTGTAA |
| **AAV9 - VP1 protein sequence (SEQ ID NO:34):** |
| MAADGYLPDWLEDNLSEGIREWWALKPGAPQPKANQQHQDNARGLVLPGYKYLGPG NGLDKGEPVNAADAAALEHDKAYDQQLKAGDNPYLKYNHADAEFQERLKEDTSFGG NLGRAVFQAKKRLLEPLGLVEEAAKTAPGKKRPVEQSPQEPDSSAGIGKSGAQPAKKRL NFGQTGDTESVPDPQPIGEPPAAPSGVGSLTMASGGGAPVADNNEGADGVGSSSGNWH CDSQWLGDRVITTSTRTWALPTYNNHLYKQISNSTSGGSSNDNAYFGYSTPWGYFDFN RFHCHFSPRDWQRLINNNWGFRPKRLNFKLFNIQVKEVTDNNGVKTIANNLTSTVQVFT DSDYQLPYVLGSAHEGCLPPFPADVFMIPQYGYLTLNDGSQAVGRSSFYCLEYFPSQML RTGNNFQFSYEFENVPFHSSYAHSQSLDRLMNPLIDQYLYYLSKTINGSGQNQQTLKFSV AGPSNMAVQGRNYIPGPSYRQQRVSTTVTQNNNSEFAWPGASSWALNGRNSLMNPGP AMASHKEGEDRFFPLSGSLIFGKQGTGRDNVDADKVMITNEEEIKTTNPVATESYGQVA TNHQSAQAQAQTGWVQNQGILPGMVWQDRDVYLQGPIWAKIPHTDGNFHPSPLMGGF GMKHPPPQILIKNTPVPADPPTAFNKDKLNSFITQYSTGQVSVEIEWELQKENSKRWNPE IQYTSNYYKSNNVEFAVNTEGVYSEPRPIGTRYLTRNL* |
| **AAV9 - VP2 nucleotide sequence (SEQ ID NO:35):** |
| ACGGCTCCTGGAAAGAAGAGGCCTGTAGAGCAGTCTCCTCAGGAACCGGACTCCTC CGCGGGTATTGGCAAATCGGGTGCACAGCCCGCTAAAAAGAGACTCAATTTCGGTC AGACTGGCGACACAGAGTCAGTCCCAGACCCTCAACCAATCGGAGAACCTCCCGCA GCCCCCTCAGGTGTGGGATCTCTTACAATGGCTTCAGGTGGTGGCGCACCAGTGGCA GACAATAACGAAGGTGCCGATGGAGTGGGTAGTTCCTCGGGAAATTGGCATTGCGA TTCCCAATGGCTGGGGGACAGAGTCATCACCACCAGCACCCGAACCTGGGCCCTGC CCACCTACAACAATCACCTCTACAAGCAAATCTCCAACAGCACATCTGGAGGATCTT CAAATGACAACGCCTACTTCGGCTACAGCACCCCCTGGGGGTATTTTGACTTCAACA GATTCCACTGCCACTTCTCACCACGTGACTGGCAGCGACTCATCAACAACAACTGGG GATTCCGGCCTAAGCGACTCAACTTCAAGCTCTTCAACATTCAGGTCAAAGAGGTTA CGGACAACAATGGAGTCAAGACCATCGCCAATAACCTTACCAGCACGGTCCAGGTC TTCACGGACTCAGACTATCAGCTCCCGTACGTGCTCGGGTCGGCTCACGAGGGCTGC CTCCCGCCGTTCCCAGCGGACGTTTTCATGATTCCTCAGTACGGGTATCTGACGCTTA ATGATGGAAGCCAGGCCGTGGGTCGTTCGTCCTTTTACTGCCTGGAATATTTCCCGT CGCAAATGCTAAGAACGGGTAACAACTTCCAGTTCAGCTACGAGTTTGAGAACGTA CCTTTCCATAGCAGCTACGCTCACAGCCAAAGCCTGGACCGACTAATGAATCCACTC |
| ATCGACCAATACTTGTACTATCTCTCAAAGACTATTAACGGTTCTGGACAGAATCAA CAAACGCTAAAATTCAGTGTGGCCGGACCCAGCAACATGGCTGTCCAGGGAAGAAA CTACATACCTGGACCCAGCTACCGACAACAACGTGTCTCAACCACTGTGACTCAAAA CAACAACAGCGAATTTGCTTGGCCTGGAGCTTCTTCTTGGGCTCTCAATGGACGTAA TAGCTTGATGAATCCTGGACCTGCTATGGCCAGCCACAAAGAAGGAGAGGACCGTT TCTTTCCTTTGTCTGGATCTTTAATTTTTGGCAAACAAGGAACTGGAAGAGACAACG TGGATGCGGACAAAGTCATGATAACCAACGAAGAAGAAATTAAAACTACTAACCCG GTAGCAACGGAGTCCTATGGACAAGTGGCCACAAACCACCAGAGTGCCAAGCACA GGCGCAGACCGGCTGGGTTCAAAACCAAGGAATACTTCCGGGTATGGTTTGGCAGG ACAGAGATGTGTACCTGCAAGGACCCATTTGGGCCAAAATTCCTCACACGGACGGC AACTTTCACCCTTCTCCGCTGATGGGAGGGTTTGGAATGAAGCACCCGCCTCCTCAG ATCCTCATCAAAAACACACCTGTACCTGCGGATCCTCCAACGGCCTTCAACAAGGAC AAGCTGAACTCTTTCATCACCCAGTATTCTACTGGCCAAGTCAGCGTGGAGATCGAG TGGGAGCTGCAGAAGGAAAACAGCAAGCGCTGGAACCCGGAGATCCAGTACACTTC CAACTATTACAAGTCTAATAATGTTGAATTTGCTGTTAATACTGAAGGTGTATATAG TGAACCCCGCCCCATTGGCACCAGATACCTGACTCGTAATCTGTAA |
| **AAV9 - VP2 protein sequence (SEQ ID NO:36):** |
| TAPGKKRPVEQSPQEPDSSAGIGKSGAQPAKKRLNFGQTGDTESVPDPQPIGEPPAAPSG VGSLTMASGGGAPVADNNEGADGVGSSSGNWHCDSQWLGDRVITTSTRTWALPTYNN HLYKQISNSTSGGSSNDNAYFGYSTPWGYFDFNRFHCHFSPRDWQRLINNNWGFRPKRL NFKLFNIQVKEVTDNNGVKTIANNLTSTVQVFTDSDYQLPYVLGSAHEGCLPPFPADVF MIPQYGYLTLNDGSQAVGRSSFYCLEYFPSQMLRTGNNFQFSYEFENVPFHSSYAHSQS LDRLMNPLIDQYLYYLSKTINGSGQNQQTLKFSVAGPSNMAVQGRNYIPGPSYRQQRVS TTVTQNNNSEFAWPGASSWALNGRNSLMNPGPAMASHKEGEDRFFPLSGSLIFGKQGT GRDNVDADKVMITNEEEIKTTNPVATESYGQVATNHQSAQAQAQTGWVQNQGILPGM VWQDRDVYLQGPIWAKIPHTDGNFHPSPLMGGFGMKHPPPQILIKNTPVPADPPTAFNK DKLNSFITQYSTGQVSVEIEWELQKENSKRWNPEIQYTSNYYKSNNVEFAVNTEGVYSE PRPIGTRYLTRNL* |
| **AAV9 - VP3 nucleotide sequence (SEQ ID NO:37):** |
| ATGGCTTCAGGTGGTGGCGCACCAGTGGCAGACAATAACGAAGGTGCCGATGGAGT GGGTAGTTCCTCGGGAAATTGGCATTGCGATTCCCAATGGCTGGGGGACAGAGTCAT CACCACCAGCACCCGAACCTGGGCCCTGCCCACCTACAACAATCACCTCTACAAGC AAATCTCCAACAGCACATCTGGAGGATCTTCAAATGACAACGCCTACTTCGGCTACA GCACCCCCTGGGGGTATTTTGACTTCAACAGATTCCACTGCCACTTCTCACCACGTG ACTGGCAGCGACTCATCAACAACAACTGGGGATTCCGGCCTAAGCGACTCAACTTC |
| AAGCTCTTCAACATTCAGGTCAAAGAGGTTACGGACAACAATGGAGTCAAGACCAT CGCCAATAACCTTACCAGCACGGTCCAGGTCTTCACGGACTCAGACTATCAGCTCCC GTACGTGCTCGGGTCGGCTCACGAGGGCTGCCTCCCGCCGTTCCCAGCGGACGTTTT CATGATTCCTCAGTACGGGTATCTGACGCTTAATGATGGAAGCCAGGCCGTGGGTCG TTCGTCCTTTTACTGCCTGGAATATTTCCCGTCGCAAATGCTAAGAACGGGTAACAA CTTCCAGTTCAGCTACGAGTTTGAGAACGTACCTTTCCATAGCAGCTACGCTCACAG CCAAAGCCTGGACCGACTAATGAATCCACTCATCGACCAATACTTGTACTATCTCTC AAAGACTATTAACGGTTCTGGACAGAATCAACAAACGCTAAAATTCAGTGTGGCCG GACCCAGCAACATGGCTGTCCAGGGAAGAAACTACATACCTGGACCCAGCTACCGA CAACAACGTGTCTCAACCACTGTGACTCAAAACAACAACAGCGAATTTGCTTGGCCT GGAGCTTCTTCTTGGGCTCTCAATGGACGTAATAGCTTGATGAATCCTGGACCTGCT ATGGCCAGCCACAAAGAAGGAGAGGACCGTTTCTTTCCTTTGTCTGGATCTTTAATT TTTGGCAAACAAGGAACTGGAAGAGACAACGTGGATGCGGACAAAGTCATGATAAC CAACGAAGAAGAAATTAAAACTACTAACCCGGTAGCAACGGAGTCCTATGGACAAG TGGCCACAAACCACCAGAGTGCCCAAGCACAGGCGCAGACCGGCTGGGTTCAAAAC CAAGGAATACTTCCGGGTATGGTTTGGCAGGACAGAGATGTGTACCTGCAAGGACC CATTTGGGCCAAAATTCCTCACACGGACGGCAACTTTCACCCTTCTCCGCTGATGGG AGGGTTTGGAATGAAGCACCCGCCTCCTCAGATCCTCATCAAAAACACACCTGTACC TGCGGATCCTCCAACGGCCTTCAACAAGGACAAGCTGAACTCTTTCATCACCCAGTA TTCTACTGGCCAAGTCAGCGTGGAGATCGAGTGGGAGCTGCAGAAGGAAAACAGCA AGCGCTGGAACCCGGAGATCCAGTACACTTCCAACTATTACAAGTCTAATAATGTTG AATTTGCTGTTAATACTGAAGGTGTATATAGTGAACCCCGCCCCATTGGCACCAGAT ACCTGACTCGTAATCTGTAA |
| **AAV9 - VP3 protein sequence (SEQ ID NO:38):** |
| MASGGGAPVADNNEGADGVGSSSGNWHCDSQWLGDRVITTSTRTWALPTYNNHLYK QISNSTSGGSSNDNAYFGYSTPWGYFDFNRFHCHFSPRDWQRLINNNWGFRPKRLNFKL FNIQVKEVTDNNGVKTIANNLTSTVQVFTDSDYQLPYVLGSAHEGCLPPFPADVFMIPQ YGYLTLNDGSQAVGRSSFYCLEYFPSQMLRTGNNFQFSYEFENVPFHSSYAHSQSLDRL MNPLIDQYLYYLSKTINGSGQNQQTLKFSVAGPSNMAVQGRNYIPGPSYRQQRVSTTVT QNNNSEFAWPGASSWALNGRNSLMNPGPAMASHKEGEDRFFPLSGSLIFGKQGTGRDN VDADKVMITNEEEIKTTNPVATESYGQVATNHQSAQAQAQTGWVQNQGILPGMVWQD RDVYLQGPIWAKIPHTDGNFHPSPLMGGFGMKHPPPQILIKNTPVPADPPTAFNKDKLNS FITQYSTGQVSVEIEWELQKENSKRWNPEIQYTSNYYKSNNVEFAVNTEGVYSEPRPIGT RYLTRNL* |

| **AAV-DJ** |
|---|
| **AAV-DJ- VP1 nucleotide sequence (SEQ ID NO:39):** |
| ATGGCTGCCGATGGTTATCTTCCAGATTGGCTCGAGGACACTCTCTCTGAAGGAATA AGACAGTGGTGGAAGCTCAAACCTGGCCCACCACCACCAAAGCCCGCAGAGCGGCA TAAGGACGACAGCAGGGGTCTTGTGCTTCCTGGGTACAAGTACCTCGGACCCTTCAA CGGACTCGACAAGGGAGAGCCGGTCAACGAGGCAGACGCCGCGGCCCTCGAGCAC GACAAAGCCTACGACCGGCAGCTCGACAGCGGAGACAACCCGTACCTCAAGTACAA CCACGCCGACGCCGAGTTCCAGGAGCGGCTCAAAGAAGATACGTCTTTTGGGGGCA ACCTCGGGCGAGCAGTCTTCCAGGCCAAAAAGAGGCTTCTTGAACCTCTTGGTCTGG TTGAGGAAGCGGCTAAGACGGCTCCTGGAAAGAAGAGGCCTGTAGAGCACTCTCCT GTGGAGCCAGACTCCTCCTCGGGAACCGGAAAGGCGGGCCAGCAGCCTGCAAGAA AAGATTGAATTTTGGTCAGACTGGAGACGCAGACTCAGTCCCAGACCCTCAACCAAT CGGAGAACCTCCCGCAGCCCCCTCAGGTGTGGGATCTCTTACAATGGCTGCAGGCG GTGGCGCACCAATGGCAGACAATAACGAGGGCGCCGACGGAGTGGGTAATTCCTCG GGAAATTGGCATTGCGATTCCACATGGATGGGCGACAGAGTCATCACCACCAGCAC CCGAACCTGGGCCCTGCCCACCTACAACAACCACCTCTACAAGCAAATCTCCAACA GCACATCTGGAGGATCTTCAAATGACAACGCCTACTTCGGCTACAGCACCCCCTGGG GGTATTTTGACTTTAACAGATTCCACTGCCACTTTTCACCACGTGACTGGCAGCGACT CATCAACAACAACTGGGGATTCCGGCCCAAGAGACTCAGCTTCAAGCTCTTCAACAT CCAGGTCAAGGAGGTCACGCAGAATGAAGGCACCAAGACCATCGCCAATAACCTCA CCAGCACCATCCAGGTGTTTACGGACTCGGAGTACCAGCTGCCGTACGTTCTCGGCT CTGCCCACCAGGGCTGCCTGCCTCCGTTCCCGGCGGACGTGTTCATGATTCCCCAGT ACGGCTACCTAACACTCAACAACGGTAGTCAGGCCGTGGGACGCTCCTCCTTCTACT GCCTGGAATACTTTCCTTCGCAGATGCTGAGAACCGGCAACAACTTCCAGTTTACTT ACACCTTCGAGGACGTGCCTTTCCACAGCAGCTACGCCCACAGCCAGAGCTTGGACC GGCTGATGAATCCTCTGATTGACCAGTACCTGTACTACTTGTCTCGGACTCAAACAA CAGGAGGCACGACAAATACGCAGACTCTGGGCTTCAGCCAAGGTGGGCCTAATACA ATGGCCAATCAGGCAAAGAACTGGCTGCCAGGACCCTGTTACCGCCAGCAGCGAGT ATCAAAGACATCTGCGGATAACAACAACAGTGAATACTCGTGGACTGGAGCTACCA AGTACCACCTCAATGGCAGAGACTCTCTGGTGAATCCGGGCCCGGCCATGGCAAGC CACAAGGACGATGAAGAAAAGTTTTTCCTCAGAGCGGGGTTCTCATCTTTGGGAAG CAAGGCTCAGAGAAAACAAATGTGGACATTGAAAAGGTCATGATTACAGACGAAGA GGAAATCAGGACAACCAATCCCGTGGCTACGGAGCAGTATGGTTCTGTATCTACCA ACCTCCAGAGAGGCAACAGACAAGCAGCTACCGCAGATGTCAACACACAAGGCGTT CTTCCAGGCATGGTCTGGCAGGACAGAGATGTGTACCTTCAGGGGCCCATCTGGGCA AAGATTCCACACACGGACGGACATTTTCACCCCTCTCCCCTCATGGGTGGATTCGGA CTTAAACACCCTCCGCCTCAGATCCTGATCAAGAACACGCCTGTACCTGCGGATCCT CCGACCACCTTCAACCAGTCAAAGCTGAACTCTTTCATCACCCAGTATTCTACTGGC CAAGTCAGCGTGGAGATCGAGTGGGAGCTGCAGAAGGAAAACAGCAAGCGCTGGA |
| ACCCCGAGATCCAGTACACCTCCAACTACTACAAATCTACAAGTGTGGACTTTGCTG TTAATACAGAAGGCGTGTACTCTGAACCCCGCCCCATTGGCACCCGTTACCTCACCC GTAATCTGTAA |
| **AAV-DJ - VP1 protein sequence (SEQ ID NO:40):** |
| MAADGYLPDWLEDTLSEGIRQWWKLKPGPPPPKPAERHKDDSRGLVLPGYKYLGPFNG LDKGEPVNEADAAALEHDKAYDRQLDSGDNPYLKYNHADAEFQERLKEDTSFGGNLG RAVFQAKKRLLEPLGLVEEAAKTAPGKKRPVEHSPVEPDSSSGTGKAGQQPARKRLNF GQTGDADSVPDPQPIGEPPAAPSGVGSLTMAAGGGAPMADNNEGADGVGNSSGNWHC DSTWMGDRVITTSTRTWALPTYNNHLYKQISNSTSGGSSNDNAYFGYSTPWGYFDFNR FHCHFSPRDWQRLINNNWGFRPKRLSFKLFNIQVKEVTQNEGTKTIANNLTSTIQVFTDS EYQLPYVLGSAHQGCLPPFPADVFMIPQYGYLTLNNGSQAVGRSSFYCLEYFPSQMLRT GNNFQFTYTFEDVPFHSSYAHSQSLDRLMNPLIDQYLYYLSRTQTTGGTTNTQTLGFSQ GGPNTMANQAKNWLPGPCYRQQRVSKTSADNNNSEYSWTGATKYHLNGRDSLVNPG PAMASHKDDEEKFFPQSGVLIFGKQGSEKTNVDIEKVMITDEEEIRTTNPVATEQYGSVS TNLQRGNRQAATADVNTQGVLPGMVWQDRDVYLQGPIWAKIPHTDGHFHPSPLMGGF GLKHPPPQILIKNTPVPADPPTTFNQSKLNSFITQYSTGQVSVEIEWELQKENSKRWNPEI QYTSNYYKSTSVDFAVNTEGVYSEPRPIGTRYLTRNL* |
| **AAV-DJ - VP2 nucleotide sequence (SEQ ID NO:41):** |
| ACGGCTCCTGGAAAGAAGAGGCCTGTAGAGCACTCTCCTGTGGAGCCAGACTCCTC CTCGGGAACCGGAAAGGCGGGCCAGCAGCCTGCAAGAAAAAGATTGAATTTTGGTC AGACTGGAGACGCAGACTCAGTCCCAGACCCTCAACCAATCGGAGAACCTCCCGCA GCCCCCTCAGGTGTGGGATCTCTTACAATGGCTGCAGGCGGTGGCGCACCAATGGC AGACAATAACGAGGGCGCCGACGGAGTGGGTAATTCCTCGGGAAATTGGCATTGCG ATTCCACATGGATGGGCGACAGAGTCATCACCACCAGCACCCGAACCTGGGCCCTG CCCACCTACAACAACCACCTCTACAAGCAAATCTCCAACAGCACATCTGGAGGATCT TCAAATGACAACGCCTACTTCGGCTACAGCACCCCCTGGGGGTATTTTGACTTTAAC AGATTCCACTGCCACTTTTCACCACGTGACTGGCAGCGACTCATCAACAACAACTGG GGATTCCGGCCCAAGAGACTCAGCTTCAAGCTCTTCAACATCCAGGTCAAGGAGGT CACGCAGAATGAAGGCACCAAGACCATCGCCAATAACCTCACCAGCACCATCCAGG TGTTTACGGACTCGGAGTACCAGCTGCCGTACGTTCTCGGCTCTGCCCACCAGGGCT GCCTGCCTCCGTTCCCGGCGGACGTGTTCATGATTCCCCAGTACGGCTACCTAACAC TCAACAACGGTAGTCAGGCCGTGGACGCTCCTCCTTCTACTGCCTGGAATACTTTC CTTCGCAGATGCTGAGAACCGGCAACAACTTCCAGTTTACTTACACCTTCGAGGACG TGCCTTTCCACAGCAGCTACGCCCACAGCCAGAGCTTGGACCGGCTGATGAATCCTC TGATTGACCAGTACCTGTACTACTTGTCTCGGACTCAAACAACAGGAGGCACGACAA |
| ATACGCAGACTCTGGGCTTCAGCCAAGGTGGGCCTAATACAATGGCCAATCAGGCA AAGAACTGGCTGCCAGGACCCTGTTACCGCCAGCAGCGAGTATCAAAGACATCTGC GGATAACAACAACAGTGAATACTCGTGGACTGGAGCTACCAAGTACCACCTCAATG GCAGAGACTCTCTGGTGAATCCGGGCCCGGCCATGGCAAGCCACAAGGACGATGAA GAAAAGTTTTTCCTCAGAGCGGGGTTCTCATCTTTGGGAAGCAAGGCTCAGAGAAA ACAAATGTGGACATTGAAAAGGTCATGATTACAGACGAAGAGGAAATCAGGACAAC CAATCCCGTGGCTACGGAGCAGTATGGTTCTGTATCTACCAACCTCCAGAGAGGCAA CAGACAAGCAGCTACCGCAGATGTCAACACACAAGGCGTTCTTCCAGGCATGGTCT GGCAGGACAGAGATGTGTACCTTCAGGGGCCCATCTGGGCAAAGATTCCACACACG GACGGACATTTTCACCCCTCTCCCCTCATGGGTGGATTCGGACTTAAACACCCTCCG CCTCAGATCCTGATCAAGAACACGCCTGTACCTGCGGATCCTCCGACCACCTTCAAC CAGTCAAAGCTGAACTCTTTCATCACCCAGTATTCTACTGGCCAAGTCAGCGTGGAG ATCGAGTGGGAGCTGCAGAAGGAAAACAGCAAGCGCTGGAACCCCGAGATCCAGT ACACCTCCAACTACTACAAATCTACAAGTGTGGACTTTGCTGTTAATACAGAAGGCG TGTACTCTGAACCCCGCCCCATTGGCACCCGTTACCTCACCCGTAATCTGTAA |
| **AAV-DJ - VP2 protein sequence (SEQ ID NO:42):** |
| TAPGKKRPVEHSPVEPDSSSGTGKAGQQPARKRLNFGQTGDADSVPDPQPIGEPPAAPS GVGSLTMAAGGGAPMADNNEGADGVGNSSGNWHCDSTWMGDRVITTSTRTWALPTY NNHLYKQISNSTSGGSSNDNAYFGYSTPWGYFDFNRFHCHFSPRDWQRLINNNWGFRP KRLSFKLFNIQVKEVTQNEGTKTIANNLTSTIQVFTDSEYQLPYVLGSAHQGCLPPFPAD VFMIPQYGYLTLNNGSQAVGRSSFYCLEYFPSQMLRTGNNFQFTYTFEDVPFHSSYAHS QSLDRLMNPLIDQYLYYLSRTQTTGGTTNTQTLGFSQGGPNTMANQAKNWLPGPCYRQ QRVSKTSADNNNSEYSWTGATKYHLNGRDSLVNPGPAMASHKDDEEKFFPQSGVLIFG KQGSEKTNVDIEKVMITDEEEIRTTNPVATEQYGSVSTNLQRGNRQAATADVNTQGVLP GMVWQDRDVYLQGPIWAKIPHTDGHFHPSPLMGGFGLKHPPPQILIKNTPVPADPPTTF NQSKLNSFITQYSTGQVSVEIEWELQKENSKRWNPEIQYTSNYYKSTSVDFAVNTEGVY SEPRPIGTRYLTRNL* |
| **AAV-DJ - VP3 nucleotide sequence (SEQ ID NO:43):** |
| ATGGCTGCAGGCGGTGGCGCACCAATGGCAGACAATAACGAGGGCGCCGACGGAG TGGGTAATTCCTCGGGAAATTGGCATTGCGATTCCACATGGATGGGCGACAGAGTCA TCACCACCAGCACCCGAACCTGGGCCCTGCCCACCTACAACAACCACCTCTACAAGC AAATCTCCAACAGCACATCTGGAGGATCTTCAAATGACAACGCCTACTTCGGCTACA GCACCCCCTGGGGGTATTTTGACTTTAACAGATTCCACTGCCACTTTTCACCACGTGA CTGGCAGCGACTCATCAACAACAACTGGGGATTCCGGCCCAAGAGACTCAGCTTCA AGCTCTTCAACATCCAGGTCAAGGAGGTCACGCAGAATGAAGGCACCAAGACCATC |
| GCCAATAACCTCACCAGCACCATCCAGGTGTTTACGGACTCGGAGTACCAGCTGCCG TACGTTCTCGGCTCTGCCCACCAGGGCTGCCTGCCTCCGTTCCCGGCGGACGTGTTC ATGATTCCCCAGTACGGCTACCTAACACTCAACAACGGTAGTCAGGCCGTGGACG CTCCTCCTTCTACTGCCTGGAATACTTTCCTTCGCAGATGCTGAGAACCGGCAACAA CTTCCAGTTTACTTACACCTTCGAGGACGTGCCTTTCCACAGCAGCTACGCCCACAG CCAGAGCTTGGACCGGCTGATGAATCCTCTGATTGACCAGTACCTGTACTACTTGTC TCGGACTCAAACAACAGGAGGCACGACAAATACGCAGACTCTGGGCTTCAGCCAAG GTGGGCCTAATACAATGGCCAATCAGGCAAAGAACTGGCTGCCAGGACCCTGTTAC CGCCAGCAGCGAGTATCAAAGACATCTGCGGATAACAACAACAGTGAATACTCGTG GACTGGAGCTACCAAGTACCACCTCAATGGCAGAGACTCTCTGGTGAATCCGGGCC CGGCCATGGCAAGCCACAAGGACGATGAAGAAAAGTTTTTCCTCAGAGCGGGGTT CTCATCTTTGGGAAGCAAGGCTCAGAGAAAACAAATGTGGACATTGAAAAGGTCAT GATTACAGACGAAGAGGAAATCAGGACAACCAATCCCGTGGCTACGGAGCAGTATG GTTCTGTATCTACCAACCTCCAGAGAGGCAACAGACAAGCAGCTACCGCAGATGTC AACACACAAGGCGTTCTTCCAGGCATGGTCTGGCAGGACAGAGATGTGTACCTTCA GGGGCCCATCTGGGCAAAGATTCCACACACGGACGGACATTTTCACCCCTCTCCCCT CATGGGTGGATTCGGACTTAAACACCCTCCGCCTCAGATCCTGATCAAGAACACGCC TGTACCTGCGGATCCTCCGACCACCTTCAACCAGTCAAAGCTGAACTCTTTCATCAC CCAGTATTCTACTGGCCAAGTCAGCGTGGAGATCGAGTGGGAGCTGCAGAAGGAAA ACAGCAAGCGCTGGAACCCCGAGATCCAGTACACCTCCAACTACTACAAATCTACA AGTGTGGACTTTGCTGTTAATACAGAAGGCGTGTACTCTGAACCCCGCCCCATTGGC ACCCGTTACCTCACCCGTAATCTGTAA |
| **AAV-DJ - VP3 protein sequence (SEQ ID NO:44):** |
| MAAGGGAPMADNNEGADGVGNSSGNWHCDSTWMGDRVITTSTRTWALPTYNNHLY KQISNSTSGGSSNDNAYFGYSTPWGYFDFNRFHCHFSPRDWQRLINNNWGFRPKRLSFK LFNIQVKEVTQNEGTKTIANNLTSTIQVFTDSEYQLPYVLGSAHQGCLPPFPADVFMIPQ YGYLTLNNGSQAVGRSSFYCLEYFPSQMLRTGNNFQFTYTFEDVPFHSSYAHSQSLDRL MNPLIDQYLYYLSRTQTTGGTTNTQTLGFSQGGPNTMANQAKNWLPGPCYRQQRVSKT SADNNNSEYSWTGATKYHLNGRDSLVNPGPAMASHKDDEEKFFPQSGVLIFGKQGSEK TNVDIEKVMITDEEEIRTTNPVATEQYGSVSTNLQRGNRQAATADVNTQGVLPGMVWQ DRDVYLQGPIWAKIPHTDGHFHPSPLMGGFGLKHPPPQILIKNTPVPADPPTTFNQSKLN SFITQYSTGQVSVEIEWELQKENSKRWNPEIQYTSNYYKSTSVDFAVNTEGVYSEPRPIG TRYLTRNL* |

### Transcription factors

| **Factor** | **Accession number** |
|---|---|
| Foxa1 | NM_008259.3 |
| Foxa2 | NM_010446.3 |
| Foxa3 | NM_008260.2 |
| Gata4 | NM_008092.3 |
| Hnf1a | BC080698.1 |
| Hnf4a | NM_008261.2 |

**Foxa1 nucleotide sequence (SEO ID NO:45):** 
ATGTTAGGGACTGTGAAGATGGAAGGGCATGAGAGCAACGACTGGAACAGCTACTA
CGCGGACACGCAGGAGGCCTACTCCTCTGTCCCTGTCAGCAACATGAACTCCGGCCT
GGGCTCTATGAACTCCATGAACACCTACATGACCATGAACACCATGACCACGAGCG
GCAACATGACCCCGGCTTCCTTCAACATGTCCTACGCCAACACGGGCTTAGGGGCCG
GCCTGAGTCCCGGTGCTGTGGCTGGCATGCCAGGGGCCTCTGCAGGCGCCATGAAC
AGCATGACTGCGGCGGGCGTCACGGCCATGGGTACGGCGCTGAGCCCGGGAGGCAT
GGGCTCCATGGGCGCGCAGCCCGCCACCTCCATGAACGGCCTGGGTCCCTACGCCG
CCGCCATGAACCCGTGCATGAGTCCCATGGCGTACGCGCCGTCCAACCTGGGCCGC
AGCCGCGCGGGGGGCGGCGGCGACGCCAAGACATTCAAGCGCAGCTACCCTCACGC
CAAGCCGCCTTACTCCTACATCTCGCTCATCACGATGGCCATCCAGCAGGCGCCCAG
CAAGATGCTCACGCTGAGCGAGATCTACCAGTGGATCATGGACCTCTTCCCCTATTA
CCGCCAGAACCAGCAGCGCTGGCAGAACTCCATCCGCCACTCGCTGTCCTTCAACGA
TTGTTTCGTCAAGGTGGCACGATCCCCGGACAAGCCAGGCAAGGGCTCCTACTGGA
CGCTGCACCCGGACTCCGGCAACATGTTCGAGAACGGCTGCTACTTGCGCCGCCAA
AAGCGCTTCAAGTGTGAGAAGCAGCCGGGGGCCGGAGGTGGAGTGGGGGCGGCG
GCTCCAAAGGGGGCCCAGAAAGTCGCAAGGACCCCTCAGGCCCGGGGAACCCCAGC
GCCGAGTCACCCCTTCACCGGGGTGTGCACGGAAAGGCTAGCCAGCTAGAGGGCGC
GCCGGCCCCAGGGCCCGCCGCCAGCCCCCAGACTCTGGACCACAGCGGGGCCACGG
CGACAGGGGGCGCTTCGGAGTTGAAGTCTCCAGCGTCTTCATCTGCGCCCCCCATAA
GCTCCGGGCCAGGGGCGCTAGCATCTGTACCCCCCTCTCACCCGGCTCACGGCCTGG
CACCCCACGAATCTCAGCTGCATCTGAAAGGGGATCCCCACTACTCCTTTAATCACC
CCTTCTCCATCAACAACCTCATGTCCTCCTCCGAGCAACAGCACAAGCTGGACTTCA
AGGCATACGAGCAGGCGCTGCAGTACTCTCCTTATGGCGCTACCTTGCCCGCCAGTC
TGCCCCTTGGCAGCGCCTCAGTGGCCACGAGGAGCCCCATCGAGCCCTCAGCCCTGG
AGCCAGCCTACTACCAAGGTGTGTATTCCAGACCCGTGCTAAATACTTCCTAG
**Foxa1 protein sequence (SEQ ID NO:46):** 
MLGTVKMEGHESNDWNSYYADTQEAYSSVPVSNMNSGLGSMNSMNTYMTMNTMTT
SGNMTPASFNMSYANTGLGAGLSPGAVAGMPGASAGAMNSMTAAGVTAMGTALSPG
104
 
GMGSMGAQPATSMNGLGPYAAAMNPCMSPMAYAPSNLGRSRAGGGGDAKTFKRSYP
HAKPPYSYISLITMAIQQAPSKMLTLSEIYQWIMDLFPYYRQNQQRWQNSIRHSLSFNDC
FVKVARSPDKPGKGSYWTLHPDSGNMFENGCYLRRQKRFKCEKQPGAGGGSGGGGSK
GGPESRKDPSGPGNPSAESPLHRGVHGKASQLEGAPAPGPAASPQTLDHSGATATGGAS
ELKSPASSSAPPISSGPGALASVPPSHPAHGLAPHESQLHLKGDPHYSFNHPFSINNLMSSS
EQQHKLDFKAYEQALQYSPYGATLPASLPLGSASVATRSPIEPSALEPAYYQGVYSRPVL
NTS*
**Foxa2 nucleotide sequence (SEQ ID NO:47):** 
ATGCTGGGAGCCGTGAAGATGGAAGGGCACGAGCCATCCGACTGGAGCAGCTACTA
CGCGGAGCCCGAGGGCTACTCTTCCGTGAGCAACATGAACGCCGGCCTGGGGATGA
ATGGCATGAACACATACATGAGCATGTCCGCGGCTGCCATGGGCGGCGGTTCCGGC
AACATGAGCGCGGGCTCCATGAACATGTCATCCTATGTGGGCGCTGGAATGAGCCC
GTCGCTAGCTGGCATGTCCCCGGGCGCCGGCGCCATGGCGGGCATGAGCGGCTCAG
CCGGGGCGGCCGGCGTGGCGGGCATGGGACCTCACCTGAGTCCGAGTCTGAGCCCG
CTCGGGGGACAGGCGGCCGGGGCCATGGGTGGCCTTGCCCCCTACGCCAACATGAA
CTCGATGAGCCCCATGTACGGGCAGGCCGGCCTGAGCCGCGCTCGGGACCCCAAGA
CATACCGACGCAGCTACACACACGCCAAACCTCCCTACTCGTACATCTCGCTCATCA
CCATGGCCATCCAGCAGAGCCCCAACAAGATGCTGACGCTGAGCGAGATCTATCAG
TGGATCATGGACCTCTTCCCTTTCTACCGGCAGAACCAGCAGCGCTGGCAGAACTCC
ATCCGCCACTCTCTCTCCTTCAACGACTGCTTTCTCAAGGTGCCCCGCTCGCCAGACA
AGCCTGGCAAGGGCTCCTTCTGGACCCTGCACCCAGACTCGGGCAACATGTTCGAG
AACGGCTGCTACCTGCGCCGCCAGAAGCGCTTCAAGTGTGAGAAGCAACTGGCACT
GAAGGAAGCCGCGGGTGCGGCCAGTAGCGGAGGCAAGAAGACCGCTCCTGGGTCC
CAGGCCTCTCAGGCTCAGCTCGGGGAGGCCGCGGGCTCGGCCTCCGAGACTCCGGC
GGGCACCGAGTCCCCCCATTCCAGCGCTTCTCCGTGTCAGGAGCACAAGCGAGGTG
GCCTAAGCGAGCTAAAGGGAGCACCTGCCTCTGCGCTGAGTCCTCCCGAGCCGGCG
CCCTCGCCTGGGCAGCAGCAGCAGGCTGCAGCCCACCTGCTGGGCCCACCTCACCA
CCCAGGCCTGCCACCAGAGGCCCACCTGAAGCCCGAGCACCATTACGCCTTCAACC
ACCCCTTCTCTATCAACAACCTCATGTCGTCCGAGCAGCAACATCACCACAGCCACC
ACCACCATCAGCCCCACAAAATGGACCTCAAGGCCTACGAACAGGTCATGCACTAC
CCAGGGGGCTATGGTTCCCCCATGCCAGGCAGCTTGGCCATGGGCCCAGTCACGAA
CAAAGCGGGCCTGGATGCCTCGCCCCTGGCTGCAGACACTTCCTACTACCAAGGAGT
GTACTCCAGGCCTATTATGAACTCATCCTAG
**Foxa2 protein sequence (SEQ ID NO:48):** 
MLGAVKMEGHEPSDWSSYYAEPEGYSSVSNMNAGLGMNGMNTYMSMSAAAMGGGS
GNMSAGSMNMSSYVGAGMSPSLAGMSPGAGAMAGMSGSAGAAGVAGMGPHLSPSLS
PLGGQAAGAMGGLAPYANMNSMSPMYGQAGLSRARDPKTYRRSYTHAKPPYSYISLIT
MAIQQSPNKMLTLSEIYQWIMDLFPFYRQNQQRWQNSIRHSLSFNDCFLKVPRSPDKPG
KGSFWTLHPDSGNMFENGCYLRRQKRFKCEKQLALKEAAGAASSGGKKTAPGSQASQ
AQLGEAAGSASETPAGTESPHSSASPCQEHKRGGLSELKGAPASALSPPEPAPSPGQQQQ
AAAHLLGPPHHPGLPPEAHLKPEHHYAFNHPFSINNLMSSEQQHHHSHHHHQPHKMDL
KAYEQVMHYPGGYGSPMPGSLAMGPVTNKAGLDASPLAADTSYYQGVYSRPIMNSS*
**Foxa3 nucleotide sequence (SEQ ID NO:49):** 
ATGCTGGGCTCAGTGAAGATGGAGGCTCATGACCTGGCCGAGTGGAGCTACTACCC
GGAGGCGGGCGAGGTGTATTCTCCAGTGAATCCTGTGCCCACCATGGCCCCTCTCAA
CTCCTACATGACCTTGAACCCACTCAGCTCTCCCTACCCTCCCGGAGGGCTTCAGGC
CTCCCCACTGCCTACAGGACCCCTGGCACCCCCAGCCCCCACTGCGCCCTTGGGGCC
CACCTTCCCAAGCTTGGGCACTGGTGGCAGCACCGGAGGCAGTGCTTCCGGGTGTGT
AGCCCCAGGGCCCGGGCTTGTACATGGAAAAGAGATGGCAAAGGGGTACCGGCGG
CCACTGGCCCACGCCAAACCACCATATTCCTACATCTCTCTCATAACCATGGCTATT
CAGCAGGCTCCAGGCAAGATGCTGACCCTGAGTGAAATCTACCAATGGATCATGGA
CCTCTTCCCGTACTACCGGGAGAACCAGCAACGTTGGCAGAACTCCATCCGGCATTC
ACTGTCCTTCAATGACTGCTTCGTCAAGGTGGCACGCTCCCCAGACAAGCCAGGCAA
AGGCTCCTACTGGGCCTTGCATCCCAGCTCTGGGAACATGTTTGAGAACGGCTGCTA
TCTCCGCCGGCAGAAGCGCTTCAAGCTGGAGGAGAAGGCAAAGAAAGGAAACAGC
GCCATATCGGCCAGCAGGAATGGTACTGCGGGGTCAGCCACCTCTGCCACCACTAC
AGCTGCCACTGCAGTCACCTCCCCGGCTCAGCCCCAGCCTACGCCATCTGAGCCCGA
GGCCCAGAGTGGGGATGATGTGGGGGGTCTGGACTGCGCCTCACCTCCTTCGTCCAC
ACCTTATTTCAGCGGCCTGGAGCTCCCGGGGGAACTAAAGTTGGATGCGCCCTATAA
CTTCAACCACCCTTTCTCTATCAACAACCTGATGTCAGAACAGACATCGACACCTTC
CAAACTGGATGTGGGGTTTGGGGGCTACGGGGCTGAGAGTGGGGAGCCTGGAGTCT
ACTACCAGAGCCTCTATTCCCGCTCTCTGCTTAATGCATCCTAG
**Foxa3 protein sequence (SEQ ID NO:50):** 
MLGSVKMEAHDLAEWSYYPEAGEVYSPVNPVPTMAPLNSYMTLNPLSSPYPPGGLQAS
PLPTGPLAPPAPTAPLGPTFPSLGTGGSTGGSASGCVAPGPGLVHGKEMAKGYRRPLAH
AKPPYSYISLITMAIQQAPGKMLTLSEIYQWIMDLFPYYRENQQRWQNSIRHSLSFNDCF
VKVARSPDKPGKGSYWALHPSSGNMFENGCYLRRQKRFKLEEKAKKGNSAISASRNGT
AGSATSATTTAATAVTSPAQPQPTPSEPEAQSGDDVGGLDCASPPSSTPYFSGLELPGEL
KLDAPYNFNHPFSINNLMSEQTSTPSKLDVGFGGYGAESGEPGVYYQSLYSRSLLNAS*
**Gata4 nucleotide sequence (SEQ ID NO:51):** 
ATGTACCAGAGCCTGGCGATGGCGGCCAATCACGGTCCTCCGCCTGGAGCTTACGA
GGCCGGAGGGCCAGGCGCGTTCATGCACTCCGCCGGAGCGGCCTCTAGTCCGGTGT
ACGTGCCCACACCCAGGGTGCCCTCAAGCGTGCTCGGCCTCAGCTACCTGCAAGGA
GGCGGTAGTGCTGCGGCGGCTGGTACGACGAGTGGCGGGTCTAGTGGAGCCGGTCC
TAGCGGGGCTGGTCCGGGAACCCAACAAGGAAGCCCTGGATGGAGCCAGGCAGGC
GCAGAGGGGGCAGCCTACACACCCCCACCCGTGAGCCCCAGGTTTAGCTTCCCAGG
CACGACAGGGAGTCTTGCAGCGGCAGCGGCAGCGGCCGCAGCCCGAGAAGCAGCC
GCGTATGGGAGCGGGGGAGGTGCTGCCGGTGCAGGCCTCGCAGGGAGGGAACAGT
ACGGTAGGCCGGGATTCGCCGGTTCCTACAGTAGCCCCTACCCCGCCTATATGGCCG
ATGTGGGGGCGAGCTGGGCTGCAGCAGCAGCAGCTTCCGCGGGACCCTTCGACTCC
CCAGTGTTGCATAGCCTGCCGGGGAGGGCTAACCCAGGTAGACACCCCAACCTGGA
CATGTTCGACGACTTCTCAGAGGGCCGAGAGTGCGTTAACTGCGGTGCTATGAGCAC
CCCACTGTGGAGGCGCGATGGGACGGGTCACTACCTGTGTAATGCATGCGGCCTGTA
CCACAAGATGAACGGCATCAACAGACCGCTCATCAAACCCCAGAGGAGGCTCAGCG
CAAGCCGCAGGGTGGGCCTGAGCTGTGCCAACTGCCAGACCACCACGACCACGCTG
TGGCGCAGGAACGCAGAAGGAGAACCCGTGTGCAACGCTTGCGGACTGTATATGAA
ACTGCACGGCGTCCCCAGGCCCTTGGCAATGAGGAAGGAGGGGATCCAAACCAGGA
AGCGAAAGCCGAAGAACCTGAACAAGAGTAAGACCCCAGCAGGTCCTGCGGGTGA
AACTCTGCCACCGTCCAGTGGTGCGAGCTCCGGTAACTCTTCAAATGCTACCAGTTC
CTCTAGCAGCTCTGAGGAAATGAGGCCCATCAAGACCGAACCCGGTTTGTCCAGCC
ATTACGGGCACAGTTCAAGCATGTCCCAAACCTTCAGCACAGTGTCCGGCCACGGCC
CGAGCATTCATCCCGTGCTTAGCGCCTTGAAGTTGAGTCCTCAGGGCTACGCCAGTC
CCGTGACTCAGACGTCCCAAGCGAGCAGCAAGCAGGACTCCTGGAACAGCCTGGTG
CTGGCCGACAGCCACGGAGACATCATCACCGCCTAG
**Gata4 protein sequence (SEQ ID NO:52):** 
MYQSLAMAANHGPPPGAYEAGGPGAFMHSAGAASSPVYVPTPRVPSSVLGLSYLQGG
GSAAAAGTTSGGSSGAGPSGAGPGTQQGSPGWSQAGAEGAAYTPPPVSPRFSFPGTTGS
LAAAAAAAAAREAAAYGSGGGAAGAGLAGREQYGRPGFAGSYSSPYPAYMADVGAS
WAAAAAASAGPFDSPVLHSLPGRANPGRHPNLDMFDDFSEGRECVNCGAMSTPLWRR
DGTGHYLCNACGLYHKMNGINRPLIKPQRRLSASRRVGLSCANCQTTTTTLWRRNAEG
EPVCNACGLYMKLHGVPRPLAMRKEGIQTRKRKPKNLNKSKTPAGPAGETLPPSSGASS
GNSSNATSSSSSSEEMRPIKTEPGLSSHYGHSSSMSQTFSTVSGHGPSIHPVLSALKLSPQG
YASPVTQTSQASSKQDSWNSLVLADSHGDIITA*
**Hnflalpha nucleotide sequence (SEQ ID NO:53):** 
ATGGTTTCTAAGCTGAGCCAGCTGCAGACGGAGCTCCTGGCTGCCCTGCTCGAGTCT
GGCCTGAGCAAAGAGGCCCTGATCCAGGCCTTGGGGGAGCCAGGGCCCTACCTGAT
GGTTGGAGAGGGTCCCCTGGACAAGGGGGAGTCCTGCGGTGGGAGTCGAGGGGACC
TGACCGAGTTGCCTAATGGCCTTGGAGAAACGCGTGGCTCTGAAGATGACACGGAT
GACGATGGGGAAGACTTCGCGCCACCCATTCTGAAAGAGCTGGAGAACCTCAGCCC
AGAGGAGGCAGCCCACCAGAAAGCCGTGGTGGAGTCACTTCTTCAGGAGGACCCAT
GGCGCGTGGCGAAGATGGTCAAGTCGTACTTGCAGCAGCACAACATCCCCCAGCGG
GAGGTGGTGGACACCACGGGTCTCAACCAGTCCCACCTGTCACAGCACCTCAACAA
GGGCACACCCATGAAGACACAGAAGCGGGCCGCTCTGTACACCTGGTACGTCCGCA
AGCAGCGAGAGGTGGCTCAGCAATTCACCCACGCGGGGCAGGGCGGACTGATTGAA
GAGCCCACAGGCGATGAGCTGCCAACTAAGAAGGGGCGTAGGAACCGGTTCAAGTG
GGGCCCCGCATCCCAGCAGATCCTGTTCCAGGCCTACGAGAGGCAAAAAGCCCCA
GCAAGGAAGAGCGAGAGACCTTGGTGGAGGAGTGTAATAGGGCGGAGTGCATCCA
GAGGGGGGTGTCACCATCGCAGGCCCAGGGGCTAGGCTCCAACCTTGTCACGGAGG
TGCGTGTCTACAACTGGTTTGCCAACCGGCGCAAGGAGGAAGCCTTCCGGCACAAG
TTGGCCATGGACACCTATAACGGACCTCCACCGGGGCCAGGCCCGGGCCCTGCGCT
GCCTGCTCACAGTTCCCCCGGCCTGCCCACAACCACCCTCTCTCCCAGTAAGGTCCA
CGGTGTACGGTACGGACAGTCTGCAACCAGTGAGGCAGCCGAGGTGCCCTCCAGCA
GCGGAGGTCCCTTAGTCACAGTGTCTGCGGCCTTACACCAGGTATCCCCCACAGGCC
TGGAGCCCAGCAGCCTGCTGAGCACAGAGGCCAAGCTGGTCTCAGCCACGGGGGGT
CCCCTGCCTCCCGTCAGCACCCTGACAGCACTGCACAGCTTGGAGCAGACATCCCG
GGTCTCAACCAGCAGCCGCAGAACCTTATCATGGCCTCGCTACCTGGGGTCATGACC
ATCGGGCCCGGGGAGCCTGCCTCCCTGGGACCCACGTTCACGAACACGGGCGCCTC
CACCCTGGTTATCGGTCTGGCCTCCACTCAGGCACAGAGCGTGCCTGTCATCAACAG
CATGGGGAGTAGCCTGACCACGCTGCAGCCGGTCCAGTTTTCCCAACCACTGCATCC
CTCCTATCAGCAGCCTCTCATGCCCCCCGTACAGAGCCACGTGGCCCAGAGCCCCTT
CATGGCAACCATGGCCCAGCTGCAGAGCCCCCACGCCTTATACAGCCACAAGCCTG
AGGTGGCCCAGTACACGCACACCAGCCTGCTCCCGCAGACCATGTTGATCACAGAC
ACCAACCTCAGCACCCTTGCCAGCCTCACACCCACCAAGCAGGTCTTCACCTCAGAC
ACAGAGGCCTCCAGTGAGCCCGGGCTTCACGAGCCACCCTCTCCAGCCACCACCATC
CACATCCCCAGCCAGGACCCGTCGAACATCCAGCACCTGCAGCCTGCTCACCGGCTC
AGCACCAGTCCCACAGTGTCCTCCAGCAGCCTGGTGTTGTATCAGAGTTCCGACTCC
AACGGGCACAGCCACCTGCTGCCATCCAACCATAGTGTCATCGAGACTTTTATCTCC
ACCCAGATGGCCTCCTCTTCCCAGTAG
**Hnflalpha protein sequence (SEQ ID NO:54):** 
MVSKLSQLQTELLAALLESGLSKEALIQALGEPGPYLMVGEGPLDKGESCGGSRGDLTE
LPNGLGETRGSEDDTDDDGEDFAPPILKELENLSPEEAAHQKAVVESLLQEDPWRVAKM
VKSYLQQHNIPQREVVDTTGLNQSHLSQHLNKGTPMKTQKRAALYTWYVRKQREVAQ
QFTHAGQGGLIEEPTGDELPTKKGRRNRFKWGPASQQILFQAYERQKSPSKEERETLVEE
CNRAECIQRGVSPSQAQGLGSNLVTEVRVYNWFANRRKEEAFRHKLAMDTYNGPPPGP
GPGPALPAHSSPGLPTTTLSPSKVHGVRYGQSATSEAAEVPSSSGGPLVTVSAALHQVSP
TGLEPSSLLSTEAKLVSATGGPLPPVSTLTALHSLEQTSPGLNQQPQNLIMASLPGVMTIG
PGEPASLGPTFTNTGASTLVIGLASTQAQSVPVINSMGSSLTTLQPVQFSQPLHPSYQQPL
MPPVQSHVAQSPFMATMAQLQSPHALYSHKPEVAQYTHTSLLPQTMLITDTNLSTLASL
TPTKQVFTSDTEASSEPGLHEPPSPATTIHIPSQDPSNIQHLQPAHRLSTSPTVSSSSLVLYQ
SSDSNGHSHLLPSNHSVIETFISTQMASSSQ*
**HNF6 (human nucleic acid sequence) (SEQ ID NO:55)** 
ccccacagtg agaggaagga aggcaacagt cgccagcagc cgatgtgaag accggactcc
gtgcgcccct cgccgcctct gcctggccac atcgatgttg tgtccgccgc ctgctcgccc
ggatcacgat gaacgcgcag ctgaccatgg aagcgatcgg cgagctgcac ggggtgagcc
atgagccggt gcccgcccct gccgacctgc tgggcggcag cccccacgcg cgcagctccg
tggcgcaccg cggcagccac ctgccccccg cgcacccgcg ctccatgggc atggcgtccc
tgctggacgg cggcagcggc ggcggagatt accaccacca ccaccgggcc cctgagcaca
gcctggccgg ccccctgcat cccaccatga ccatggcctg cgagactccc ccaggtatga
gcatgcccac cacctacacc accttgaccc ctctgcagcc gctgcctccc atctccacag
tctcggacaa gttcccccac catcaccacc accaccatca ccaccaccac ccgcaccacc
accagcgcct ggcgggcaac gtgagcggta gcttcacgct catgcgggat gagcgcgggc
tggcctccat gaataacctc tataccccct accacaagga cgtggccggc atgggccaga
gcctctcgcc cctctccagc tccggtctgg gcagcatcca caactcccag caagggctcc
cccactatgc ccacccgggg gccgccatgc ccaccgacaa gatgctcacc cccaacggct
tcgaagccca ccacccggcc atgctcggcc gccacgggga gcagcacctc acgcccacct
cggccggcat ggtgcccatc aacggccttc ctccgcacca tccccacgcc cacctgaacg
cccagggcca cgggcaactc ctgggcacag cccgggagcc caacccttcg gtgaccggcg
cgcaggtcag caatggaagt aattcagggc agatggaaga gatcaatacc aaagaggtgg
cgcagcgtat caccaccgag ctcaagcgct acagcatccc acaggccatc ttcgcgcaga
gggtgctctg ccgctcccag ggcaccctct cggacctgct gcgcaacccc aaaccctgga
gcaaactcaa atccggccgg gagaccttcc ggaggatgtg gaagtggctg caggagccgg
agttccagcg catgtccgcg ctccgcttag cagcatgcaa aaggaaagaa caagaacatg
ggaaggatag aggcaacaca cccaaaaagc ccaggttggt cttcacagat gtccagcgtc
gaactctaca tgcaatattc aaggaaaata agcgtccatc caaagaattg caaatcacca
tttcccagca gctggggttg gagctgagca ctgtcagcaa cttcttcatg aacgcaagaa
ggaggagtct ggacaagtgg caggacgagg gcagctccaa ttcaggcaac tcatcttctt
catcaagcac ttgtaccaaa gcatgaagga agaaccacaa actaaaacct cggtggaaaa
gctttaaatt aaaaaaaatt tttaaaagac caggacctca agatagcagg tttatactta
gaaatatttg aagaaaaaaa agcgttattt atagtccaaa gaaaccaaag acttagctca
cctgcattct gactttgttt ggagacacac acttcagcag ggcggcgact tggcaagaca
 
aatgatgagc aggaaaacac cactggatct cacaccttca atccatgacc atcctcgctg
tgcttggctg tttagtggtt tggagcatag tgattttgag ccattgagcg gacatctttt
aagatcgaac tttctcatct gttctaccat gccacgaagg tgtatggtgt ctcagtacta
ccacc
**HNF6 (human amino acid sequence) (SEQ ID NO:56)** 
MNAQLTMEAIGELHGVSHEPVPAPADLLGGSPHARSSVAHRGSH
LPPAHPRSMGMASLLDGGSGGGDYHHHHRAPEHSLAGPLHPTMTMACETPPGMSMPTT
YTTLTPLQPLPPISTVSDKFPHHHHHHHHHHHPHHHQRLAGNVSGSFTLMRDERGLAS
MNNLYTPYHKDVAGMGQSLSPLSSSGLGSIHNSQQGLPHYAHPGAAMPTDKMLTPNGF
EAHHPAMLGRHGEQHLTPTSAGMVPINGLPPHHPHAHLNAQGHGQLLGTAREPNPSVT
GAQVSNGSNSGQMEEINTKEVAQRITTELKRYSIPQAIFAQRVLCRSQGTLSDLLRNP
KPWSKLKSGRETFRRMWKWLQEPEFQRMSALRLAACKRKEQEHGKDRGNTPKKPRLVF
TDVQRRTLHAIFKENKRPSKELQITISQQLGLELSTVSNFFMNARRRSLDKWQDEGSS
NSGNSSSSSSTCTKA
**HLF (human nucleic acid sequence) (SEQ ID NO:57)** 
1 atggagaaaa tgtcccgacc gctccccctg aatcccacct ttatcccgcc tccctacggc
61 gtgctcaggt ccctgctgga gaacccgctg aagctccccc ttcaccacga agacgcattt
121 agtaaagata aagacaagga aaagaagctg gatgatgaga gtaacagccc gacggtcccc
181 cagtcggcat tcctggggcc taccttatgg gacaaaaccc ttccctatga cggagatact
241 ttccagttgg aatacatgga cctggaggag tttttgtcag aaaatggcat tccccccagc
301 ccatctcagc atgaccacag ccctcaccct cctgggctgc agccagcttc ctcggctgcc
361 ccctcggtca tggacctcag cagccgggcc tctgcacccc ttcaccctgg catcccatct
421 ccgaactgta tgcagagccc catcagacca ggtcagctgt tgccagcaaa ccgcaataca
481 ccaagtccca ttgatcctga caccatccag gtcccagtgg gttatgagcc agacccagca
541 gatcttgccc tttccagcat ccctggccag gaaatgtttg accctcgcaa acgcaagttc
601 tctgaggaag aactgaagcc acagcccatg atcaagaaag ctcgcaaagt cttcatccct
661 gatgacctga aggatgacaa gtactgggca aggcgcagaa agaacaacat ggcagccaag
721 cgctcccgcg acgcccggag gctgaaagag aaccagatcg ccatccgggc ctcgttcctg
781 gagaaggaga actcggccct ccgccaggag gtggctgact tgaggaagga gctgggcaaa
841 tgcaagaaca tacttgccaa gtatgaggcc aggcacgggc ccctgtag
**HLF (human amino acid sequence) (SEQ ID NO:58)** 
MEKMSRPLPLNPTFIPPPYGVLRSLLENPLKLPLHHEDAFSKDK
DKEKKLDDESNSPTVPQSAFLGPTLWDKTLPYDGDTFQLEYMDLEEFLSENGIPPSPS
QHDHSPHPPGLQPASSAAPSVMDLSSRASAPLHPGIPSPNCMQSPIRPGQLLPANRNT
PSPIDPDTIQVPVGYEPDPADLALSSIPGQEMFDPRKRKFSEEELKPQPMIKKARKVF
IPDDLKDDKYWARRRKNNMAAKRSRDARRLKENQIAIRASFLEKENSALRQEVADLRK
ELGKCKNILAKYEARHGPL
**CEBPA nucleic acid sequence (SEQ ID NO:59)** 
1 tataaaagct gggccggcgc gggccgggcc attcgcgacc cggaggtgcg cgggcgcggg
61 cgagcagggt ctccgggtgg gcggcggcga cgccccgcgc aggctggagg ccgccgaggc
121 tcgccatgcc gggagaactc taactccccc atggagtcgg ccgacttcta cgaggcggag
181 ccgcggcccc cgatgagcag ccacctgcag agccccccgc acgcgcccag cagcgccgcc
241 ttcggctttc cccggggcgc gggccccgcg cagcctcccg ccccacctgc cgccccggag
301 ccgctgggcg gcatctgcga gcacgagacg tccatcgaca tcagcgccta catcgacccg
361 gccgccttca acgacgagtt cctggccgac ctgttccagc acagccggca gcaggagaag
421 gccaaggcgg ccgtgggccc cacgggcggc ggcggcggcg gcgactttga ctacccgggc
481 gcgcccgcgg gccccggcgg cgccgtcatg cccgggggag cgcacgggcc cccgcccggc
541 tacggctgcg cggccgccgg ctacctggac ggcaggctgg agcccctgta cgagcgcgtc
601 ggggcgccgg cgctgcggcc gctggtgatc aagcaggagc cccgcgagga ggatgaagcc
661 aagcagctgg cgctggccgg cctcttccct taccagccgc cgccgccgcc gccgccctcg
721 cacccgcacc cgcacccgcc gcccgcgcac ctggccgccc cgcacctgca gttccagatc
781 gcgcactgcg gccagaccac catgcacctg cagcccggtc accccacgcc gccgcccacg
841 cccgtgccca gcccgcaccc cgcgcccgcg ctcggtgccg ccggcctgcc gggccctggc
901 agcgcgctca aggggctggg cgccgcgcac cccgacctcc gcgcgagtgg cggcagcggc
961 gcgggcaagg ccaagaagtc ggtggacaag aacagcaacg agtaccgggt gcggcgcgag
1021 cgcaacaaca tcgcggtgcg caagagccgc gacaaggcca agcagcgcaa cgtggagacg
1081 cagcagaagg tgctggagct gaccagtgac aatgaccgcc tgcgcaagcg ggtggaacag
1141 ctgagccgcg aactggacac gctgcggggc atcttccgcc agctgccaga gagctccttg
1201 gtcaaggcca tgggcaactg cgcgtgaggc gcgcggctgt gggaccgccc tgggccagcc
1261 tccggcgggg acccagggag tggtttgggg tcgccggatc tcgaggcttg cccgagccgt
1321 gcgagccagg actaggagat tccggtgcct cctgaaagcc tggcctgctc cgcgtgtccc
1381 ctcccttcct ctgcgccgga cttggtgcgt ctaagatgag ggggccaggc ggtggcttct
1441 ccctgcgagg aggggagaat tcttggggct gagctgggag cccggcaact ctagtattta
1501 ggataacctt gtgccttgga aatgcaaact caccgctcca atgcctactg agtaggggga
1561 gcaaatcgtg ccttgtcatt ttatttggag gtttcctgcc tccttcccga ggctacagca
1621 gacccccatg agagaaggag gggagcaggc ccgtggcagg aggagggctc agggagctga
1681 gatcccgaca agcccgccag ccccagccgc tcctccacgc ctgtccttag aaaggggtgg
1741 aaacataggg acttggggct tggaacctaa ggttgttccc ctagttctac atgaaggtgg
1801 agggtctcta gttccacgcc tctcccacct ccctccgcac acaccccacc ccagcctgct
1861 ataggctggg cttccccttg ggcggaactc cactgcgatg ggggtcacca ggtgaccagt
1921 gggagccccc accccgagtc acaccagaaa gctaggtcgt gggtcagctc tgaggatgta
1981 taccccctggt gggagaggga gacctagaga tctggctgtg ggcgggcat gggggtgaa
2041 gggccactgg gaccctcagc cttgtttgta ctgtatgcct tcagcattgc ctaggaacac
2101 gaagcacgat cagtccatcc cagagggacc ggagttatga caagctttcc aaatattttg
2161 ctttatcagc cgatatcaac acttgtatct ggcctctgtg ccccagcagt gccttgtgca
2221 atgtgaatgt gcgcgtctct gctaaaccac cattttattt ggtttttgtt ttgttttggt
2281 tttgctcgga tacttgccaa aatgagactc tccgtcggca gctgggggaa gggtctgaga
2341 ctccctttcc ttttggtttt gggattactt ttgatcctgg gggaccaatg aggtgagggg
2401 ggttctcctt tgccctcagc tttccccagc ccctccggcc tgggctgccc acaaggcttg
2461 tcccccagag gccctggctc ctggtcggga agggaggtgg cctcccgcca acgcatcact
2521 ggggctggga gcagggaagg acggcttggt tctcttcttt tggggagaac gtagagtctc
2581 actctagatg ttttatgtat tatatctata atataaacat atcaaagtca a
**CEBPA amino acid sequence (SEQ ID NO:60)** 
MPGGAHGPPPGYGCAAAGYLDGRLEPLYERVGAPALRPLVIKQE
PREEDEAKQLALAGLFPYQPPPPPPPSHPHPHPPPAHLAAPHLQFQIAHCGQTTMHLQ
PGHPTPPPTPVPSPHPAPALGAAGLPGPGSALKGLGAAHPDLRASGGSGAGKAKKSVD
KNSNEYRVRRERNNIAVRKSRDKAKQRNVETQQKVLELTSDNDRLRKRVEQLSRELDT
LRGIFRQLPESSLVKAMGNCA
**PROX1 nucleic acid sequence (SEQ ID NO:61)** 
1 gtgtcttaaa gtaaatcttg ttgtggagcg gagccctcag ctgagggagc gctctgaaat
61 aatacaccat tgcagccggg gaaagcagag cggcgcaaaa gagctctcgc cgggtccgcc
121 tgctccctct ccgcttcgct cctcttctct tctttaccct tctcctctct cctcctctgc
181 tgctctctcc tctcctccgc tcttctctct cctcctctcc tgctctctcc tcttccctta
241 gctcctcttc ttttcttctc ctcttcttcc ctctcctcgc ctctcccctg ctcctcttct
301 ctcgtctccc ctcccctccc gcctctctct cccctctccc tctcccactc gccccgctcg
361 ctcgctcgct gtcgcacaga ctcaccgtcc cttgtccaat tatcatattc atcacccgca
421 agatatcacc gtgtgtgcac tcgcgtgttt tcctctctct gccgggggaa aaaaaagaga
481 gagagagaga tagagagaga gagagagaga gagagagaga ggctcggtcc cactgctccc
541 tgcaccgcgg tcccgggatt cttgagctgt gcccagctga cgagcttttg aagatggcac
601 aataaccgtc cagtgatgcc tgaccatgac agcacagccc tcttaagccg gcaaaccaag
661 aggagaagag ttgacattgg agtgaaaagg acggtaggga cagcatctgc atttttgct
721 aaggcaagag caacgttttt tagtgccatg aatccccaag gttctgagca ggatgttgag
781 tattcagtgg tgcagcatgc agatggggaa aagtcaaatg tactccgcaa gctgctgaag
841 agggcgaact cgtatgaaga tgccatgatg ccttttccag gagcaaccat aatttcccag
901 ctgttgaaaa ataacatgaa caaaaatggt ggcacggagc ccagtttcca agccagcggt
961 ctctctagta caggctccga agtacatcag gaggatatat gcagcaactc ttcaagagac
1021 agccccccag agtgtctttc cccttttggc aggcctacta tgagccagtt tgatatggat
1081 cgcttatgtg atgagcacct gagagcaaag cgcgcccggg ttgagaatat aattcggggt
1141 atgagccatt cccccagtgt ggcattaagg ggcaatgaaa atgaaagaga gatggccccg
1201 cagtctgtga gtccccgaga aagttacaga gaaaacaaac gcaagcaaaa gcttccccag
1261 cagcagcaac agagtttcca gcagctggtt tcagcccgaa aagaacagaa gcgagaggag
1321 cgccgacagc tgaaacagca gctggaggac atgcagaaac agctgcgcca gctgcaggaa
1381 aagttctacc aaatctatga cagcactgat tcggaaaatg atgaagatgg taacctgtct
1441 gaagacagca tgcgctcgga gatcctggat gccagggccc aggactctgt cggaaggtca
1501 gataatgaga tgtgcgagct agacccagga cagtttattg accgagctcg agccctgatc
1561 agagagcagg aaatggctga aaacaagccg aagcgagaag gcaacaacaa agaaagagac
1621 catgggccaa actccttaca accggaaggc aaacatttgg ctgagacctt gaaacaggaa
1681 ctgaacactg ccatgtcgca agttgtggac actgtggtca aagtcttttc ggccaagccc
1741 tcccgccagg ttcctcaggt cttcccacct ctccagatcc cccaggccag atttgcagtc
1801 aatggggaaa accacaattt ccacaccgcc aaccagcgcc tgcagtgctt tggcgacgtc
1861 atcattccga accccctgga cacctttggc aatgtgcaga tggccagttc cactgaccag
1921 acagaagcac tgcccctggt tgtccgcaaa aactcctctg accagtctgc ctccggccct
1981 gccgctggcg gccaccacca gcccctgcac cagtcgcctc tctctgccac cacgggcttc
2041 accacgtcca ccttccgcca ccccttcccc cttcccttga tggcctatcc atttcagagc
2101 ccattaggtg ctccctccgg ctccttctct ggaaaagaca gagcctctcc tgaatcctta
2161 gacttaacta gggataccac gagtctgagg accaagatgt catctcacca cctgagccac
2221 cacccttgtt caccagcaca cccgcccagc accgccgaag ggctctcctt gtcgctcata
 
2281 aagtccgagt gcggcgatct tcaagatatg tctgaaatat caccttattc gggaagtgca
2341 atgcaggaag gattgtcacc caatcacttg aaaaaagcaa agctcatgtt tttttatacc
2401 cgttatccca gctccaatat gctgaagacc tacttctccg acgtaaagtt caacagatgc
2461 attacctctc agctcatcaa gtggtttagc aatttccgtg agttttacta cattcagatg
2521 gagaagtacg cacgtcaagc catcaacgat ggggtcacca gtactgaaga gctgtctata
2581 accagagact gtgagctgta cagggctctg aacatgcact acaataaagc aaatgacttt
2641 gaggttccag agagattcct ggaagttgct cagatcacat tacgggagtt tttcaatgcc
2701 attatcgcag gcaaagatgt tgatccttcc tggaagaagg ccatatacaa ggtcatctgc
2761 aagctggata gtgaagtccc tgagattttc aaatccccga actgcctaca agagctgctt
2821 catgagtaga aatttcaaca actctttttg aatgtatgaa gagtagcagt cccctttgga
2881 tgtccaagtt atatgtgtct agattttgat ttcatatata tgtgtatggg aggcatggat
2941 atgttatgaa atcagctggt aattcctcct catcacgttt ctctcatttt cttttgtttt
3001 ccattgcaag gggatggttg ttttctttct gcctttagtt tgcttttgcc caaggccctt
3061 aacatttgga cacttaaaat agggttaatt ttcagggaaa aagaatgttg gcgtgtgtaa
3121 agtctctatt agcaatgaag ggaatttgtt aacgatgcat ccacttgatt gatgacttat
3181 tgcaaatggc ggttggctga ggaaaaccca tgacacagca caactctaca gacagtgatg
3241 tgtctcttgt ttctactgct aagaaggtct gaaaatttaa tgaaaccact tcatacattt
3301 aagtattttg tttggtttga actcaatcag tagcttttcc ttacatgttt aaaaataatt
3361 ccaatgacag atgagcagct cacttttcca aagtacccca aaaggccaaa ttaaaaaaga
3421 aaaataatca ctctcaagcc ttgtctaaga aaagaggcaa actctgaaag tcgtaccagt
3481 ttcttctgga ggcaaagcaa ttttgcacaa aaccagctct ctcaagatga gactagaaat
3541 tcatacctgg tcttgtagcc acctctctaa acttgaaaat aggttcttct tcataagtga
3601 gcttacatca ttcttcataa agaaaaatcc tataacttgt tatcattttt gcttcagata
3661 ctaaaaggca ctaagtttcc aatttacgct gctcaacttt gtttatatgc ttaaaaggat
3721 tctgtttact taacaatttt ttcccctaaa atactatttt ctgaatactt ccttccagta
3781 aggaataaag gaaagcccaa cttggccata aaattcttgc ctacactaga agtttgttga
3841 cagccattag ctgacttgat cgtcatctcc taagaggaac acatatattt tcacaagcaa
3901 ttccacacta tcctgatggg tatgcaaagt ggtgacagtc taactcagtg tttcttcatt
3961 ttaggtataa cattttaaag caattgataa tgcctcttcc aattcagaag ctagtattga
4021 ccaaaatgtg agaagagtgt atagcatagg aaaatttggg gttaacccaa aagacacaat
4081 tccagcacac ataagaaagc tagctgctat tttatgcttt cttccatggt tctcctcttt
4141 tttccctttt atttttccct gtttttcaat gatgtacagt gttccctact tgcattgaaa
4201 aaactcgtat ggcattcaca ctttttttct taggtgggtt tttgtgtcca gatgcagtaa
4261 gaattcattg ttcatcctaa aactgttttc cagacccttc cttcccctta ggtaatttga
4321 tatacacctc ctaaaatgac acagtaacaa atctggtatt tagaacatat agaacataaa
4381 tgccattttt taattcaact ttaataagaa ttacatttga ctttggagaa tacaggtctt
4441 gacccatgtg actgactagc tgacccgatc gctgtaattt aacgtcattt ataaattctg
4501 ctgatggaca ggaatgtatg aactcaatta ttgtcagcac aaagccttaa aacctgctga
4561 ctttaaatta aatggtgcag tcctatgatg ccctgcacca tccaggggac taacagggcc
4621 tcgcagtgta gacagagggt gcagccacac gggcgggggc accagccacc tcactctgca
4681 cccgcggcct cacacatctc ccagctcaca ctctactaat gcacagagtc attagatcca
4741 atttgttatt tttctcactt gctttaaaaa aaagcagttt ggataatcat gacattggaa
4801 taaagtggga aggaaaaatt ccatcagcac aaaataggga agtaatccca acttgtagtc
4861 acagttttct gactggcttt gttttaaaag aggatggcag tccttgttcg tgtcagtgtg
4921 ccactgggtt tttgctgttc cgtgtaattc atatcaactt tgtgttgcca tttgcaaggt
4981 aaaaggcaaa gctgtagtgt attcacctat gtagacagat tgctagatat ctttttgatc
 
5041 tggggcgagt tcaatattga ttccagactt atttggattt ttttagtatt attttcccct
5101 ccctttctaa tttaaataga caaattaagc aaaagtgtgt gttcacaacc aaatgttgat
5161 gcccttatct actgataata tcctctcaat gttcactgag gcatagaaat tatttcagag
5221 tagaaattgc agcatgagga taaactcacc tctttgttct gaaaatagaa ctttatcact
5281 atgctttccg gtggttttcc cttttacaat cgaaatcttg tgcctcccaa gtgcattgga
5341 aaatgacaaa agcctgtctc tccaaattcc tatttaacag tttgattttt tttttttaat
5401 caccatcttt caaatcttag ctcaactctc accaagtgaa aattggctac ttgggagaaa
5461 gttaactttc tatggtggga tggtgaagga tgagggacag tttacatagg aaaagaaaaa
5521 aaaaagtcta aagtccatgt tgaaaaacca cactaccact tattttctgc taaccctaaa
5581 ttatttttgc gtatacgctt gaggttatag tctgtgccta gacctaaaat gcaccagcgg
5641 gggggatttt aaaaaatcct tcaaaatacc agttttttcc caacaagtac aattgttctt
5701 gtgccttctg tggctttcga tttcatcttt ttgactttat ttccaattac tacagctgca
5761 ataaacacta gatttttttt ctggctgttt gacataacgt tgatagctat gcatattttg
5821 tgtcttttta aaacaaagcg ggagaatacg tttttgaaga agagaatttt tagaacagtt
5881 tgataccgca aattattttt tcctcaattg tttgagcagc attcgagttt tgaaaattct
5941 tgtagaagcc aattttttgt aactgtggtg caaatcttgt gttttcttag cctaatgaaa
6001 agtagtatag aagcaatatt tcataccatg tgctatatat gtgtgcgcag atgtgtgaac
6061 ataaaatcac atacacacat atacacacat gtaaaaatat acatatatat atatgcgtgt
6121 gaagtggaaa gcttaccttt tcctatctag atttaagaac ctattttaga catttgttat
6181 gttttgtgaa aagaatgttc tatttgcaac aaaacattta attcttactg tatctctggc
6241 tgtttaatga ggacgtttca cattaaatgg taaaacacat ggaagatgtt agaatgtagt
6301 aattatttaa gtaaacgttc acccacatat tcctgaagtt tgctttgtgc ctccgagtat
6361 tatttaatta aagaagtgtt ttatgtttgc agaatctttg tcactgtact agggatgtgg
6421 gtgaatatca tttaaaaaaa tttaaaacaa caaaaaaaaa gcaaaacaga aacactaaag
6481 caagagggga acttttataa agcaatgtaa atatttaacc tcatggctgt cattatgtaa
6541 gacatgagat tttaataaat aactacattc tcacgacatc tgttgaattt actaggaaca
6601 ctacagtgac tgtatagaca gttgaaagca ttcttgaaaa tcctgctctc tccttttaaa
6661 agttaacaat ctcttttatc agatgtcaag ggcaagggta atgcagtttc tgtaaattta
6721 tgaaatttct ttttctatgt acatgaagac atttagtaag taacaccccc ccttcccatg
6781 cgcacatgtg cgcatacaca cacacacaca cacacacaca cacacaaaca cacacactgt
6841 cataaagcta atgatttggg gactttaaaa aataggatgt cctccaggaa caatcataaa
6901 tttatgaaag aaagagtagt ttacagactc ccctgaaaga agcagtgtat atgtgaagac
6961 agtgcaaaaa tctctttgcc atgtatatta tagcgtattc attggtgtga atagtacaaa
7021 tgtttccttc tggtacaaac tctgtgtttg caaatttaca agaagcattg ttttcaaaaa
7081 gctcccctta aaaaatgtaa ctggtttata tgagtaagca gttaccgtat tgcacttaaa
7141 tgttatgttg aaggaaatgc agttttgttt tctgtagatc tgttggttgt aaaccatcta
7201 taaaactaaa gctaaaatgc tcatattcag agctgggatc aaaactggta tttaaccttt
7261 gcatcttctt ataattatcc ttctaagaat ataacagaat gtggaagtgt ctggactttg
7321 agtcttttca actgagcctt ctctcaaatc tgacaccccc tcagaatgca caaacataag
7381 cagaaaaggc aaacaagctt accttctttt gtgaaaacgt attcattctg tattttttta
7441 aatattcaat tcccctaaaa atggggagaa aatattttaa aattgtatat tacgacttca
7501 aatttagaac taagaaaaaa atgtatttgg gattggtctc agcgctacct agaagaatca
7561 aaggtcatgg cttccctcaa tattgtccca gccatttctc atatgtatat agtataaacc
7621 gtgacaaaac actgccttta tattatttag caatatgttg taaatagcat tattaagctc
7681 ttttttgtaa taaagaccct ttgatttgaa tatagtacaa taactgaact gataaagtca
7741 atttttgatt tttgtttgtt ttttttagct agaggcaatt tcaattgtga attttgttg
 
7801 ttgtctattg ttctgaagac tttgcataat ttattggttt aatttatcct aatttatttg
7861 atgaaggtgt acaattttgt attaccaagg atgtactgta atattaattg atatgataaa
7921 cacaatgaga ctccctgtcc atattaaaaa gaaaataaaa aggtgcagta gacaattgat
7981 tttaaaggaa aagttaaaaa aattagtttg gcagctacta aattttaaaa caggaaaaaa
8041 aaaagttgtt gtggggaggg tgggaaaggg gttttacttt gtgtgtttta agcttttgta
8101 tactctccaa acttttacct tttgctttgt accacttaaa ggatacagta gtccaattgc
8161 cttgtgtgcc ttccatctcc tcttaaactg aatgtatgtg cagtatatat gcaagcttgt
8221 gcaaaataaa atatacatta caagctcagt gccgtttgat tttcttaaag aaagagtgac
8281 ttttaatttt tggacctgta tccaattgta ggacagtagg ctagttgtgc cagtaatgtc
8341 aagtatggag attttctttc actacaattc ttcattctgt tagcctaacg tgcagctcct
8401 agaaacaacc tcttttactt tagatgcttg gaataattgc ttggatttct ctctctgaaa
8461 catctttcag gcttaacttt atttagccct gaaacttaaa aaaaa
**PROX1 amino acid sequence (SEQ ID NO:62)** 
MPDHDSTALLSRQTKRRRVDIGVKRTVGTASAFFAKARATFFSA
MNPQGSEQDVEYSVVQHADGEKSNVLRKLLKRANSYEDAMMPFPGATIISQLLKNNM
NKNGGTEPSFQASGLSSTGSEVHQEDICSNSSRDSPPECLSPFGRPTMSQFDMDRLCDE
HLRAKRARVENIIRGMSHSPSVALRGNENEREMAPQSVSPRESYRENKRKQKLPQQQQ
QSFQQLVSARKEQKREERRQLKQQLEDMQKQLRQLQEKFYQIYDSTDSENDEDGNLSE
DSMRSEILDARAQDSVGRSDNEMCELDPGQFIDRARALIREQEMAENKPKREGNNKER
DHGPNSLQPEGKHLAETLKQELNTAMSQVVDTVVKVFSAKPSRQVPQVFPPLQIPQAR
FAVNGENHNFHTANQRLQCFGDVIIPNPLDTFGNVQMASSTDQTEALPLVVRKNSSDQ
SASGPAAGGHHQPLHQSPLSATTGFTTSTFRHPFPLPLMAYPFQSPLGAPSGSFSGKD
RASPESLDLTRDTTSLRTKMSSHHLSHHPCSPAHPPSTAEGLSLSLIKSECGDLQDMS
EISPYSGSAMQEGLSPNHLKKAKLMFFYTRYPSSNMLKTYFSDVKFNRCITSQLIKWF
SNFREFYYIQMEKYARQAINDGVTSTEELSITRDCELYRALNMHYNKANDFEVPERFL
EVAQITLREFFNAIIAGKDVDPSWKKAIYKVICKLDSEVPEIFKSPNCLQELLHE
**ATF5A nucleic acid sequence (SEQ ID NO:63)** 
1 tatgccgacg ggattttgtg ttttctcgga agtcgctgag aaaagtgaat gctagaactt
61 gtgggcgttt tcaccggtcc gaagtgtgaa atttatctcg cccagtgcat ccgctgggcg
121 gaggaatctt tagcactggg ggctgaccgc gtgacgcagc tggttgctag gtgtgggtga
181 cctgatgcat tgactcaatt agccgattct cagtcatcaa ctccatcaca acaaagcaag
241 accaacagca cttcagtccg agacactccg aaggagacat ggctttttaa tttagtacag
301 tcttcgatcc taaagaaaca ccgtgctttg ctcttgcaac gcaccccaag ctcaacacat
361 ttaagcaaga atggagtttc ataatgcgca ccaagccctg ttttctgtgg ctcaactctc
421 aaacttcact gtcgtgcact ctaagctcag gcatttgaat cgtaggacca acggacagga
481 tgatgacaat gtcagcaccc atttggaaga ctctactcgt ctgcccggca gaccccctca
541 ctctctctca cccacaggct aaccacagcc aatcggaggg gcgcagaggg gaggggccag
601 aggagaacca gcacttaatt ggtgatggtc ttagtgactg gatgacggaa gaagtagatt
661 tctcctctta cctcccaacc cctcactcct ctccctcccc aaatgcatcc cttcccccct
721 cgcccctaca gaatgacatc caggtgccct cagatttgga ggtcatgacc tctctgctgc
 
781 aagaggagct tgctcagctg gaggattact tcctgtctga cccactccca gaaaaagcct
841 ccaaactggg caaatgcgac aagggtccaa ctgcagttgg tccatcgtcg tattaccagt
901 tgccctacgc atcatattct acttccaacc aatccgaatc cagccctcta cttgttaccc
961 tggcaactgg ggaacttgac ttgctgagct tctgtggggg tcccattggc cgtaccaaga
1021 ttccaagaca tgccccctac agctgcagcc gccccaacag caacgtctgc agccgcaaga
1081 gagtttccga tggggtgagg gtgggtgaca gctacgagag tagcatctgg agttccaaag
1141 gaaattcctc aggtaactca gctgttgcgc ctggtgggag ctacagctgt gctgaagatg
1201 aacgggtggt aggcaaaggc tactgcctag gcagtacagt agagatcaga aggtgtgcca
1261 ttttacccaa agaggagaag aattgccgct acacagaaga ggccgtcggc gcgaacaagg
1321 ctggtggcgg ctacaatttt agtggaccaa ttcaaattcc ccataagaaa gatgaaatga
1381 tgtatggcat cagagaagtc aatttaagtg gcataggagg aagcacagag atggagatga
1441 tgagtgaacc gaagaatggt gcttctgaca tgaaggccaa catatcctgg aagacagaac
1501 ccagcgaaag ctgttttctc caaaatgcgt ctcaagaaga ggcctatcac agtttccttg
1561 gggccatcaa tgagccagta aaggaggaaa gcttagagat tcaccggcaa cataacttcc
1621 actgcggctt tctcgaaggc caaggccccg actgcctgag cggtgaccgc cacagacctg
1681 aaatggggtc cccgtgtgcc agaggagcct gcgtgctgaa agaagacccc tgcattgtga
1741 aatcagacct agaggtgcct ctaatcgaag ggcaccatgg tgaacgcaaa cagaagaaga
1801 gggatcagaa caagactgca gctcacagat atcgtcaacg gaaacgagca gagttggact
1861 cattggagga acagcttcat ggtctggagg gcagaaatcg tgagctccgg gacaaggcag
1921 aatcggtgga acgggagatc cagtacgtga aagacctcct gatcgaggtg tacaaggccc
1981 gtagccaacg cctcaagcag gaaaccagcg cctgaccaaa agctcgacca cctggcgatg
2041 gtcctgcaag tagaattgat agcagtctga aaggcaaagg agattggaat gttttctgac
2101 tgaatgtgtt tttttaggat atggtggagg ttgtactatt gcacgttact cagtcattta
2161 attttcgtta caaactgaca aagtccatca acagctgcag cacagacttc cacttttctc
2221 agaaataact ccaaatcagt cgatatgcac tgtgattatt atgtctttga ctgtaatgac
2281 agccgttttt ccttttctct tgtttttcca agagtggcga gacggctcat cctctt
**ATF5A amino acid sequence (SEQ ID NO:64)** 
MMTMSAPIWKTLLVCPADPLTLSHPQANHSQSEGRRGEGPEENQ
HLIGDGLSDWMTEEVDFSSYLPTPHSSPSPNASLPPSPLQNDIQVPSDLEVMTSLLQE
ELAQLEDYFLSDPLPEKASKLGKCDKGPTAVGPSSYYQLPYASYSTSNQSESSPLLVT
LATGELDLLSFCGGPIGRTKIPRHAPYSCSRPNSNVCSRKRVSDGVRVGDSYESSIWS
SKGNSSGNSAVAPGGSYSCAEDERVVGKGYCLGSTVEIRRCAILPKEEKNCRYTEEAV
GANKAGGGYNFSGPIQIPHKKDEMMYGIREVNLSGIGGSTEMEMMSEPKNGASDMKAN
ISWKTEPSESCFLQNASQEEAYHSFLGAINEPVKEESLEIHRQHNFHCGFLEGQGPDC
LSGDRHRPEMGSPCARGACVLKEDPCIVKSDLEVPLIEGHHGERKQKKRDQNKTAAHR
YRQRKRAELDSLEEQLHGLEGRNRELRDKAESVEREIQYVKDLLIEVYKARSQRLKQE
TSA
**Hnf4alpha nucleotide sequence (SEQ ID NO:65):** 
ATGCGACTCTCTAAAACCCTTGCCGGCATGGATATGGCCGACTACAGCGCTGCCCTG
GACCCAGCCTACACCACCCTGGAGTTTGAAAATGTGCAGGTGTTGACCATGGGCAAT
GACACGTCCCCATCTGAAGGTGCCAACCTCAATTCATCCAACAGCCTGGGCGTCAGT
GCCCTGTGCGCCATCTGTGGCGACCGGGCCACCGGCAAACACTACGGAGCCTCGAG
CTGTGACGGCTGCAAGGGGTTCTTCAGGAGGAGCGTGAGGAAGAACCACATGTACT
CCTGCAGGTTTAGCCGACAATGTGTGGTAGACAAAGATAAGAGGAACCAGTGTCGT
TACTGCAGGCTTAAGAAGTGCTTCCGGGCTGGCATGAAGAAGGAAGCTGTCCAAAA
TGAGCGGGACCGGATCAGCACGCGGAGGTCAAGCTACGAGGACAGCAGCCTGCCCT
CCATCAACGCGCTCCTGCAGGCAGAGGTTCTGTCCCAGCAGATCACCTCTCCCATCT
CTGGGATCAATGGCGACATTCGGGCAAAGAAGATTGCCAACATCACAGACGTGTGT
GAGTCTATGAAGGAGCAGCTGCTGGTCCTGGTCGAGTGGGCCAAGTACATCCCGGC
CTTCTGCGAACTCCTTCTGGATGACCAGGTGGCGCTGCTCAGGGCCCACGCCGGTGA
GCATCTGCTGCTTGGAGCCACCAAGAGGTCCATGGTGTTTAAGGACGTGCTGCTCCT
AGGCAATGACTACATCGTCCCTCGGCACTGTCCAGAGCTAGCGGAGATGAGCCGTG
TGTCCATCCGCATCCTCGATGAGCTGGTCCTGCCCTTCCAAGAGCTGCAGATTGATG
ACAATGAATATGCCTGCCTCAAAGCCATCATCTTCTTTGATCCAGATGCCAAGGGGC
TGAGTGACCCGGGCAAGATCAAGCGGCTGCGGTCACAGGTGCAAGTGAGCCTGGAG
GATTACATCAACGACCGGCAGTACGACTCTCGGGGCCGCTTTGGAGAGCTGCTGCTG
CTGTTGCCCACGCTGCAGAGCATCACCTGGCAGATGATCGAACAGATCCAGTTCATC
AAGTCTTCGGCATGGCCAAGATTGACAACCTGCTGCAGGAGATGCTTCTCGGAGGGT
CTGCCAGTGATGCACCCCACACCCACCACCCCCTGCACCCTCACCTGATGCAAGAAC
ACATGGGCACCAATGTCATTGTTGCTAACACGATGCCCTCTCACCTCAGCAATGGAC
AGATGTGTGAGTGGCCCCGACCCAGGGGGCAGGCAGCCACTCCCGAGACTCCACAG
CCATCACCACCAAGTGGCTCGGGATCTGAATCCTACAAGCTCCTGCCAGGAGCCATC
ACCACCATCGTCAAGCCTCCCTCTGCCATTCCCCAGCCAACGATCACCAAGCAAGAA
GCCATCTAG
**Hnf4a protein sequence (SEQ ID NO:66):**

MRLSKTLAGMDMADYSAALDPAYTTLEFENVQVLTMGNDTSPSEGANLNSSNSLGVS
ALCAICGDRATGKHYGASSCDGCKGFFRRSVRKNHMYSCRFSRQCVVDKDKRNQCRY
CRLKKCFRAGMKKEAVQNERDRISTRRSSYEDSSLPSINALLQAEVLSQQITSPISGINGDI
RAKKIANITDVCESMKEQLLVLVEWAKYIPAFCELLLDDQVALLRAHAGEHLLLGATK
RSMVFKDVLLLGNDYIVPRHCPELAEMSRVSIRILDELVLPFQELQIDDNEYACLKAIIFF
DPDAKGLSDPGKIKRLRSQVQVSLEDYINDRQYDSRGRFGELLLLLPTLQSITWQMIEQI
QFIKLFGMAKIDNLLQEMLLGGSASDAPHTHHPLHPHLMQEHMGTNVIVANTMPSHLS
NGQMCEWPRPRGQAATPETPQPSPPSGSGSESYKLLPGAITTIVKPPSAIPQPTITKQEAI*

Optionally, additional nucleotide sequences may be operably linked to the nucleotide sequence(s) encoding the therapeutic protein, such as nucleotide sequences encoding signal peptides (e.g. for targeting transport of the peptide to the extracellular space), nuclear localization signals, expression enhancers, and the like.

### REFERENCES

1. Dataller, R. & Brenner, D.A. Liver fibrosis. J Clin Invest 115, 209-218 (2005).
2. Iredale, J.P. Models of liver fibrosis: exploring the dynamic nature of inflammation and repair in a solid organ. J Clin Invest 117, 539-548 (2007).
3. Friedman, S.L. Mechanisms of hepatic fibrogenesis. Gastroenterology 134, 1655-1669(2008).
4. Schuppan, D. & Afdhal, N.H. Liver cirrhosis. Lancet 371, 838-851 (2008).
5. Kim, W.R., et al. OPTN/SRTR 2012 Annual Data Report: liver. Am J Transplant 14 Suppl 1, 69-96 (2014).
6. Hughes, R.D., Mitry, R.R. & Dhawan, A. Current status of hepatocyte transplantation. Transplantation 93, 342-347 (2012).
7. Lim, Y.S. & Kim, W.R. The global impact of hepatic fibrosis and end-stage liver disease. Clin Liver Dis 12, 733-746, vii (2008).
8. Zhang, D.Y. & Friedman, S.L. Fibrosis-dependent mechanisms of hepatocarcinogenesis. Hepatology 56, 769-775 (2012).
9. Mederacke, I., et al. Fate tracing reveals hepatic stellate cells as dominant contributors to liver fibrosis independent of its aetiology. Nat Commun 4, 2823 (2013).
10. Iwaisako, K., et al. Origin of myofibroblasts in the fibrotic liver in mice. Proc Natl Acad Sci USA 111, E3297-3305 (2014).
11. Huang, P., et al. Induction of functional hepatocyte-like cells from mouse fibroblasts by defined factors. Nature 475, 386-389 (2011).
12. Sekiya, S. & Suzuki, A. Direct conversion of mouse fibroblasts to hepatocyte-like cells by defined factors. Nature 475, 390-393 (2011).
13. Vandenberghe, L.H., Wilson, J.M. & Gao, G. Tailoring the AAV vector capsid for gene therapy. Gene Ther 16, 311-319 (2009).
14. Liedtke, C., et al. Experimental liver fibrosis research: update on animal models, legal issues and translational aspects. Fibrogenesis Tissue Repair 6, 19 (2013).
15. Grimm, D. & Kay, M.A. From virus evolution to vector revolution: use of naturally occurring serotypes of adeno-associated virus (AAV) as novel vectors for human gene therapy. Curr Gene Ther 3, 281-304 (2003).
16. Rutledge, E.A., Halbert, C.L. & Russell, D.W. Infectious clones and vectors derived from adeno-associated virus (AAV) serotypes other than AAV type 2. J Virol 72, 309-319 (1998).
17. Blankinship, M.J., et al. Efficient transduction of skeletal muscle using vectors based on adeno-associated virus serotype 6. Mol Ther 10, 671-678 (2004).
18. Gao, G., et al. Clades of Adeno-associated viruses are widely disseminated in human tissues. J Virol 78, 6381-6388 (2004).
19. Borner, K., et al. Robust RNAi enhancement via human Argonaute-2 overexpression from plasmids, viral vectors and cell lines. Nucleic Acids Res 41, e199 (2013).
20. Li, M., et al. High-efficiency transduction of fibroblasts and mesenchymal stem cells by tyrosine-mutant AAV2 vectors for their potential use in cellular therapy. Hum Gene Ther 21, 1527-1543 (2010).
21. Grimm, D., et al. In vitro and in vivo gene therapy vector evolution via multispecies interbreeding and retargeting of adeno-associated viruses. J Virol 82, 5887-5911 (2008).
22. Schaub, J.R., Malato, Y., Gormond, C. & Willenbring, H. Evidence against a Stem Cell Origin of New Hepatocytes in a Common Mouse Model of Chronic Liver Injury. Cell Rep 8, 933-939 (2014).
23. Henderson, N.C., et al. Targeting of alphav integrin identifies a core molecular pathway that regulates fibrosis in several organs. Nat Med 19, 1617-1624 (2013).
24. Derman, E. Isolation of a cDNA clone for mouse urinary proteins: age- and sex-related expression of mouse urinary protein genes is transcriptionally controlled. Proc Natl Acad Sci U S A 78, 5425-5429 (1981).
25. Zhu, S., et al. Mouse liver repopulation with hepatocytes generated from human fibroblasts. Nature 508, 93-97 (2014).
26. Azuma, H., et al. Robust expansion of human hepatocytes in Fah-/-/Rag2-/-/Il2rg-/- mice. NatBiotechnol 25, 903-910 (2007).
27. Wang, X., et al. Kinetics of liver repopulation after bone marrow transplantation. Am J Pathol 161, 565-574 (2002).
28. Espejel, S., et al. Induced pluripotent stem cell-derived hepatocytes have the functional and proliferative capabilities needed for liver regeneration in mice. J Clin Invest 120, 3120-3126 (2010).
29. Li, H., et al. In vivo genome editing restores haemostasis in a mouse model of haemophilia. Nature 475, 217-221 (2011).
30. Liu, L., et al. The microenvironment in hepatocyte regeneration and function in rats with advanced cirrhosis. Hepatology 55, 1529-1539 (2012).
31. Kotterman, M.A. & Schaffer, D.V. Engineering adeno-associated viruses for clinical gene therapy. Nat Rev Genet 15, 445-451 (2014).
32. Matsushita, T., et al. Adeno-associated virus vectors can be efficiently produced without helper virus. Gene Ther 5, 938-945 (1998).
33. Grimm, D., et al. Fatality in mice due to oversaturation of cellular microRNA/short hairpin RNA pathways. Nature 441, 537-541 (2006).
34. Zolotukhin, S., et al. Recombinant adeno-associated virus purification using novel methods improves infectious titer and yield. Gene Ther 6, 973-985 (1999).
35. Malato, Y., et al. Fate tracing of mature hepatocytes in mouse liver homeostasis and regeneration. J Clin Invest 121, 4850-4860 (2011).
36. Srinivas, S., et al. Cre reporter strains produced by targeted insertion of EYFP and ECFP into the ROSA26 locus. BMC Dev Biol 1, 4 (2001).
37. Madisen, L., et al. A robust and high-throughput Cre reporting and characterization system for the whole mouse brain. Nat Neurosci 13, 133-140 (2010).
38. Blouin, A., Bolender, R.P. & Weibel, E.R. Distribution of organelles and membranes between hepatocytes and nonhepatocytes in the rat liver parenchyma. A stereological study. J Cell Biol 72, 441-455 (1977).
39. Tanguay, R.M., et al. Different molecular basis for fumarylacetoacetate hydrolase deficiency in the two clinical forms of hereditary tyrosinemia (type I). Am J Hum Genet 47, 308-316 (1990).

The following examples are provided to describe the invention in greater detail. They are intended to illustrate, not to limit, the invention. Various publications, including patents, published applications, technical articles and scholarly articles are cited throughout the specification.

### EXAMPLES

### EXAMPLE 1

**AAV vector plasmid sequences used in preparation of viral particles.**
pAAV-CMV-**Foxa1 (SEQ ID NO: 67)** 
ctaaattgtaagcgttaatattttgttaaaattcgcgttaaatttttgttaaatcagctcattttttaaccaataggccgaaatcggcaaaatcccttataa
atcaaaagaatagaccgagatagggttgagtgttgttccagtttggaacaagagtccactattaaagaacgtggactccaacgtcaaagggcgaaaa
accgtctatcagggcgatggcccactacgtgaaccatcaccctaatcaagttttttggggtcgaggtgccgtaaagcactaaatcggaaccctaaagg
gagcccccgatttagagcttgacggggaaagccggcgaacgtggcgagaaaggaagggaagaaagcgaaaggagcgggcgctagggcgctggca
agtgtagcggtcacgctgcgcgtaaccaccacacccgccgcgcttaatgcgccgctacagggcgcgtcccattcgccattcaggctgcgcaactgttg
ggaagggcgatcggtgcgggcctcttcgctattacgccagctggcgaaagggggatgtgctgcaaggcgattaagttgggtaacgccagggttttccc
agtcacgacgttgtaaaacgacggccagtgagcgcgcgtaatacgactcactatagggcgaattggagctctctagaatgcaggggggggggggg
gggggccactccctctctgcgcgctcgctcgctcactgaggccgggcgaccaaaggtcgcccgacgcccgggctttgcccgggcggcctcagtgagc
gagcgagcgcgcagagagggagtggccaactccatcactaggggttcctagatctgatatcgtcgaggttacataacttacggtaaatggcccgcctg
gctgaccgcccaacgacccccgcccattgacgtcaataatgacgtatgttcccatagtaacgccaatagggactttccattgacgtcaatgggtggagt
atttacggtaaactgcccacttggcagtacatcaagtgtatcatatgccaagtacgccccctattgacgtcaatgacggtaaatggcccgcctggcatt
atgcccagtacatgaccttatgggactttcctacttggcagtacatctacgtattagtcatcgctattaccatggtgatgcggttttggcagtacatcaat
gggcgtggatagcggtttgactcacggggatttccaagtctccaccccattgacgtcaatgggagtttgttttggcaccaaaatcaacgggactttcca
aaatgtcgtaacaactccgccccattgacgcaaatgggcggtaggcgtgtacggtgggaggtctatataagcagagctggtttagtgaaccgtcagat
ccggatccaccggtaccatgttagggactgtgaagatggaagggcatgagagcaacgactggaacagctactacgcggacacgcaggaggcctact
cctctgtccctgtcagcaacatgaactccggcctgggctctatgaactccatgaacacctacatgaccatgaacaccatgaccacgagcggcaacatg
accccggcttccttcaacatgtcctacgccaacacgggcttaggggccggcctgagtcccggtgctgtggctggcatgccaggggcctctgcaggcgcc
atgaacagcatgactgcggcgggcgtcacggccatgggtacggcgctgagcccgggaggcatgggctccatgggcgcgcagcccgccacctccatg
aacggcctgggtccctacgccgccgccatgaacccgtgcatgagtcccatggcgtacgcgccgtccaacctgggccgcagccgcgcgggggggcggcg
gcgacgccaagacattcaagcgcagctaccctcacgccaagccgccttactcctacatctcgctcatcacgatggccatccagcaggcgcccagcaag
atgctcacgctgagcgagatctaccagtggatcatggacctcttcccctattaccgccagaaccagcagcgctggcagaactccatccgccactcgctg
tccttcaacgattgtttcgtcaaggtggcacgatccccggacaagccaggcaagggctcctactggacgctgcacccggactccggcaacatgttcga
gaacggctgctacttgcgccgccaaaagcgcttcaagtgtgagaagcagccgggggccggaggtgggagtgggggcggcggctccaaagggggcc
cagaaagtcgcaaggacccctcaggcccggggaaccccagcgccgagtcaccccttcaccggggtgtgcacggaaaggctagccagctagagggc
gcgccggccccagggcccgccgccagcccccagactctggaccacagcggggccacggcgacagggggcgcttcggagttgaagtctccagcgtctt
catctgcgcccccataagctccgggccagggggcgctagcatctgtaccccctctcacccggctcacggcctggcaccccacgaatctcagctgcatc
tgaaaggggatccccactactcctttaatcaccccttctccatcaacaacctcatgtcctcctccgagcaacagcacaagctggacttcaaggcatacg
agcaggcgctgcagtactctccttatggcgctaccttgcccgccagtctgccccttggcagcgcctcagtggccacgaggagccccatcgagccctcag
ccctggagccagcctactaccaaggtgtgtattccagacccgtgctaaatacttcctagctcgagtgcTGCATCCTAGCTCGTCGactagcaat
aaaggatcgtttattttcattggaagcgtgtgttggttttttgatcactcgacgcgccgatatcagatctggcaaacctagatgatggaggtacccactcc
ctctatgcgcgctcgctcactcactcggccctgccggccagaggccggcagtctggagacctttggtctccagggccgagtgagtgagcgagcgcgca
tagagggagtgggtaggacgcgtcctgcaggatgcatactagtggtacccagcttttgttccctttagtgagggttaattgcgcgcttggcgtaatcatg
gtcatagctgtttcctgtgtgaaattgttatccgctcacaattccacacaacatacgagccggaagcataaagtgtaaagcctggggtgcctaatgagt
 
gagctaactcacattaattgcgttgcgctcactgcccgctttccagtcgggaaacctgtcgtgccagctgcattaatgaatcggccaacgcgcggggag
aggcggtttgcgtattgggcgctcttccgcttcctcgctcactgactcgctgcgctcggtcgttcggctgcggcgagcggtatcagctcactcaaaggcg
gtaatacggttatccacagaatcaggggataacgcaggaaagaacatgtgagcaaaaggccagcaaaaggccaggaaccgtaaaaaggccgcgtt
gctggcgtttttccataggctccgcccccctgacgagcatcacaaaaatcgacgctcaagtcagaggtggcgaaacccgacaggactataaagatac
caggcgtttccccctggaagctccctcgtgcgctctcctgttccgaccctgccgcttaccggatacctgtccgcctttctcccttcgggaagcgtggcgctt
tctcatagctcacgctgtaggtatctcagttcggtgtaggtcgttcgctccaagctgggctgtgtgcacgaaccccccgttcagcccgaccgctgcgcctt
atccggtaactatcgtcttgagtccaacccggtaagacacgacttatcgccactggcagcagccactggtaacaggattagcagagcgaggtatgtag
gcggtgctacagagttcttgaagtggtggcctaactacggctacactagaaggacagtatttggtatctgcgctctgctgaagccagttaccttcggaa
aaagagttggtagctcttgatccggcaaacaaaccaccgctggtagcggtggtttttttgtttgcaagcagcagattacgcgcagaaaaaaaggatct
caagaagatcctttgatcttttctacggggtctgacgctcagtggaacgaaaactcacgttaagggattttggtcatgagattatcaaaaaggatcttca
cctagatccttttaaattaaaaatgaagttttaaatcaatctaaagtatatatgagtaaacttggtctgacagttaccaatgcttaatcagtgaggcacc
tatctcagcgatctgtctatttcgttcatccatagttgcctgactccccgtcgtgtagataactacgatacgggagggcttaccatctggccccagtgctg
caatgataccgcgagacccacgctcaccggctccagatttatcagcaataaaccagccagccggaagggccgagcgcagaagtggtcctgcaacttt
atccgcctccatccagtctattaattgttgccgggaagctagagtaagtagttcgccagttaatagtttgcgcaacgttgttgccattgctacaggcatcg
tggtgtcacgctcgtcgtttggtatggcttcattcagctccggttcccaacgatcaaggcgagttacatgatcccccatgttgtgcaaaaaagcggttag
ctccttcggtcctccgatcgttgtcagaagtaagttggccgcagtgttatcactcatggttatggcagcactgcataattctcttactgtcatgccatccgt
aagatgcttttctgtgactggtgagtactcaaccaagtcattctgagaatagtgtatgcggcgaccgagttgctcttgcccggcgtcaatacgggataat
accgcgccacatagcagaactttaaaagtgctcatcattggaaaacgttcttcggggcgaaaactctcaaggatcttaccgctgttgagatccagttcg
atgtaacccactcgtgcacccaactgatcttcagcatcttttactttcaccagcgtttctgggtgagcaaaaacaggaaggcaaaatgccgcaaaaaa
gggaataagggcgacacggaaatgttgaatactcatactcttcctttttcaatattattgaagcatttatcagggttattgtctcatgagcggatacatat
ttgaatgtatttagaaaaataaacaaataggggttccgcgcacatttccccgaaaagtgccac
pAAV-CMV-**Foxa2 (SEQ ID NO:68)** 
ctaaattgtaagcgttaatattttgttaaaattcgcgttaaatttttgttaaatcagctcattttttaaccaataggccgaaatcggcaaaatcccttataa
atcaaaagaatagaccgagatagggttgagtgttgttccagtttggaacaagagtccactattaaagaacgtggactccaacgtcaaagggcgaaaa
accgtctatcagggcgatggcccactacgtgaaccatcaccctaatcaagttttttggggtcgaggtgccgtaaagcactaaatcggaaccctaaagg
gagcccccgatttagagcttgacggggaaagccggcgaacgtggcgagaaaggaagggaagaaagcgaaaggagcgggcgctagggcgctggca
agtgtagcggtcacgctgcgcgtaaccaccacacccgccgcgcttaatgcgccgctacagggcgcgtcccattcgccattcaggctgcgcaactgttg
ggaagggcgatcggtgcgggcctcttcgctattacgccagctggcgaaagggggatgtgctgcaaggcgattaagttgggtaacgccagggttttccc
agtcacgacgttgtaaaacgacggccagtgagcgcgcgtaatacgactcactatagggcgaattggagctctctagaatgcaggggggggggggg
gggggccactccctctctgcgcgctcgctcgctcactgaggccgggcgaccaaaggtcgcccgacgcccgggctttgcccgggcggcctcagtgagc
gagcgagcgcgcagagagggagtggccaactccatcactaggggttcctagatctgatatcgtcgaggttacataacttacggtaaatggcccgcctg
gctgaccgcccaacgacccccgcccattgacgtcaataatgacgtatgttcccatagtaacgccaatagggactttccattgacgtcaatgggtggagt
atttacggtaaactgcccacttggcagtacatcaagtgtatcatatgccaagtacgccccctattgacgtcaatgacggtaaatggcccgcctggcatt
atgcccagtacatgaccttatgggactttcctacttggcagtacatctacgtattagtcatcgctattaccatggtgatgcggttttggcagtacatcaat
gggcgtggatagcggtttgactcacggggatttccaagtctccaccccattgacgtcaatgggagtttgttttggcaccaaaatcaacgggactttcca
aaatgtcgtaacaactccgccccattgacgcaaatgggcggtaggcgtgtacggtgggaggtctatataagcagagctggtttagtgaaccgtcagat
ccggatccaccggtaccatgctgggagccgtgaagatggaagggcacgagccatccgactggagcagctactacgcggagcccgagggctactcttc
cgtgagcaacatgaacgccggcctggggatgaatggcatgaacacatacatgagcatgtccgcggctgccatgggcggcggttccggcaacatgag
cgcgggctccatgaacatgtcatcctatgtgggcgctggaatgagcccgtcgctagctggcatgtccccgggcgccggcgccatggcgggcatgagcg
gctcagccggggcggccggcgtggcgggcatgggacctcacctgagtccgagtctgagcccgctcgggggacaggcggccggggccatgggtggcc
ttgcccccctacgccaacatgaactcgatgagccccatgtacgggcaggccggcctgagccgcgctcgggaccccaagacataccgacgcagctacac
acacgccaaacctccctactcgtacatctcgctcatcaccatggccatccagcagagccccaacaagatgctgacgctgagcgagatctatcagtgga
tcatggacctcttccctttctaccggcagaaccagcagcgctggcagaactccatccgccactctctctccttcaacgactgctttctcaaggtgccccgc
tcgccagacaagcctggcaagggctccttctggaccctgcacccagactcgggcaacatgttcgagaacggctgctacctgcgccgccagaagcgctt
caagtgtgagaagcaactggcactgaaggaagccgcgggtgcggccagtagcggaggcaagaagaccgctcctgggtcccaggcctctcaggctca
gctcggggaggccgcgggctcggcctccgagactccggcgggcaccgagtcccccattccagcgcttctccgtgtcaggagcacaagcgaggtggc
ctaagcgagctaaagggagcacctgcctctgcgctgagtcctcccgagccggcgccctcgcctgggcagcagcagcaggctgcagcccacctgctgg
gcccacctcaccacccaggcctgccaccagaggcccacctgaagcccgagcaccattacgccttcaaccaccccttctctatcaacaacctcatgtcgt
ccgagcagcaacatcaccacagccaccaccaccatcagccccacaaaatggacctcaaggcctacgaacaggtcatgcactacccaggggggctatg
gttcccccatgccaggcagcttggccatgggcccagtcacgaacaaagcgggcctggatgcctcgcccctggctgcagacacttcctactaccaagga
gtgtactccaggcctattatgaactcatcctagctcgagtgcggccgcaTGCATCCTAGCTCGTCGactagcaataaaggatcgtttattttcatt
ggaagcgtgtgttggttttttgatcactcgacgcgccgatatcagatctggcaaacctagatgatggaggtacccactccctctatgcgcgctcgctcac
tcactcggccctgccggccagaggccggcagtctggagacctttggtctccagggccgagtgagtgagcgagcgcgcatagagggagtgggtaggac
gcgtcctgcaggatgcatactagtggtacccagcttttgttccctttagtgagggttaattgcgcgcttggcgtaatcatggtcatagctgtttcctgtgtg
aaattgttatccgctcacaattccacacaacatacgagccggaagcataaagtgtaaagcctggggtgcctaatgagtgagctaactcacattaattg
cgttgcgctcactgcccgctttccagtcgggaaacctgtcgtgccagctgcattaatgaatcggccaacgcgcggggagaggcggtttgcgtattgggc
gctcttccgcttcctcgctcactgactcgctgcgctcggtcgttcggctgcggcgagcggtatcagctcactcaaaggcggtaatacggttatccacaga
atcaggggataacgcaggaaagaacatgtgagcaaaaggccagcaaaaggccaggaaccgtaaaaaggccgcgttgctggcgtttttccataggct
ccgcccccctgacgagcatcacaaaaatcgacgctcaagtcagaggtggcgaaacccgacaggactataaagataccaggcgtttccccctggaag
ctccctcgtgcgctctcctgttccgaccctgccgcttaccggatacctgtccgcctttctcccttcgggaagcgtggcgctttctcatagctcacgctgtag
gtatctcagttcggtgtaggtcgttcgctccaagctgggctgtgtgcacgaaccccccgttcagcccgaccgctgcgccttatccggtaactatcgtcttg
agtccaacccggtaagacacgacttatcgccactggcagcagccactggtaacaggattagcagagcgaggtatgtaggcggtgctacagagttctt
gaagtggtggcctaactacggctacactagaaggacagtatttggtatctgcgctctgctgaagccagttaccttcggaaaaagagttggtagctcttg
atccggcaaacaaaccaccgctggtagcggtggtttttttgtttgcaagcagcagattacgcgcagaaaaaaaggatctcaagaagatcctttgatctt
ttctacggggtctgacgctcagtggaacgaaaactcacgttaagggattttggtcatgagattatcaaaaaggatcttcacctagatccttttaaattaa
aaatgaagttttaaatcaatctaaagtatatatgagtaaacttggtctgacagttaccaatgcttaatcagtgaggcacctatctcagcgatctgtctatt
tcgttcatccatagttgcctgactccccgtcgtgtagataactacgatacgggagggcttaccatctggccccagtgctgcaatgataccgcgagaccc
acgctcaccggctccagatttatcagcaataaaccagccagccggaagggccgagcgcagaagtggtcctgcaactttatccgcctccatccagtcta
ttaattgttgccgggaagctagagtaagtagttcgccagttaatagtttgcgcaacgttgttgccattgctacaggcatcgtggtgtcacgctcgtcgttt
 
ggtatggcttcattcagctccggttcccaacgatcaaggcgagttacatgatcccccatgttgtgcaaaaaagcggttagctccttcggtcctccgatcg
ttgtcagaagtaagttggccgcagtgttatcactcatggttatggcagcactgcataattctcttactgtcatgccatccgtaagatgcttttctgtgactg
gtgagtactcaaccaagtcattctgagaatagtgtatgcggcgaccgagttgctcttgcccggcgtcaatacgggataataccgcgccacatagcaga
actttaaaagtgctcatcattggaaaacgttcttcggggcgaaaactctcaaggatcttaccgctgttgagatccagttcgatgtaacccactcgtgcac
ccaactgatcttcagcatcttttactttcaccagcgtttctgggtgagcaaaaacaggaaggcaaaatgccgcaaaaaagggaataagggcgacacg
gaaatgttgaatactcatactcttcctttttcaatattattgaagcatttatcagggttattgtctcatgagcggatacatatttgaatgtatttagaaaaa
taaacaaataggggttccgcgcacatttccccgaaaagtgccac
pAAV-CMV-**Foxa3 (SEQ ID NO:69)** 
cctgcaggcagctgcgcgctcgctcgctcactgaggccgcccgggcaaagcccgggcgtcgggcgacctttggtcgcccggcctcagtgagcgagcg
agcgcgcagagagggagtggccaactccatcactaggggttcctgcggccgcacgcgtcgacattgattattgactagttattaatagtaatcaattac
ggggtcattagttcatagcccatatatggagttccgcgttacataacttacggtaaatggcccgcctggctgaccgcccaacgacccccgcccattgac
gtcaataatgacgtatgttcccatagtaacgccaatagggactttccattgacgtcaatgggtggactatttacggtaaactgcccacttggcagtacat
caagtgtatcatatgccaagtacgccccctattgacgtcaatgacggtaaatggcccgcctggcattatgcccagtacatgaccttatgggactttccta
cttggcagtacatctacgtattagtcatcgctattaccatggtgatgcggttttggcagtacatcaatgggcgtggatagcggtttgactcacggggattt
ccaagtctccaccccattgacgtcaatgggagtttgttttggcaccaaaatcaacgggactttccaaaatgtcgtaacaactccgccccattgacgcaa
atgggcggtaggcgtgtacggtgggaggtctatataagcagagctctctggctaactagagaacccactgcTTACTGGCTTATCGAAATTAA
TACgactcactatagggagacccaagctggctagttaagcttggtaccgagctcggatctctagaagctggaacggccagagaggccttacccatca
acaagtttgtacaaaaaagcaggcttgaaggaattcggtaccatgctgggctcagtgaagatggaggctcatgacctggccgagtggagctactacc
cggaggcgggcgaggtgtattctccagtgaatcctgtgcccaccatggcccctctcaactcctacatgaccttgaacccactcagctctccctaccctcc
cggagggcttcaggcctccccactgcctacaggacccctggcacccccagcccccactgcgcccttggggcccaccttcccaagcttgggcactggtg
gcagcaccggaggcagtgcttccgggtgtgtagccccagggcccgggcttgtacatggaaaagagatggcaaaggggtaccggcggccactggccc
acgccaaaccaccatattcctacatctctctcataaccatggctattcagcaggctccaggcaagatgctgaccctgagtgaaatctaccaatggatca
tggacctcttcccgtactaccgggagaaccagcaacgttggcagaactccatccggcattcactgtccttcaatgactgcttcgtcaaggtggcacgctc
cccagacaagccaggcaaaggctcctactgggccttgcatcccagctctgggaacatgtttgagaacggctgctatctccgccggcagaagcgcttca
agctggaggagaaggcaaagaaaggaaacagcgccatatcggccagcaggaatggtactgcggggtcagccacctctgccaccactacagctgcc
actgcagtcacctccccggctcagccccagcctacgccatctgagcccgaggcccagagtggggatgatgtgggggggtctggactgcgcctcacctcc
ttcgtccacaccttatttcagcggcctggagctcccgggggaactaaagttggatgcgccctataacttcaaccaccctttctctatcaacaacctgatgt
cagaacagacatcgacaccttccaaactggatgtggggtttgggggctacggggctgagagtggggagcctggagtctactaccagagcctctattcc
cgctctctgcttaatgcatcctagctcgagtgcggccgcaacccagctttcttgtacaaagtggttgatgggtaaggcctctctggcctcgacctcgaga
gatctacgggtggcatccctgtgacccctccccagtgcctctcctggccctggaagttgccactccagtgcccaccagccttgtcctaataaaattaagt
tgcatcattttgtctgactaggtgtccttctataatattatggggtggaggggggtggtatggagcaaggggcaagttgggaagacaacctgtagggcc
tgcggggtctattgggaaccaagctggagtgcagtggcacaatcttggctcactgcaatctccgcctcctgggttcaagcgattctcctgcctcagcctc
ccgagttgttgggattccaggcatgcatgaccaggctcagctaatttttgtttttttggtagagacggggtttcaccatattggccaggctggtctccaac
tcctaatctcaggtgatctacccaccttggcctcccaaattgctgggattacaggcgtgaaccactgctcccttccctgtccttctgattttgtaggtaacc
acgtgcggaccgagcggccgcaggaacccctagtgatggagttggccactccctctctgcgcgctcgctcgctcactgaggccgggcgaccaaaggt
cgcccgacgcccgggctttgcccgggcggcctcagtgagcgagcgagcgcgcagctgcctgcaggggcgcctgatgcggtattttctccttacgcatct
gtgcggtatttcacaccgcatacgtcaaagcaaccatagtacgcgccctgtagcggcgcattaagcgcggcgggtgtggtggttacgcgcagcgtgac
cgctacacttgccagcgccctagcgcccgctcctttcgctttcttcccttcctttctcgccacgttcgccggctttccccgtcaagctctaaatcgggggctc
cctttagggttccgatttagtgctttacggcacctcgaccccaaaaaacttgatttgggtgatggttcacgtagtgggccatcgccctgatagacggtttt
tcgccctttgacgttggagtccacgttctttaatagtggactcttgttccaaactggaacaacactcaaccctatctcgggctattcttttgatttataagg
gattttgccgatttcggcctattggttaaaaaatgagctgatttaacaaaaatttaacgcgaattttaacaaaatattaacgtttacaattttatggtgca
ctctcagtacaatctgctctgatgccgcatagttaagccagccccgacacccgccaacacccgctgacgcgccctgacgggcttgtctgctcccggcat
ccgcttacagacaagctgtgaccgtctccgggagctgcatgtgtcagaggttttcaccgtcatcaccgaaacgcgcgagacgaaagggcctcgtgata
cgcctatttttataggttaatgtcatgataataatggtttcttagacgtcaggtggcacttttcggggaaatgtgcgcggaacccctatttgtttatttttct
aaatacattcaaatatgtatccgctcatgagacaataaccctgataaatgcttcaataatattgaaaaaggaagagtatgagtattcaacatttccgtg
tcgcccttattcccttttttgcggcattttgccttcctgtttttgctcacccagaaacgctggtgaaagtaaaagatgctgaagatcagttgggtgcacga
gtgggttacatcgaactggatctcaacagcggtaagatccttgagagttttcgccccgaagaacgttttccaatgatgagcacttttaaagttctgctat
gtggcgcggtattatcccgtattgacgccgggcaagagcaactcggtcgccgcatacactattctcagaatgacttggttgagtactcaccagtcacag
aaaagcatcttacggatggcatgacagtaagagaattatgcagtgctgccataaccatgagtgataacactgcggccaacttacttctgacaacgatc
ggaggaccgaaggagctaaccgcttttttgcacaacatgggggatcatgtaactcgccttgatcgttgggaaccggagctgaatgaagccataccaa
acgacgagcgtgacaccacgatgcctgtagcaatggcaacaacgttgcgcaaactattaactggcgaactacttactctagcttcccggcaacaatta
atagactggatggaggcggataaagttgcaggaccacttctgcgctcggcccttccggctggctggtttattgctgataaatctggagccggtgagcgt
gggtctcgcggtatcattgcagcactggggccagatggtaagccctcccgtatcgtagttatctacacgacggggagtcaggcaactatggatgaacg
aaatagacagatcgctgagataggtgcctcactgattaagcattggtaactgtcagaccaagtttactcatatatactttagattgatttaaaacttcatt
tttaatttaaaaggatctaggtgaagatcctttttgataatctcatgaccaaaatcccttaacgtgagttttcgttccactgagcgtcagaccccgtagaa
 
aagatcaaaggatcttcttgagatcctttttttctgcgcgtaatctgctgcttgcaaacaaaaaaaccaccgctaccagcggtggtttgtttgccggatca
agagctaccaactctttttccgaaggtaactggcttcagcagagcgcagataccaaatactgtccttctagtgtagccgtagttaggccaccacttcaa
gaactctgtagcaccgcctacatacctcgctctgctaatcctgttaccagtggctgctgccagtggcgataagtcgtgtcttaccgggttggactcaaga
cgatagttaccggataaggcgcagcggtcgggctgaacggggggttcgtgcacacagcccagcttggagcgaacgacctacaccgaactgagatac
ctacagcgtgagctatgagaaagcgccacgcttcccgaagggagaaaggcggacaggtatccggtaagcggcagggtcggaacaggagagcgcac
gagggagcttccagggggaaacgcctggtatctttatagtcctgtcgggtttcgccacctctgacttgagcgtcgatttttgtgatgctcgtcaggggggg
cggagcctatggaaaaacgccagcaacgcggcctttttacggttcctggccttttgctggccttttgctcacatgt
pAAV-CMV-**Gata4 (SEQ ID NO:70)** 
cctgcaggcagctgcgcgctcgctcgctcactgaggccgcccgggcaaagcccgggcgtcgggcgacctttggtcgcccggcctcagtgagcgagcg
agcgcgcagagagggagtggccaactccatcactaggggttcctgcggccgcacgcgtcgacattgattattgactagttattaatagtaatcaattac
ggggtcattagttcatagcccatatatggagttccgcgttacataacttacggtaaatggcccgcctggctgaccgcccaacgacccccgcccattgac
gtcaataatgacgtatgttcccatagtaacgccaatagggactttccattgacgtcaatgggtggactatttacggtaaactgcccacttggcagtacat
caagtgtatcatatgccaagtacgccccctattgacgtcaatgacggtaaatggcccgcctggcattatgcccagtacatgaccttatgggactttccta
cttggcagtacatctacgtattagtcatcgctattaccatggtgatgcggttttggcagtacatcaatgggcgtggatagcggtttgactcacggggattt
ccaagtctccaccccattgacgtcaatgggagtttgttttggcaccaaaatcaacgggactttccaaaatgtcgtaacaactccgccccattgacgcaa
atgggcggtaggcgtgtacggtgggaggtctatataagcagagctctctggctaactagagaacccactgcTTACTGGCTTATCGAAATTAA
TACgactcactatagggagacccaagctggctagttaagcttggtaccgagctcggatctctagaagctggaacggccagagaggccttacccatca
acaagtttgtacaaaaaagcaggcttgaaggaattcggtaccatgtaccagagcctggcgatggcggccaatcacggtcctccgcctggagcttacg
aggccggagggccaggcgcgttcatgcactccgccggagcggcctctagtccggtgtacgtgcccacacccagggtgccctcaagcgtgctcggcctc
agctacctgcaaggaggcggtagtgctgcggcggctggtacgacgagtggcgggtctagtggagccggtcctagcggggctggtccgggaacccaa
caaggaagccctggatggagccaggcaggcgcagagggggcagcctacacacccccacccgtgagccccaggtttagcttcccaggcacgacagg
gagtcttgcagcggcagcggcagcggccgcagcccgagaagcagccgcgtatgggagcgggggaggtgctgccggtgcaggcctcgcagggaggg
aacagtacggtaggccgggattcgccggttcctacagtagcccctaccccgcctatatggccgatgtgggggcgagctgggctgcagcagcagcagct
tccgcgggacccttcgactccccagtgttgcatagcctgccggggagggctaacccaggtagacaccccaacctggacatgttcgacgacttctcaga
gggccgagagtgcgttaactgcggtgctatgagcacccccactgtggaggcgcgatgggacgggtcactacctgtgtaatgcatgcggcctgtaccaca
agatgaacggcatcaacagaccgctcatcaaaccccagaggaggctcagcgcaagccgcagggtgggcctgagctgtgccaactgccagaccacca
cgaccacgctgtggcgcaggaacgcagaaggagaacccgtgtgcaacgcttgcggactgtatatgaaactgcacggcgtccccaggcccttggcaat
gaggaaggaggggatccaaaccaggaagcgaaagccgaagaacctgaacaagagtaagaccccagcaggtcctgcgggtgaaactctgccaccg
tccagtggtgcgagctccggtaactcttcaaatgctaccagttcctctagcagctctgaggaaatgaggcccatcaagaccgaacccggtttgtccagc
cattacgggcacagttcaagcatgtcccaaaccttcagcacagtgtccggccacggcccgagcattcatcccgtgcttagcgccttgaagttgagtcct
cagggctacgccagtcccgtgactcagacgtcccaagcgagcagcaagcaggactcctggaacagcctggtgctggccgacagccacggagacatc
atcaccgcctagctcgagtgcggccgcaacccagctttcttgtacaaagtggttgatgggtaaggcctctctggcctcgacctcgagagatctacgggt
ggcatccctgtgacccctccccagtgcctctcctggccctggaagttgccactccagtgcccaccagccttgtcctaataaaattaagttgcatcattttg
tctgactaggtgtccttctataatattatggggtggaggggggtggtatggagcaaggggcaagttgggaagacaacctgtagggcctgcggggtcta
ttgggaaccaagctggagtgcagtggcacaatcttggctcactgcaatctccgcctcctgggttcaagcgattctcctgcctcagcctcccgagttgttg
ggattccaggcatgcatgaccaggctcagctaatttttgtttttttggtagagacggggtttcaccatattggccaggctggtctccaactcctaatctca
ggtgatctacccaccttggcctcccaaattgctgggattacaggcgtgaaccactgctcccttccctgtccttctgattttgtaggtaaccacgtgcggac
cgagcggccgcaggaacccctagtgatggagttggccactccctctctgcgcgctcgctcgctcactgaggccgggcgaccaaaggtcgcccgacgcc
cgggctttgcccgggcggcctcagtgagcgagcgagcgcgcagctgcctgcaggggcgcctgatgcggtattttctccttacgcatctgtgcggtatttc
acaccgcatacgtcaaagcaaccatagtacgcgccctgtagcggcgcattaagcgcggcgggtgtggtggttacgcgcagcgtgaccgctacacttg
ccagcgccctagcgcccgctcctttcgctttcttcccttcctttctcgccacgttcgccggctttccccgtcaagctctaaatcgggggctccctttagggtt
ccgatttagtgctttacggcacctcgaccccaaaaaacttgatttgggtgatggttcacgtagtgggccatcgccctgatagacggtttttcgccctttga
cgttggagtccacgttctttaatagtggactcttgttccaaactggaacaacactcaaccctatctcgggctattcttttgatttataagggattttgccga
tttcggcctattggttaaaaaatgagctgatttaacaaaaatttaacgcgaattttaacaaaatattaacgtttacaattttatggtgcactctcagtaca
atctgctctgatgccgcatagttaagccagccccgacacccgccaacacccgctgacgcgccctgacgggcttgtctgctcccggcatccgcttacaga
caagctgtgaccgtctccgggagctgcatgtgtcagaggttttcaccgtcatcaccgaaacgcgcgagacgaaagggcctcgtgatacgcctattttta
taggttaatgtcatgataataatggtttcttagacgtcaggtggcacttttcggggaaatgtgcgcggaacccctatttgtttatttttctaaatacattca
aatatgtatccgctcatgagacaataaccctgataaatgcttcaataatattgaaaaaggaagagtatgagtattcaacatttccgtgtcgcccttattc
ccttttttgcggcattttgccttcctgtttttgctcacccagaaacgctggtgaaagtaaaagatgctgaagatcagttgggtgcacgagtgggttacatc
gaactggatctcaacagcggtaagatccttgagagttttcgccccgaagaacgttttccaatgatgagcacttttaaagttctgctatgtggcgcggtat
tatcccgtattgacgccgggcaagagcaactcggtcgccgcatacactattctcagaatgacttggttgagtactcaccagtcacagaaaagcatctta
cggatggcatgacagtaagagaattatgcagtgctgccataaccatgagtgataacactgcggccaacttacttctgacaacgatcggaggaccgaa
ggagctaaccgcttttttgcacaacatgggggatcatgtaactcgccttgatcgttgggaaccggagctgaatgaagccataccaaacgacgagcgtg
acaccacgatgcctgtagcaatggcaacaacgttgcgcaaactattaactggcgaactacttactctagcttcccggcaacaattaatagactggatg
gaggcggataaagttgcaggaccacttctgcgctcggcccttccggctggctggtttattgctgataaatctggagccggtgagcgtgggtctcgcggt
 
atcattgcagcactggggccagatggtaagccctcccgtatcgtagttatctacacgacggggagtcaggcaactatggatgaacgaaatagacaga
tcgctgagataggtgcctcactgattaagcattggtaactgtcagaccaagtttactcatatatactttagattgatttaaaacttcatttttaatttaaaa
ggatctaggtgaagatcctttttgataatctcatgaccaaaatcccttaacgtgagttttcgttccactgagcgtcagaccccgtagaaaagatcaaag
gatcttcttgagatcctttttttctgcgcgtaatctgctgcttgcaaacaaaaaaaccaccgctaccagcggtggtttgtttgccggatcaagagctacca
actctttttccgaaggtaactggcttcagcagagcgcagataccaaatactgtccttctagtgtagccgtagttaggccaccacttcaagaactctgtag
caccgcctacatacctcgctctgctaatcctgttaccagtggctgctgccagtggcgataagtcgtgtcttaccgggttggactcaagacgatagttacc
ggataaggcgcagcggtcgggctgaacggggggttcgtgcacacagcccagcttggagcgaacgacctacaccgaactgagatacctacagcgtga
gctatgagaaagcgccacgcttcccgaagggagaaaggcggacaggtatccggtaagcggcagggtcggaacaggagagcgcacgagggagcttc
cagggggaaacgcctggtatctttatagtcctgtcgggtttcgccacctctgacttgagcgtcgatttttgtgatgctcgtcaggggggcggagcctatg
gaaaaacgccagcaacgcggcctttttacggttcctggccttttgctggccttttgctcacatgt
pAAV-CMV-**Hnf1a (SEQ ID NO:71)** 
cctgcaggcagctgcgcgctcgctcgctcactgaggccgcccgggcaaagcccgggcgtcgggcgacctttggtcgcccggcctcagtgagcgagcg
agcgcgcagagagggagtggccaactccatcactaggggttcctgcggccgcacgcgtcgacattgattattgactagttattaatagtaatcaattac
ggggtcattagttcatagcccatatatggagttccgcgttacataacttacggtaaatggcccgcctggctgaccgcccaacgacccccgcccattgac
gtcaataatgacgtatgttcccatagtaacgccaatagggactttccattgacgtcaatgggtggactatttacggtaaactgcccacttggcagtacat
caagtgtatcatatgccaagtacgccccctattgacgtcaatgacggtaaatggcccgcctggcattatgcccagtacatgaccttatgggactttccta
cttggcagtacatctacgtattagtcatcgctattaccatggtgatgcggttttggcagtacatcaatgggcgtggatagcggtttgactcacggggattt
ccaagtctccaccccattgacgtcaatgggagtttgttttggcaccaaaatcaacgggactttccaaaatgtcgtaacaactccgccccattgacgcaa
atgggcggtaggcgtgtacggtgggaggtctatataagcagagctctctggctaactagagaacccactgcTTACTGGCTTATCGAAATTAA
TACgactcactatagggagacccaagctggctagttaagcttggtaccgagctcggatctctagaagctggaacggccagagaggccttacccatca
acaagtttgtacaaaaaagcaggcttgaaggaattcggtaccatggtttctaagctgagccagctgcagacggagctcctggctgccctgctcgagtct
ggcctgagcaaagaggccctgatccaggccttgggggagccagggccctacctgatggttggagagggtcccctggacaagggggagtcctgcggt
gggagtcgaggggacctgaccgagttgcctaatggccttggagaaacgcgtggctctgaagatgacacggatgacgatggggaagacttcgcgcca
cccattctgaaagagctggagaacctcagcccagaggaggcagcccaccagaaagccgtggtggagtcacttcttcaggaggacccatggcgcgtg
gcgaagatggtcaagtcgtacttgcagcagcacaacatcccccagcgggaggtggtggacaccacgggtctcaaccagtcccacctgtcacagcacc
tcaacaagggcacacccatgaagacacagaagcgggccgctctgtacacctggtacgtccgcaagcagcgagaggtggctcagcaattcacccacg
cggggcagggcggactgattgaagagcccacaggcgatgagctgccaactaagaaggggcgtaggaaccggttcaagtggggccccgcatcccag
cagatcctgttccaggcctacgagaggcaaaaaagccccagcaaggaagagcgagagaccttggtggaggagtgtaataggcggagtgcatcca
gagggggtgtcaccatcgcaggcccaggggctaggctccaaccttgtcacggaggtgcgtgtctacaactggtttgccaaccggcgcaaggaggaa
gccttccggcacaagttggccatggacacctataacggacctccaccggggccaggcccgggccctgcgctgcctgctcacagttcccccggcctgcc
cacaaccaccctctctcccagtaaggtccacggtgtacggtacggacagtctgcaaccagtgaggcagccgaggtgccctccagcagcggaggtccc
ttagtcacagtgtctgcggccttacaccaggtatcccccacaggcctggagcccagcagcctgctgagcacagaggccaagctggtctcagccacggg
gggtcccctgcctcccgtcagcaccctgacagcactgcacagcttggagcagacatctccgggtctcaaccagcagccgcagaaccttatcatggcct
cgctacctggggtcatgaccatcgggcccggggagcctgcctccctgggacccacgttcacgaacacgggcgcctccaccctggttatcggtctggcct
ccactcaggcacagagcgtgcctgtcatcaacagcatggggagtagcctgaccacgctgcagccggtccagttttcccaaccactgcatccctcctatc
agcagcctctcatgcccccgtacagagccacgtggcccagagccccttcatggcaaccatggcccagctgcagagcccccacgccttatacagccac
aagcctgaggtggcccagtacacgcacaccagcctgctcccgcagaccatgttgatcacagacaccaacctcagcacccttgccagcctcacaccca
ccaagcaggtcttcacctcagacacagaggcctccagtgagcccgggcttcacgagccaccctctccagccaccaccatccacatccccagccagga
cccgtcgaacatccagcacctgcagcctgctcaccggctcagcaccagtcccacagtgtcctccagcagcctggtgttgtatcagagttccgactccaa
cgggcacagccacctgctgccatccaaccatagtgtcatcgagacttttatctccacccagatggcctcctcttcccagtagctcgagtgcggccgcaa
cccagctttcttgtacaaagtggttgatgggtaaggcctctctggcctcgacctcgagagatctacgggtggcatccctgtgacccctccccagtgcctc
tcctggccctggaagttgccactccagtgcccaccagccttgtcctaataaaattaagttgcatcattttgtctgactaggtgtccttctataatattatgg
ggtggaggggggtggtatggagcaaggggcaagttgggaagacaacctgtagggcctgcggggtctattgggaaccaagctggagtgcagtggcac
aatcttggctcactgcaatctccgcctcctgggttcaagcgattctcctgcctcagcctcccgagttgttgggattccaggcatgcatgaccaggctcagc
taatttttgtttttttggtagagacggggtttcaccatattggccaggctggtctccaactcctaatctcaggtgatctacccaccttggcctcccaaattg
ctgggattacaggcgtgaaccactgctcccttccctgtccttctgattttgtaggtaaccacgtgcggaccgagcggccgcaggaacccctagtgatgg
agttggccactccctctctgcgcgctcgctcgctcactgaggccgggcgaccaaaggtcgcccgacgcccgggctttgcccgggcggcctcagtgagc
gagcgagcgcgcagctgcctgcaggggcgcctgatgcggtattttctccttacgcatctgtgcggtatttcacaccgcatacgtcaaagcaaccatagt
acgcgccctgtagcggcgcattaagcgcggcgggtgtggtggttacgcgcagcgtgaccgctacacttgccagcgccctagcgcccgctcctttcgctt
tcttcccttcctttctcgccacgttcgccggctttccccgtcaagctctaaatcgggggctccctttagggttccgatttagtgctttacggcacctcgaccc
caaaaaacttgatttgggtgatggttcacgtagtgggccatcgccctgatagacggtttttcgccctttgacgttggagtccacgttctttaatagtggac
tcttgttccaaactggaacaacactcaaccctatctcgggctattcttttgatttataagggattttgccgatttcggcctattggttaaaaaatgagctga
tttaacaaaaatttaacgcgaattttaacaaaatattaacgtttacaattttatggtgcactctcagtacaatctgctctgatgccgcatagttaagccag
ccccgacacccgccaacacccgctgacgcgccctgacgggcttgtctgctcccggcatccgcttacagacaagctgtgaccgtctccgggagctgcat
gtgtcagaggttttcaccgtcatcaccgaaacgcgcgagacgaaagggcctcgtgatacgcctatttttataggttaatgtcatgataataatggtttct
tagacgtcaggtggcacttttcggggaaatgtgcgcggaacccctatttgtttatttttctaaatacattcaaatatgtatccgctcatgagacaataacc
ctgataaatgcttcaataatattgaaaaaggaagagtatgagtattcaacatttccgtgtcgcccttattcccttttttgcggcattttgccttcctgttttt
gctcacccagaaacgctggtgaaagtaaaagatgctgaagatcagttgggtgcacgagtgggttacatcgaactggatctcaacagcggtaagatcc
 
ttgagagttttcgccccgaagaacgttttccaatgatgagcacttttaaagttctgctatgtggcgcggtattatcccgtattgacgccgggcaagagca
actcggtcgccgcatacactattctcagaatgacttggttgagtactcaccagtcacagaaaagcatcttacggatggcatgacagtaagagaattat
gcagtgctgccataaccatgagtgataacactgcggccaacttacttctgacaacgatcggaggaccgaaggagctaaccgcttttttgcacaacatg
ggggatcatgtaactcgccttgatcgttgggaaccggagctgaatgaagccataccaaacgacgagcgtgacaccacgatgcctgtagcaatggcaa
caacgttgcgcaaactattaactggcgaactacttactctagcttcccggcaacaattaatagactggatggaggcggataaagttgcaggaccactt
ctgcgctcggcccttccggctggctggtttattgctgataaatctggagccggtgagcgtgggtctcgcggtatcattgcagcactggggccagatggta
agccctcccgtatcgtagttatctacacgacggggagtcaggcaactatggatgaacgaaatagacagatcgctgagataggtgcctcactgattaag
cattggtaactgtcagaccaagtttactcatatatactttagattgatttaaaacttcatttttaatttaaaaggatctaggtgaagatcctttttgataatc
tcatgaccaaaatcccttaacgtgagttttcgttccactgagcgtcagaccccgtagaaaagatcaaaggatcttcttgagatcctttttttctgcgcgta
atctgctgcttgcaaacaaaaaaaccaccgctaccagcggtggtttgtttgccggatcaagagctaccaactctttttccgaaggtaactggcttcagc
agagcgcagataccaaatactgtccttctagtgtagccgtagttaggccaccacttcaagaactctgtagcaccgcctacatacctcgctctgctaatcc
tgttaccagtggctgctgccagtggcgataagtcgtgtcttaccgggttggactcaagacgatagttaccggataaggcgcagcggtcgggctgaacg
gggggttcgtgcacacagcccagcttggagcgaacgacctacaccgaactgagatacctacagcgtgagctatgagaaagcgccacgcttcccgaa
gggagaaaggcggacaggtatccggtaagcggcagggtcggaacaggagagcgcacgagggagcttccagggggaaacgcctggtatctttatag
tcctgtcgggtttcgccacctctgacttgagcgtcgatttttgtgatgctcgtcaggggggcggagcctatggaaaaacgccagcaacgcggcctttta
cggttcctggccttttgctggccttttgctcacatgt
pAAV-CMV-**Hnf4a (SEQ ID NO:72)**

ctaaattgtaagcgttaatattttgttaaaattcgcgttaaatttttgttaaatcagctcattttttaaccaataggccgaaatcggcaaaatcccttataa
atcaaaagaatagaccgagatagggttgagtgttgttccagtttggaacaagagtccactattaaagaacgtggactccaacgtcaaagggcgaaaa
accgtctatcagggcgatggcccactacgtgaaccatcaccctaatcaagttttttggggtcgaggtgccgtaaagcactaaatcggaaccctaaagg
gagcccccgatttagagcttgacggggaaagccggcgaacgtggcgagaaaggaagggaagaaagcgaaaggagcgggcgctagggcgctggca
agtgtagcggtcacgctgcgcgtaaccaccacacccgccgcgcttaatgcgccgctacagggcgcgtcccattcgccattcaggctgcgcaactgttg
ggaagggcgatcggtgcgggcctcttcgctattacgccagctggcgaaagggggatgtgctgcaaggcgattaagttgggtaacgccagggttttccc
agtcacgacgttgtaaaacgacggccagtgagcgcgcgtaatacgactcactatagggcgaattggagctctctagaatgcagggggggggggggg
ggggggccactccctctctgcgcgctcgctcgctcactgaggccgggcgaccaaaggtcgcccgacgcccgggctttgcccgggcggcctcagtgagc
gagcgagcgcgcagagagggagtggccaactccatcactaggggttcctagatctgatatcgtcgaggttacataacttacggtaaatggcccgcctg
gctgaccgcccaacgacccccgcccattgacgtcaataatgacgtatgttcccatagtaacgccaatagggactttccattgacgtcaatgggtggagt
atttacggtaaactgcccacttggcagtacatcaagtgtatcatatgccaagtacgccccctattgacgtcaatgacggtaaatggcccgcctggcatt
atgcccagtacatgaccttatgggactttcctacttggcagtacatctacgtattagtcatcgctattaccatggtgatgcggttttggcagtacatcaat
gggcgtggatagcggtttgactcacggggatttccaagtctccaccccattgacgtcaatgggagtttgttttggcaccaaaatcaacgggactttcca
aaatgtcgtaacaactccgccccattgacgcaaatgggcggtaggcgtgtacggtgggaggtctatataagcagagctggtttagtgaaccgtcagat
ccggatccaccggtaccatgcgactctctaaacccttgccggcatggatatggccgactacagcgctgccctggacccagcctacaccaccctggag
tttgaaaatgtgcaggtgttgaccatgggcaatgacacgtccccatctgaaggtgccaacctcaattcatccaacagcctgggcgtcagtgccctgtgc
gccatctgtggcgaccggccaccggcaaacactacggagcctcgagctgtgacggctgcaaggggttcttcaggaggagcgtgaggaagaaccac
atgtactcctgcaggtttagccgacaatgtgtggtagacaaagataagaggaaccagtgtcgttactgcaggcttaagaagtgcttccgggctggcat
gaagaaggaagctgtccaaaatgagcgggaccggatcagcacgcggaggtcaagctacgaggacagcagcctgccctccatcaacgcgctcctgca
ggcagaggttctgtcccagcagatcacctctcccatctctgggatcaatggcgacattcgggcaaagaagattgccaacatcacagacgtgtgtgagt
ctatgaaggagcagctgctggtcctggtcgagtgggccaagtacatcccggccttctgcgaactccttctggatgaccaggtggcgctgctcagggccc
acgccggtgagcatctgctgcttggagccaccaagaggtccatggtgtttaaggacgtgctgctcctaggcaatgactacatcgtccctcggcactgtc
cagagctagcggagatgagccgtgtgtccatccgcatcctcgatgagctggtcctgcccttccaagagctgcagattgatgacaatgaatatgcctgcc
tcaaagccatcatcttctttgatccagatgccaaggggctgagtgacccgggcaagatcaagcggctgcggtcacaggtgcaagtgagcctggagga
ttacatcaacgaccggcagtacgactctcggggccgctttggagagctgctgctgctgttgcccacgctgcagagcatcacctggcagatgatcgaac
agatccagttcatcaagctcttcggcatggccaagattgacaacctgctgcaggagatgcttctcggagggtctgccagtgatgcacccacacccacc
acccccctgcaccctcacctgatgcaagaacacatgggcaccaatgtcattgttgctaacacgatgccctctcacctcagcaatggacagatgtgtgagt
ggccccgacccagggggcaggcagccactcccgagactccacagccatcaccaccaagtggctcgggatctgaatcctacaagctcctgccaggagc
catcaccaccatcgtcaagcctccctctgccattccccagccaacgatcaccaagcaagaagccatctagctcgtcgactagcaataaaggatcgttta
ttttcattggaagcgtgtgttggttttttgatcactccgcgccgatatcagatctggcaaacctagatgatggaggtacccactccctctatgcgcgctcg
ctcactcactcggccctgccggccagaggccggcagtctggagacctttggtctccagggccgagtgagtgagcgagcgcgcatagagggagtgggt
aggacgcgtcctgcaggatgcatactagtggtacccagcttttgttccctttagtgagggttaattgcgcgcttggcgtaatcatggtcatagctgtttcc
tgtgtgaaattgttatccgctcacaattccacacaacatacgagccggaagcataaagtgtaaagcctggggtgcctaatgagtgagctaactcacat
taattgcgttgcgctcactgcccgctttccagtcgggaaacctgtcgtgccagctgcattaatgaatcggccaacgcgcggggagaggcggtttgcgta
ttgggcgctcttccgcttcctcgctcactgactcgctgcgctcggtcgttcggctgcggcgagcggtatcagctcactcaaaggcggtaatacggttatcc
acagaatcaggggataacgcaggaaagaacatgtgagcaaaaggccagcaaaaggccaggaaccgtaaaaaggccgcgttgctggcgtttttcca
taggctccgcccccctgacgagcatcacaaaaatcgacgctcaagtcagaggtggcgaaacccgacaggactataaagataccaggcgtttcccccct
ggaagctccctcgtgcgctctcctgttccgaccctgccgcttaccggatacctgtccgcctttctcccttcgggaagcgtggcgctttctcatagctcacgc
tgtaggtatctcagttcggtgtaggtcgttcgctccaagctgggctgtgtgcacgaaccccccgttcagcccgaccgctgcgccttatccggtaactatc
gtcttgagtccaacccggtaagacacgacttatcgccactggcagcagccactggtaacaggattagcagagcgaggtatgtaggcggtgctacaga
gttcttgaagtggtggcctaactacggctacactagaaggacagtatttggtatctgcgctctgctgaagccagttaccttcggaaaaagagttggtag
ctcttgatccggcaaacaaaccaccgctggtagcggtggtttttttgtttgcaagcagcagattacgcgcagaaaaaaaggatctcaagaagatccttt
gatcttttctacggggtctgacgctcagtggaacgaaaactcacgttaagggattttggtcatgagattatcaaaaaggatcttcacctagatcctttta
aattaaaaatgaagttttaaatcaatctaaagtatatatgagtaaacttggtctgacagttaccaatgcttaatcagtgaggcacctatctcagcgatct
gtctatttcgttcatccatagttgcctgactccccgtcgtgtagataactacgatacgggagggcttaccatctggccccagtgctgcaatgataccgcg
agacccacgctcaccggctccagatttatcagcaataaaccagccagccggaagggccgagcgcagaagtggtcctgcaactttatccgcctccatcc
agtctattaattgttgccgggaagctagagtaagtagttcgccagttaatagtttgcgcaacgttgttgccattgctacaggcatcgtggtgtcacgctc

gtcgtttggtatggcttcattcagctccggttcccaacgatcaaggcgagttacatgatcccccatgttgtgcaaaaaagcggttagctccttcggtcctc
cgatcgttgtcagaagtaagttggccgcagtgttatcactcatggttatggcagcactgcataattctcttactgtcatgccatccgtaagatgcttttctg
tgactggtgagtactcaaccaagtcattctgagaatagtgtatgcggcgaccgagttgctcttgcccggcgtcaatacgggataataccgcgccacata
gcagaactttaaaagtgctcatcattggaaaacgttcttcggggcgaaaactctcaaggatcttaccgctgttgagatccagttcgatgtaacccactc
gtgcacccaactgatcttcagcatcttttactttcaccagcgtttctgggtgagcaaaaacaggaaggcaaaatgccgcaaaaaagggaataagggc
gacacggaaatgttgaatactcatactcttcctttttcaatattattgaagcatttatcagggttattgtctcatgagcggatacatatttgaatgtattta
gaaaaataaacaaataggggttccgcgcacatttccccgaaaagtgccac

### Example 2: Reprogramming of mouse fibroblasts by viral overexpression of hepatic transcription factor genes.

### Introduction

Most of the collagen in liver fibrosis is produced by myofibroblasts (MFs), a mesenchymal liver cell type generated in large numbers by hepatic stellate cells (HSCs) or portal fibroblasts when they become activated in response to liver injury. Because MFs are essential for liver fibrosis formation and progression, we reasoned that *in vivo* reprogramming of MFs into hepatocytes would be an effective therapy for liver fibrosis by replenishing the hepatocyte mass and limiting collagen production. Previous reports show reprogramming of mouse fibroblasts *in vitro* into hepatocyte-like cells, so-called induced hepatocytes (iHeps), by lentiviral or retroviral overexpression of the hepatic transcription factor (TF) genes *Foxa3, Gata4* and *Hnf1a*11 or *Foxa1*/*Foxa2* and *Hnf4a*12, respectively.

### Methods

**AAV-capsid vector construction.** AAV helper plasmids (pAAV-capsid) co-expressing the *rep* gene from AAV2 and *cap* genes from AAV1, AAV2, AAV6 to AAV9 or the AAV2/8/9 chimera AAV-DJ have been reported A helper plasmid for the production of the AAV1P4 vector was created through PCR-based introduction of two unique SfiI restriction sites into the AAV1 *cap* gene and subsequent insertion of a double-stranded DNA oligonucleotide encoding a seven amino acid re-targeting peptide (P4). Details of the cloning strategy and peptide sequence will be reported separately (Börner, K., *et al.*, manuscript in preparation). A helper plasmid for the production of the AAV2(Y444,500,730F) vector was created by three rounds of site-directed mutagenesis using the QuickChange II Kit (Stratagene) as in the original report of this triple tyrosine-to-phenylalanine mutant.

**AAV-TFs vector construction.** Transcription factor (TF) cDNAs were derived from Gateway compatible plasmids (GeneCopoeia): *Foxa1:* GC-Mm03068, *Foxa2*: GC-Mm30428, *Foxa3:* GCMm03070, *Gata4:* GC-Mm02662, *Hnf1a*: GC-Mm21209 and *Hnf4a*: GC-Mm03071. *Hnf1a*, *Foxa3* and *Gata4* cDNAs were inserted into the single-stranded AAV backbone plasmid pAAVCMV-Gateway (Applied Viromics) by LR reaction using Gateway LR Clonase enzyme mix (Life Technologies). *Foxa1, Foxa2* and *Hnf4a* cDNAs were inserted into a modified version of the double-stranded (DS) plasmid pAAV-CMV-EYFP-U6/DS (bi-cistronic AAV vector backbone encoding a CMV promoter-driven EYFP and a U6 promoter for shRNA expression) in which the U6 promoter was deleted by XhoI/AscI (New England Biolabs) digestion. *Foxa1, Foxa2* and *Hnf4a* cDNAs were amplified by PCR from GeneCopoeia plasmids and AgeI and SalI restriction sites were added in 5' and 3', respectively, for subsequent insertion into pAAVCMV-EYFP/DS. All plasmids were sequenced after amplification using the EndoFree Plasmid Mega Kit (Qiagen). Sequence-validated plasmids were functionally validated by transfection of 2 µg plasmid into HEK293T cells (Agilent Technologies) using 1 µL MegaTran transfection reagent (Origene) followed 48 hours later by RNA isolation for qRT-PCR analysis of TF expression (data not shown). Mycoplasma contamination of HEK293T cells was ruled out using the MycoAlert Detection Kit (Lonza).

**AAV vector production.** AAV vectors were produced by transfecting HEK293T cells in 20 15-cm dishes with a combination of three plasmids-pAAV-CMV-EYFP-U6/DS or pAAV-CMVTF, adenoviral helper plasmid pVAE2AE4-5 and pAAV-capsid-using the calcium phosphate method. Virus was harvested three days after transfection.

**AAV vector purification.** HEK293T cells were lysed by five cycles of freezing (-196°C) and thawing (37°C) and sonicated for 1 minute and 20 seconds. Samples were digested with benzonase (EMD Millipore) at 50 U/mL for 1 hour at 37°C and centrifuged at 5,000 g for 15 minutes at 4°C. Viral particles were purified using an iodixanol (Sigma-Aldrich) density gradient. In a 29.9 mL OptiSeal Tube (Beckman-Coulter), the crude virus and gradient were loaded as follows: 8 mL crude virus, 7 mL 15% iodixanol, 5 mL 25% iodixanol, 4 mL 40% iodixanol and 4 mL 60% iodixanol. Samples were centrifuged for 2.5 hours at 250,000 g at 4°C in a 70 Ti rotor using an Optima L-90K ultracentrifuge (Beckman-Coulter). After ultracentrifugation viral particles accumulating in the 40% phase of the iodixanol gradient were eluted by aspiration of the phase. AAV vector titers were determined by qRT-PCR as previously described.

**Mice.** All procedures involving mice were approved by the Institutional Animal Care and Use Committee at the University of California San Francisco. All mice were housed under barrier conditions. 8-10-week-old wildtype mice (C57BL/6), mice heterozygous for Pdgfrb-Cre23 and homozygous for R26R-EYFP (C57BL/6) or heterozygous for R26R-RFP (C57BL/6 x 129S6) were used. P2 immune-deficient, fumarylacetoacetate hydrolase (FAH)-deficient FRG mice were used as hepatic stellate cell (HSC) recipients. FRG mice were maintained on 2-(2-nitro-4-fluoromethylbenzoyl)-1,3-cyclohexanedione (NTBC; Yecuris) in the drinking water at 16 mg/L. Littermates were equally distributed between experimental and control groups. Male and female mice were equally distributed between experimental and control groups. Blinding was not done.

**AAV vector transduction.** Each mouse was intravenously injected via the tail vein with 4 × 10¹¹ viral genomes. To reduce iodixanol thickness, AAV vectors were diluted in phosphate-buffered saline (PBS)/5% sorbitol at a ratio of 5:1. Injections were performed slowly and the volume was limited to 500 µL to avoid hydrodynamic effects. Cells were transduced *in vitro* by addition of AAV vectors to fetal bovine serum (FBS)-free culture medium for 48 hours.

**Liver fibrosis model.** Mice received intraperitoneal injections of 0.5 µL/g body weight carbon tetrachloride (CCl4) diluted 1:4 in corn oil (both Sigma-Aldrich) bi-weekly for the indicated number of weeks.

**Hepatic stellate cell isolation.** Primary HSCs were isolated by a two-step pronase/collagenase *in situ* liver perfusion. Mice were anesthetized with Avertin (Sigma-Aldrich). The portal vein was canulated using a 24-gauge catheter (Surflo, Terumo). Liver perfusion was started with Liver Perfusion Media (LPM, Life Technologies) and the infrahepatic inferior vena cava was cut for drainage. DMEM/F12 medium (Life Technologies) was used to prepare the pronase/collagenase solutions. After perfusion with 50 mL of LPM for 10 minutes, the liver was perfused with 14 mL of high-pronase solution (from *Streptomyces griseus,* Roche) at 2.85 g/L for 4 minutes, followed by perfusion with 42 mL of collagenase solution (crude, Crescent Chemical) at 0.15 g/L for 10 minutes. The liver was removed from the mouse after complete digestion, placed into a 100-mm petri dish containing warmed low-pronase solution at 0.45 g/L, minced using sharp scissors and transferred into a conical tube containing warmed low-pronase solution. The petri dish was rinsed with 2.5 mL of DNase solution at 0.08 g/L, which was then added to the low-pronase solution containing the minced liver. The volume was adjusted to 50 mL using DMEM/F12. The cell suspension was mixed at 37°C using a magnetic stirrer for 10 minutes. After two washes at 10°C at 1,000 g, the cell suspension was purified by discontinuous density-gradient centrifugation with 8.2 and 15.6% Nycodenz layers (Accurate Chemical). Ultracentrifugation was performed at 70,000 g (slow acceleration and no brake) and 10°C in a Beckman-Coulter Optima L-90K ultracentrifuge for 25 minutes. HSCs were collected after removal of the media layer, washed twice in DMEM/F12 and used for RNA extraction, cell culture or transplantation.

**Hepatic stellate cell culture.** Primary HSCs were plated on 6-well plastic dishes (Corning) immediately after isolation and maintained in DMEM/F12/10% FBS (Gemini BioProducts), 1% Glutamax (Life Technologies) and 1% antibiotic-antimycotic (Gemini BioProducts) at 37°C in 5% CO2 atmosphere. The culture medium was replaced every 2-3 days. HSC-derived myofibroblasts (MFs) were transduced in FBS-free culture medium and analyzed 48 hours later.

**Hepatic stellate cell transplantation.** P2 FRG pups were transplanted with 250,000 HSCs in 10 µl DMEM/F12/10% FBS by percutaneous intrahepatic injection using a 31-gauge needle (BD PrecisionGlide). Four weeks after injection of AAV6-6TFs, NTBC withdrawal was started consisting of repeated cycles of NTBC off for ten days and NTBC on for three days (4 mg/L in the drinking water). FRG mice received antibiotic prophylaxis in the drinking water with trimethoprim/sulfamethoxazole (TMP/SMX; Sigma-Aldrich) at 0.2 g/L TMP and 1 g/L SMX continuously.

**Tissue immunostaining.** Tissue samples were fixed in zinc-containing neutral-buffered formalin (Anatech) or 10% formalin (Sigma-Aldrich) at 4°C overnight. After storage in 70% ethanol and paraffin embedding, tissues were cut into 5-µm-thick sections and placed on Superfrost Plus slides (Fisher Scientific). Sections were deparaffinized and incubated in boiling Antigen Retrieval Citra Solution (BioGenex) for 10 minutes. After cooling down, sections were blocked in 10% donkey serum (Jackson ImmunoResearch) for 1 hour and then incubated with primary antibodies overnight at 4°C and secondary antibodies for 1 hour at room temperature (See Tables 4 and 5 below).

**Table 4. Primary antibodies.**

| **Antigen** | **Species** | **Dilution** | **Supplier** | **Catalog #** |
|---|---|---|---|---|
| Collagen 1 | Rabbit | 1/100 | Abcam | Ab34710 |
| Desmin | Rabbit | 1/100 | Thermo Scientific | RB-9014-P0 |
| FAH | Rabbit | 1/15,000 | Université Laval³ | |
| GFP | Chicken | 1/200 | Abcam | Ab13970 |
| Ki67 | Rat | 1/100 | Affymetrix/eBioscience | 14-5698-82 |
| MUP | Goat | 1/200 | Cedarlane | GAM/MUP |
| α-SMA | Rabbit | 1/100 | Abcam | Ab5694 |
| Vimentin | Rabbit | 1/100 | Abcam | Ab45939 |

**Table 5. Secondary antibodies.**

| **Species** | **Reactivity** | **Fluorochrome** | **Dilution** | **Supplier** | **Catalog #** |
|---|---|---|---|---|---|
| Donkey | Chicken | Cy3 | 1/200 | Jackson Immunoresearch | 703-165-155 |
| Donkey | Goat | Alexa Fluor 488 | 1/200 | | 705-545-147 |
| Donkey | Rabbit | Alexa Fluor 488 | 1/200 | | 711-545-152 |
| Donkey | Rat | Alexa Fluor 647 | 1/200 | | 712-605-150 |

Nuclear DNA was stained with 300 nM DAPI (Millipore).

Pdgfrb-Cre, R26R-RFP samples were perfused with LPM for 5 minutes through the portal vein and fixed *in situ* using 2% paraformaldehyde (Sigma-Aldrich) for 5 minutes. Tissues were then fixed in 4% paraformaldehyde for 2 hours at room temperature and cryoprotected in 30% sucrose (Sigma-Aldrich) at 4°C overnight before embedding and freezing in optimum cutting temperature compound (OCT; Tissue-Tek, Sakura Finetek). Frozen tissues were cut into 5-7 µm sections using a Leica 3050S Cryostat, air dried and stored at -20°C prior to staining. Images were captured using a QImaging Retiga 2000R camera mounted on an Olympus BX51 microscope.

**Cell immunostaining.** Primary HSC-derived MFs transduced with AAV6-EYFP for 48 hours were fixed in 2% paraformaldehyde for 10 minutes at room temperature and then washed in PBS three times for 10 minutes. Nuclear DNA was stained with 300 nM DAPI. Images were captured using a QImaging QIClick-F-M-12 camera mounted on an Olympus IX71 microscope.

**Calculation of nodule size.** The number of MF-derived hepatocytes (MF-iHeps) counted in a nodule in a two-dimensional liver section was multiplied by a previously determined correction factor27 to estimate the total number of MF-iHeps forming the nodule in three dimensions.

**Statistical analysis.** Data are expressed as means ± standard error of the mean (s.e.m.), which were calculated from the average of 3 independent samples unless otherwise specified. Statistical differences between experimental and control groups were determined by two-way analysis of variance followed by Student's *t* test (unpaired, two-tailed). A *P* value of less than 0.05 was considered significant.

**qRT-PCR.** Total RNA was extracted with the phenol-chloroform (both Sigma-Aldrich) method. First-strand reverse transcription was performed with 1 µg of RNA using qScript cDNA supermix (Quanta Biosciences). qRT-PCR was performed using VeriQuest Fast SYBR qPCR Master Mix (Affymetrix) on an Applied Biosciences Viia7 Real-Time PCR system (Life Technologies). Primers used for amplification of the genes of interest appear below:

| Gene name | Forward primer (5'-3') with [Seq ID No] | Reverse primer (5'-3') with [Seq ID No] |
|---|---|---|
| *Acta2* | GTCCCAGACATCAGGGAGTAA [73] | TCGGATACTTCAGCGTCAGGA [74] |
| *Alb* | GCAGATGACAGGGCGGAACTTG [75] | AAAATCAGCAGCAATGGCAGGC [76] |
| *Col1a1* | TAGGCCATTGTGTATGCAGC [77] | ACATGTTCAGCTTTGTGGACC [78] |
| *Colla2* | GGTGAGCCTGGTCAAACGG [79] | ACTGTGTCCTTTCACGCCTTT [80] |
| *Des* | GAGAAACCAGCCCCGAGCAAAG [81] | AGCCTCGCTGACAACCTCTCCA [82] |
| *Foxa1* | GCCGCCTTACTCCTACATCTCG [83] | GGGGATCGTGCCACCTTGA [84] |
| *Foxa2* | CACCTGAGTCCGAGTCTGAGC [85] | GTACGAGTAGGGAGGTTTGGC [86] |
| *Foxa3* | GCGGGCGAGGTGTATTCTC [87] | GAGCTGAGTGGGTTCAAGGTC [88] |
| *Gapdh* | TGTTGAAGTCACAGGAGACAACCT [89] | AACCTGCCAAGTATGATGACATCA [90] |
| *Gata4* | GGGATTCGCCGGTTCCTACAG [91] | CTACCTGGGTTAGCCCTCCCC [92] |
| *Hnf1a* | CCCTTAGTCACAGTGTCTGC [93] | CCATGATAAGGTTCTGCGGCTGCT [94] |
| *Hnf4a* | CATCAACGACCGGCAGTAC [95] | GCAGCAGGTTGTCAATCTTG [96] |
| *Serpinala* | CACTTCCCCAGACTGTCCAT [97] | AGGGGAGCATTTTCCTCTGT [98] |

**Imaging.** Large images were captured using a Hamamatsu ORCA-Flash 4.0 camera mounted on a Nikon Ti-E microscope and processed using NIS-Elements 4.2 software.

**Calculation of reprogramming efficiency and liver repopulation.** Reprogramming efficiency was calculated by identifying the number of EYFP-positive MF-iHep clones divided by the total number of EYFP-positive HSCs/MFs. Liver repopulation is the number of EYFP-positive MF-iHeps divided by the total number of hepatocytes. Counts were performed on two-dimensional sections of five entire left liver lobes of Pdgfrb-Cre, R26R-EYFP mice. EYFP-positive MF-iHeps were counted directly. The total number of EYFP-positive HSCs/MFs and the total number of hepatocytes were estimated based on the total number of nuclei counted using ImageJ (NIH) software. For this, the total number of liver cells was derived from the total number of nuclei by accounting for cell type heterogeneity and the presence of both mononucleated and binucleated hepatocytes in the liver of adult mice. Considering that 50% of all liver cells are hepatocytes and 64% of hepatocytes are binucleated, 50 hepatocytes correspond to 82 nuclei because 32 binucleated hepatocytes contribute 64 nuclei and 18 mononucleated hepatocytes contribute 18 nuclei. Because nonhepatocyte liver cell types are mononucleated, 100 liver cells correspond to 50 + 82 = 132 nuclei. Consequently, the total number of liver cells is the total number of nuclei divided by 1.32. The total number of EYFP-positive HSCs/MFs is 5% of the total number of liver cells adjusted to 69.9% ± 7.5% labeling efficiency in Pdgfrb-Cre, R26R-EYFP mice.

**Collagen quantification.** Collagen staining was quantified using ImageJ software. Within the Analyze tab, under "Set Measurements", "Perimeter" and "Limit to threshold" were selected. Within the Image tab, under "Adjust" and "Color Threshold", a brightness of 85 was selected. Finally, within the Analyze tab, "Measure" was selected to obtain perimeter results in pixels.

**Laser-capture microdissection followed by qRT-PCR.** 10-µm-thick cryosections of formalinfixed, OCT-embedded liver samples were attached to PALM MembraneSlide slides (Zeiss). After OCT removal in nuclease-free water and dehydration in decreasing ethanol dilutions, MFiHeps and primary hepatocytes were isolated using a PALM MicroBeam IV system (Zeiss). PALM RoboSoftware 4.3 SP1 was used to manually identify RFP-positive MF-iHeps by direct fluorescence. After microdissection, cells were catapulted into PALM AdhesiveCaps (Zeiss). Multiple MF-iHep clones were pooled. The same method was used to isolate primary hepatocytes from the same mice and noninjured control mice. Samples were incubated for 16 hours in proteinase K (AB Biosystems) after which RNA was extracted and purified using the Arcturus Paradise Extraction and Isolation Kit (AB Biosystems). First-strand reverse transcription was performed with 10 µl of RNA using the Maxima First Strand cDNA Synthesis Kit for RT-qPCR with dsDNase (ThermoScientific). qRT-PCR was performed as described above.

### Results

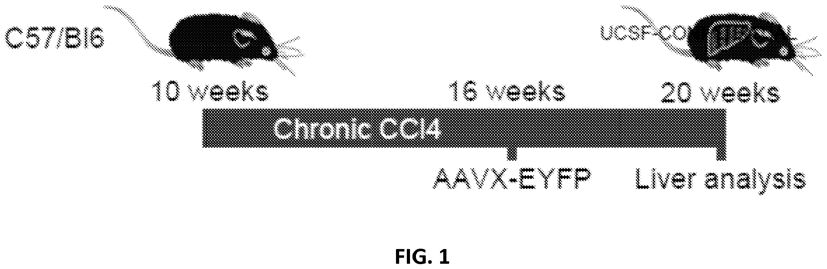

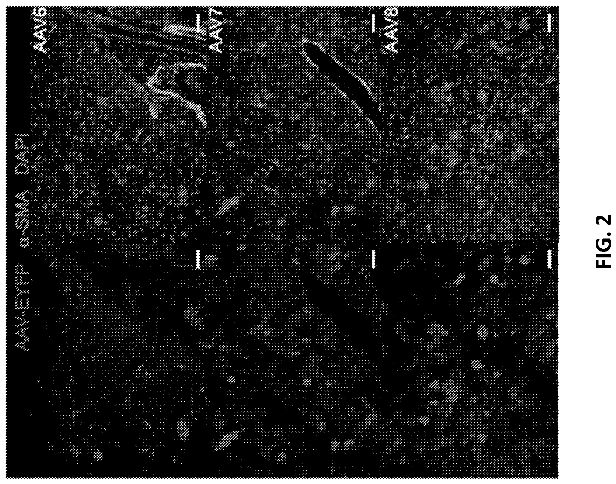

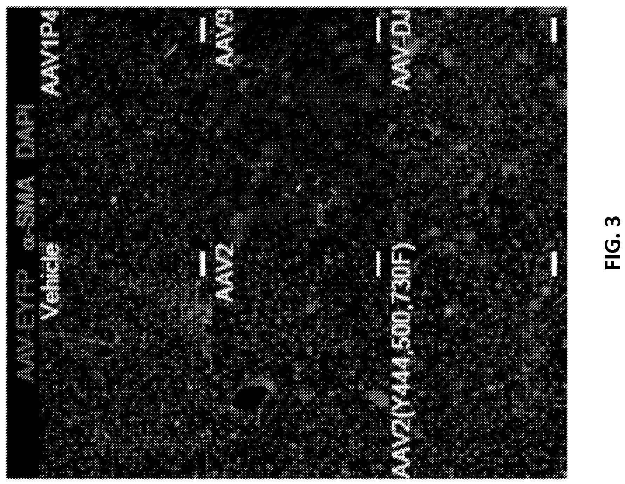

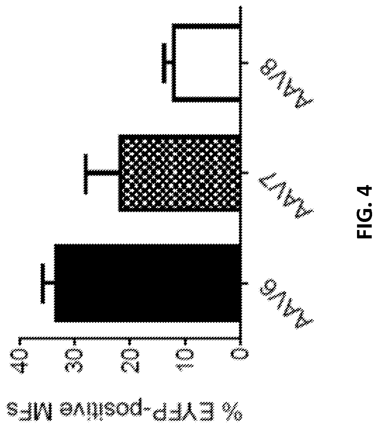

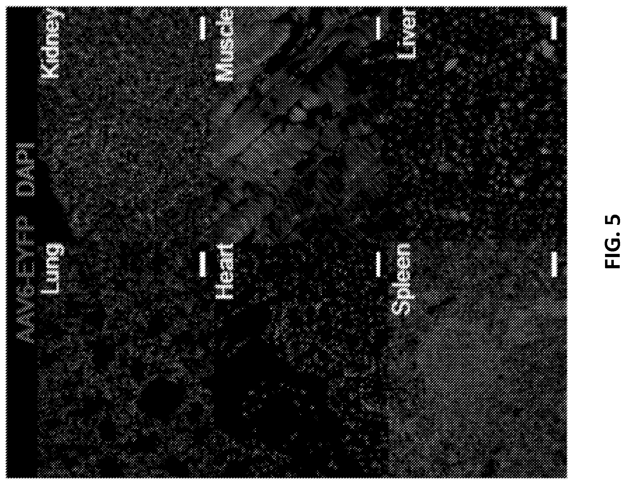

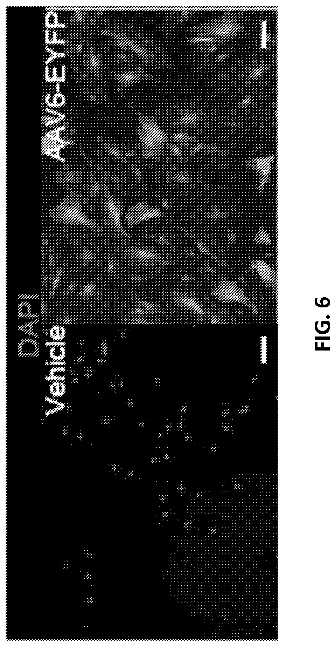

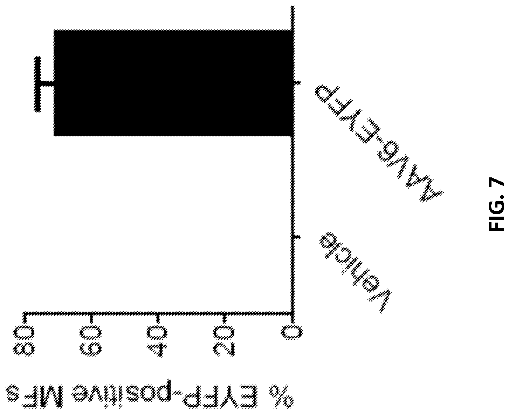

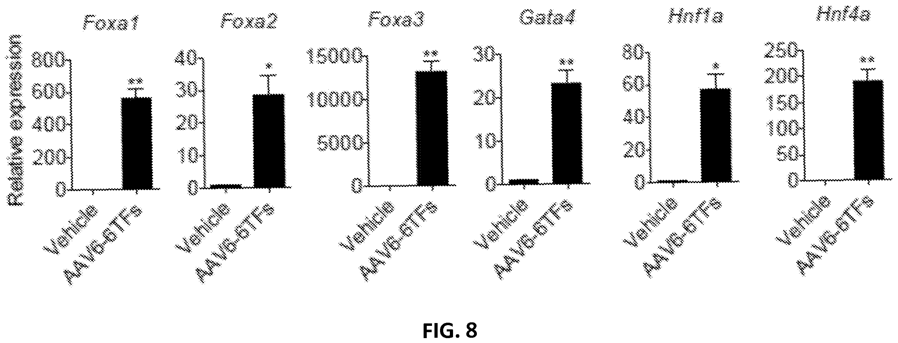

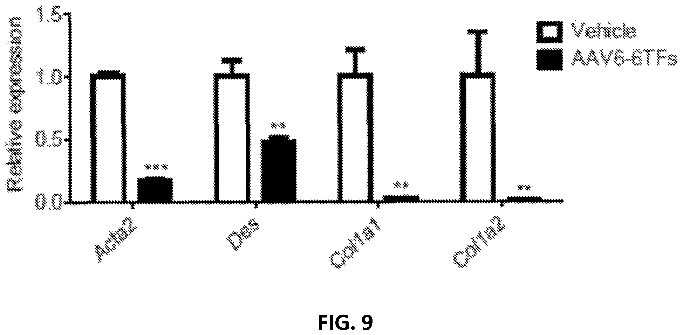

To establish *in vivo* delivery of TFs to MFs in a clinically translatable fashion, we used adenoassociated viral (AAV) vectors because they are not toxic and do not integrate into the genome. Moreover, many naturally occurring AAV capsids have a narrow cell tropism that can be further refined by molecular engineering. Since AAV capsids with MF tropism were unknown, we performed an *in vivo* screen in a liver fibrosis mouse model generated by treating C57BL/6 wildtype mice bi-weekly with carbon tetrachloride (CCl4) for six weeks (total of 12 doses). We focused on AAV capsids reported to be effective in transducing fibroblasts or other mesenchymal cell types, including the naturally occurring AAV2, AAV6, AAV7, AAV8 and AAV9 capsids and the engineered capsids AAV1P4 (seven amino acid re-targeting peptide displayed in an exposed capsid loop), AAV2(Y444,500,730F) (mutation of three exposed tyrosines to phenylalanines) and AAV-DJ (chimera of AAV2/8/9). We intravenously injected each mouse with 4 × 10¹¹ viral genomes of an AAV-EYFP vector pseudotyped with one of the eight capsids and analyzed their livers four weeks later (FIG. 1). We found that the AAV6, AAV7 and AAV8 capsids had a relevant MF tropism (FIG. 2, FIG. 3), with AAV6 transducing > 30% of α-smooth muscle actin (α-SMA)-positive MFs *in vivo* (FIG. 4). AAV6 capsid also showed organ tropism restricted to liver and skeletal muscle (FIG. 5). We confirmed the MF tropism of the AAV6 capsid *in vitro* by showing > 70% transduction efficiency in primary HSC-derived MFs (FIG. 6, FIG. 7). We also used these cells to test the function of AAV6 capsid-pseudotyped vectors expressing the TF genes *Foxa1, Foxa2, Foxa3*, *Gata4, Hnf1a* or *Hnf4a* (combination referred to as AAV6-6TFs). As expected, quantitative reverse transcription PCR (qRT-PCR) showed overexpression of the TF genes (FIG. 8), and decreased expression of the MF marker genes *Acta2, Des, Col1a1* and *Col1a2* in transduced MFs (FIG. 9).

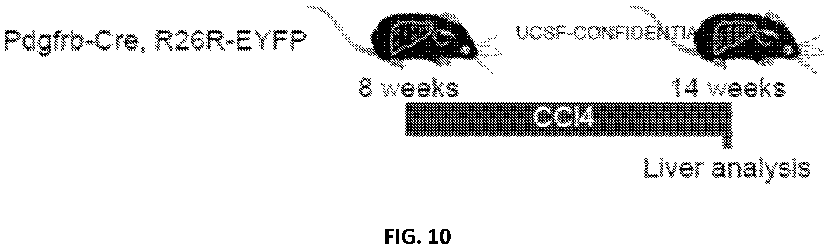

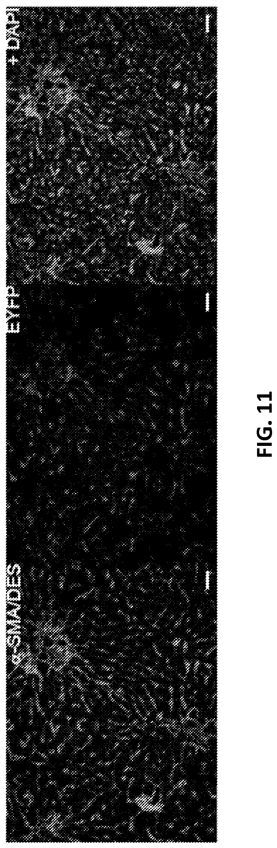

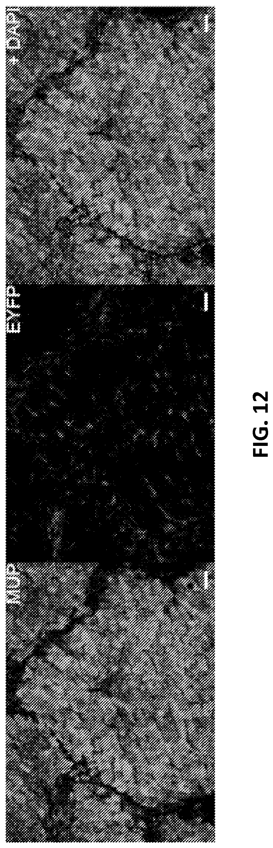

To establish *in vivo* efficacy of AAV6-6TFs, we used a genetic MF lineage-tracing mouse model based on activation of a reporter (R26R-EYFP) by Cre recombinase expressed from the platelet-derived growth-factor receptor β (Pdgfrb) promoter. We and others have previously used this model to label MFs, as well as HSCs, with high efficiency and specificity. We confirmed these results by showing 69.9% ± 7.5% labeling efficiency of MFs and absence of MF-derived hepatocytes-identified by major urinary protein (MUP) expression-in Pdgfrb-Cre, R26REYFP mice treated with 12 doses of CCl4 as described for the AAV capsid screening (FIG. 10, FIG. 11, FIG. 12).

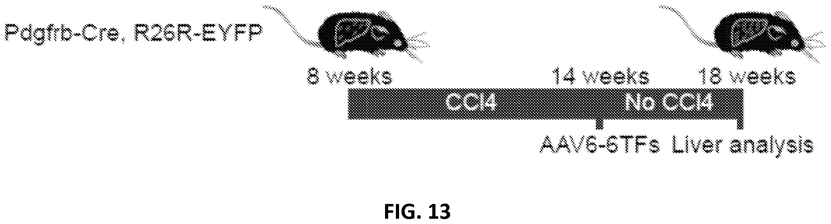

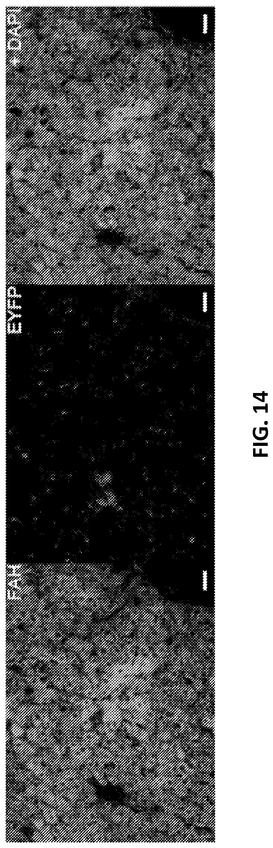

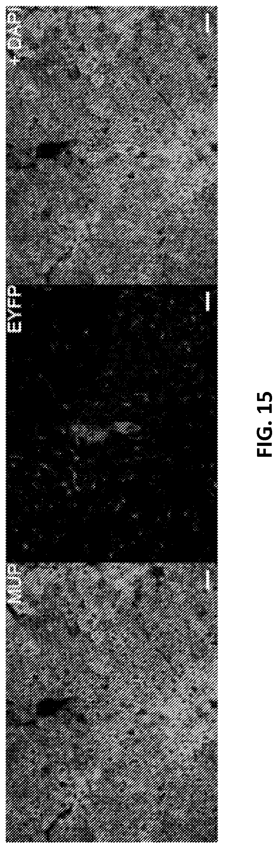

Next, we intravenously injected CCl4-treated Pdgfrb-Cre, R26R-EYFP mice with AAV6-6TFs and analyzed their livers four weeks later (FIG. 13). We found small clusters of 2-3 iHeps derived from MFs (MF-iHeps) as identified by expression of the MF lineage-tracing marker EYFP and the hepatocyte markers fumarylacetoacetate hydrolase (FAH) and MUP, the latter suggesting that the cells were mature hepatocytes (FIG. 14, FIG. 15).

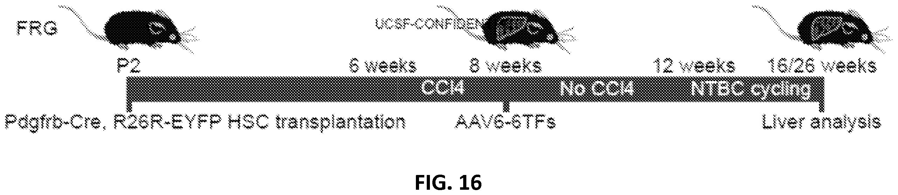

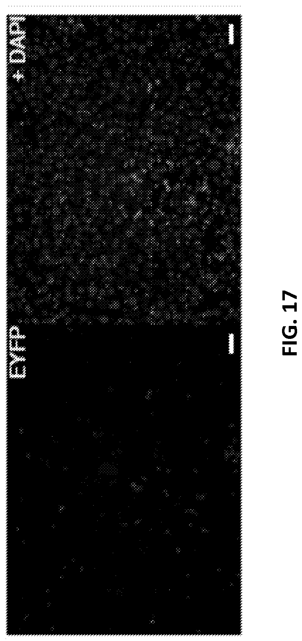

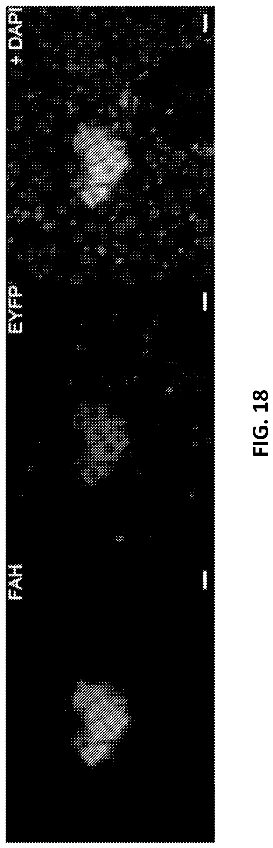

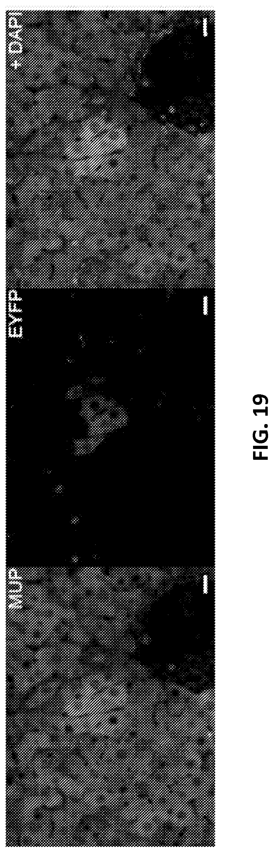

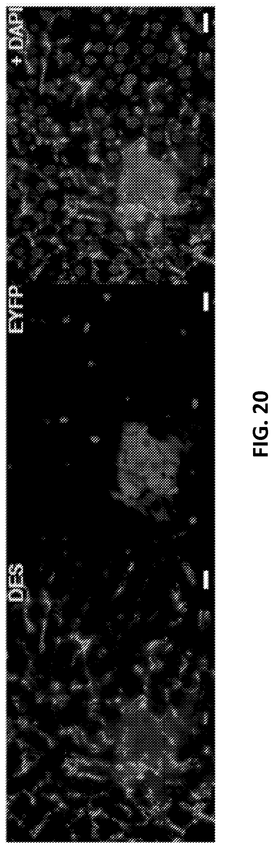

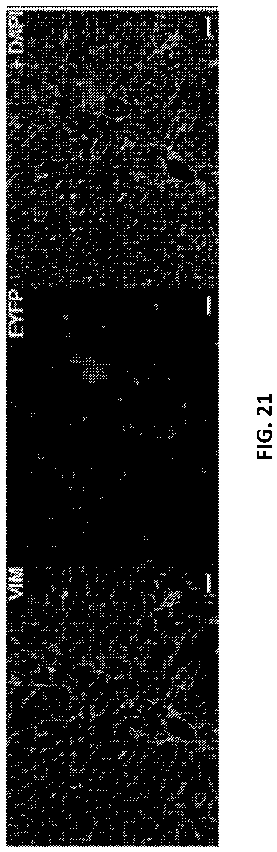

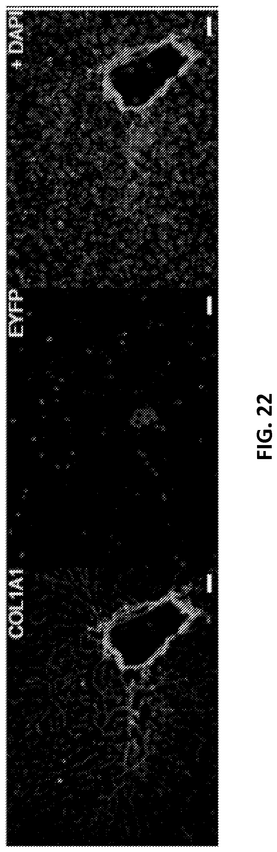

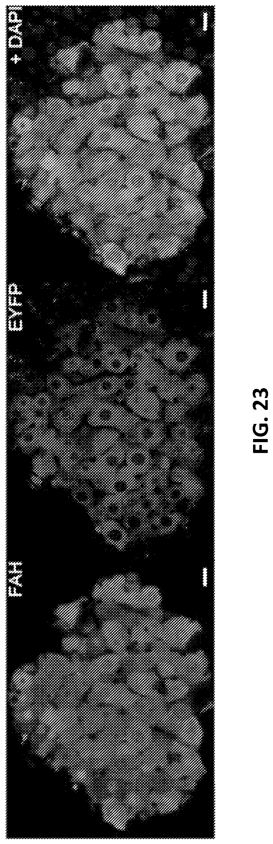

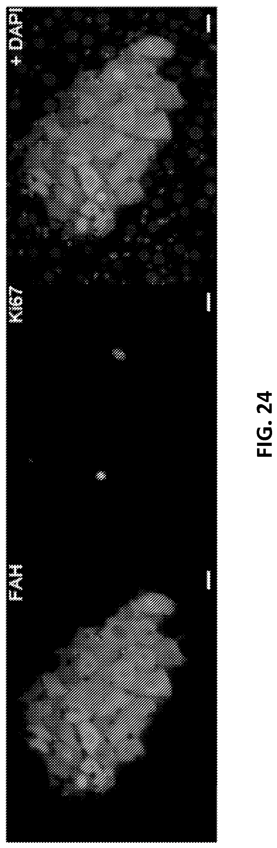

To determine both function and proliferation of MF-iHeps, we tested their liver repopulation capability in FAH-deficient mice, a model of nonfibrotic liver failure that provides a selective growth advantage for wildtype, mature-not immature or partially differentiated-hepatocytes. For this, we generated FAH-deficient mice harboring a large fraction (20.2 ± 10.3%) of lineage traceable HSCs by intrahepatic injection of two-day-old (P2) FAH-deficient pups with 250,000 HSCs isolated from Pdgfrb-Cre, R26R-EYFP mice (FIG. 16, FIG. 17). We used immune-deficient, FAH-deficient mice (FRG mice) to exclude rejection of the donor cells. When the mice reached the age of six weeks, we treated them with five doses of CCl4 over the course of two weeks to prompt HSCs to become MFs and expand. Next, we intravenously injected the mice with AAV6-6TFs. Four weeks later, we subjected the mice to repeated cycles of 2-(2-nitro-4-fluoromethylbenzoyl)-1,3-cyclohexanedione (NTBC)-a drug that prevents liver failure in FAH-deficient mice-off for ten days followed by a recovery phase on NTBC for three days. After two NTBC withdrawal cycles, we found clusters of 4-6 MF-iHeps identified by coexpression of EYFP and FAH or MUP (FIG. 18, FIG. 19). At this stage, we detected traces of desmin (DES) but not vimentin (VIM) or collagen 1 (COL1A1) expression in MF-iHeps, suggesting near-complete reprogramming FIG. 20, FIG. 21, FIG. 22). After five NTBC withdrawal cycles, we found nodules containing up to 64 MF-iHeps in two-dimensional liver sections (FIG. 23), which corresponds to 512 cells in three dimensions. Since each nodule was derived from a single reprogrammed MF, reaching this nodule size required nine rounds of cell division per MFiHep, assuming that all cells in a repopulating nodule proliferate equally. Considering that mainly cells in the periphery of a nodule proliferate-both in primary hepatocyte and MF-iHep (FIG. 24) nodules-some MF-iHeps likely divided more than nine times. In addition to establishing that MF-iHeps replicate the hallmarks of primary hepatocytes-mature function and extensive proliferation-these results show that MF-iHeps are stably reprogrammed because AAV vectors are nonintegrating and therefore lost from transduced cells after a few rounds of cell division.

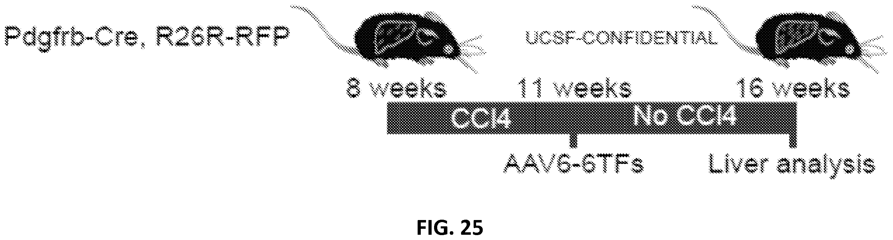

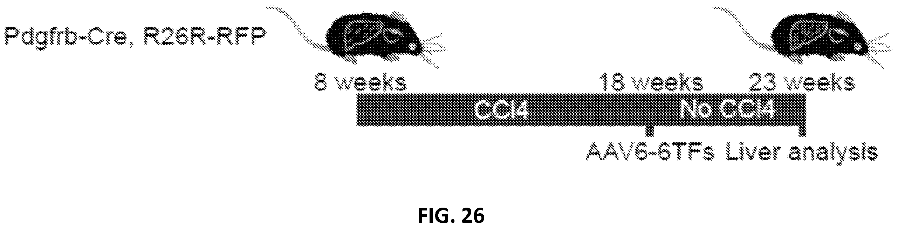

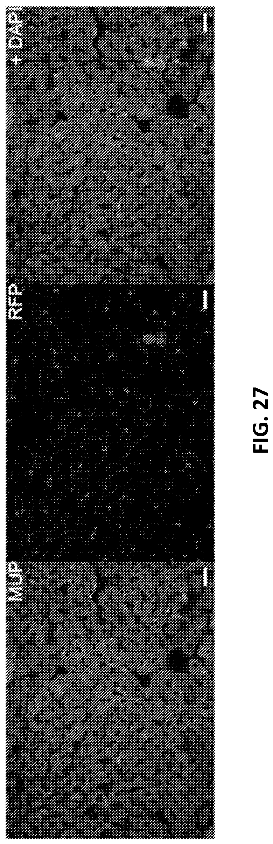

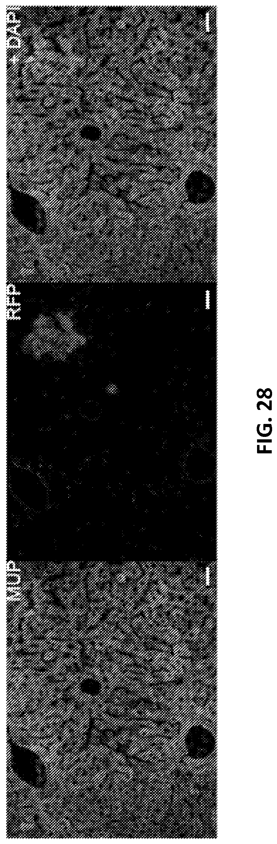

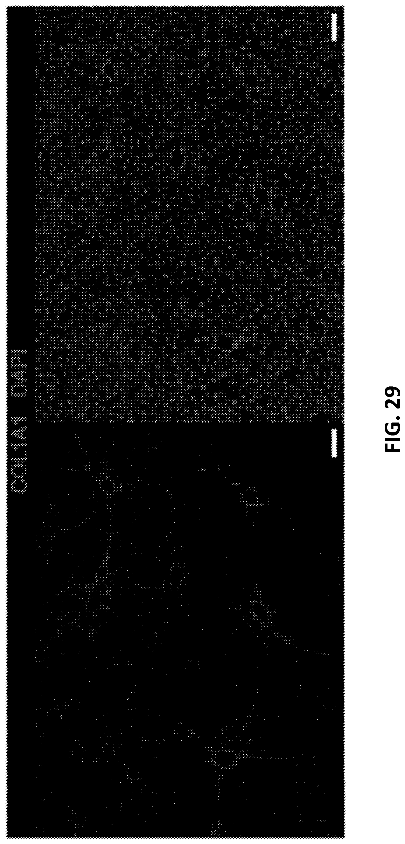

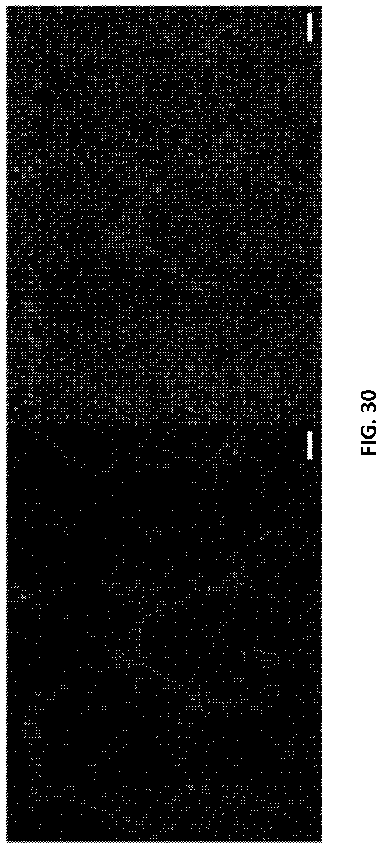

We also investigated the regenerative capabilities of MF-iHeps in the fibrotic liver. For this, we modeled early and advanced liver fibrosis by treating Pdgfrb-Cre, R26R-RFP mice with six or 20 doses of CCl4, respectively, followed by intravenous injection of AAV6-6TFs (FIG. 25, FIG. 26). Five weeks later we found that MF-iHep clusters were much larger in advanced fibrosis than in early fibrosis (FIG. 27, FIG. 28, FIG. 29, FIG. 30, and FIG. 31), reflecting a growth advantage of "newborn" MF-iHeps over the damaged primary hepatocytes, and leading to 0.19 ± 0.01% liver repopulation.

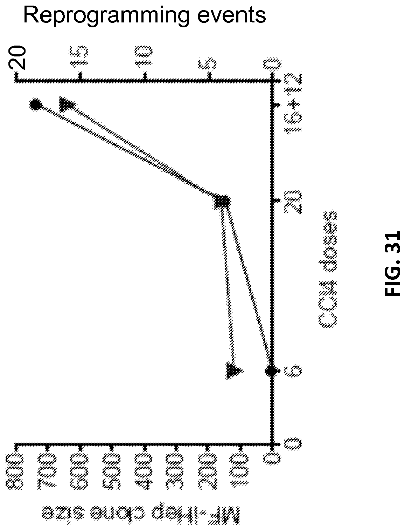

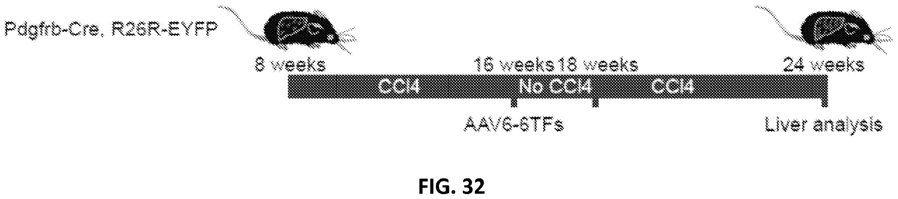

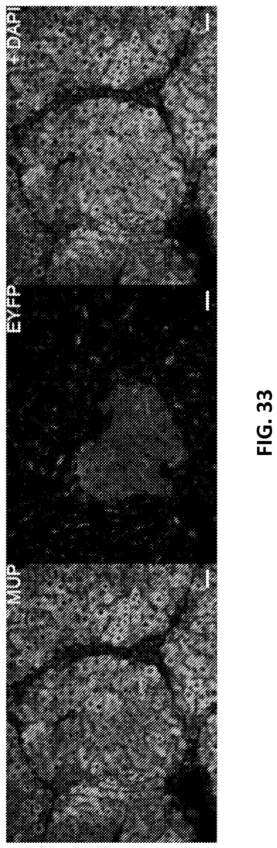

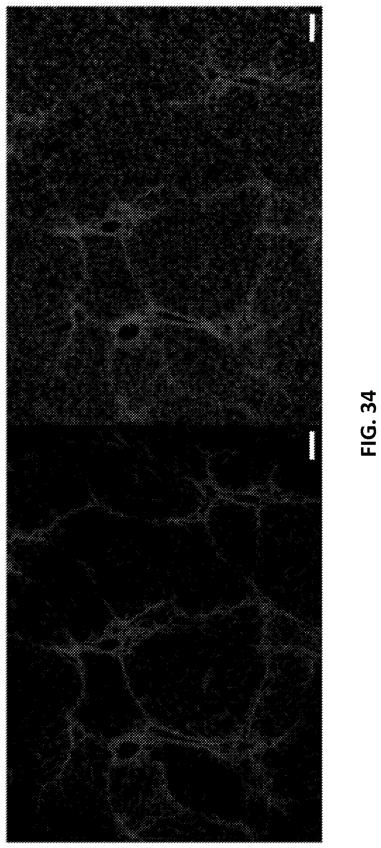

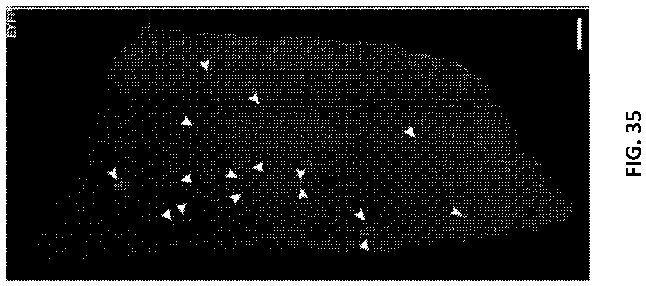

Finally, we investigated the regenerative capabilities of MF-iHeps under persistent liver injury conditions, the most common clinical scenario. For this, we treated Pdgfrb-Cre, R26R-EYFP mice with 16 doses of CCl4, followed by 12 additional doses of CCl4 after intravenous injection of AAV6-6TFs (FIG. 32). We found that MF-iHeps continued to have a growth advantage-producing nodules of up to 732 cells in three dimensions-despite chronic CCl4 exposure and the structural and molecular changes associated with more advanced, bridging liver fibrosis (FIG. 33, FIG. 34 and FIG. 31). In addition, we found that the efficiency of MF-into-MF-iHep reprogramming increased from 0.18 ± 0.01% in advanced fibrosis to 0.5 ± 0.05% in persistent liver injury, which, aided by clonal expansion, led to 0.63 ± 0.06% liver repopulation (FIG. 31, FIG. 35). Considering that cell cycle arrest and death have been reported as barriers to iHep generation *in vitro,* we attribute the increased reprogramming efficiency in mice with persistent liver injury to increased stimulation of proliferation and/or survival of newly formed MF-iHeps.

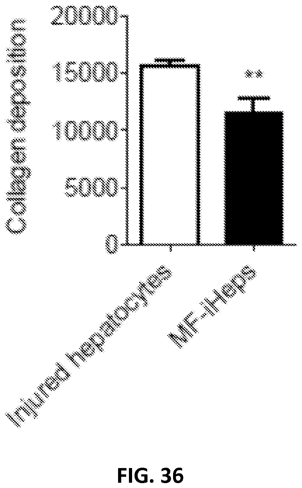

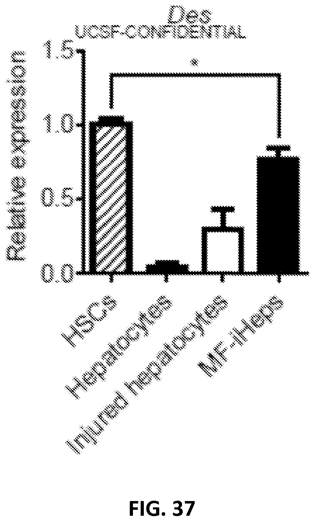

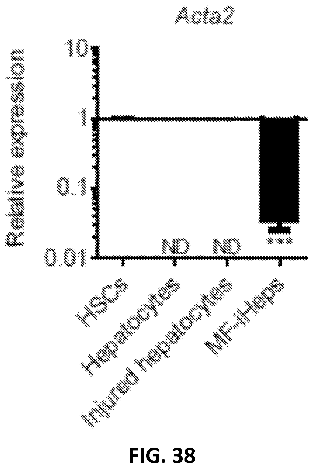

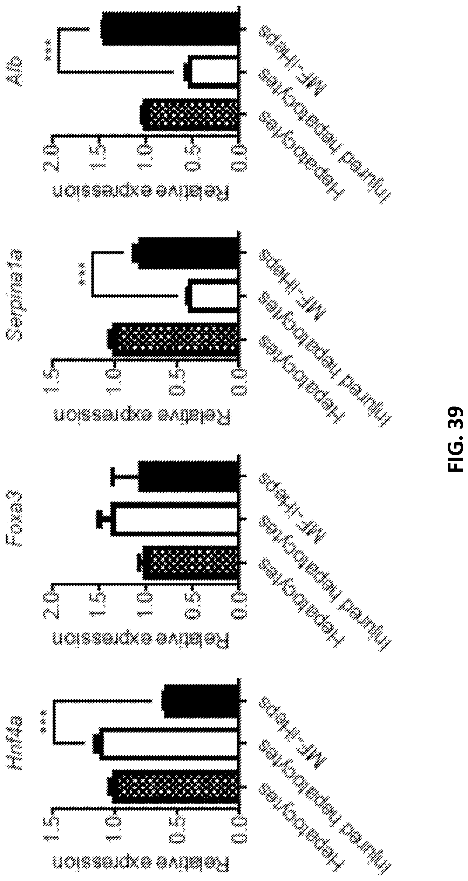

As hypothesized, we found that reprogramming MFs into hepatocytes reduced collagen deposition in the liver (FIG. 36). This finding suggested that MF-iHeps had lost fibrogenic function, which we confirmed by analyzing laser-capture microdissected cells by qRT-PCR. Although MF-iHeps expressed *Des,* a gene expressed in both HSCs and MFs, its levels were significantly lower than in HSCs (FIG. 37). Moreover, the MF specific gene *Acta2* was expressed at very low levels in MF-iHeps, indicating negligible memory of MF origin (FIG. 38). Most importantly, *Alb* and *Serpina1a,* markers of synthetic hepatocyte function, which is suppressed in the fibrotic liver, were normal in MF-iHeps (FIG. 39).

### EXAMPLE 3. Tropism Determination

AAV viral particles were made using the methods described in Example 2. Capsid proteins used for packaging the viral particles are below. Tropism was determined in liver fibroblasts are shown below:

| **AAV** | | **Tropism** | |
|---|---|---|---|
| **Capsid** | **Accession number** | **Yes** | **No** |
| AAV1 | NC_002077 | | X |
| AAV2/ AAV2(Y444,500,730F) | NC_001401 | | X |
| AAV6 | AF028704 | X | |
| AAV7 | NC_006260 | X | |
| AAV8 | NC_006261 | X | |
| AAV9 | AY530579 | | X |
| AAV-DJ | Source: CellBiolabs Inc. | | X |

### EXAMPLE 4

### Human cell targeting in Animal Models (PROPHETIC)

AAV6 virions disclosed herein will be purified.

Human translation: We know from in vitro studies that AAV6 also transduces human myofibroblasts; we will establish reprogramming into hepatocytes of human myofibroblasts engrafted in livers of immune-deficient mice.

### EXAMPLE 5- Maximizing AAV6 myofibroblast transduction efficiency and specificity (PROPHETIC)

We will screen libraries of 1 million or so synthetic AAV capsids generated by DNA shuffling of all naturally occurring AAV capsids to identify capsids with high myofibroblast tropism that do not target other cell types like skeletal muscle and heart. In the main library AAV6 sequences will be overrepresented to build on its existing myofibroblast tropism. We will also screen a library of AAV6 capsid variants generated by genetic insertion of short peptides into surface-exposed capsid loops. To screen the libraries, we will intravenously inject them into mice in which myofibroblasts are genetically labeled or in which human myofibroblasts have been engrafted, isolate these cells by FACS and recover capsid variants that transduced them using an established PCR strategy. By using the recovered capsid variants to generate new AAV vectors and subjecting them to additional rounds of in vivo selection we will identify the capsid variants that are most efficient in myofibroblast transduction. Because most humans have neutralizing AAV antibodies, to achieve maximum efficiency, we will subject capsid variants identified by in vivo screening to a round of in vitro selection in commercially available pooled human antisera to eliminate capsids displaying prevalent epitopes.

### EXAMPLE 6 Maximizing reprogramming efficiency (PROPHETIC)

We will test additional genes (transcription factors, chromatin modifiers, cell cycle regulators) for their ability to promote reprogramming of human myofibroblasts into hepatocytes in vivo.

### EXAMPLE 7 Detargeting: (PROPHETIC)

It is possible that the synthetic AAV capsids identified as described above are detargeted from other tissues the wildtype AAV6 capsid is known to transduce after intravenous injection, i.e., skeletal muscle and heart. However, to be safe, we will definitely avoid transcription factor expression and thus potentially reprogramming in other tissues by suppressing it outside of the liver. For this, we will add binding sites of microRNAs specific to these tissues, e.g., miR-1 for both skeletal muscle and heart, to the 3' end of the transcription factor sequence so that transcription factor mRNAs are degraded in these tissues. By avoiding premature termination of transcription factor expression in myofibroblasts, this detargeting approach is better than using an myofibroblast-specific promoter.

### EXAMPLE 8 - Human Administration of viral particles (PROPHETIC)

AAV6 viral particles will be made using the methods disclosed herein.

We will determine the durability of transgene expression, the vector dose-response relationship, and the level of persistent or late toxicity.

Methods will be based on N Engl J Med. 2014 Nov 20;371(21).

### METHODS:

We will evaluate stability of transgene expression and long-term safety in 10 patients with liver fibrosis: 6 patients who will be administered dose-escalation trial, with 2 patients each receiving a low, intermediate, or high dose, and 4 additional patients who will receive the high dose about 2×10¹² vector genomes per kilogram of body weight. The patients will subsequently undergo extensive clinical and laboratory monitoring.

The results of this study will determine the frequency of intravenous infusion of vector in all 10 patients with liver fibrosis. Dosing will be based on clinical experience with intravenously injected AAV8 vectors for gene therapy of hemophilia, ranging from 2 × 10¹¹ viral genomes (vg)/kg body weight (BW) (low dose) to 6 × 10¹¹ vg/kg BW (intermediate dose) and 2 × 10¹² vg/kg BW (high dose). All doses were tolerated without complications. Brief, moderate rises in liver enzymes in a subset of patients will resolve after prednisolone application.

## Claims

1. A viral vector comprising:
a viral capsid comprising a nucleic acid molecule encapsulated within the viral capsid, wherein the nucleic acid molecule comprises:
a first nucleic acid sequence that encodes HNF4α; and
a second nucleic acid sequence that encodes one or more transcription factors selected from the group consisting of: FOXA1, FOXA2, FOXA3, HNF1α, HNF6, GATA4, HLF, CEBPA, PROX1, and ATF5A;
wherein the viral capsid comprises the viral capsid polypeptides VP1 of AAV6, VP2 of AAV6, and VP3 of AAV6.

2. A composition comprising
a) one or a plurality of viral vectors of claim 1; and/or
b) a plurality of viral vectors comprising
i) a first viral vector comprising a viral capsid comprising a nucleic acid molecule encapsulated within the viral capsid, wherein the nucleic acid molecule comprises a nucleic acid sequence that encodes mammalian HNF4α and wherein the viral capsid comprises the viral capsid polypeptides VP1 of AAV6, VP2 of AAV6, and VP3 of AAV6; and
ii) a second viral vector comprising a viral capsid comprising a nucleic acid molecule encapsulated within the viral capsid, wherein the nucleic acid molecule comprises a nucleic acid sequence that encodes one or more mammalian transcription factors selected from the group consisting of: FOXA1, FOXA2, FOXA3, HNF1α, HNF6, GATA4, HLF, CEBPA, PROX1, and ATF5A and wherein the viral capsid comprises the viral capsid polypeptides VP1 of AAV6, VP2 of AAV6, and VP3 of AAV6.

3. The viral vector of claim 1 or the composition of claim 2, wherein the viral capsid comprises a combination of a polypeptide comprising to the amino acid sequence of SEQ ID NO: 2 (VP1 of AAV6), a polypeptide comprising the amino acid sequence of SEQ ID NO: 4 (VP2 of AAV6), and a polypeptide comprising to the amino acid sequence of SEQ ID NO: 6 (VP3 of AAV6).

4. The viral vector of claim 1 or the composition of claim 2, wherein the viral vector does not comprise an expressible gene from a lentivirus.

5. The viral vector of claim 1 or the composition of claim 2, wherein the nucleic acid molecule is free of a regulatory sequence from a lentivirus.

6. The viral vector of claim 1 or the composition of claim 2, wherein the second nucleic acid sequence encodes FOXA2.

7. The viral vector of claim 1 or the composition of claim 2, wherein the HNF4α comprises the amino acid sequence of SEQ ID NO: 66, the FOXA1 comprises the amino acid sequence of SEQ ID NO: 46, the FOXA2 comprises the amino acid sequence of SEQ ID NO: 48, the FOXA3 comprises the amino acid sequence of SEQ ID NO: 50, the HNF1α comprises the amino acid sequence of SEQ ID NO: 54, the HNF6 comprises the amino acid sequence of SEQ ID NO: 56, the GATA4 comprises the amino acid sequence of SEQ ID NO: 52, the HLF comprises the amino acid sequence of SEQ ID NO: 58, the CEBPA comprises the amino acid sequence of SEQ ID NO: 60, the PROX1 comprises the amino acid sequence of SEQ ID NO: 62, and the ATF5A comprises the amino acid sequence of SEQ ID NO: 64.

8. A pharmaceutical composition comprising:
a therapeutically effective amount of the viral vector of claim 1 or the composition of claim 2; and
a pharmaceutically acceptable carrier.

9. The pharmaceutical composition of claim 8, wherein the pharmaceutical composition is free of a short-hairpin RNA (shRNA), free of a nucleic acid sequence encoding a shRNA, free of a short inhibitory RNA (siRNA), and free of a nucleic acid sequence encoding a siRNA.

10. The pharmaceutical composition of claim 8 for use in a method of treating and/or preventing liver fibrosis in a subject in need thereof.

11. The pharmaceutical composition for use according to claim 10, wherein the pharmaceutical composition is suitable for administration via intravenous injection.

12. The pharmaceutical composition of claim 8 for use in a method of restoring tissue-specific function to fibrotic tissue in an organ in a subject suspected of having, diagnosed as having, or genetically predisposed to acquiring fibrotic tissue in an organ.

13. The pharmaceutical composition for use according to claim 12, wherein the pharmaceutical composition of claim 8 is free of a lentiviral vector or a lentiviral regulatory sequence.

14. The pharmaceutical composition of claim 8 for use in a method of inducing differentiation of a myofibroblast into a hepatocyte *in vivo* or a method of inducing proliferation of hepatocytes in a liver of a subject.

## Patentansprüche

1. Virusvektor, umfassend:
ein Viruskapsid, das ein in dem Viruskapsid verkapseltes Nukleinsäuremolekül umfasst, wobei das Nukleinsäuremolekül Folgendes umfasst:
eine erste Nukleinsäuresequenz, die HNF4α codiert; und
eine zweite Nukleinsäuresequenz, die einen oder mehrere Transkriptionsfaktoren codiert, die ausgewählt sind aus der Gruppe bestehend aus: FOXA1, FOXA2, FOXA3, HNF1α, HNF6, GATA4, HLF, CEBPA, PROX1 und ATF5A;
wobei das Viruskapsid die Viruskapsid-Polypeptide VP1 von AAV6, VP2 von AAV6 und VP3 von AAV6 umfasst.

2. Zusammensetzung, umfassend
a) einen oder mehrere Virusvektoren nach Anspruch 1; und/oder
b) mehrere Virusvektoren, die Folgendes umfassen:
i) einen ersten Virusvektor, der ein Viruskapsid umfasst, das ein in dem Viruskapsid verkapseltes Nukleinsäuremolekül umfasst, wobei das Nukleinsäuremolekül eine Nukleinsäuresequenz umfasst, die Säuger-HNF4α codiert, und wobei das Viruskapsid die Viruskapsid-Polypeptide VP1 von AAV6, VP2 von AAV6 und VP3 von AAV6 umfasst; und
ii) einen zweiten Virusvektor, der ein Viruskapsid umfasst, das ein in dem Viruskapsid verkapseltes Nukleinsäuremolekül umfasst, wobei das Nukleinsäuremolekül eine Nukleinsäuresequenz umfasst, die einen oder mehrere Säuger-Transkriptionsfaktoren codiert, die ausgewählt sind aus der Gruppe bestehend aus FOXA1, FOXA2, FOXA3, HNF1α, HNF6, GATA4, HLF, CEBPA, PROX1 und ATF5A, und wobei das Viruskapsid die Viruskapsid-Polypeptide VP1 von AAV6, VP2 von AAV6 und VP3 von AAV6 umfasst.

3. Virusvektor nach Anspruch 1 oder Zusammensetzung nach Anspruch 2, wobei das Viruskapsid eine Kombination eines Polypeptids, das zu der Aminosäuresequenz unter SEQ ID NO: 2 (VP1 von AAV6) umfasst, eines Polypeptids, das die Aminosäuresequenz unter SEQ ID NO: 4 (VP2 von AAV6) umfasst, und eines Polypeptids, das zu der Aminosäuresequenz unter SEQ ID NO: 6 (VP3 von AAV6) umfasst, umfasst.

4. Virusvektor nach Anspruch 1 oder Zusammensetzung nach Anspruch 2, wobei der Virusvektor kein exprimierbares Gen aus einem Lentivirus umfasst.

5. Virusvektor nach Anspruch 1 oder Zusammensetzung nach Anspruch 2, wobei das Nukleinsäuremolekül frei von einer Regulatorsequenz aus einem Lentivirus ist.

6. Virusvektor nach Anspruch 1 oder Zusammensetzung nach Anspruch 2, wobei die zweite Nukleinsäuresequenz FOXA2 codiert.

7. Virusvektor nach Anspruch 1 oder Zusammensetzung nach Anspruch 2, wobei der HNF4α die Aminosäuresequenz unter SEQ ID NO: 66, der FOXA1 die Aminosäuresequenz unter SEQ ID NO: 46, der FOXA2 die Aminosäuresequenz unter SEQ ID NO: 48, der FOXA3 die Aminosäuresequenz unter SEQ ID NO: 50, der HNF1α die Aminosäuresequenz unter SEQ ID NO: 54, der HNF6 die Aminosäuresequenz unter SEQ ID NO: 56, der GATA4 die Aminosäuresequenz unter SEQ ID NO: 52, der HLF die Aminosäuresequenz unter SEQ ID NO: 58, der CEBPA die Aminosäuresequenz unter SEQ ID NO: 60, der PROX1 die Aminosäuresequenz unter SEQ ID NO: 62 und der ATF5A die Aminosäuresequenz unter SEQ ID NO: 64 umfasst.

8. Pharmazeutische Zusammensetzung, umfassend: eine therapeutisch wirksame Menge des Virusvektors nach Anspruch 1 oder der Zusammensetzung nach Anspruch 2; und einen pharmazeutisch unbedenklichen Träger.

9. Pharmazeutische Zusammensetzung nach Anspruch 8, wobei die pharmazeutische Zusammensetzung frei von einer shRNA (short-hairpin RNA), frei von einer eine shRNA codierenden Nukleinsäuresequenz, frei von einer siRNA (short inhibitory RNA) und frei von einer eine siRNA codierenden Nukleinsäuresequenz ist.

10. Pharmazeutische Zusammensetzung nach Anspruch 8 zur Verwendung bei einem Verfahren zur Behandlung und/oder Vorbeugung von Leberfibrose bei einem behandlungsbedürftigen Individuum.

11. Pharmazeutische Zusammensetzung zur Verwendung gemäß Anspruch 10, wobei die pharmazeutische Zusammensetzung zur Verabreichung über intravenöse Injektion geeignet ist.

12. Pharmazeutische Zusammensetzung nach Anspruch 8 zur Verwendung bei einem Verfahren zur Wiederherstellung von gewebespezifischer Funktion bei fibrotischem Gewebe in einem Organ bei einem Individuum, bei dem fibrotisches Gewebe in einem Organ vermutet wird, diagnostiziert wurde oder eine genetische Veranlagung zur Erwerbung von fibrotischem Gewebe in einem Organ besteht.

13. Pharmazeutische Zusammensetzung zur Verwendung gemäß Anspruch 12, wobei die pharmazeutische Zusammensetzung nach Anspruch 8 frei von einem Lentivirusvektor oder einer lentiviralen Regulatorsequenz ist.

14. Pharmazeutische Zusammensetzung nach Anspruch 8 zur Verwendung bei einem Verfahren zum Induzieren der Differenzierung eines Myofibroblasten zu einem Hepatozyten *in vivo* oder einem Verfahren zum Induzieren der Proliferation von Hepatozyten in einer Leber eines Individuums.

## Revendications

1. Vecteur viral comprenant :
une capside virale comprenant une molécule d'acide nucléique encapsulée dans la capside virale, la molécule d'acide nucléique comprenant :
une première séquence d'acide nucléique qui code pour HNF4α ; et
une deuxième séquence d'acide nucléique qui code pour un ou plusieurs facteurs de transcription choisis dans le groupe constitué de : FOXA1, FOXA2, FOXA3, HNF1α, HNF6, GATA4, HLF, CEBPA, PROX1 et ATF5A ;
la capside virale comprenant les polypeptides de capside virale VP1 d'AAV6, VP2 d'AAV6 et VP3 d'AAV6.

2. Composition comprenant
a) un ou plusieurs vecteurs viraux selon la revendication 1 ; et/ou
une pluralité de vecteurs viraux comprenant
un premier vecteur viral comprenant une capside virale comprenant une molécule d'acide nucléique encapsulée dans la capside virale, la molécule d'acide nucléique comprenant une séquence d'acide nucléique qui code pour HNF4α de mammifère et la capside virale comprenant les polypeptides de capside virale VP1 d'AAV6, VP2 d'AAV6 et VP3 d'AAV6 ; et
ii) un deuxième vecteur viral comprenant une capside virale comprenant une molécule d'acide nucléique encapsulée dans la capside virale, la molécule d'acide nucléique comprenant une séquence d'acide nucléique codant pour un ou plusieurs facteurs de transcription de mammifère choisis dans le groupe constitué de : FOXA1, FOXA2, FOXA3, HNF1α, HNF6, GATA4, HLF, CEBPA, PROX1 et ATF5A et la capside virale comprenant les polypeptides de capside virale VP1 d'AAV6, VP2 d'AAV6 et VP3 d'AAV6.

3. Vecteur viral selon la revendication 1 ou composition selon la revendication 2, la capside virale comprenant une combinaison d'un polypeptide comprenant la séquence d'acides aminés de SEQ ID NO : 2 (VP1 d'AAV6), d'un polypeptide comprenant la séquence d'acides aminés de SEQ ID NO : 4 (VP2 d'AAV6), et d'un polypeptide comprenant la séquence d'acides aminés de SEQ ID NO : 6 (VP3 d'AAV6) .

4. Vecteur viral selon la revendication 1 ou composition selon la revendication 2, le vecteur viral ne comprenant pas de gène exprimable provenant d'un lentivirus.

5. Vecteur viral selon la revendication 1 ou composition selon la revendication 2, la molécule d'acide nucléique étant exempte d'une séquence régulatrice provenant d'un lentivirus.

6. Vecteur viral selon la revendication 1 ou composition selon la revendication 2, la deuxième séquence d'acide nucléique codant pour FOXA2.

7. Vecteur viral selon la revendication 1 ou composition selon la revendication 2, HNF4α comprenant la séquence d'acides aminés de SEQ ID NO : 66, FOXA1 comprenant la séquence d'acides aminés de SEQ ID NO : 46, FOXA2 comprenant la séquence d'acides aminés de SEQ ID NO : 48, FOXA3 comprenant la séquence d'acides aminés de SEQ ID NO : 50, HNFla comprenant la séquence d'acides aminés de SEQ ID NO : 54, HNF6 comprenant la séquence d'acides aminés de SEQ ID NO : 56, GATA4 comprenant la séquence d'acides aminés de SEQ ID NO : 52, HLF comprenant la séquence d'acides aminés de SEQ ID NO : 58, CEBPA comprenant la séquence d'acides aminés de SEQ ID NO : 60, PROX1 comprenant la séquence d'acides aminés de SEQ ID NO : 62, et ATF5A comprenant la séquence d'acides aminés de SEQ ID NO : 64.

8. Composition pharmaceutique comprenant :
une quantité thérapeutiquement efficace du vecteur viral selon la revendication 1 ou de la composition selon la revendication 2 ; et
un véhicule pharmaceutiquement acceptable.

9. Composition pharmaceutique selon la revendication 8, la composition pharmaceutique étant exempte d'un ARN en épingle à cheveux court (ARNsh), exempte d'une séquence d'acide nucléique codant pour un ARNsh, exempte d'un petit ARN inhibiteur (ARNsi) et exempte d'une séquence d'acide nucléique codant pour un ARNsi.

10. Composition pharmaceutique selon la revendication 8 destinée à être utilisée dans une méthode de traitement et/ou de prévention de la fibrose hépatique chez un sujet en ayant besoin.

11. Composition pharmaceutique destinée à être utilisée selon la revendication 10, la composition pharmaceutique étant adaptée pour administration par injection intraveineuse.

12. Composition pharmaceutique selon la revendication 8 destinée à être utilisée dans une méthode de restauration de la fonction spécifique tissulaire d'un tissu fibrotique dans un organe chez un sujet suspecté d'avoir, diagnostiqué comme ayant, ou génétiquement prédisposé à acquérir un tissu fibrotique dans un organe.

13. Composition pharmaceutique destinée à être utilisée selon la revendication 12, la composition pharmaceutique de la revendication 8 étant exempte d'un vecteur lentiviral ou d'une séquence régulatrice lentivirale.

14. Composition pharmaceutique selon la revendication 8 destinée à être utilisée dans une méthode d'induction de la différenciation *in vivo* d'un myofibroblaste en hépatocyte ou dans une méthode d'induction de la prolifération d'hépatocytes dans le foie d'un sujet.
